# EUROPEAN PATENT SPECIFICATION

(11) **EP 4 486 882 B1**
(45) Date of publication and mention of the grant of the patent: **11.11.2026**
(21) Application number: 23764064.4
(22) Date of filing: 01.03.2023
(51) Int. Cl.: C12N 9/22, C12N 15/113

(54) **ENGINEERED NUCLEASES, COMPOSITIONS, AND METHODS OF USE THEREOF**
MANIPULIERTE NUKLEASEN, ZUSAMMENSETZUNGEN UND VERFAHREN ZUR VERWENDUNG DAVON
NUCLÉASES MODIFIÉES, COMPOSITIONS ET LEURS PROCÉDÉS D'UTILISATION

(30) Priority: 01.03.2022 US 202263315159 P; 19.10.2022 US 202263380178 P; 28.11.2022 US 202263385171 P
(43) Date of publication of application: 08.01.2025
(73) Proprietor: Epicrispr Biotechnologies, Inc., South San Francisco, CA 94080 (US)
(72) Inventor: YANG, Xiao, San Francisco, California 94080 (US); QI, Lei S., San Francisco, California 94080 (US); CUTILLAS, Vincent, San Francisco, California 94080 (US); ALVAREZ, Gabriella, San Francisco, California 94080 (US); TCHEAU, Tabitha, San Francisco, California 94080 (US); HART, Daniel, San Francisco, California 94080 (US)
(74) Representative: Zacco Denmark A/S
(86) International application number: PCT/US2023/063446
(87) International publication number: WO 2023/168242

(56) References cited:
- WO-A1-2019/089808
- WO-A1-2019/089820
- WO-A1-2021/023887
- WO-A1-2021/084533
- WO-A1-2022/051250
- WO-A2-2021/086083
- KR-A- 20230 007 218

## Description

### BACKGROUND

Various nucleases (e.g., endonucleases) can be utilized to edit a target sequence in a cell, or regulate expression or activity of the target gene in the cell. For example, a heterologous nuclease can be introduced (e.g., delivered, expressed, etc.) to the cell, and the heterologous nuclease, either alone or along with an additional agent, can effect such editing or regulation of the target gene. For example, clustered regularly interspaced short palindromic repeats (CRISPR)/CRISPR-associated protein (Cas) is a family of nucleases that are involved in specifically binding, cleaving, and/or editing a target deoxyribonucleic acid (DNA) sequence or ribonucleic acid (RNA) sequence (e.g., foreign DNA sequence or RNA sequence). The programmable nature of these nucleases has facilitated their use as a versatile technology that is revolutionizing the field of target gene manipulation, e.g., as gene therapy to treat or ameliorate a condition (e.g., a disease) of a subject.

WO 2022/051250 A1 discloses a synthetic miniature CRISPR/Cas (CASMINI) system for eukaryotic genome engineering. The Cas proteins and guide RNA molecules are engineered to exhibit increased activity in eukaryotic cells.

### SUMMARY

Various endonucleases, such as CRISPR/Cas proteins (e.g., Cas12f proteins utilized thus far), can have smaller sizes as compared to Cas9 or Cas12a. However, the sizes of the endonuclease may not be small enough to package them along with at least one additional agent (e.g., a guide RNA, a transgene encoding a therapeutic polynucleotide or protein, etc.) in a delivery mode (e.g., viral vectors, such as adeno-associated virus (AAV) vectors). Thus, various aspects of the present disclosure, for example, provide engineered nucleases that are smaller, yet effective, in binding, cleaving, and/or editing a target polynucleotide sequence, compositions thereof, and methods of use thereof.

Disclosed is an engineered polypeptide comprising an engineered nuclease, wherein the engineered nuclease comprises an amino acid sequence that is at least 80% identical to the polypeptide sequence of SEQ ID NO: 1, wherein the amino acid sequence comprises at least one deletion from the amino acid resides 2-100, as compared to the polypeptide sequence of SEQ ID NO: 1.

Disclosed is an engineered polypeptide comprising an engineered nuclease, wherein the engineered nuclease comprises an amino acid sequence that is greater than 92% identical to the polypeptide sequence of SEQ ID NO: 12.

Disclosed is an engineered polypeptide comprising an engineered nuclease variant, wherein the engineered nuclease variant comprises an amino acid sequence that is at least 70% identical to the polypeptide sequence of SEQ ID NO: 12, wherein the amino acid sequence comprises a modification as compared to the polypeptide sequence of SEQ ID NO: 1, wherein the modification comprises one or more members selected from the group consisting of A21Q, V23I, N32E, D29E, N33R, E35K, K36Q, I37A, A38G, E40D, K73G, A74T, R75G, K76E, Q83K, G87K, E151A, A340S, H353K, A374K, I387E, N423D, K473Q, T474L, T474R, H497K, L515R, N519T, K521D, K521N, L522I, and at least one deletion from the amino acid residues 400-529 of SEQ ID NO: 1.

Another aspect of the present disclosure provides an engineered polypeptide comprising an engineered nuclease variant, wherein the engineered nuclease variant is a chimeric polypeptide comprising: a first polypeptide sequence comprising at least 3 contiguous amino acid residues in common with a first Cas protein; and a second polypeptide sequence comprising at least 3 contiguous amino acid residues in common with a second Cas protein, wherein the second Cas protein is different from the first Cas protein, wherein the first Cas protein comprises an amino acid sequence that is at least 80% identical to the polypeptide sequence of SEQ ID NO: 1.

Disclosed is an engineered polypeptide comprising an engineered nuclease variant, wherein the engineered nuclease is a chimeric polypeptide comprising: a first polypeptide sequence (CP1) comprising at least 3 contiguous amino acid residues in common with a portion of a first Cas protein; a second polypeptide sequence (CP2) comprising at least 3 contiguous amino acid residues in common with a portion of a second Cas protein that is different from the first Cas protein; and a third polypeptide sequence (CPx) comprising at least 3 contiguous amino acid residues in common with: (i) an additional portion of the first Cas protein, wherein the portion and the additional portion of the first Cas protein are not directly adjacent to each other in the first Cas protein; (ii) an additional portion of the second Cas protein, wherein the portion and the additional portion of the second Cas protein are not directly adjacent to each other in the second Cas protein; or (iii) a portion of a third Cas protein that is different from the first Cas protein and the second Cas protein, wherein the chimeric polypeptide has a length of less than or equal to about 1,000 amino acids.

Disclosed is an engineered polypeptide comprising an engineered nuclease variant, wherein the engineered nuclease variant is a chimeric polypeptide comprising: a first polypeptide comprising at least 3 contiguous amino acid residues in common with a first Cas protein; and a second polypeptide comprising at least 3 contiguous amino acid residues in common with a second Cas protein, wherein the second Cas protein is different from the first Cas protein, wherein a length of the second polypeptide sequence is less than about 20% than that of the first polypeptide sequence.

Disclosed is an engineered polypeptide comprising an engineered nuclease variant, wherein the engineered nuclease variant: (i) comprises an amino acid sequence that is at least 80% identical to the polypeptide sequence of a member selected from TABLE 3B, TABLE 4B, or TABLE 5C; (ii) is not any one of SEQ ID NOs: 1-3, 10, and 13-19; and (iii) has a length of less than or equal to about 800 amino acids.

Disclosed is an engineered polypeptide comprising an engineered nuclease variant operatively coupled to a gene modulator, wherein the engineered nuclease variant: (i) comprises an amino acid sequence that is at least 70% identical to the polypeptide sequence of SEQ ID NO: 1; and (ii) when operatively coupled to the gene modulator, induces an enhanced modulation of a target gene in a cell, as compared to that by a control engineered polypeptide comprising SEQ ID NO: 10 operatively coupled to the gene modulator.

The invention is set out in the appended set of claims. In particular, the present invention provides an engineered polypeptide comprising an engineered nuclease, wherein said engineered nuclease comprises the amino acid sequence of any one of SEQ ID NOs: 132, 134, 135, 140, or 146.

### BRIEF DESCRIPTION OF THE DRAWINGS

FIG. 1 schematically illustrates structural alignment between Un1Cas12f1 and AsCas12f, to identify one or more domains in Un1Cas12f1 that may not be conserved in one or more additional Cas12f homologous structures.
FIG. 2 schematically illustrates structural alignment between Un1Cas12f1 and Un2Cas12f1, to identify one or more domains in Un1Cas12f1 that may not be conserved in one or more additional Cas12f homologous structures.
FIG. 3A schematically illustrates selection of various domains of Un1Cas12f1 that are to be at least partially deleted to generate one or more engineered nucleases. FIG. 3B schematically illustrates deletion landscape approach to generate engineered nuclease variants of Un1Cas12f1.
FIG. 4 schematically illustrates example deletion landscape of a Cas protein (e.g., dCas9), to identify one or more domains in the Cas protein that may be deleted with minimal or substantially no effect on the Cas protein's activity (e.g., the ability to induce transcription expression when the modified variant of dCas9 is operatively coupled to a gene repressor).
FIG. 5 shows enhanced expression of endogenous IFN gamma (top plot) and endogenous CD2 (bottom plot) in cells by various engineered nuclease variants disclosed herein. The engineered nuclease variants were engineered to exhibit reduced nuclease activity (e.g., dCas variants), and were fused with a gene activating modulator.
FIG. 6 shows reduced expression of a target gene (e.g., GFP) in cells by various engineered nuclease variants disclosed herein. The engineered nuclease variants were engineered to exhibit reduced nuclease activity (e.g., dCas variants), and were fused with a gene repressing modulator. Two different gene repressing modulators were used: gene repressor A (top plot) and gene repressor B (bottom plot).
FIG. 7 shows enhanced expression of endogenous CD2 (top plot), endogenous IFN gamma (middle plot), and endogenous CXCR4 (bottom plot) in cells by various engineered nuclease variants disclosed herein. The engineered nuclease variants were engineered to exhibit reduced nuclease activity (e.g., dCas variants), and were fused with a gene activating modulator.
FIG. 8 schematically illustrates a guide nucleic acid molecule configured to form a complex with a Cas protein.
FIG. 9 schematically illustrates regions of a scaffold region of a guide nucleic acid molecule that can be modified (e.g., mutated or deleted) to engineer the guide nucleic acid molecule.
FIG. 10 shows reduced expression of a target gene (e.g., GFP) in cells by a plurality of engineered guide RNA variants disclosed herein. The plurality of engineered guide RNA variants was modified at least in the scaffold region, as compared to a control guide RNA sequence ("SQ"). The reduced gene expression was performed with the same dCas protein coupled to a gene repressing modulator.
FIG. 11 shows reduced expression of a target gene (e.g., GFP) in cells by an additional plurality of engineered guide RNA variants disclosed herein. The additional plurality of engineered guide RNA variants was modified at least in the scaffold region, as compared to a control guide RNA sequence ("SQ"). The reduced gene expression was performed with the same dCas protein coupled to a gene repressing modulator.
FIG. 12 shows enhanced expression of endogenous CD2 in cells by a different plurality of engineered guide RNA variants disclosed herein. The different plurality of engineered guide RNA variants was modified at least in the scaffold region, as compared to a control guide RNA sequence ("SQ"). The enhanced gene expression was performed with the same dCas protein coupled to a gene activating modulator.
FIG. 13 schematically illustrates examples of the engineered guide nucleic acid molecules disclosed herein.
FIG. 14 shows comparison of sizes of various DNA vectors, each encoding a Cas protein and a respective single guide nucleic acid molecule.
FIG. 15 shows increased expression of endogenous IFN gamma (IFNγ) in cells by the truncation nuclease variant t1 disclosed herein. The engineered nuclease variant t1 was fused with a gene activating modulator, VPR.
FIG. 16 shows reduced expression of endogenous CXCR4 in cells by the chimeric nuclease variants disclosed herein. The engineered nuclease variant t1 was fused with a gene repressing modulator (ZNF10-KRAB-hDNMT3L).

### DETAILED DESCRIPTION

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

The term "about" or "approximately" generally means within an acceptable error range for the particular value as determined by one of ordinary skill in the art, which will depend in part on how the value is measured or determined, i.e., the limitations of the measurement system. For example, "about" can mean within 1 or more than 1 standard deviation, per the practice in the art. Alternatively, "about" can mean a range of up to 20%, up to 10%, up to 5%, or up to 1% of a given value. Alternatively, particularly with respect to biological systems or processes, the term can mean within an order of magnitude, preferably within 5-fold, and more preferably within 2- fold, of a value. Where particular values are described in the application and claims, unless otherwise stated, the term "about" meaning within an acceptable error range for the particular value should be assumed.

The use of the alternative (e.g., "or") should be understood to mean either one, both, or any combination thereof of the alternatives. The term "and/or" should be understood to mean either one, or both of the alternatives.

The term "cell" generally refers to a biological cell. A cell can be the basic structural, functional and/or biological unit of a living organism. A cell can originate from any organism having one or more cells. Some non-limiting examples include: a prokaryotic cell, eukaryotic cell, a bacterial cell, an archaeal cell, a cell of a single-cell eukaryotic organism, a protozoa cell, a cell from a plant (e.g., cells from plant crops, fruits, vegetables, grains, soy bean, corn, maize, wheat, seeds, tomatoes, rice, cassava, sugarcane, pumpkin, hay, potatoes, cotton, cannabis, tobacco, flowering plants, conifers, gymnosperms, ferns, clubmosses, hornworts, liverworts, mosses), an algal cell, (e.g., Botryococcus braunii, Chlamydomonas reinhardtii, Nannochloropsis gaditana, Chlorella pyrenoidosa, Sargassum patens C. Agardh, and the like), seaweeds (e.g., kelp), a fungal cell (e.g., a yeast cell, a cell from a mushroom), an animal cell, a cell from an invertebrate animal (e.g., fruit fly, cnidarian, echinoderm, nematode, etc.), a cell from a vertebrate animal (e.g., fish, amphibian, reptile, bird, mammal), a cell from a mammal (e.g., a pig, a cow, a goat, a sheep, a rodent, a rat, a mouse, a non-human primate, a human, etc.), and etcetera. Sometimes a cell is not originating from a natural organism (e.g., a cell can be a synthetically made, sometimes termed an artificial cell).

The term "nucleotide," as used herein, generally refers to a base-sugar-phosphate combination. A nucleotide can comprise a synthetic nucleotide. A nucleotide can comprise a synthetic nucleotide analog. Nucleotides can be monomeric units of a nucleic acid sequence (e.g., deoxyribonucleic acid (DNA) and ribonucleic acid (RNA)). The term nucleotide can include ribonucleoside triphosphates adenosine triphosphate (ATP), uridine triphosphate (UTP), cytosine triphosphate (CTP), guanosine triphosphate (GTP) and deoxyribonucleoside triphosphates such as dATP, dCTP, dITP, dUTP, dGTP, dTTP, or derivatives thereof. Such derivatives can include, for example, [αS]dATP, 7-deaza-dGTP and 7-deaza-dATP, and nucleotide derivatives that confer nuclease resistance on the nucleic acid molecule containing them. The term nucleotide as used herein can refer to dideoxyribonucleoside triphosphates (ddNTPs) and their derivatives. Illustrative examples of dideoxyribonucleoside triphosphates can include, but are not limited to, ddATP, ddCTP, ddGTP, ddITP, and ddTTP. A nucleotide may be unlabeled or detectably labeled by well-known techniques. Labeling can also be carried out with quantum dots. Detectable labels can include, for example, radioactive isotopes, fluorescent labels, chemiluminescent labels, bioluminescent labels and enzyme labels. Fluorescent labels of nucleotides may include but are not limited fluorescein, 5-carboxyfluorescein (FAM), 2'7'-dimethoxy-4'5-dichloro-6-carboxyfluorescein (JOE), rhodamine, 6-carboxyrhodamine (R6G), N,N,N',N'-tetramethyl-6-carboxyrhodamine (TAMRA), 6-carboxy-X-rhodamine (ROX), 4-(4'dimethylaminophenylazo) benzoic acid (DABCYL), Cascade Blue, Oregon Green, Texas Red, Cyanine and 5-(2'-aminoethyl)aminonaphthalene-1-sulfonic acid (EDANS). Specific examples of fluorescently labeled nucleotides can include [R6G]dUTP, [TAMRA]dUTP, [R110]dCTP, [R6G] dCTP, [TAMRA] dCTP, [JOE] ddATP, [R6G] ddATP, [FAM] ddCTP, [R110]ddCTP, [TAMRA]ddGTP, [ROX]ddTTP, [dR6G]ddATP, [dR110]ddCTP, [dTAMRA]ddGTP, and [dROX]ddTTP available from Perkin Elmer, Foster City, Calif. FluoroLink DeoxyNucleotides, FluoroLink Cy3-dCTP, FluoroLink Cy5-dCTP, FluoroLink Fluor X-dCTP, FluoroLink Cy3-dUTP, and FluoroLink Cy5-dUTP available from Amersham, Arlington Heights, Ill.; Fluorescein-15-dATP, Fluorescein-12-dUTP, Tetramethyl-rodamine-6-dUTP, IR770-9-dATP, Fluorescein-12-ddUTP, Fluorescein-12-UTP, and Fluorescein-15-2'-dATP available from Boehringer Mannheim, Indianapolis, Ind.; and Chromosome Labeled Nucleotides, BODIPY-FL-14-UTP, BODIPY-FL-4-UTP, BODIPY-TMR-14-UTP, BODIPY-TMR-14-dUTP, BODIPY-TR-14-UTP, BODIPY-TR-14-dUTP, Cascade Blue-7-UTP, Cascade Blue-7-dUTP, fluorescein-12-UTP, fluorescein-12-dUTP, Oregon Green 488-5-dUTP, Rhodamine Green-5-UTP, Rhodamine Green-5-dUTP, tetramethylrhodamine-6-UTP, tetramethylrhodamine-6-dUTP, Texas Red-5-UTP, Texas Red-5-dUTP, and Texas Red-12-dUTP available from Molecular Probes, Eugene, Oreg. Nucleotides can also be labeled or marked by chemical modification. A chemically-modified single nucleotide can be biotin-dNTP. Some non-limiting examples of biotinylated dNTPs can include, biotin-dATP (e.g., bio-N6-ddATP, biotin-14-dATP), biotin-dCTP (e.g., biotin-11-dCTP, biotin-14-dCTP), and biotin-dUTP (e.g., biotin-11-dUTP, biotin-16-dUTP, biotin-20-dUTP).

The term "polynucleotide," "oligonucleotide," or "nucleic acid," as used interchangeably herein, generally refers to a polymeric form of nucleotides of any length, either deoxyribonucleotides or ribonucleotides, or analogs thereof, either in single-, double-, or multi-stranded form. A polynucleotide can be exogenous or endogenous to a cell. A polynucleotide can exist in a cell-free environment. A polynucleotide can be a gene or fragment thereof. A polynucleotide can be DNA. A polynucleotide can be RNA. A polynucleotide can have any three dimensional structure, and can perform any function, known or unknown. A polynucleotide can comprise one or more analogs (e.g., altered backbone, sugar, or nucleobase). If present, modifications to the nucleotide structure can be imparted before or after assembly of the polymer. Some non-limiting examples of analogs include: 5-bromouracil, peptide nucleic acid, xeno nucleic acid, morpholinos, locked nucleic acids, glycol nucleic acids, threose nucleic acids, dideoxynucleotides, cordycepin, 7-deaza-GTP, florophores (e.g., rhodamine or fluorescein linked to the sugar), thiol containing nucleotides, biotin linked nucleotides, fluorescent base analogs, CpG islands, methyl-7-guanosine, methylated nucleotides, inosine, thiouridine, pseudouridine, dihydrouridine, queuosine, and wyosine. Non-limiting examples of polynucleotides include coding or non-coding regions of a gene or gene fragment, loci (locus) defined from linkage analysis, exons, introns, messenger RNA (mRNA), transfer RNA (tRNA), ribosomal RNA (rRNA), short interfering RNA (siRNA), short-hairpin RNA (shRNA), micro-RNA (miRNA), ribozymes, cDNA, recombinant polynucleotides, branched polynucleotides, plasmids, vectors, isolated DNA of any sequence, isolated RNA of any sequence, cell-free polynucleotides including cell-free DNA (cfDNA) and cell-free RNA (cfRNA), nucleic acid probes, and primers. The sequence of nucleotides can be interrupted by non-nucleotide components.

The term "gene" generally refers to a nucleic acid (e.g., DNA such as genomic DNA and cDNA) and its corresponding nucleotide sequence that is involved in encoding an RNA transcript. The term as used herein with reference to genomic DNA includes intervening, non-coding regions as well as regulatory regions and can include 5' and 3' ends. In some uses, the term encompasses the transcribed sequences, including 5' and 3' untranslated regions (5'-UTR and 3'-UTR), exons and introns. In some genes, the transcribed region will contain "open reading frames" that encode polypeptides. In some uses of the term, a "gene" comprises only the coding sequences (e.g., an "open reading frame" or "coding region") necessary for encoding a polypeptide. In some cases, genes do not encode a polypeptide, for example, ribosomal RNA genes (rRNA) and transfer RNA (tRNA) genes. In some cases, the term "gene" includes not only the transcribed sequences, but in addition, also includes non-transcribed regions including upstream and downstream regulatory regions, enhancers and promoters. A gene can refer to an "endogenous gene" or a native gene in its natural location in the genome of an organism. A gene can refer to an "exogenous gene" or a non-native gene. A non-native gene can refer to a gene not normally found in the host organism but which is introduced into the host organism by gene transfer. A non-native gene can also refer to a gene not in its natural location in the genome of an organism. A non-native gene can also refer to a naturally occurring nucleic acid or polypeptide sequence that comprises mutations, insertions and/or deletions (e.g., non-native sequence).

The term "deletion" generally refers to the removal (or loss) of one or more (or a specified number of) amino acids (e.g., contiguous or non-contiguous amino acids) from a polypeptide sequence, or the removal (or loss) one or more (or a specified number of) nucleic acid bases (e.g., contiguous or non-contiguous nucleic acid bases) from a polynucleotide sequence (e.g., that encodes the polypeptide sequence. The term "internal deletion" generally refers to a deletion that does not include the N- or C-terminus of a polypeptide or the 5' or 3' end of a polynucleotide. A deletion (e.g., an internal deletion) can be identified by comparing to a reference sequence, e.g., by specifying the start and end positions of the deletion relative to the reference sequence. A deletion (e.g., an internal deletion) is different and distinct from a substitution. For example, deletion of at least one amino acid is not followed by an insertion of at least one different amino acid at the same position as the at least one amino acid as compared to a reference polypeptide sequence, such that the size (e.g., a number of the amino acid residue(s)) of a modified (or engineered) polypeptide sequence comprising the deletion of the at least one amino acid is smaller than the reference polypeptide sequence by the size of the at least one amino acid that has been deleted.

The term "sequence identity" generally refers to an exact nucleotide-to-nucleotide or amino acid-to-amino acid correspondence of two polynucleotides or polypeptide sequences, respectively. Typically, techniques for determining sequence identity include determining the nucleotide sequence of a polynucleotide and/or determining the amino acid sequence encoded thereby, and comparing these sequences to a second nucleotide or amino acid sequence. Two or more sequences (polynucleotide or amino acid) can be compared by determining their "percent identity." The percent identity of two sequences, whether nucleic acid or amino acid sequences, is the number of exact matches between two aligned sequences divided by the length of the longer sequence and multiplied by 100. Percent identity may also be determined, for example, by comparing sequence information using the advanced BLAST computer program, including version 2.2.9, available from the National Institutes of Health. The BLAST program is based on the alignment method of Karlin and Altschul, Proc. Natl. Acad. Sci. USA, 87:2264-2268 (1990) and as discussed in Altschul, et al., J. Mol. Biol., 215:403-410 (1990); Karlin And Altschul, Proc. Natl. Acad. Sci. USA, 90:5873-5877 (1993); and Altschul et al., Nucleic Acids Res., 25:3389-3402 (1997). The program may be used to determine percent identity over the entire length of the proteins being compared. Default parameters are provided to optimize searches with short query sequences in, for example, with the blastp program. The program also allows use of an SEG filter to mask-off segments of the query sequences as determined by the SEG program of Wootton and Federhen, Computers and Chemistry 17:149-163 (1993). Ranges of desired degrees of sequence identity are approximately 50% to 100% and integer values therebetween. In general, this disclosure encompasses sequences with at least 50%, at least 55%, at least 60%, at least 65%, at least 70%, at least 75%, at least 80%, at least 85%, at least 90%, at least 95%, or at least 98% sequence identity with any sequence provided herein.

The term "expression" generally refers to one or more processes by which a polynucleotide is transcribed from a DNA template (such as into an mRNA or other RNA transcript) and/or the process by which a transcribed mRNA is subsequently translated into peptides, polypeptides, or proteins. Transcripts and encoded polypeptides can be collectively referred to as "gene product." If the polynucleotide is derived from genomic DNA, expression can include splicing of the mRNA in a eukaryotic cell. "Upregulated," with reference to expression, generally refers to an increased expression level of a polynucleotide (e.g., RNA such as mRNA) and/or polypeptide sequence relative to its expression level in a wild-type state while "down-regulated" generally refers to a decreased expression level of a polynucleotide (e.g., RNA such as mRNA) and/or polypeptide sequence relative to its expression in a wild-type state. Expression of a transfected gene can occur transiently or stably in a cell. During "transient expression" the transfected gene is not transferred to the daughter cell during cell division. Since its expression is restricted to the transfected cell, expression of the gene is lost over time. In contrast, stable expression of a transfected gene can occur when the gene is co-transfected with another gene that confers a selection advantage to the transfected cell. Such a selection advantage may be a resistance towards a certain toxin that is presented to the cell.

The term "expression profile" generally refers to quantitative (e.g., abundance) and qualitative expression of one or more genes in a sample (e.g., a cell). The one or more genes can be expressed and ascertained in the form of a nucleic acid molecule (e.g., an mRNA or other RNA transcript). Alternatively or in addition to, the one or more genes can be expressed and ascertained in the form of a polypeptide (e.g., a protein measured via Western blot). An expression profile of a gene may be defined as a shape of an expression level of the gene over a time period (e.g., at least or up to about 1 hour, at least or up to about 2 hours, at least or up to about 3 hours, at least or up to about 4 hours, at least or up to about 5 hours, at least or up to about 6 hours, at least or up to about 7 hours, at least or up to about 8 hours, at least or up to about 9 hours, at least or up to about 10 hours, at least or up to about 11 hours, at least or up to about 12 hours, at least or up to about 16 hours, at least or up to about 18 hours, at least or up to about 24 hours, at least or up to about 36 hours, at least or up to about 48 hours, at least up to about 3 days, at least up to about 4 days, at least up to about 5 days, at least up to about 6 days, at least up to about 7 days, at least up to about 8 days, at least up to about 9 days, at least up to about 10 days, at least up to about 11 days, at least up to about 12 days, at least up to about 13 days, at least up to about 14 days, etc.). Alternatively, an expression profile of a gene may be defined as an expression level of the gene at a time point of interest (e.g., the expression level of the gene measured at least or up to about 1 hour, at least or up to about 2 hours, at least or up to about 3 hours, at least or up to about 4 hours, at least or up to about 5 hours, at least or up to about 6 hours, at least or up to about 7 hours, at least or up to about 8 hours, at least or up to about 9 hours, at least or up to about 10 hours, at least or up to about 11 hours, at least or up to about 12 hours, at least or up to about 16 hours, at least or up to about 18 hours, at least or up to about 24 hours, at least or up to about 36 hours, at least or up to about 48 hours, at least up to about 3 days, at least up to about 4 days, at least up to about 5 days, at least up to about 6 days, at least up to about 7 days, at least up to about 8 days, at least up to about 9 days, at least up to about 10 days, at least up to about 11 days, at least up to about 12 days, at least up to about 13 days, or at least up to about 14 days after treating a cell to induce such expression level.)

The term "peptide," "polypeptide," or "protein," as used interchangeably herein, generally refers to a polymer of at least two amino acid residues joined by peptide bond(s). This term does not connote a specific length of polymer, nor is it intended to imply or distinguish whether the peptide is produced using recombinant techniques, chemical or enzymatic synthesis, or is naturally occurring. The terms apply to naturally occurring amino acid polymers as well as amino acid polymers comprising at least one modified amino acid. In some cases, the polymer can be interrupted by non-amino acids. The terms include amino acid chains of any length, including full length proteins, and proteins with or without secondary and/or tertiary structure (e.g., domains). The terms also encompass an amino acid polymer that has been modified, for example, by disulfide bond formation, glycosylation, lipidation, acetylation, phosphorylation, oxidation, and any other manipulation such as conjugation with a labeling component. The terms "amino acid" and "amino acids," as used herein, generally refer to natural and non-natural amino acids, including, but not limited to, modified amino acids and amino acid analogues. Modified amino acids can include natural amino acids and non-natural amino acids, which have been chemically modified to include a group or a chemical moiety not naturally present on the amino acid. Amino acid analogues can refer to amino acid derivatives. The term "amino acid" includes both D-amino acids and L-amino acids.

The term "derivative," "variant," or "fragment," as used herein with reference to a polypeptide, generally refers to a polypeptide related to a wild type polypeptide, for example either by amino acid sequence, structure (e.g., secondary and/or tertiary), activity (e.g., enzymatic activity) and/or function. Derivatives, variants and fragments of a polypeptide can comprise one or more amino acid variations (e.g., mutations, insertions, and deletions), truncations, modifications, or combinations thereof compared to a wild type polypeptide.

The term "engineered," "chimeric," or "recombinant," as used herein with respect to a polypeptide molecule (e.g., a protein), generally refers to a polypeptide molecule having a heterologous amino acid sequence or an altered amino acid sequence as a result of the application of genetic engineering techniques to nucleic acids which encode the polypeptide molecule, as well as cells or organisms which express the polypeptide molecule. The term "engineered" or "recombinant," as used herein with respect to a polynucleotide molecule (e.g., a DNA or RNA molecule), generally refers to a polynucleotide molecule having a heterologous nucleic acid sequence or an altered nucleic acid sequence as a result of the application of genetic engineering techniques. Genetic engineering techniques include, but are not limited to, PCR and DNA cloning technologies; transfection, transformation and other gene transfer technologies; homologous recombination; site-directed mutagenesis; and gene fusion. In some cases, an engineered or recombinant polynucleotide (e.g., a genomic DNA sequence) can be modified or altered by a gene editing moiety.

For example, an engineered nuclease (e.g., an engineered Cas protein) as disclosed herein is not a naturally occurring nuclease (e.g., not a naturally occurring Cas protein). The terms "engineered nuclease" and "engineered nuclease variant" may be used interchangeable herein.

The terms "engineered" and "modified" are used interchangeably herein. The terms "engineering" and "modifying" are used interchangeably herein. The terms "engineered cell" or "modified cell" are used interchangeably herein. The terms "engineered characteristic" and "modified characteristic" are used interchangeably herein.

The term "enhanced expression," "increased expression," or "upregulated expression" generally refers to production of a moiety of interest (e.g., a polynucleotide or a polypeptide) to a level that is above a normal level of expression of the moiety of interest in a host strain (e.g., a host cell). The normal level of expression can be substantially zero (or null) or higher than zero. The moiety of interest can comprise an endogenous gene or polypeptide construct of the host strain. The moiety of interest can comprise a heterologous gene or polypeptide construct that is introduced to or into the host strain. For example, a heterologous gene encoding a polypeptide of interest can be knocked-in (KI) to a genome of the host strain for enhanced expression of the polypeptide of interest in the host strain.

The term "enhanced activity," "increased activity," or "upregulated activity" generally refers to activity of a moiety of interest (e.g., a polynucleotide or a polypeptide) that is modified to a level that is above a normal level of activity of the moiety of interest in a host strain (e.g., a host cell). The normal level of activity can be substantially zero (or null) or higher than zero. The moiety of interest can comprise a polypeptide construct of the host strain. The moiety of interest can comprise a heterologous polypeptide construct that is introduced to or into the host strain. For example, a heterologous gene encoding a polypeptide of interest can be knocked-in (KI) to a genome of the host strain for enhanced activity of the polypeptide of interest in the host strain.

The term "reduced expression," "decreased expression," or "downregulated expression" generally refers to a production of a moiety of interest (e.g., a polynucleotide or a polypeptide) to a level that is below a normal level of expression of the moiety of interest in a host strain (e.g., a host cell). The normal level of expression is higher than zero. The moiety of interest can comprise an endogenous gene or polypeptide construct of the host strain. In some cases, the moiety of interest can be knocked-out or knocked-down in the host strain. In some examples, reduced expression of the moiety of interest can include a complete inhibition of such expression in the host strain.

The term "reduced activity," "decreased activity," or "downregulated activity" generally refers to activity of a moiety of interest (e.g., a polynucleotide or a polypeptide) that is modified to a level that is below a normal level of activity of the moiety of interest in a host strain (e.g., a host cell). The normal level of activity is higher than zero. The moiety of interest can comprise an endogenous gene or polypeptide construct of the host strain. In some cases, the moiety of interest can be knocked-out or knocked-down in the host strain. In some examples, reduced activity of the moiety of interest can include a complete inhibition of such activity in the host strain.

The term "subject," "individual," or "patient," as used interchangeably herein, generally refers to a vertebrate, preferably a mammal such as a human. Mammals include, but are not limited to, murines, simians, humans, farm animals, sport animals, and pets. Tissues, cells and their progeny of a biological entity obtained in vivo or cultured in vitro are also encompassed.

The term "treatment" or "treating" generally refers to an approach for obtaining beneficial or desired results including but not limited to a therapeutic benefit and/or a prophylactic benefit. For example, a treatment can comprise administering a system or cell population disclosed herein. By therapeutic benefit is meant any therapeutically relevant improvement in or effect on one or more diseases, conditions, or symptoms under treatment. For prophylactic benefit, a composition can be administered to a subject at risk of developing a particular disease, condition, or symptom, or to a subject reporting one or more of the physiological symptoms of a disease, even though the disease, condition, or symptom may not have yet been manifested.

The term "effective amount" or "therapeutically effective amount" generally refers to the quantity of a composition, for example a composition comprising heterologous polypeptides, heterologous polynucleotides, and/or modified cells (e.g., modified stem cells), that is sufficient to result in a desired activity upon administration to a subject in need thereof. Within the context of the present disclosure, the term "therapeutically effective" generally refers to that quantity of a composition that is sufficient to delay the manifestation, arrest the progression, relieve or alleviate at least one symptom of a disorder treated by the methods of the present disclosure.

### Overview

Disclosed are engineered nucleases that are smaller, yet effective, in binding, cleaving, and/or editing a target polynucleotide sequence, compositions thereof, and methods of use thereof. Such engineered nucleases (e.g., engineered CRISPR/Cas nuclease) can, for example, effect manipulation of expression or activity of a target gene (e.g., a target endogenous gene) in a cell, e.g., to treat or ameliorate a condition (e.g., a disease) of a subject. Gene expression can underpin various physiological and pathological effects in cells and tissues, contributing to many diseases and conditions, and thus compositions and methods utilizing the engineered nucleases of the present disclosure can modulate expression of specific genes in a desirable way to have therapeutic benefit.

### Engineered nucleases, compositions, and methods thereof

Disclosed is an engineered nuclease comprising an amino acid sequence that is at least 50% identical to the polypeptide sequence of SEQ ID NO: 1. The amino acid sequence of the engineered nuclease can comprise at least one deletion, as compared to (e.g., when aligned to) the polypeptide sequence of SEQ ID NO: 1 (or SEQ ID NO: 10). The at least one deletion can be selectively removed in accordance with the present disclosure. As disclosed herein, SEQ ID NO: 1 encodes the polypeptide sequence of Un1Cas12f1 (or Cas14a1). As disclosed herein, SEQ ID NO: 10 encodes an engineered variant of Un1Cas12f1 with reduced nuclease activity. Thus, the amino acid sequence of the engineered nuclease as disclosed herein can be a mutant sequence (or a mutant variant) of Un1Cas12f1 (or a deactivated variant thereof).

Without wishing to be bound by theory, the at least one deletion of the amino acid sequence of the engineered nuclease, as disclosed herein, can be found in one or more regions of the native Un1Cas12f1 nuclease that do not structurally align to a control CRISPR/Cas protein. The control CRISPR/Cas protein can be from Class 1 CRISPR system or Class 2 CRISPR system (e.g., as a wild type CRISPR/Cas protein). Class 1 CRISPR system can be divided into types I, III, and IV, and Class 2 CRISPR system can be divided into types II, V, and VI. In some cases, the control CRISPR/Cas protein can be a type V Cas protein, e.g., a type V-A Cas protein, a type V-B Cas protein, a type V-C Cas protein, a type V-D Cas protein, a type V-E Cas protein, a type V-F Cas protein, a type V-G Cas protein, a type V-H Cas protein, a type V-I Cas protein, a type V-J Cas protein, a type V-K Cas protein, or a type V-U Cas protein. In some cases, the control CRISPR/Cas protein can be a type V-J protein, such as a wild-type CasΦ (Cas 12J) protein. In some cases, the control CRISPR/Cas protein can be Un2Cas12f1 (SEQ ID NO: 2) or AsCas12f (SEQ ID NO: 3).

Without wishing to be bound by theory, the at least one deletion of the amino acid sequence of the engineered nuclease, as disclosed herein, can be determined by performing a deletion landscape study (e.g., iterative and/or comprehensive deletion) of the Cas nuclease encoded by the polypeptide sequence of SEQ ID NO: 1 (or SEQ ID NO: 10).

Without wishing to be bound by theory, a plurality of different variants of the engineered nuclease, as disclosed herein, can exhibit different activities (e.g., different binding affinities to a control sgRNA, different binding affinities to a control target gene, different target gene cleaving level, different target gene activation level, different target gene repression level, etc.). In some embodiments, a first variant of the plurality of different variants can comprise the at least one deletion at the amino acid resides 1-100 (e.g., when aligned to the polypeptide sequence of SEQ ID NO: 1), and a second variant of the plurality of different variants can comprise the at least one deletion at the amino acid resides 101-529 (e.g., when aligned to the polypeptide sequence of SEQ ID NO: 1), and the first variant and the second variant can exhibit different activities (e.g., the first variant can effect enhanced target gene activation and/or expression as compared to that of the second variant, or vice versa). Alternatively, the first variant and the second variant can exhibit comparable activities. In some embodiments, a first variant of the plurality of different variants can comprise a single deletion at the amino acid resides 1-100 (e.g., when aligned to the polypeptide sequence of SEQ ID NO: 1), and a second variant of the plurality of different variants can comprise a plurality of deletions at the amino acid resides 1-100 (e.g., when aligned to the polypeptide sequence of SEQ ID NO: 1), and the first variant and the second variant can exhibit different activities (e.g., the second variant can effect enhanced target gene activation and/or expression as compared to that of the first variant, or vice versa). Alternatively, the first variant and the second variant can exhibit comparable activities.
**SEQ ID NO: 1 (Un1Cas12f1)** 
1 MAKNTITKTL KLRIVRPYNS AEVEKIVADE KNNREKIALE KNKDKVKEAC
51 SKHLKVAAYC TTQVERNACL FCKARKLDDK FYQKLRGQFP DAVFWQEISE
101 IFRQLQKQAA EIYNQSLIEL YYEIFIKGKG IANASSVEHY LSDVCYTRAA
151 ELFKNAAIAS GLRSKIKSNF RLKELKNMKS GLPTTKSDNF PIPLVKQKGG
201 QYTGFEISNH NSDFIIKIPF GRWQVKKEID KYRPWEKFDF EQVQKSPKPI
251 SLLLSTQRRK RNKGWSKDEG TEAEIKKVMN GDYQTSYIEV KRGSKIGEKS
301 AWMLNLSIDV PKIDKGVDPS IIGGIDVGVK SPLVCAINNA FSRYSISDND
351 LFHFNKKMFA RRRILLKKNR HKRAGHGAKN KLKPITILTE KSERFRKKLI
401 ERWACEIADF FIKNKVGTVQ MENLESMKRK EDSYFNIRLR GFWPYAEMQN
451 KIEFKLKQYG IEIRKVAPNN TSKTCSKCGH LNNYFNFEYR KKNKFPHFKC
501 EKCNFKENAD YNAALNISNP KLKSTKEEP
**SEQ ID NO: 10 (deactivated nuclease variant of Un1Cas12f1, i.e., dCasMINI)** 
1 MAKNTITKTL KLRIVRPYNS AEVEKIVADE KNNREKIALE KNKDKVKEAC
51 SKHLKVAAYC TTQVERNACL FCKARKLDDK FYQKLRGQFP DAVFWQEISE
101 IFRQLQKQAA EIYNQSLIEL YYEIFIKGKG IANASSVEHY LSRVCYRRAA
151 ELFKNAAIAS GLRSKIKSNF RLKELKNMKS GLPTTKSDNF PIPLVKQKGG
201 QYTGFEISNH NSDFIIKIPF GRWQVKKEID KYRPWEKFDF EQVQKSPKPI
251 SLLLSTQRRK RNKGWSKDEG TEAEIKKVMN GDYQTSYIEV KRGSKICEKS
301 AWMLNLSIDV PKIDKGVDPS IIGGIAVGVR SPLVCAINNA FSRYSISDND
351 LFHFNKKMFA RRRILLKKNR HKRAGHGAKN KLKPITILTE KSERFRKKLI
401 ERWACEIADF FIKNKVGTVQ MENLESMKRK EDSYFNIRLR GFWPYAEMQN
451 KIEFKLKQYG IEIRKVAPNN TSKTCSKCGH LNNYFNFEYR KKNKFPHFKC
501 EKCNFKENAA YNAALNISNP KLKSTKERP
**SEQ ID NO: 2 (Un2Cas12f1)** 
1 MEVQKTVMKT LSLRILRPLY SQEIEKEIKE EKERRKQAGG TGELDGGFYK
51 KLEKKHSEMF SFDRLNLLLN QLQREIAKVY NHAISELYIA TIAQGNKSNK
101 HYISSIVYNR AYGYFYNAYI ALGICSKVEA NFRSNELLTQ QSALPTAKSD
151 NFPIVLHKQK GAEGEDGGFR ISTEGSDLIF EIPIPFYEYN GENRKEPYKW
201 VKKGGQKPVL KLILSTFRRQ RNKGWAKDEG TDAEIRKVTE GKYQVSQIEI
251 NRGKKLGEHQ KWFANFSIEQ PIYERKPNRS IVGGLDVGIR SPLVCAINNS
 
301 FSRYSVDSND VFKFSKQVFA FRRRLLSKNS LKRKGHGAAH KLEPITEMTE
351 KNDKFRKKII ERWAKEVTNF FVKNQVGIVQ IEDLSTMKDR EDHFFNQYLR
401 GFWPYYQMQT LIENKLKEYG IEVKRVQAKY TSQLCSNPNC RYWNNYFNFE
451 YRKVNKFPKF KCEKCNLEIS ADYNAARNLS TPDIEKFVAK ATKGINLPEK
**SEQ ID NO: 3 (AsCas12f)**

1 MIKVYRYEIV KPLDLDWKEF GTILRQLQQE TRFALNKATQ LAWEWMGFSS
51 DYKDNHGEYP KSKDILGYTN VHGYAYHTIK TKAYRLNSGN LSQTIKRATD
101 RFKAYQKEIL RGDMSIPSYK RDIPLDLIKE NISVNRMNHG DYIASLSLLS
151 NPAKQEMNVK RKISVIIIVR GAGKTIMDRI LSGEYQVSAS QIIHDDRKNK
201 WYLNISYDFE PQTRVLDLNK IMGIDLGVAV AVYMAFQHTP ARYKLEGGEI
251 ENFRRQVESR RISMLRQGKY AGGARGGHGR DKRIKPIEQL RDKIANFRDT
301 TNHRYSRYIV DMAIKEGCGT IQMEDLTNIR DIGSRFLQNW TYYDLQQKII
351 YKAEEAGIKV IKIDPQYTSQ RCSECGNIDS GNRIGQAIFK CRACGYEANA
401 DYNAARNIAI PNIDKIIAES IK

Throughout the present disclosure, (i) a sequence comparison between the amino acid sequence of the engineered nuclease disclosed herein and the polypeptide sequence of SEQ ID NO: 1 may be comparable (e.g., substantially identical) to (ii) a sequence comparison between the amino acid sequence of the engineered nuclease disclosed herein and the polypeptide sequence of SEQ ID NO: 10.

In some embodiments, the amino acid sequence of the engineered nuclease disclosed herein can be at least or up to about 50%, at least or up to about 55%, at least or up to about 60%, at least or up to about 62%, at least or up to about 64%, at least or up to about 65%, at least or up to about 66%, at least or up to about 68%, at least or up to about 70%, at least or up to about 72%, at least or up to about 74%, at least or up to about 75%, at least or up to about 76%, at least or up to about 78%, at least or up to about 80%, at least or up to about 82%, at least or up to about 84%, at least or up to about 85%, at least or up to about 86%, at least or up to about 88%, at least or up to about 90%, at least or up to about 91%, at least or up to about 92%, at least or up to about 93%, at least or up to about 94%, at least or up to about 95%, at least or up to about 96%, at least or up to about 97%, at least or up to about 98%, at least or up to about 99% identical to (i) the polypeptide sequence of SEQ ID NO: 1 or (ii) the polypeptide sequence of any one of the Cas proteins selected from TABLE 2. For example, the amino acid sequence of the engineered nuclease can be between about 80% and about 100% identical to (i) the polypeptide sequence of SEQ ID NO: 1 or (ii) the polypeptide sequence of any one of the Cas proteins selected from TABLE 2.

The amino acid sequence of the engineered nuclease of the present invention is not identical to (i) the polypeptide sequence of SEQ ID NO: 1 or (ii) the polypeptide sequence of any one of the Cas proteins selected from TABLE 2.

In some embodiments, the at least one deletion of the amino acid sequence of the engineered nuclease disclosed herein can comprise an N-terminus deletion, a C-terminus deletion, and/or an internal deletion. For example, the at least one deletion may not comprise an N-terminus deletion, but rather an internal deletion and/or a C-terminus deletion.

In some embodiments, the at least one deletion of the amino acid sequence of the engineered nuclease disclosed herein can be from the amino acid residues 1-100 (e.g., the amino acid residues 2-100), the amino acid resides 101-429, the amino acid residues 101-200, the amino acid residues 201-300, the amino acid residues 301-400, the amino acid residues 401-500, or the amino acid residues 500-529, and/or amino acid residues 430-529, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease disclosed herein can be from at least one deletion from the amino acid resides 2-100, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease can be from the amino acid residues 2-98, the amino acid residues 2-96, the amino acid residues 2-95, the amino acid residues 2-94, the amino acid residues 2-92, the amino acid residues 2-90, the amino acid residues 2-88, the amino acid residues 2-86, the amino acid residues 2-85, the amino acid residues 2-84, the amino acid residues 2-82, the amino acid residues 2-80, the amino acid residues 2-78, the amino acid residues 2-76, the amino acid residues 2-75, the amino acid residues 2-74, the amino acid residues 2-72, the amino acid residues 2-70, the amino acid residues 2-68, the amino acid residues 2-66, the amino acid residues 2-65, the amino acid residues 2-64, the amino acid residues 2-62, the amino acid residues 2-60, the amino acid residues 2-58, the amino acid residues 2-56, the amino acid residues 2-55, the amino acid residues 2-54, the amino acid residues 2-52, the amino acid residues 2-50, the amino acid residues 2-48, the amino acid residues 2-46, the amino acid residues 2-45, the amino acid residues 2-44, the amino acid residues 2-42, the amino acid residues 2-40, the amino acid residues 2-38, the amino acid residues 2-36, the amino acid residues 2-35, the amino acid residues 2-34, the amino acid residues 2-32, the amino acid residues 2-30, the amino acid residues 2-28, the amino acid residues 2-26, the amino acid residues 2-25, the amino acid residues 2-24, the amino acid residues 2-22, the amino acid residues 2-20, the amino acid residues 2-18, the amino acid residues 2-16, the amino acid residues 2-15, the amino acid residues 2-14, the amino acid residues 2-12, the amino acid residues 2-10, the amino acid residues 2-8, the amino acid residues 2-6, the amino acid residues 2-5, or the amino acid residues 2-4, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease can be from the amino acid residues 2-100, the amino acid residues 4-100, the amino acid residues 5-100, the amino acid residues 6-100, the amino acid residues 8-100, the amino acid residues 10-100, the amino acid residues 12-100, the amino acid residues 14-100, the amino acid residues 15-100, the amino acid residues 16-100, the amino acid residues 18-100, the amino acid residues 20-100, the amino acid residues 22-100, the amino acid residues 24-100, the amino acid residues 25-100, the amino acid residues 26-100, the amino acid residues 28-100, the amino acid residues 30-100, the amino acid residues 32-100, the amino acid residues 34-100, the amino acid residues 35-100, the amino acid residues 36-100, the amino acid residues 38-100, the amino acid residues 40-100, the amino acid residues 42-100, the amino acid residues 44-100, the amino acid residues 45-100, the amino acid residues 46-100, the amino acid residues 48-100, the amino acid residues 50-100, the amino acid residues 52-100, the amino acid residues 54-100, the amino acid residues 55-100, the amino acid residues 56-100, the amino acid residues 58-100, the amino acid residues 60-100, the amino acid residues 62-100, the amino acid residues 64-100, the amino acid residues 65-100, the amino acid residues 66-100, the amino acid residues 68-100, the amino acid residues 70-100, the amino acid residues 72-100, the amino acid residues 75-100, the amino acid residues 76-100, the amino acid residues 78-100, the amino acid residues 80-100, the amino acid residues 82-100, the amino acid residues 84-100, the amino acid residues 85-100, the amino acid residues 86-100, the amino acid residues 88-100, the amino acid residues 90-100, the amino acid residues 92-100, the amino acid residues 94-100, the amino acid residues 95-100, the amino acid residues 96-100, or the amino acid residues 98-100, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease can be from the amino acid residues 30-100, the amino acid residues 30-98, the amino acid residues 30-96, the amino acid residues 30-95, the amino acid residues 30-94, the amino acid residues 30-92, the amino acid residues 30-90, the amino acid residues 30-88, the amino acid residues 30-86, the amino acid residues 30-85, the amino acid residues 30-84, the amino acid residues 30-82, the amino acid residues 30-80, the amino acid residues 30-78, the amino acid residues 30-76, the amino acid residues 30-75, the amino acid residues 30-74, the amino acid residues 30-72, the amino acid residues 30-70, the amino acid residues 30-68, the amino acid residues 30-66, the amino acid residues 30-65, the amino acid residues 30-64, the amino acid residues 30-62, the amino acid residues 30-60, the amino acid residues 30-58, the amino acid residues 30-56, the amino acid residues 30-55, the amino acid residues 30-54, the amino acid residues 30-52, the amino acid residues 30-50, the amino acid residues 30-48, the amino acid residues 30-46, the amino acid residues 30-45, the amino acid residues 30-44, the amino acid residues 30-42, the amino acid residues 30-40, the amino acid residues 30-38, the amino acid residues 30-36, the amino acid residues 30-34, or the amino acid residues 30-32, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease can comprise one or more deletions from (e.g., deletions of substantially all of the amino acid residues from) the amino acid residues 55-56, the amino acid residues 54-57, the amino acid residues 54-58, the amino acid residues 53-59, the amino acid residues 52-60, the amino acid residues 51-61, the amino acid residues 50-62, the amino acid residues 49-63, the amino acid residues 48-64, the amino acid residues 47-65, the amino acid residues 46-66, the amino acid residues 45-67, the amino acid residues 44-68, the amino acid residues 43-69, the amino acid residues 42-70, the amino acid residues 41-71, the amino acid residues 40-72, the amino acid residues 39-73, the amino acid residues 38-74, the amino acid residues 37-73, the amino acid residues 36-74, or the amino acid residues 35-75, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease can be from one or more members (e.g., a single member, at least 2 members, at least 3 members, at least 4 members, at least 5 members, or more) selected from the group consisting of the amino acid resides 2-10, the amino acid resides 11-20, the amino acid resides 21-30, the amino acid resides 31-40, the amino acid resides 41-50, the amino acid resides 51-60, the amino acid resides 61-70, and the amino acid resides 71-80, as compared to the polypeptide sequence of SEQ ID NO: 1. In some embodiments, the at least one deletion of the amino acid sequence of the engineered nuclease can be from one or more members (e.g., a single member, at least 2 members, at least 3 members, at least 4 members, at least 5 members, or more) selected from the group consisting of the amino acid resides 2-5, the amino acid resides 6-10, the amino acid resides 11-15, the amino acid resides 16-20, the amino acid resides 21-25, the amino acid resides 26-30, the amino acid resides 31-35, the amino acid resides 36-40, the amino acid resides 41-45, the amino acid resides 46-50, the amino acid resides 51-55, the amino acid resides 56-60, the amino acid resides 61-65, the amino acid resides 66-70, the amino acid resides 71-75, and the amino acid resides 76-80, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease disclosed herein can be from one or more members (e.g., two or more members, three or more members, four or more members, five or more members, etc.) selected from the group consisting of the amino acid resides 10-90, the amino acid residues 15-85, the amino acid residues 20-80, the amino acid residues 25-75, the amino acid residues 30-70, the amino acid residues 35-75, the amino acid residues 40-70, the amino acid residues 45-65, or the amino acid residues 50-60, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease disclosed herein can be from one or more members (e.g., two or more members, three or more members, four or more members, five or more members, etc.) selected from the group consisting of the amino acid residues 20-30, the amino acid resides 25-35, the amino acid residues 30-40, the amino acid residues 35-45, the amino acid residues 40-50, the amino acid residues 45-55, the amino acid residues 50-60, the amino acid residues 55-65, the amino acid residues 60-70, the amino acid residues 65-75, the amino acid residues 70-80, the amino acid residues 75-85, and the amino acid residues 80-90, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease disclosed herein can be from one or more members (e.g., two or more members, three or more members, four or more members, five or more members, etc.) selected from the group consisting of the amino acid residues 20-25, the amino acid residues 25-30, the amino acid residues 30-35, the amino acid residues 35-40, the amino acid residues 40-45, the amino acid residues 45-50, the amino acid residues 50-55, the amino acid residues 55-60, the amino acid residues 60-65, the amino acid residues 65-70, the amino acid residues 70-75, the amino acid residues 75-80, and the amino acid residues 85-90, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease disclosed herein can be from the amino acid resides 430-529, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease can be from the amino acid residues 430-528, the amino acid residues 430-526, the amino acid residues 430-525, the amino acid residues 430-524, the amino acid residues 430-522, the amino acid residues 430-520, the amino acid residues 430-518, the amino acid residues 430-516, the amino acid residues 430-515, the amino acid residues 430-514, the amino acid residues 430-512, the amino acid residues 430-510, the amino acid residues 430-508, the amino acid residues 430-506, the amino acid residues 430-505, the amino acid residues 430-504, the amino acid residues 430-502, the amino acid residues 430-500, the amino acid residues 430-498, the amino acid residues 430-496, the amino acid residues 430-495, the amino acid residues 430-494, the amino acid residues 430-492, the amino acid residues 430-490, the amino acid residues 430-488, the amino acid residues 430-486, the amino acid residues 430-485, the amino acid residues 430-484, the amino acid residues 430-482, the amino acid residues 430-480, the amino acid residues 430-478, the amino acid residues 430-476, the amino acid residues 430-475, the amino acid residues 430-474, the amino acid residues 430-472, the amino acid residues 430-470, the amino acid residues 430-468, the amino acid residues 430-466, the amino acid residues 430-465, the amino acid residues 430-464, the amino acid residues 430-462, the amino acid residues 430-460, the amino acid residues 430-458, the amino acid residues 430-456, the amino acid residues 430-455, the amino acid residues 430-454, the amino acid residues 430-452, the amino acid residues 430-450, the amino acid residues 430-448, the amino acid residues 430-446, the amino acid residues 430-445, the amino acid residues 430-444, the amino acid residues 430-442, the amino acid residues 430-440, the amino acid residues 430-438, the amino acid residues 430-436, the amino acid residues 430-435, the amino acid residues 430-434, or the amino acid residues 430-432, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease can be from the amino acid residues 430-529, the amino acid residues 432-529, the amino acid residues 434-529, the amino acid residues 435-529, the amino acid residues 436-529, the amino acid residues 438-529, the amino acid residues 440-529, the amino acid residues 442-529, the amino acid residues 444-529, the amino acid residues 445-529, the amino acid residues 446-529, the amino acid residues 448-529, the amino acid residues 450-529, the amino acid residues 452-529, the amino acid residues 454-529, the amino acid residues 455-529, the amino acid residues 456-529, the amino acid residues 458-529, the amino acid residues 460-529, the amino acid residues 462-529, the amino acid residues 464-529, the amino acid residues 465-529, the amino acid residues 466-529, the amino acid residues 468-529, the amino acid residues 470-529, the amino acid residues 472-529, the amino acid residues 474-529, the amino acid residues 475-529, the amino acid residues 476-529, the amino acid residues 478-529, the amino acid residues 480-529, the amino acid residues 482-529, the amino acid residues 484-529, the amino acid residues 485-529, the amino acid residues 486-529, the amino acid residues 488-529, the amino acid residues 490-529, the amino acid residues 492-529, the amino acid residues 494-529, the amino acid residues 495-529, the amino acid residues 496-529, the amino acid residues 498-529, the amino acid residues 500-529, the amino acid residues 502-529, the amino acid residues 504-529, the amino acid residues 505-529, the amino acid residues 506-529, the amino acid residues 508-529, the amino acid residues 510-529, the amino acid residues 512-529, the amino acid residues 514-529, the amino acid residues 515-529, the amino acid residues 516-529, the amino acid residues 518-529, the amino acid residues 520-529, the amino acid residues 522-529, the amino acid residues 524-529, the amino acid residues 525-529, the amino acid residues 526-529, or the amino acid residues 528-529, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease can be from one or more members (e.g., a single member, at least 2 members, at least 3 members, at least 4 members, at least 5 members, or more) selected from the group consisting of the amino acid resides 450-459, the amino acid resides 460-469, the amino acid resides 470-479, the amino acid resides 480-489, the amino acid resides 490-499, the amino acid resides 500-509, the amino acid resides 510-519, and the amino acid resides 520-529, as compared to (i) the polypeptide sequence of SEQ ID NO: 1 or (ii) the polypeptide sequence of any one of the Cas proteins selected from TABLE 2 (when applicable). In some embodiments, the at least one deletion of the amino acid sequence of the engineered nuclease can be from one or more members (e.g., a single member, at least 2 members, at least 3 members, at least 4 members, at least 5 members, or more) selected from the group consisting of the amino acid resides 450-459, the amino acid resides 460-465, the amino acid resides 466-469, the amino acid resides 470-475, the amino acid resides 476-479, the amino acid resides 480-485, the amino acid resides 486-489, the amino acid resides 490-495, the amino acid resides 496-499, the amino acid resides 500-505, the amino acid resides 506-509, the amino acid resides 510-515, the amino acid resides 516-519, the amino acid resides 520-525, and the amino acid resides 526-529, as compared to (i) the polypeptide sequence of SEQ ID NO: 1 or (ii) the polypeptide sequence of any one of the Cas proteins selected from TABLE 2 (when applicable).

In some embodiments, the at least one deletion of the amino acid sequence of the engineered nuclease can comprise deletion of a single amino acid residue, as compared to (i) the polypeptide sequence of SEQ ID NO: 1 or (ii) the polypeptide sequence of any one of the Cas proteins selected from TABLE 2 (when applicable). In some embodiments, the at least one deletion of the amino acid sequence of the engineered nuclease can comprise deletion of a plurality of amino acid residues, as compared to (i) the polypeptide sequence of SEQ ID NO: 1 or (ii) the polypeptide sequence of any one of the Cas proteins selected from TABLE 2 (when applicable). The plurality of amino acid residues that are deleted can be adjacent to each other (e.g., consecutive) when aligned to (i) the polypeptide sequence of SEQ ID NO: 1 or (ii) the polypeptide sequence of any one of the Cas proteins selected from TABLE 2 (when applicable). Alternatively or in addition to, the plurality of amino acid residues that are deleted may not be adjacent to each other, when aligned to (i) the polypeptide sequence of SEQ ID NO: 1 or (ii) the polypeptide sequence of any one of the Cas proteins selected from TABLE 2 (when applicable). The plurality of amino acid residues can comprise at least or up to about 2 amino acid residues, at least or up to about 3 amino acid residues, at least or up to about 4 amino acid residues, at least or up to about 5 amino acid residues, at least or up to about 6 amino acid residues, at least or up to about 7 amino acid residues, at least or up to about 8 amino acid residues, at least or up to about 9 amino acid residues, at least or up to about 10 amino acid residues, at least or up to about 11 amino acid residues, at least or up to about 12 amino acid residues, at least or up to about 13 amino acid residues, at least or up to about 14 amino acid residues, at least or up to about 15 amino acid residues, at least or up to about 16 amino acid residues, at least or up to about 17 amino acid residues, at least or up to about 18 amino acid residues, at least or up to about 19 amino acid residues, at least or up to about 20 amino acid residues, at least or up to about 22 amino acid residues, at least or up to about 24 amino acid residues, at least or up to about 25 amino acid residues, at least or up to about 26 amino acid residues, at least or up to about 28 amino acid residues, at least or up to about 30 amino acid residues, at least or up to about 32 amino acid residues, at least or up to about 34 amino acid residues, at least or up to about 35 amino acid residues, at least or up to about 36 amino acid residues, at least or up to about 38 amino acid residues, at least or up to about 40 amino acid residues, at least or up to about 42 amino acid residues, at least or up to about 44 amino acid residues, at least or up to about 45 amino acid residues, at least or up to about 46 amino acid residues, at least or up to about 48 amino acid residues, at least or up to about 50 amino acid residues, at least or up to about 52 amino acid residues, at least or up to about 54 amino acid residues, at least or up to about 55 amino acid residues, at least or up to about 56 amino acid residues, at least or up to about 58 amino acid residues, at least or up to about 60 amino acid residues, at least or up to about 62 amino acid residues, at least or up to about 64 amino acid residues, at least or up to about 65 amino acid residues, at least or up to about 66 amino acid residues, at least or up to about 68 amino acid residues, at least or up to about 70 amino acid residues, at least or up to about 72 amino acid residues, at least or up to about 74 amino acid residues, at least or up to about 75 amino acid residues, at least or up to about 76 amino acid residues, at least or up to about 78 amino acid residues, at least or up to about 80 amino acid residues, at least or up to about 82 amino acid residues, at least or up to about 84 amino acid residues, at least or up to about 85 amino acid residues, at least or up to about 86 amino acid residues, at least or up to about 88 amino acid residues, at least or up to about 90 amino acid residues, at least or up to about 92 amino acid residues, at least or up to about 94 amino acid residues, at least or up to about 95 amino acid residues, at least or up to about 96 amino acid residues, at least or up to about 98 amino acid residues, or at least or up to about 100 amino acid residues.

In some embodiments, the deletion of the plurality of amino acid residues to generate the amino acid sequence of the engineered nuclease can comprise deletion of a plurality of non-consecutive amino acid residues, e.g., as compared to (i) the polypeptide sequence of SEQ ID NO: 1 or (ii) the polypeptide sequence of any one of the Cas proteins selected from TABLE 2 (when applicable). In some cases, the deletion of the plurality of non-consecutive amino acid residues in the amino acid sequence of the engineered nuclease can comprise deletion of a first amino acid residue (e.g., a first single amino acid residue or a first set of multiple amino acid residues, such as a first set of consecutive amino acid residues) and a second amino acid residue (e.g., a second single amino acid residue or a second set of multiple amino acid residues, such as a second set of consecutive amino acid residues) that are not consecutive to each other, when aligned to (i) the polypeptide sequence of SEQ ID NO: 1 or (ii) the polypeptide sequence of any one of the Cas proteins selected from TABLE 2 (when applicable). For example, the position of the first amino acid residue and the second amino acid residue (e.g., when aligned to the polypeptide sequence of SEQ ID NO: 1) can be separated by at least or up to about 1, at least or up to about 2, at least or up to about 3, at least or up to about 4, at least or up to about 5, at least or up to about 10, at least or up to about 15, at least or up to about 20, at least or up to about 30, at least or up to about 40, at least or up to about 50, at least or up to about 60, at least or up to about 70, at least or up to about 80, at least or up to about 90, at least or up to about 100, at least or up to about 120, at least or up to about 150, at least or up to about 200, at least or up to about 250, at least or up to about 300, at least or up to about 350, at least or up to about 400, at least or up to about 450, at least or up to about 500, at least or up to about 510, at least or up to about 520, or at least or up to about 525 amino acid residues.

In some embodiments, the deletion of the plurality of amino acid residues to generate the amino acid sequence of the engineered nuclease can comprise deletion of a plurality of consecutive amino acid residues, e.g., as compared to (i) the polypeptide sequence of SEQ ID NO: 1 or (ii) the polypeptide sequence of any one of the Cas proteins selected from TABLE 2 (when applicable). In some cases, the deletion of the plurality of consecutive amino acid residues can comprise deletion of at least or up to about 2 consecutive amino acid residues, at least or up to about 3 consecutive amino acid residues, at least or up to about 4 consecutive amino acid residues, at least or up to about 5 consecutive amino acid residues, at least or up to about 6 consecutive amino acid residues, at least or up to about 7 consecutive amino acid residues, at least or up to about 8 consecutive amino acid residues, at least or up to about 9 consecutive amino acid residues, at least or up to about 10 consecutive amino acid residues, at least or up to about 11 consecutive amino acid residues, at least or up to about 12 consecutive amino acid residues, at least or up to about 13 consecutive amino acid residues, at least or up to about 14 consecutive amino acid residues, at least or up to about 15 consecutive amino acid residues, at least or up to about 16 consecutive amino acid residues, at least or up to about 17 consecutive amino acid residues, at least or up to about 18 consecutive amino acid residues, at least or up to about 19 consecutive amino acid residues, at least or up to about 20 consecutive amino acid residues, at least or up to about 21 consecutive amino acid residues, at least or up to about 22 consecutive amino acid residues, at least or up to about 23 consecutive amino acid residues, at least or up to about 24 consecutive amino acid residues, at least or up to about 25 consecutive amino acid residues, at least or up to about 26 consecutive amino acid residues, at least or up to about 27 consecutive amino acid residues, at least or up to about 28 consecutive amino acid residues, at least or up to about 29 consecutive amino acid residues, at least or up to about 30 consecutive amino acid residues, at least or up to about 31 consecutive amino acid residues, at least or up to about 32 consecutive amino acid residues, at least or up to about 34 consecutive amino acid residues, at least or up to about 35 consecutive amino acid residues, at least or up to about 36 consecutive amino acid residues, at least or up to about 37 consecutive amino acid residues, at least or up to about 38 consecutive amino acid residues, at least or up to about 39 consecutive amino acid residues, at least or up to about 40 consecutive amino acid residues, at least or up to about 45 consecutive amino acid residues, at least or up to about 50 consecutive amino acid residues, at least or up to about 55 consecutive amino acid residues, at least or up to about 60 consecutive amino acid residues, at least or up to about 65 consecutive amino acid residues, at least or up to about 70 consecutive amino acid residues, at least or up to about 75 consecutive amino acid residues, at least or up to about 80 consecutive amino acid residues, at least or up to about 85 consecutive amino acid residues, at least or up to about 80 consecutive amino acid residues 90, at least or up to about 95 consecutive amino acid residues, or at least or up to about 100 consecutive amino acid residues, e.g., as compared to (i) the polypeptide sequence of SEQ ID NO: 1 or (ii) the polypeptide sequence of any one of the Cas proteins selected from TABLE 2 (when applicable).

For example, the deletion can comprise a single deletion of a plurality of consecutive amino acid residues. In another example, the deletion can comprise a first deletion of a first plurality of consecutive amino acid resides and a second deletion of a second plurality of consecutive amino acid resides, and the first plurality of consecutive amino acid resides and the second plurality of consecutive amino acid resides may not be consecutive (e.g., may not be adjacent to each other), when aligned to (i) the polypeptide sequence of SEQ ID NO: 1 or (ii) the polypeptide sequence of any one of the Cas proteins selected from TABLE 2 (when applicable).

In some embodiments, the amino acid sequence of the engineered nuclease as disclosed herein can comprise addition of one or more heterologous amino acid residues (e.g., one or more polypeptide sequences), as compared to (i) the polypeptide sequence of SEQ ID NO: 1 or (ii) the polypeptide sequence of any one of the Cas proteins selected from TABLE 2 (when applicable). The one or more heterologous amino acid residues may be at the position of the at least one deletion of the engineered nuclease. Alternatively or in addition to, the one or more heterologous amino acid residues may not be at the position of the at least one deletion of the engineered nuclease. For example, the one or more heterologous amino acid residues may be upstream and/or downstream of the position of the at least one deletion of the engineered nuclease.

In some embodiments, the one or more heterologous amino acid residues of the engineered nuclease may comprise a single amino acid residue. In some embodiments, the one or more heterologous amino acid residues of the engineered nuclease may comprise a plurality of amino acid residues, such as, at least or up to about 2 amino acid residues, at least or up to about 3 amino acid residues, at least or up to about 4 amino acid residues, at least or up to about 5 amino acid residues, at least or up to about 6 amino acid residues, at least or up to about 7 amino acid residues, at least or up to about 8 amino acid residues, at least or up to about 9 amino acid residues, at least or up to about 10 amino acid residues, at least or up to about 11 amino acid residues, at least or up to about 12 amino acid residues, at least or up to about 13 amino acid residues, at least or up to about 14 amino acid residues, at least or up to about 15 amino acid residues, at least or up to about 16 amino acid residues, at least or up to about 17 amino acid residues, at least or up to about 18 amino acid residues, at least or up to about 19 amino acid residues, at least or up to about 20 amino acid residues, at least or up to about 22 amino acid residues, at least or up to about 24 amino acid residues, at least or up to about 25 amino acid residues, at least or up to about 26 amino acid residues, at least or up to about 28 amino acid residues, at least or up to about 30 amino acid residues, at least or up to about 32 amino acid residues, at least or up to about 34 amino acid residues, at least or up to about 35 amino acid residues, at least or up to about 36 amino acid residues, at least or up to about 38 amino acid residues, at least or up to about 40 amino acid residues, at least or up to about 42 amino acid residues, at least or up to about 44 amino acid residues, at least or up to about 45 amino acid residues, at least or up to about 46 amino acid residues, at least or up to about 48 amino acid residues, at least or up to about 50 amino acid residues, at least or up to about 52 amino acid residues, at least or up to about 54 amino acid residues, at least or up to about 55 amino acid residues, at least or up to about 56 amino acid residues, at least or up to about 58 amino acid residues, at least or up to about 60 amino acid residues, at least or up to about 62 amino acid residues, at least or up to about 64 amino acid residues, at least or up to about 65 amino acid residues, at least or up to about 66 amino acid residues, at least or up to about 68 amino acid residues, at least or up to about 70 amino acid residues, at least or up to about 72 amino acid residues, at least or up to about 74 amino acid residues, at least or up to about 75 amino acid residues, at least or up to about 76 amino acid residues, at least or up to about 78 amino acid residues, at least or up to about 80 amino acid residues, at least or up to about 82 amino acid residues, at least or up to about 84 amino acid residues, at least or up to about 85 amino acid residues, at least or up to about 86 amino acid residues, at least or up to about 88 amino acid residues, at least or up to about 90 amino acid residues, at least or up to about 92 amino acid residues, at least or up to about 94 amino acid residues, at least or up to about 95 amino acid residues, at least or up to about 96 amino acid residues, at least or up to about 98 amino acid residues, or at least or up to about 100 amino acid residues.

In some embodiments, the plurality of amino acid residues of the one or more heterologous amino acid residues may be consecutive amino acid residues. In some embodiments, the plurality of amino acid residues of the one or more heterologous amino acid residues may comprise a plurality of non-consecutive amino acid residues.

In some embodiments, the one or more heterologous amino acid residues of the engineered nuclease may comprise a heterologous polypeptide sequence (e.g., heterologous to (i) the polypeptide sequence of SEQ ID NO: 1 or (ii) the polypeptide sequence of any one of the Cas proteins selected from TABLE 2 (when applicable)). The heterologous polypeptide sequence can exhibit a net positive charge (e.g., net +1 charge, net +2 charge, net +3 charge, net +4 charge, net +5 charge, etc., e.g., as measured in a buffer at about pH 7.4). The heterologous polypeptide sequence can exhibit a net negative charge (e.g., net -1 charge, net -2 charge, net -3 charge, net -4 charge, net -5 charge, etc., e.g., as measured in a buffer at about pH 7.4). The heterologous polypeptide sequence can exhibit a neutral charge, e.g., as measured in a buffer at about pH 7.4. In some cases, the heterologous polypeptide sequence can comprise an amino acid sequence that is at least about 50%, at least about 60%, at least about 65%, at least about 70%, at least about 75%, at least about 80%, at least about 85%, at least about 90%, at least about 95%, at least about 99%, or about 100% identical to the polypeptide sequence of SEQ ID NO: 11.
**SEQ ID NO: 11 (heterologous polypeptide)**
1 ERRKQAGGTG E

In some embodiments, the amino acid sequence of the engineered nuclease as disclosed herein can comprise deletion of one or more amino acid residues from (i) the amino acid resides 2-100 as compared to the polypeptide sequence of SEQ ID NO: 1 and/or (ii) the amino acid resides 430-529 as compared to the polypeptide sequence of SEQ ID NO: 1, and the amino acid sequence can further comprise deletion of one or more additional amino acid residues (e.g., at least 1, at least 2, at least 3, at least 4, at least 5, at least 6, at least 7, at least 8, at least 9, at least 10, at least 15, at least 20, or more additional amino acid residues) from the amino acid residues 101-429 as disclosed herein, as compared to the polypeptide sequence of SEQ ID NO: 1.

In some embodiments, the amino acid sequence of the engineered nuclease as disclosed herein can comprise deletion of one or more amino acid residues from (i) the amino acid resides 2-100 as compared to the polypeptide sequence of any one of the Cas proteins selected from TABLE 2 and/or (ii) the last 100 C-terminal amino acid resides as compared to the polypeptide sequence of any one of the Cas proteins selected from TABLE 2, and the amino acid sequence can further comprise deletion of one or more additional amino acid residues (e.g., at least 1, at least 2, at least 3, at least 4, at least 5, at least 6, at least 7, at least 8, at least 9, at least 10, at least 15, at least 20, or more additional amino acid residues) from the amino acid residues there between, as compared to the polypeptide sequence of any one of the Cas proteins selected from TABLE 2.

In some embodiments, the engineered polypeptide as disclosed herein can comprise one or more heterologous amino acid residues (e.g., other than mutations) as compared to the native Un1Cas12f1 nuclease as provided in SEQ ID NO: 1. The presence of the one or more heterologous amino acid residues can enhance, for example, activity, stability, expression, binding to the respective guide nucleic acid molecule, etc. of the engineered polypeptide.

In some embodiments, of the engineered polypeptide as disclosed herein (e.g., a deactivated Cas nuclease variant) can comprises an amino acid sequence that is at least or up to about 50%, at least or up to about 55%, at least or up to about 60%, at least or up to about 65%, at least or up to about 70%, at least or up to about 75%, at least or up to about 80%, at least or up to about 85%, at least or up to about 90%, at least or up to about 91%, at least or up to about 92%, at least or up to about 93%, at least or up to about 94%, at least or up to about 95%, at least or up to about 96%, at least or up to about 97%, at least or up to about 98%, at least or up to about 99%, or about 100% identical to the polypeptide sequence of SEQ ID NO: 12.
**SEQ ID NO: 12 (example engineered nuclease polypeptide)**

1 MAKNTITKTL KLRIVRPYNS AEVEKIVADE KERRKQAGGT GELDDKFYQK
51 LRGQFPDAVF WQEISEIFRQ LQKQAAEIYN QSLIELYYEI FIKGKGIANA
101 SSVEHYLSRV CYRRAAELFK NAAIASGLRS KIKSNFRLKE LKNMKSGLPT
151 TKSDNFPIPL VKQKGGQYTG FEISNHNSDF IIKIPFGRWQ VKKEIDKYRP
201 WEKFDFEQVQ KSPKPISLLL STQRRKRNKG WSKDEGTEAE IKKVMNGDYQ
251 TSYIEVKRGS KICEKSAWML NLSIDVPKID KGVDPSIIGG IAVGVRSPLV
301 CAINNAFSRY SISDNDLFHF NKKMFARRRI LLKKNRHKRA GHGAKNKLKP
351 ITILTEKSER FRKKLIERWA CEIADFFIKN KVGTVQMENL ESMKRKEDSY
401 FNIRLRGFWP YAEMQNKIEF KLKQYGIEIR KVAPNNTSKT CSKCGHLNNY
451 FNFEYRKKNK FPHFKCEKCN FKENAAYNAA LNISNPKLKS TKERP

In some embodiments, the engineered polypeptide as disclosed herein can comprise an amino acid sequence that is at least or up to about 50%, at least or up to about 55%, at least or up to about 60%, at least or up to about 65%, at least or up to about 70%, at least or up to about 75%, at least or up to about 80%, at least or up to about 85%, at least or up to about 90%, at least or up to about 91%, at least or up to about 92%, at least or up to about 93%, at least or up to about 94%, at least or up to about 95%, at least or up to about 96%, at least or up to about 97%, at least or up to about 98%, at least or up to about 99%, or about 100% identical to the polypeptide sequence of an engineered nuclease variant from TABLE 3B (e.g., one or more of SEQ ID NOs: 20-27), TABLE 4B (e.g., one or more of SEQ ID NOs: 28-111), and/or TABLE 5C (e.g., one or more of SEQ ID NOs: 112-201).

In some embodiments, the engineered polypeptide as disclosed herein may not be identical to any one of: SEQ ID NO: 1 and any Cas protein from TABLE 2 (SEQ ID NOs: 2, 3, and 13-19).

In some embodiments, the engineered polypeptide can comprise at least one amino acid modification as compared to the polypeptide sequence of SEQ ID NO: 1 or SEQ ID NO: 10 selected from the group consisting of A21Q, V23I, N32E, D29E, N33R, E35K, K36Q, I37A, A38G, E40D, K73G, A74T, R75G, K76E, Q83K, G87K, E151A, A340S, H353K, A374K, I387E, N423D, K473Q, T474L, T474R, H497K, L515R, N519T, K521D, K521N, L522I, and any deletion of one or more amino acid residues relative to the polypeptide sequence of SEQ ID NO: 1 or SEQ ID NO: 10 as disclosed herein (e.g., at least one deletion from the amino acid residues 400-529 of SEQ ID NO: 1 or SEQ ID NO: 10). The one or more members can comprise at least or up to about 1, at least or up to about 2, at least or up to about 3, at least or up to about 4, at least or up to about 5, at least or up to about 6, at least or up to about 7, at least or up to about 8, at least or up to about 9, at least or up to about 10, at least or up to about 11, at least or up to about 12, at least or up to about 13, at least or up to about 14, at least or up to about 15, at least or up to about 20 amino acid, at least or up to about 25 amino acid, or at least or up to about 30 amino acid modifications selected from the group consisting of A21Q, V23I, N32E, D29E, N33R, E35K, K36Q, I37A, A38G, E40D, K73G, A74T, R75G, K76E, Q83K, G87K, E151A, A340S, H353K, A374K, I387E, N423D, K473Q, T474L, T474R, H497K, L515R, N519T, K521D, K521N, L522I, as compared to the polypeptide sequence of SEQ ID NO: 1 or SEQ ID NO: 10. In some cases, the at least one amino acid modification can comprise at least one set of modifications selected from TABLE 5A. In some cases, the at least one amino acid modification can comprise at least one combination of modifications selected from TABLE 5B. For example, the at least one combination of modifications selected from TABLE 5B may not be cA2.55 or cA2.84.

In some embodiments, the engineered polypeptide as disclosed herein can comprise at least one amino acid modification as compared to the polypeptide sequence of SEQ ID NO: 1 or SEQ ID NO: 10. The at least one amino acid modification can comprise one or more members (e.g., at least 1, 2, 3, 4, 5, 6, 7, 8, 9, 10, 11, 12, 13, 14, 15, or more members) selected from the group consisting of A21Q, V23I, D29E, N33R, E40D, Q83K, G87K, E151A, A340S, H353K, A374K, I387E, N423D, K473Q, T474L, T474R, H497K, L515R, N519T, K521D, K521N, and L522I.

In some embodiments, the engineered polypeptide as disclosed herein can comprise at least one amino acid modification as compared to the polypeptide sequence of SEQ ID NO: 1 or SEQ ID NO: 10. The at least one amino acid modification can comprise one or more members (e.g., at least 1, 2, 3, 4, 5, 6, 7, 8, 9, or 10 members) selected from the group consisting of N32E, N33R, E35K, K36Q, I37A, A38G, K73G, A74T, R75G, and K76E.

In some embodiments, the engineered nuclease variant of the engineered polypeptide as disclosed herein can be a chimeric polypeptide comprising different polynucleotide sequence domains derived from different Cas proteins. The chimeric polypeptide can comprise a first polypeptide sequence comprising at least 3 contiguous amino acid residues in common with a first Cas protein and a second polypeptide sequence comprising at least 3 contiguous amino acid residues in common with a second Cas protein, which second Cas protein is different from the first Cas protein. The first Cas protein and the second Cas protein can be different naturally occurring Cas proteins. The first Cas protein and the second Cas protein can have a size (or amino acid sequence length) that is different from each other by no more than 500, no more than 400, no more than 350, no more than 300, no more than 250, no more than 200, no more than 150, no more than 140, no more than 130, no more than 120, no more than 110, no more than 100, no more than 90, no more than 80, no more than 70, no more than 60, no more than 50, no more than 40, no more than 30, or no more than 20 amino acid residues.

In some cases, a length of the first polypeptide sequence can be substantially the same as a length of the second polypeptide sequence. Alternatively, the length of the first polypeptide sequence can be different from the length of the second polypeptide sequence. The length of the second polypeptide sequence can be less than or equal to about 90%, less than or equal to about 85%, less than or equal to about 80%, less than or equal to about 75%, less than or equal to about 70%, less than or equal to about 65%, less than or equal to about 60%, less than or equal to about 55%, less than or equal to about 50%, less than or equal to about 45%, less than or equal to about 40%, less than or equal to about 35%, less than or equal to about 30%, less than or equal to about 25%, less than or equal to about 20%, less than or equal to about 18%, less than or equal to about 16%, less than or equal to about 15%, less than or equal to about 14%, less than or equal to about 12%, less than or equal to about 10%, less than or equal to about 9%, less than or equal to about 8%, less than or equal to about 7%, less than or equal to about 6%, less than or equal to about 5%, less than or equal to about 4%, less than about 3%, less than or equal to about 2%, or less than or equal to about 1% of the length of the first polypeptide sequence. The length of the second polypeptide sequence can be at least or up to about 1%, at least or up to about 2%, at least or up to about 3%, at least or up to about 4%, at least or up to about 5%, at least or up to about 6%, at least or up to about 7%, at least or up to about 8%, at least or up to about 9%, at least or up to about 10%, at least or up to about 12%, at least or up to about 14%, at least or up to about 15%, at least or up to about 16%, at least or up to about 18%, at least or up to about 20%, at least or up to about 25%, at least or up to about 30%, at least or up to about 35%, at least or up to about 40%, at least or up to about 45%, at least or up to about 50%, at least or up to about 55%, at least or up to about 60%, at least or up to about 70%, at least or up to about 80%, at least or up to about 90%, or at least or up to about 95% of the length of the first polypeptide sequence.

In some cases, the first Cas protein and the second Cas protein can be two different members selected from: SEQ ID NO: 1 and any Cas protein from TABLE 2 (SEQ ID NOs: 2, 3, and 13-19). The first Cas protein or the second Cas protein can comprise an amino acid sequence that is at least or up to about 5%, at least or up to about 10%, at least or up to about 15%, at least or up to about 20%, at least or up to about 30%, at least or up to about 40%, at least or up to about 50%, at least or up to about 60%, at least or up to about 70%, at least or up to about 75%, at least or up to about 80%, at least or up to about 85%, at least or up to about 90%, at least or up to about 92%, at least or up to about 95%, at least or up to about 96%, at least or up to about 97%, at least or up to about 98%, at least or up to about 99%, or substantially about 100% identical to the polypeptide sequence of a member selected from: SEQ ID NO: 1 and any Cas protein from TABLE 2 (SEQ ID NOs: 2, 3, and 13-19). For example, such member can be SEQ ID NO: 1 or SEQ ID NO: 2.

In some cases, the first polypeptide of the chimeric polypeptide of the engineered nuclease variant can comprise at least or up to about 4 continuous amino acid residues, at least or up to about 5 contiguous amino acid residues, at least or up to about 6 continuous amino acid residues, at least or up to about 7 continuous amino acid residues, at least or up to about 8 continuous amino acid residues, at least or up to about 9 continuous amino acid residues, at least or up to about 10 contiguous amino acid residues, at least or up to about 11 continuous amino acid residues, at least or up to about 12 continuous amino acid residues, at least or up to about 13 continuous amino acid residues, at least or up to about 14 continuous amino acid residues, at least or up to about 15 continuous amino acid residues, at least or up to about 18 continuous amino acid residues, at least or up to about 20 contiguous amino acid residues, at least or up to about 30 contiguous amino acid residues, at least or up to about 40 contiguous amino acid residues, or at least or up to about 50 contiguous amino acid residues in common with the first Cas protein.

In some cases, the second polypeptide of the chimeric polypeptide of the engineered nuclease variant can comprise at least or up to about 4 continuous amino acid residues, at least or up to about 5 contiguous amino acid residues, at least or up to about 6 continuous amino acid residues, at least or up to about 7 continuous amino acid residues, at least or up to about 8 continuous amino acid residues, at least or up to about 9 continuous amino acid residues, at least or up to about 10 contiguous amino acid residues, at least or up to about 11 continuous amino acid residues, at least or up to about 12 continuous amino acid residues, at least or up to about 13 continuous amino acid residues, at least or up to about 14 continuous amino acid residues, at least or up to about 15 continuous amino acid residues, at least or up to about 18 continuous amino acid residues, at least or up to about 20 contiguous amino acid residues, at least or up to about 30 contiguous amino acid residues, at least or up to about 40 contiguous amino acid residues, or at least or up to about 50 contiguous amino acid residues in common with the second Cas protein.

In some cases, in the second polypeptide of the chimeric polypeptide of the engineered nuclease variant, a length of the first polypeptide can be greater than a length of the second polypeptide by at least or up to about 1 amino acid, at least or up to about 2 amino acids, at least or up to about 5 amino acids, at least or up to about 10 amino acids, at least or up to about 15 amino acids, at least or up to about 20 amino acids, at least or up to about 25 amino acids, at least or up to about 30 amino acids, at least or up to about 35 amino acids, at least or up to about 40 amino acids, at least or up to about 45 amino acids, at least or up to about 50 amino acids, at least or up to about 55 amino acids, at least or up to about 60 amino acids, at least or up to about 70 amino acids, at least or up to about 80 amino acids, at least or up to about 90 amino acids, at least or up to about 100 amino acids, at least or up to about 110 amino acids, at least or up to about 120 amino acids, at least or up to about 130 amino acids, at least or up to about 140 amino acids, at least or up to about 150 amino acids, at least or up to about 200 amino acids, at least or up to about 250 amino acids, at least or up to about 300 amino acids, at least or up to about 350 amino acids, at least or up to about 400 amino acids, at least or up to about 450 amino acids, or at least or up to about 500 amino acids.

In some cases, the first polypeptide can be derived from the N-terminal 50%, the N-terminal 45%, the N-terminal 40%, the N-terminal 35%, the N-terminal 30%, the N-terminal 25%, the N-terminal 20%, the N-terminal 15%, the N-terminal 10%, or the N-terminal 5% of the first Cas protein. Alternatively or in addition to, the first polypeptide can be derived from the C-terminal 50%, the C-terminal 45%, the C-terminal 40%, the C-terminal 35%, the C-terminal 30%, the C-terminal 25%, the C-terminal 20%, the C-terminal 15%, the C-terminal 10%, or the C-terminal 5% of the first Cas protein. In some cases, the second polypeptide can be derived from the N-terminal 50%, the N-terminal 45%, the N-terminal 40%, the N-terminal 35%, the N-terminal 30%, the N-terminal 25%, the N-terminal 20%, the N-terminal 15%, the N-terminal 10%, or the N-terminal 5% of the second Cas protein. Alternatively or in addition to, the second polypeptide can be derived from the C-terminal 50%, the C-terminal 45%, the C-terminal 40%, the C-terminal 35%, the C-terminal 30%, the C-terminal 25%, the C-terminal 20%, the C-terminal 15%, the C-terminal 10%, or the C-terminal 5% of the second Cas protein.

In some cases, the first polypeptide can be derived from the first 5 amino acid residues, the first 10 amino acid residues, the first 15 amino acid residues, the first 20 amino acid residues, the first 30 amino acid residues, the first 40 amino acid residues, the first 50 amino acid residues, the first 60 amino acid residues, the first 70 amino acid residues, the first 80 amino acid residues, the first 90 amino acid residues, the first 100 amino acid residues, the first 150 amino acid residues, the first 200 amino acid residues, the first 250 amino acid residues, or the first 300 amino acid residues from the N-terminus of the first Cas protein. Alternatively or in addition to, the first polypeptide can be derived from the first 5 amino acid residues, the first 10 amino acid residues, the first 15 amino acid residues, the first 20 amino acid residues, the first 30 amino acid residues, the first 40 amino acid residues, the first 50 amino acid residues, the first 60 amino acid residues, the first 70 amino acid residues, the first 80 amino acid residues, the first 90 amino acid residues, the first 100 amino acid residues, the first 150 amino acid residues, the first 200 amino acid residues, the first 250 amino acid residues, or the first 300 amino acid residues from the C-terminus of the first Cas protein.

In some cases, the second polypeptide can be derived from the first 5 amino acid residues, the first 10 amino acid residues, the first 15 amino acid residues, the first 20 amino acid residues, the first 30 amino acid residues, the first 40 amino acid residues, the first 50 amino acid residues, the first 60 amino acid residues, the first 70 amino acid residues, the first 80 amino acid residues, the first 90 amino acid residues, the first 100 amino acid residues, the first 150 amino acid residues, the first 200 amino acid residues, the first 250 amino acid residues, or the first 300 amino acid residues from the N-terminus of the second Cas protein. Alternatively or in addition to, the second polypeptide can be derived from the first 5 amino acid residues, the first 10 amino acid residues, the first 15 amino acid residues, the first 20 amino acid residues, the first 30 amino acid residues, the first 40 amino acid residues, the first 50 amino acid residues, the first 60 amino acid residues, the first 70 amino acid residues, the first 80 amino acid residues, the first 90 amino acid residues, the first 100 amino acid residues, the first 150 amino acid residues, the first 200 amino acid residues, the first 250 amino acid residues, or the first 300 amino acid residues from the C-terminus of the second Cas protein.

In some cases, the engineered nuclease variant of the engineered polypeptide as disclosed herein can comprise a third polypeptide sequence comprising at least 3 contiguous amino acid residues (or more as disclosed herein) in common with the first Cas protein. In such cases, the first polypeptide sequence and the third polypeptide sequence may or may not be contiguous to each other within the chimeric polypeptide. Alternatively or in addition to, the third polypeptide sequence can comprise at least 3 contiguous amino acid residues (or more as disclosed herein) in common with the second Cas protein. The first polypeptide sequence and the third polypeptide sequence may not be contiguous to each other in the chimeric polypeptide. Alternatively, the first polypeptide sequence and the third polypeptide sequence may be contiguous to each other in the chimeric polypeptide. The second polypeptide sequence and the third polypeptide sequence may not be contiguous to each other in the chimeric polypeptide. Alternatively, the second polypeptide sequence and the third polypeptide sequence may be contiguous to each other in the chimeric polypeptide. Yet in another alternative or additionally, the third polypeptide sequence can comprise at least 3 contiguous amino acid residues in common with a third Cas protein that is different from the first Cas protein and the second Cas protein. For example, the first Cas protein, the second Cas protein, and the third Cas protein can be three different members selected from SEQ ID NO: 1 and any Cas protein selected from TABLE 2.

In some cases, the third polypeptide of the chimeric polypeptide of the engineered nuclease variant can comprise at least or up to about 4 continuous amino acid residues, at least or up to about 5 contiguous amino acid residues, at least or up to about 6 continuous amino acid residues, at least or up to about 7 continuous amino acid residues, at least or up to about 8 continuous amino acid residues, at least or up to about 9 continuous amino acid residues, at least or up to about 10 contiguous amino acid residues, at least or up to about 11 continuous amino acid residues, at least or up to about 12 continuous amino acid residues, at least or up to about 13 continuous amino acid residues, at least or up to about 14 continuous amino acid residues, at least or up to about 15 continuous amino acid residues, at least or up to about 18 continuous amino acid residues, at least or up to about 20 contiguous amino acid residues, at least or up to about 30 contiguous amino acid residues, at least or up to about 40 contiguous amino acid residues, or at least or up to about 50 contiguous amino acid residues in common with the third Cas protein.

In some cases, the third polypeptide can be derived from the first 5 amino acid residues, the first 10 amino acid residues, the first 15 amino acid residues, the first 20 amino acid residues, the first 30 amino acid residues, the first 40 amino acid residues, the first 50 amino acid residues, the first 60 amino acid residues, the first 70 amino acid residues, the first 80 amino acid residues, the first 90 amino acid residues, the first 100 amino acid residues, the first 150 amino acid residues, the first 200 amino acid residues, the first 250 amino acid residues, or the first 300 amino acid residues from the N-terminus of the third Cas protein. Alternatively or in addition to, the third polypeptide can be derived from the first 5 amino acid residues, the first 10 amino acid residues, the first 15 amino acid residues, the first 20 amino acid residues, the first 30 amino acid residues, the first 40 amino acid residues, the first 50 amino acid residues, the first 60 amino acid residues, the first 70 amino acid residues, the first 80 amino acid residues, the first 90 amino acid residues, the first 100 amino acid residues, the first 150 amino acid residues, the first 200 amino acid residues, the first 250 amino acid residues, or the first 300 amino acid residues from the C-terminus of the third Cas protein.

In some cases, within the chimeric polypeptide of the engineered nuclease variant as disclosed herein, the first polypeptide sequence, the second polypeptide sequence, and the third polypeptide sequence can be arranged in any of the following structures, from N-terminus to C-terminus of the chimeric polypeptide: CP1-CP2-CPx (I), CP1-CPx-CP2 (II), CP2-CP1-CPx (III), CP2-CPx-CP1 (IV), CPx-CP1-CP2 (V), or CPx-CP2-CP1 (VI), in which "-" can be either an amino acid linker (e.g., comprising one or more amino acid sequences) or a direct covalent bond. The amino acid linker as disclosed herein can comprise a single amino acid, at least or up to about 2 amino acids, at least or up to about 3 amino acids, at least or up to about 4 amino acids, at least or up to about 5 amino acids, at least or up to about 8 amino acids, at least or up to about 10 amino acids, at least or up to about 12 amino acids, at least or up to about 15 amino acids, at least or up to about 16 amino acids, or at least or up to about 20 amino acids. The amino acid linker can comprise at least one Glycine, at least one Serine, or at least one Glycine-Serine dipeptide.

In some embodiments, the amino acid sequence of the engineered nuclease as disclosed herein (e.g., the chimeric polypeptide as disclosed herein) can have a length of at most 528 amino acids, at most 527 amino acids, at most 526 amino acids, at most 525 amino acids, at most 524 amino acids, at most 523 amino acids, at most 522 amino acids, at most 521 amino acids, at most 520 amino acids, at most 519 amino acids, at most 518 amino acids, at most 517 amino acids, at most 516 amino acids, at most 515 amino acids, at most 514 amino acids, at most 513 amino acids, at most 512 amino acids, at most 511 amino acids, at most 510 amino acids, at most 509 amino acids, at most 508 amino acids, at most 507 amino acids, at most 506 amino acids, at most 505 amino acids, at most 504 amino acids, at most 503 amino acids, at most 502 amino acids, at most 501 amino acids, at most about 500 amino acids, at most about 495 amino acids, at most about 490 amino acids, at most about 485 amino acids, at most about 480 amino acids, at most about 475 amino acids, at most about 470 amino acids, at most about 465 amino acids, at most about 460 amino acids, at most about 455 amino acids, at most about 450 amino acids, at most about 445 amino acids, at most about 440 amino acids, at most about 435 amino acids, at most about 430 amino acids, at most about 425 amino acids, at most about 420 amino acids, at most about 415 amino acids, at most about 410 amino acids, at most about 405 amino acids, at most about 400 amino acids, at most about 395 amino acids, at most about 390 amino acids, at most about 385 amino acids, at most about 380 amino acids, at most about 375 amino acids, at most about 370 amino acids, at most about 365 amino acids, at most about 360 amino acids, at most about 355 amino acids, at most about 350 amino acids, at most about 345 amino acids, at most about 340 amino acids, at most about 335 amino acids, at most about 330 amino acids, at most about 325 amino acids, at most about 320 amino acids, at most about 315 amino acids, at most about 310 amino acids, at most about 305 amino acids, or at most about 300 amino acids.

In some embodiments, the engineered nuclease comprising the amino acid sequence as disclosed herein can have a length of at most about 1000 amino acids, at most about 950 amino acids, at most about 900 amino acids, at most about 850 amino acids, at most about 800 amino acids, at most about 750 amino acids, at most about 700 amino acids, at most about 650 amino acids, at most about 640 amino acids, at most about 630 amino acids, at most about 620 amino acids, at most about 610 amino acids, at most about 600 amino acids, at most about 590 amino acids, at most about 580 amino acids, at most about 570 amino acids, at most about 560 amino acids, at most about 550 amino acids, at most about 540 amino acids, at most about 530 amino acids, at most about 520 amino acids, at most about 510 amino acids, at most about 500 amino acids, at most about 490 amino acids, at most about 480 amino acids, at most about 470 amino acids, at most about 460 amino acids, at most about 450 amino acids, at most about 440 amino acids, at most about 430 amino acids, at most about 420 amino acids, at most about 410 amino acids, at most about 400 amino acids, at most about 350 amino acids, or at most about 300 amino acids.

In some embodiments, at least a portion of the engineered nuclease variant as disclosed herein may be derived from (e.g., via engineering of) a naturally occurring Cas protein (e.g., the first Cas protein, the second Cas protein, or the third Cas protein as described herein). In some cases, the naturally occurring Cas protein can have a length of at most about 800 amino acids, at most about 750 amino acids, at most about 700 amino acids, at most about 650 amino acids, at most about 600 amino acids, at most about 550 amino acids, at most about 540 amino acids, at most about 530 amino acids, at most about 510 amino acids, at most about 500 amino acids, at most about 490 amino acids, at most about 480 amino acids, at most about 470 amino acids, at most about 460 amino acids, at most about 450 amino acids, or at most about 400 amino acids. The naturally occurring Cas protein, for example, can be a member from SEQ ID NO: 1 and any Cas protein selected from TABLE 2.

In some embodiments, the first polypeptide sequence, the second polypeptide sequence, and/or the third polypeptide sequence of the chimeric polypeptide of the engineered nuclease variant as disclosed herein may not be derived from Cas12a. In some embodiments, the chimeric polypeptide of the engineered nuclease variant may not be derived from Cas12a. In some embodiments, the chimeric polypeptide of the engineered nuclease variant may be entirely derived from one or more Cas12f type orthologous (e.g., SEQ ID NO:1 or a Cas protein selected from TABLE 2).

In some embodiments, the engineered nuclease comprising the amino acid sequence as disclosed herein can be is mutated and/or modified to yield a nuclease deficient protein or a protein with decreased nuclease activity relative to a wild-type Cas protein. A nuclease deficient protein can retain the ability to bind a target gene (e.g., DNA), but may lack or have reduced nucleic acid cleavage activity. In some embodiments, the engineered nuclease comprising the amino acid sequence as disclosed herein can exhibit reduced nuclease activity (e.g., nuclease deficient or nuclease null) as compared to the Cas nuclease encoded by SEQ ID NO: 1 or a Cas protein selected from TABLE 2. The reduced nuclease activity can be at most about 95%, at most about 90%, at most about 80%, at most about 70%, at most about 60%, at most about 50%, at most about 40%, at most about 30%, at most about 20%, at most about 10%, at most about 5%, at most about 1%, at most about 0.5%, at most about 0.1%, or less than that of the Cas nuclease encoded by SEQ ID NO: 1 or that of the Cas protein selected from TABLE 2. In some cases, the engineered nuclease can comprise a substitution at D326 and/or D510, as compared to the polypeptide sequence of SEQ ID NO: 1. For example, the D326 and/or the D510 substitution(s) can be alanine substitutions (e.g., D326A and/or D510A).

In some embodiments, the amino acid sequence of the engineered nuclease as disclosed herein can comprise one or more substitutions in the native amino acid sequence, where the positions of at least some of these substitutions follow one or more particular rules determined to have surprising advantages for the engineered nuclease. In some cases, the particular substitution rules have been selected for their ability to produce variants of the engineered nuclease, e.g., that can be capable of functioning within eukaryotic cells. According to these particular rules, all or some of the one or more substitutions in the native amino acid sequence are either (1) within or no more than 30 amino acids downstream of a (D/E/K/N)X(R/F)(E/K)N motif of the native amino acid sequence, (2) at or no more than 30 amino acids upstream or downstream of position 241 of the native amino acid sequence, (3) at or no more than 30 amino acids upstream or downstream of position 516 of the native amino acid sequence, and/or (4) having an electrically charged amino acid in the native amino acid sequence.

In some embodiments, the amino acid sequence of the engineered nuclease as disclosed herein can comprise one or more substitutions at amino acid positions within or no more than a threshold length (e.g., 30 amino acid residues) upstream and/or downstream of a (D/E/K/N)X(R/F)(E/K)N motif, as compared to (i) the polypeptide sequence of SEQ ID NO: 1 or (ii) the amino acid sequence of the engineered nuclease as disclosed herein without the one or more substitutions. In some cases, at least one of the one or more substitutions can be, for example, within or no more than 28 amino acids, 26 amino acids, 24 amino acids, 22 amino acids, 20 amino acids, 18 amino acids, 16 amino acids, 14 amino acids, 12 amino acids, or 10 amino acids of the motif. In some cases, at least one of the one or more substitutions can be to an R, A, S, or G amino acid residue. In some cases, the one or more substitutions can include substitutions are at one or more positions selected from the group consisting of D143, T147, E151, and K154 (e.g., D143R, T147R, E151R, and/or K154R). In some cases, the one or more substitutions can include substitutions are at one or more positions selected from the group consisting of N504, E507, N516, N519, E527, and E528 (e.g., N504R, E507R, N516R, N519R, E527R, and/or E528R). In some cases, the one or more substitutions can include substitutions are at one or more positions selected from the group consisting of K11, K73, D143, T147, E151, K154, E241, D318, K330, K457, E425, E462, N504, E507, N516, N519, E527, and E528 (e.g., K11R, K73R, D143R, T147R, E151R, K154R, E241R, D318R, K330R, E425N, K457R, E462R, N504R, E507R, N516R, N519R, E527R, and/or E528R).

In some embodiments, the amino acid sequence of the engineered nuclease comprising the one or more substitutions upstream and/or downstream of the (D/E/K/N)X(R/F)(E/K)N motif, as disclosed herein, can exhibit a cationic charge (e.g., a positive) that is greater than that of a control amino acid sequence of the engineered nuclease lacking the one or more substitutions, by at least or up to about 1 cationic charge, at least or up to about 2 cationic charges, at least or up to about 3 cationic charges, at least or up to about 4 cationic charges, at least or up to about 5 cationic charges, at least or up to about 6 cationic charges, at least or up to about 7 cationic charges, at least or up to about 8 cationic charges, at least or up to about 9 cationic charges, at least or up to about 10 cationic charges, at least or up to about 11 cationic charges, at least or up to about 12 cationic charges, at least or up to about 13 cationic charges, at least or up to about 14 cationic charges, at least or up to about 15 cationic charges, at least or up to about 16 cationic charges, at least or up to about 17 cationic charges, or at least or up to about 18 cationic charges.

Without wishing to be bound by theory, the amino acid sequence of the engineered nuclease comprising the one or more substitutions upstream and/or downstream of the (D/E/K/N)X(R/F)(E/K)N motif, as disclosed herein, can exhibit enhanced (e.g., higher) binding affinity to (i) a guide nucleic acid sequence (e.g., a guide RNA sequence) and/or (ii) a target polynucleotide sequence (e.g., a target gene in a cell, such as a target endogenous gene) of the Cas/guide nucleic complex, as compared to (A) a control amino acid sequence of the engineered nuclease lacking the one or more substitutions and/or (B) the CRISPR/Cas protein encoded by SEQ ID NO: 1, by at least or up to about 1%, at least or up to about 5%, at least or up to about 10%, at least or up to about 15%, at least or up to about 20%, at least or up to about 25%, at least or up to about 30%, at least or up to about 40%, at least or up to about 50%, at least or up to about 60%, at least or up to about 70%, at least or up to about 80%, at least or up to about 90%, at least or up to about 100%, at least or up to about 110%, at least or up to about 120%, at least or up to about 125%, at least or up to about 150%, at least or up to about 200%, at least or up to about 250%, at least or up to about 300%, at least or up to about 350%, at least or up to about 400%, at least or up to about 450%, or at least or up to about 500%, as ascertained by surface plasmon resonance (SPR) or isothermal titration calorimetry (IRC) assays.

Without wishing to be bound by theory, the amino acid sequence of the engineered nuclease comprising the one or more substitutions upstream and/or downstream of the (D/E/K/N)X(R/F)(E/K)N motif, as disclosed herein, can exhibit enhanced (e.g., higher) binding affinity to (i) a guide nucleic acid sequence (e.g., a guide RNA sequence) and/or (ii) a target polynucleotide sequence (e.g., a target gene in a cell, such as a target endogenous gene) of the Cas/guide nucleic complex, as compared to (A) a control amino acid sequence of the engineered nuclease lacking the one or more substitutions and/or (B) the CRISPR/Cas protein encoded by SEQ ID NO: 1, by at least or up to about 0.1-fold, at least or up to about 0.2-fold, at least or up to about 0.5-fold, at least or up to about 1-fold, at least or up to about 2-fold, at least or up to about 3-fold, at least or up to about 4-fold, at least or up to about 5-fold, at least or up to about 6-fold, at least or up to about 7-fold, at least or up to about 8-fold, at least or up to about 9-fold, at least or up to about 10-fold, at least or up to about 15-fold, at least or up to about 20-fold, at least or up to about 25-fold, at least or up to about 30-fold, at least or up to about 35-fold, or at least or up to about 40-fold, as ascertained by surface plasmon resonance (SPR) or isothermal titration calorimetry (IRC) assays.

In some embodiments, the present disclosure provides a system comprising the engineered nuclease as disclosed herein. In some embodiments, the system can comprise recombinantly expressed (or generated) form of the engineered nuclease. In some embodiments, the system can comprise one or more polynucleotides encoding at least the engineered nuclease. In some embodiments, the system can comprise a cell (or a population of cells) comprising (e.g., engineered to comprise, such as transfected or transduced to express) at least the engineered nuclease.

### Methods of use of the engineered nuclease

In some embodiments, the engineered nuclease as disclosed herein can be used to effect binding, cleaving, and/or editing a target polynucleotide sequence, e.g., to regulate expression and/or activity level of the target polynucleotide sequence of a polypeptide (e.g., a protein) encoded by the target polynucleotide sequence or operatively coupled to the target polynucleotide sequence. In some cases, a heterologous polypeptide comprising the engineered nuclease can be introduced to a cell (e.g., a mammalian cell) to effect binding, cleaving, and/or editing a target polynucleotide sequence of the cell (e.g., endogenous gene or heterologous gene of the cell). In some embodiments, the engineered nuclease as disclosed herein, or a protein comprising the engineered nuclease can be referred to as an actuator moiety.

In some embodiments, the engineered nuclease as disclosed herein can retain at least a portion (e.g., substantially all of) of the nuclease activity of the nuclease encoded by the polypeptide sequence of SEQ ID NO: 1 or that of the nuclease activity of a Cas protein selected from TABLE 2.

In some embodiments, is the engineered nuclease as disclosed herein can be nuclease-deficient. In some embodiments, the engineered nuclease can be a nuclease-null DNA binding protein that does not induce transcriptional activation or repression of a target DNA sequence unless it is present in a complex with one or more heterologous gene effectors of the disclosure. In some embodiments, the engineered nuclease can be a nuclease-null DNA binding protein that can induce transcriptional activation or repression of a target DNA sequence (e.g., which can be altered or augmented by the presence of a heterologous gene effector as provided herein). The terms "gene effector" and "gene modulator" may be used interchangeably herein. The terms "gene effector polypeptide" and "gene modulator polypeptides" may be used interchangeably herein.

In some embodiments, the engineered nuclease as disclosed herein can be an RNA nuclease such as an engineered (e.g., programmable or targetable) RNA nuclease. In some embodiments, the engineered nuclease as disclosed herein can be a nuclease-null RNA binding protein that does not induce transcriptional activation or repression of a target RNA sequence unless it is present in a complex with one or more heterologous gene effectors of the disclosure. In some embodiments, the engineered nuclease as disclosed herein can be a nuclease-null RNA binding protein that can induce transcriptional activation or repression of a target RNA sequence (e.g., which can be altered or augmented by the presence of a heterologous gene effector as provided herein).

In some embodiments, the engineered nuclease can be a nucleic acid-guided targeting system. In some embodiments, the engineered nuclease can be a DNA-guided targeting system. In some embodiments, the engineered nuclease can be an RNA-guided targeting system. The nucleic acid-guided targeting system can comprise and utilize, for example, a guide nucleic acid sequence that facilitates specific binding of a CRISPR-Cas system (e.g., a nuclease deficient form thereof, such as dCas9 or dCas14) to a target gene (e.g., target endogenous gene) or target gene regulatory sequence. For example, the target gene may be any one of the genes listed in TABLE 1, and the target gene regulatory sequence may be operatively coupled to any one of the genes listed in TABLE 1. Binding specificity can be determined by use of a guide nucleic acid, such as a single guide RNA (sgRNA) or a part thereof. In some embodiments, the use of different sgRNAs allows the compositions and methods of the disclosure to be used with (e.g., targeted to) different target genes (e.g., target endogenous genes) or target gene regulatory sequences.

In some embodiments, the engineered nuclease can form a complex with a guide nucleic acid, such as a guide RNA or a part thereof. In some embodiments, the engineered nuclease can form a complex with a single guide nucleic acid, such as a single guide RNA (sgRNA). In some embodiments, the engineered nuclease can be a RNA-binding protein (RBP) optionally complexed with a guide nucleic acid, such as a guide RNA (e.g., sgRNA), which is able to form a complex with a Cas protein. In some embodiments, the engineered nuclease can be a nuclease-null DNA binding protein that can induce transcriptional activation or repression of a target DNA sequence. In some embodiments, the engineered nuclease can be a nuclease-null RNA binding protein derived from a RNA.

A guide nucleic acid used in compositions and methods of the disclosure can be, for example, at least 8, at least 9, at least 10, at least 11, at least 12, at least 13, at least 14, at least 15, at least 16, at least 17, at least 18, at least 19, at least 20, at least 21, at least 22, at least 23, at least 24, at least 25, at least 26, at least 27, at least 28, at least 29, at least 30, at least 31, at least 32, at least 33, at least 34, at least 35, at least 36, at least 37, at least 38, at least 39, or at least 40 nucleotides.

In some embodiments, a guide nucleic acid used in compositions and methods of the disclosure is at most at most 10, at most 11, at most 12, at most 13, at most 14, at most 15, at most 16, at most 17, at most 18, at most 19, at most 20, at most 21, at most 22, at most 23, at most 24, at most 25, at most 26, at most 27, at most 28, at most 29, at most 30, at most 31, at most 32, at most 33, at most 34, at most 35, at most 36, at most 37, at most 38, at most 39, or at most 40 nucleotides.

In some embodiments, a guide nucleic acid used in compositions and methods of the disclosure is between about 8 and about 40 nucleotides, between about 10 and about 40 nucleotides, between about 11 and about 40 nucleotides, between about 12 and about 40 nucleotides, between about 13 and about 40 nucleotides, between about 14 and about 40 nucleotides, between about 15 and about 40 nucleotides, between about 16 and about 40 nucleotides, between about 17 and about 40 nucleotides, between about 18 and about 40 nucleotides, between about 19 and about 40 nucleotides, between about 20 and about 40 nucleotides, between about 22 and about 40 nucleotides, between about 24 and about 40 nucleotides, between about 26 and about 40 nucleotides, between about 28 and about 40 nucleotides, between about 30 and about 40 nucleotides, between about 8 and about 30 nucleotides, between about 10 and about 30 nucleotides, between about 11 and about 30 nucleotides, between about 12 and about 30 nucleotides, between about 13 and about 30 nucleotides, between about 14 and about 30 nucleotides, between about 15 and about 30 nucleotides, between about 16 and about 30 nucleotides, between about 17 and about 30 nucleotides, between about 18 and about 30 nucleotides, between about 19 and about 30 nucleotides, between about 20 and about 30 nucleotides, between about 22 and about 30 nucleotides, between about 24 and about 30 nucleotides, between about 26 and about 30 nucleotides, between about 28 and about 30 nucleotides, between about 8 and about 25 nucleotides, between about 10 and about 25 nucleotides, between about 11 and about 25 nucleotides, between about 12 and about 25 nucleotides, between about 13 and about 25 nucleotides, between about 14 and about 25 nucleotides, between about 15 and about 25 nucleotides, between about 16 and about 25 nucleotides, between about 17 and about 25 nucleotides, between about 18 and about 25 nucleotides, between about 19 and about 25 nucleotides, between about 20 and about 25 nucleotides, between about 22 and about 25 nucleotides, between about 24 and about 25 nucleotides, between about 8 and about 20 nucleotides, between about 10 and about 20 nucleotides, between about 11 and about 20 nucleotides, between about 12 and about 20 nucleotides, between about 13 and about 20 nucleotides, between about 14 and about 20 nucleotides, between about 15 and about 20 nucleotides, between about 16 and about 20 nucleotides, between about 17 and about 20 nucleotides, between about 18 and about 20 nucleotides, between about 19 and about 20 nucleotides, between about 8 and about 18 nucleotides, between about 10 and about 18 nucleotides, between about 11 and about 18 nucleotides, between about 12 and about 18 nucleotides, between about 13 and about 18 nucleotides, between about 14 and about 18 nucleotides, between about 15 and about 18 nucleotides, between about 16 and about 18 nucleotides, between about 8 and about 16 nucleotides, between about 10 and about 16 nucleotides, between about 11 and about 16 nucleotides, between about 12 and about 16 nucleotides, between about 13 and about 16 nucleotides, between about 14 and about 16 nucleotides, or between about 15 and about 16 nucleotides. In some embodiments, a guide nucleic acid can be a guide RNA or a part thereof.

The engineered nuclease as disclosed herein can be modified to enhance regulation of gene expression by compositions and methods of the disclosure, e.g., as part of a complex disclosed herein. The engineered nuclease can be modified to increase or decrease nucleic acid binding affinity, nucleic acid binding specificity, enzymatic activity, and/or binding to other factors, such as heterodimerization or oligomerization domains and induce ligands. The engineered nuclease can also be modified to change any other activity or property of the protein, such as stability. For example, one or more nuclease domains of the engineered nuclease can be modified, deleted, or inactivated, or at least a portion of the engineered nuclease can be truncated to remove domains that are not essential for the desired function of the protein or complex. The engineered nuclease can be modified to modulate (e.g., enhance or reduce) the activity of the engineered nuclease for regulating gene expression by a complex of the disclosure that comprises a heterologous gene effector.

For example, the engineered nuclease can be coupled (e.g., fused, covalently coupled, or non-covalently coupled) to a heterologous gene effector (e.g., an epigenetic modification domain, a transcriptional activation domain, and/or a transcriptional repressor domain). The engineered nuclease can be coupled (e.g., fused, covalently coupled, or non-covalently coupled) to an oligomerization or dimerization domain as disclosed herein (e.g., a heterodimerization domain). The engineered nuclease can be coupled (e.g., fused, covalently coupled, or non-covalently coupled) to a heterologous polypeptide that provides increased or decreased stability. The engineered nuclease can be coupled (e.g., fused, covalently coupled, or non-covalently coupled) to a sequence that can facilitate degradation of the engineered nuclease or a complex containing the engineered nuclease. The engineered nuclease can be coupled (e.g., fused, covalently coupled, or non-covalently coupled) to a gene editing moiety (e.g., heterologous protein, or domain or functional fragment thereof), that edits, mutates, or modifies (either directly or indirectly) a target polynucleotide sequence.

The engineered nuclease can be coupled (e.g., fused, covalently coupled, or non-covalently coupled) to any suitable number of partners, for example, at least one, at least two, at least three, at least four, or at least five, at least six, at least seven, or at least 8 partners. In some embodiments, the engineered nuclease of the disclosure is coupled (e.g., fused, covalently coupled, or non-covalently coupled) to at most two, at most three, at most four, at most five, at most six, at most seven, at most eight, or at most ten partners. In some embodiments, the engineered nuclease of the disclosure is coupled (e.g., fused, covalently coupled, or non-covalently coupled) to 1 - 5, 1 - 4, 1 - 3, 1 - 2, 2 - 5, 2 - 4, 2 - 3, 3 - 5, 3 - 4, or 4 - 5 partners. In some embodiments, the engineered nuclease of the disclosure is coupled (e.g., fused, covalently coupled, or non-covalently coupled) to one partner. In some embodiments, the engineered nuclease of the disclosure is coupled (e.g., fused, covalently coupled, or non-covalently coupled) to two partners. In some embodiments, the engineered nuclease of the disclosure is coupled (e.g., fused, covalently coupled, or non-covalently coupled) to three partners. In some embodiments, the engineered nuclease of the disclosure is coupled (e.g., fused, covalently coupled, or non-covalently coupled) to four partners. In some embodiments, the engineered nuclease of the disclosure is coupled (e.g., fused, covalently coupled, or non-covalently coupled) to five partners. In some embodiments, the engineered nuclease of the disclosure is coupled (e.g., fused, covalently coupled, or non-covalently coupled) to six partners.

The engineered nuclease as disclosed herein can be a fusion protein, e.g., a fusion comprising the engineered nuclease and one or more of the partners as disclosed herein. The fused domain or heterologous polypeptide can be located at the N-terminus, the C-terminus, or internally within the engineered nuclease.

A partner of the engineered nuclease (e.g., covalently or non-covalently coupled to a nuclease deficient or null variant of the engineered nuclease as disclosed herein) can be a transcriptional effector (e.g., a transcriptional activator or a transcriptional repressor). The transcriptional effector can be heterologous to the cell as provided herein.

In some embodiments, the transcriptional effector can be a histone epigenetic modifier (or a histone modifier). In some cases, the histone epigenetic modifier can modulate histones through methylation (e.g., a histone methylation modifier, such as an amino acid methyltransferase, e.g., KRAB). In some cases, the histone epigenetic modifier can modulate histones through acetylation. In some cases, the histone epigenetic modifier can modulate histones through phosphorylation. In some cases, the histone epigenetic modifier can modulate histones through ADP-ribosylation. In some cases, the histone epigenetic modifier can modulate histones through glycosylation. In some cases, the histone epigenetic modifier can modulate histones through SUMOylation. In some cases, the histone epigenetic modifier can modulate histones through ubiquitination. In some cases, the histone epigenetic modifier can modulate histones by remodeling histone structure, e.g., via an ATP hydrolysis-dependent process.

In some embodiments, the transcriptional effector can be a gene epigenetic modifier (or a gene modifier). In some cases, a gene modifier can modulate genes through methylation (e.g., a gene methylation modifier, such as a DNA methyltransferase or DNMT). In some cases, a gene modifier can modulate genes through acetylation.

In some embodiments, the transcriptional effector is from a family of related histone acetyltransferases. Non-limiting examples of histone acetyltransferases include GNAT subfamily, MYST subfamily, p300/CBP subfamily, HAT1 subfamily, GCN5, PCAF, Tip60, MOZ, MORF, MOF, HBO1, p300, CBP, HAT1, ATF-2, SRC1, and TAFII250.

In some embodiments, the transcriptional effector can comprise an epigenetic modifier. In some embodiments, the transcriptional effector comprises a histone epigenetic modifier (e.g., a histone lysine methyltransferase., a histone lysine demethylase, or a DNA methylase). Non-limiting examples of an epigenetic modifier can include EZH subfamily, Non-SET subfamily, Other SET subfamily, PRDM subfamily, SET1 subfamily, SET2 subfamily, SUV39 subfamily, SYMD subfamily, ASH1L, EHMT1, EHMT2, EZH1, EZH2, MLL, MLL2, MLL3, MLL4, MLL5, NSD1, NSD2, NSD3, PRDM1, PRDM10, PRDM11, PRDM12, PRDM13, PRDM14, PRDM15, PRDM16, PRDM2, PRDM4, PRDM5, PRDM6, PRDM7, PRDM8, PRDM9, SET1, SET1L, SET2L, SETD2, SETD3, SETD4, SETD5, SETD6, SETD7, SETD8, SETDB1, SETDB2, SETMAR, SUV39H1, SUV39H2, SUV420H1, SUV420H2, SYMD1, SYMD2, SYMD3, SYMD4, and SYMD5.

Examples of proteins (or fragments thereof) that can be used as a fusion partner to increase transcription include but are not limited to: transcriptional activators such as VP16, VP64, VP48, VP160, p65 subdomain (e.g., from NFkB), and activation domain of EDLL and/or TAL activation domain (e.g., for activity in plants), SET1A, SET1B, MLL1 to 5, ASH1, SYMD2, NSD1, JHDM2a/b, UTX, JMJD3, GCN5, PCAF, CBP, p300, TAF1, TIP60/PLIP, MOZMYST3, MORFMYST4, SRC1, ACTR, PI 60, CLOCK, Ten-Eleven Translocation (TET) dioxygenase 1 (TET1CD), TET1, DME, DML1, DML2, ROS1, etc. An additional example of such gene activating modulator is VP64-p65-Rta fusion polypeptide (VPR).
Examples of proteins (or fragments thereof) that can be used as a fusion partner to decrease transcription include but are not limited to: transcriptional repressors such as the Kruppel associated box (KRAB or SKD); KOX1 repression domain; the Mad mSIN3 interaction domain (SID); the ERF repressor domain (ERD), the SRDX repression domain (e.g, for repression in plants), and the like; histone lysine methyltransferases such as Pr-SET7/8, SUV4- 20H1, RIZ1, and the like; histone lysine demethylases such as JMJD2A/JHDM3A, JMJD2B, JMJD2C/GASC1, JMJD2D, JARJD 1 A/RBP2, JARID1B/PLU-1, JARID 1C/SMCX, JARIDID/SMCY, and the like; histone lysine deacetylases such as HDAC1, HDAC2, HDAC3, HDAC8, HDAC4, HDAC5, HDAC7, HDAC9, SIRT1, SIRT2, HDAC11, and the like; DNA methylases such as Hhal DNA m5c-methyltransferase (M.Hhal), DNA methyltransferase 1 (DNMT1), DNA methyltransferase 3a (DNMT3a), DNA methyltransferase 3b (DNMT3b), METI, DRM3 (plants), ZMET2, CMT1, CMT2 (plants), and the like; and periphery recruitment elements such as Lamin A, Lamin B, and the like.

In various aspects, an engineered nuclease provided herein may effect editing or mutating of a target polynucleotide, as described herein. In some embodiments, editing or mutating a target polynucleotide sequence involves changing one or more nucleotides in a target polynucleotide to one or more different nucleotides. In some embodiments, editing or mutating a target polynucleotide involves changing a guanine (G) to a different nucleotide. In some cases, a guanine (G) may be changed to an adenine (A). In some cases, a guanine (G) may be changed to a thymine (T). In some cases, a guanine (G) may be changed to a cytosine (C). In some cases, a guanine (G) may be changed to a uracil (U). In some cases, a guanine (G) may be changed to an inosine (I). In some embodiments, editing or mutating a target polynucleotide involves changing a cytosine (C) to a different nucleotide. In some cases, a cytosine (C) may be changed to a guanine (G). In some cases, a cytosine (C) may be changed to an adenine (A). In some cases, a cytosine (C) may be changed to a thymine (T). In some cases, a cytosine (C) may be changed to a uracil (U). In some cases, a cytosine (C) may be changed to an inosine (I). In some embodiments, editing or mutating a target polynucleotide involves changing a thymine (T) to a different nucleotide. In some cases, a thymine (T) may be changed to a cytosine (C). In some cases, a thymine (T) may be changed to a guanine (G). In some cases, a thymine (T) may be changed to an adenine (A). In some cases, a thymine (T) may be changed to a uracil (U). In some cases, a thymine (T) may be changed to an inosine (I). In some embodiments, editing or mutating a target polynucleotide involves changing an adenine (A) to a different nucleotide. In some cases, an adenine (A) may be changed to a guanine (G). In some cases, an adenine (A) may be changed to a cytosine (C). In some cases, an adenine (A) may be changed to a thymine (T). In some cases, an adenine (A) may be changed to a uracil (U). In some cases, an adenine (A) may be changed to an inosine (I). In some embodiments, editing or mutating a target polynucleotide involves changing a uracil (U) to a different nucleotide. In some cases, a uracil (U) may be changed to a guanine (G). In some cases, a uracil (U) may be changed to a cytosine (C). In some cases, a uracil (U) may be changed to a thymine (T). In some cases, a uracil (U) may be changed to an adenine (A). In some cases, a uracil (U) may be changed to an inosine (I). In some embodiments, editing or mutating a target polynucleotide involves changing an inosine (I) to a different nucleotide. In some cases, an inosine (I) may be changed to a guanine (G). In some cases, an inosine (I) may be changed to a cytosine (C). In some cases, an inosine (I) may be changed to a thymine (T). In some cases, an inosine (I) may be changed to an adenine (A). In some cases, an inosine (I) may be changed to a uracil (U).

In some embodiments, editing or mutating a target polynucleotide involves introducing one or more point mutations into a target polynucleotide. In some embodiments, editing or mutating a target polynucleotide involves introducing one or more deletions (e.g., of one or more nucleotides) into a target polynucleotide. In some embodiments, editing or mutating a target polynucleotide involves introducing one or more insertions (e.g., of one or more nucleotides) into a target polynucleotide. In some embodiments, editing or mutating a target polynucleotide involves introducing one or more inversions (e.g., of two or more nucleotides) in a target polynucleotide. In some embodiments, editing or mutating a target polynucleotide involves introducing one or more translocations (e.g., of one or more nucleotides) in a target polynucleotide. In some embodiments, editing or mutating a target polynucleotide involves introducing one or more transpositions in a target polynucleotide.

In some cases, an engineered nuclease as described herein may be coupled to a partner (e.g., a gene editing moiety) that effects editing or mutating of a target polynucleotide, as described herein. In some cases, a nuclease-deficient or nuclease-null engineered nuclease as provided herein is covalently or non-covalently coupled to a gene editing moiety (e.g., a protein, or functional domain or functional fragment thereof) that effects editing or mutating of a target polynucleotide. In some embodiments, the gene editing moiety (e.g., a protein, or functional domain or functional fragment thereof) that effects editing or mutating of a target polynucleotide is a gene editing moiety that changes one or more nucleotides to a different nucleotide. In some embodiments, the gene editing moiety (e.g., a protein, or functional domain or functional fragment thereof) that effects editing or mutating of a target polynucleotide is a gene editing moiety that changes a guanine (G) to a different nucleotide. In some cases, the gene editing moiety that effects editing or mutating of a target polynucleotide changes a guanine (G) to a cytosine (C). In some cases, the gene editing moiety that effects editing or mutating of a target polynucleotide changes a guanine (G) to a thymine (T). In some cases, the gene editing moiety that effects editing or mutating of a target polynucleotide changes a guanine (G) to an adenine (A). In some cases, the gene editing moiety that effects editing or mutating of a target polynucleotide changes a guanine (G) to a uracil (U). In some cases, the gene editing moiety that effects editing or mutating of a target polynucleotide changes a guanine (G) to an inosine (I). In some embodiments, the gene editing moiety (e.g., a protein, or functional domain or functional fragment thereof) that effects editing or mutating of a target polynucleotide sequence is a gene editing moiety that changes a cytosine (C) to a different nucleotide. In some cases, the gene editing moiety that effects editing or mutating of a target polynucleotide changes a cytosine (C) to a guanine (G). In some cases, the gene editing moiety that effects editing or mutating of a target polynucleotide changes a cytosine (C) to a thymine (T). In some cases, the gene editing moiety that effects editing or mutating of a target polynucleotide changes a cytosine (C) to an adenine (A). In some cases, the gene editing moiety that effects editing or mutating of a target polynucleotide changes a cytosine (C) to a uracil (U). In some cases, the gene editing moiety that effects editing or mutating of a target polynucleotide changes a cytosine (C) to an inosine (I). In some embodiments, the gene editing moiety (e.g., a protein, or functional domain or functional fragment thereof) that effects editing or mutating of a target polynucleotide sequence is a gene editing moiety that changes a thymine (T) to a different nucleotide. In some cases, the gene editing moiety that effects editing or mutating of a target polynucleotide changes a thymine (T) to a cytosine (C). In some cases, the gene editing moiety that effects editing or mutating of a target polynucleotide changes a thymine (T) to a guanine (G). In some cases, the gene editing moiety that effects editing or mutating of a target polynucleotide changes a thymine (T) to an adenine (A). In some cases, the gene editing moiety that effects editing or mutating of a target polynucleotide changes a thymine (T) to a uracil (U). In some cases, the gene editing moiety that effects editing or mutating of a target polynucleotide changes a thymine (T) to an inosine (I). In some embodiments, the gene editing moiety (e.g., a protein, or functional domain or functional fragment thereof) that effects editing or mutating of a target polynucleotide sequence is a gene editing moiety that changes an adenine (A) to a different nucleotide. In some cases, the gene editing moiety that effects editing or mutating of a target polynucleotide changes an adenine (A) to a cytosine (C). In some cases, the gene editing moiety that effects editing or mutating of a target polynucleotide changes an adenine (A) to a thymine (T). In some cases, the gene editing moiety that effects editing or mutating of a target polynucleotide changes an adenine (A) to a guanine (G). In some cases, the gene editing moiety that effects editing or mutating of a target polynucleotide changes an adenine (A) to a uracil (U). In some cases, the gene editing moiety that effects editing or mutating of a target polynucleotide changes an adenine (A) to an inosine (I). In some embodiments, the gene editing moiety (e.g., a protein, or functional domain or functional fragment thereof) that effects editing or mutating of a target polynucleotide sequence is a gene editing moiety that changes a uracil (U) to a different nucleotide. In some cases, the gene editing moiety that effects editing or mutating of a target polynucleotide changes a uracil (U) to a cytosine (C). In some cases, the gene editing moiety that effects editing or mutating of a target polynucleotide changes a uracil (U) to a thymine (T). In some cases, the gene editing moiety that effects editing or mutating of a target polynucleotide changes a uracil (U) to an adenine (A). In some cases, the gene editing moiety that effects editing or mutating of a target polynucleotide changes a uracil (U) to a guanine (G). In some cases, the gene editing moiety that effects editing or mutating of a target polynucleotide changes a uracil (U) to an inosine (I). In some embodiments, the gene editing moiety (e.g., a protein, or functional domain or functional fragment thereof) that effects editing or mutating of a target polynucleotide sequence is a gene editing moiety that changes an inosine (I) to a different nucleotide. In some cases, the gene editing moiety that effects editing or mutating of a target polynucleotide changes an inosine (I) to a cytosine (C). In some cases, the gene editing moiety that effects editing or mutating of a target polynucleotide changes an inosine (I) to a thymine (T). In some cases, the gene editing moiety that effects editing or mutating of a target polynucleotide changes an inosine (I) to an adenine (A). In some cases, the gene editing moiety that effects editing or mutating of a target polynucleotide changes an inosine (I) to a uracil (U). In some cases, the gene editing moiety that effects editing or mutating of a target polynucleotide changes an inosine (I) to a guanine (G).

In some embodiments, the engineered nuclease as described herein (e.g., a nuclease-deficient or nuclease-null engineered nuclease, as described herein) may be coupled (e.g., covalently or non-covalently) to a partner (e.g., a gene editing moiety) that introduces one or more point mutations into a target polynucleotide. In some embodiments, the engineered nuclease as described herein (e.g., a nuclease-deficient or nuclease-null engineered nuclease, as described herein) may be coupled (e.g., covalently or non-covalently) to a partner (e.g., a gene editing moiety) that introduces one or more deletions (e.g., of one or more nucleotides) into a target polynucleotide. In some embodiments, the engineered nuclease as described herein (e.g., a nuclease-deficient or nuclease-null engineered nuclease, as described herein) may be coupled (e.g., covalently or non-covalently) to a partner (e.g., a gene editing moiety) that introduces one or more insertions (e.g., of one or more nucleotides) into a target polynucleotide. In some embodiments, the engineered nuclease as described herein (e.g., a nuclease-deficient or nuclease-null engineered nuclease, as described herein) may be coupled (e.g., covalently or non-covalently) to a partner (e.g., a gene editing moiety) that introduces one or more inversions (e.g., of two or more nucleotides) in a target polynucleotide. In some embodiments, the engineered nuclease as described herein (e.g., a nuclease-deficient or nuclease-null engineered nuclease, as described herein) may be coupled (e.g., covalently or non-covalently) to a partner (e.g., a gene editing moiety) that introduces one or more translocations (e.g., of one or more nucleotides) in a target polynucleotide. In some embodiments, the engineered nuclease as described herein (e.g., a nuclease-deficient or nuclease-null engineered nuclease, as described herein) may be coupled (e.g., covalently or non-covalently) to a partner (e.g., a gene editing moiety) that introduces one or more transpositions in a target polynucleotide.

In some embodiments, the gene editing moiety may be a base-editing protein or a base-editing enzyme. In some embodiments, the base-editing protein or base-editing enzyme is a deaminase. In some cases, the deaminase is a cytidine deaminase. In some cases, the cytidine deaminase catalyzes the reaction of a cytosine (C) to a uracil (U), which has the base-pairing properties of thymine. In some embodiments, for example where the polynucleotide is double-stranded (e.g., double-stranded DNA), the uridine base can then be substituted with a thymidine base (e.g., by cellular repair machinery) to give rise to a C•G to a T•A transition. In some embodiments, the deaminase is an adenine deaminase. In some cases, the adenine deaminase catalyzes the reaction of an adenosine (A) to an inosine (I). Non-limiting examples of deaminases suitable for use herein include, without limitation, APOBEC 1 deaminase, APOBEC2 deaminase, APOBEC3 deaminase, APOBEC3A deaminase, APOBEC3B deaminase, APOBEC3C deaminase, APOBEC3D deaminase, APOBEC3E deaminase, APOBEC3F deaminase, APOBEC3G deaminase, APOBEC3H deaminase, APOBEC4 deaminase, activation-induced cytidine deaminase (AID), adenosine deaminase 1 (ADAR1), adenosine deaminase 2 (ADAR2), adenosine deaminase 3 (ADAR3), or TadA.

In some embodiments, the engineered nucleases described herein may be used for prime editing. For example, an engineered nuclease as described herein may be coupled to a reverse transcriptase enzyme (e.g., an engineered M-MLV reverse transcriptase) and a prime editing RNA (pegRNA). In such cases, the engineered nuclease may comprise nickase activity. In some embodiments, prime editing may be used to mediate targeted insertions, deletions, or base-to-base conversions.

In some embodiments, the engineered nucleases described herein may be used for gene writing.

In some embodiments, the engineered nucleases described herein may be coupled to a partner (e.g., a protein, or functional domain or functional fragment thereof) that methylates a target substrate. In some cases, the partner (e.g., a protein, or functional domain or functional fragment thereof) that methylates a target substrate is a methyltransferase. In some cases, the methyltransferase is a DNA methyltransferase, a histone methyltransferase, or an RNA methyltransferase. In some cases, the DNA methyltransferase is DNMT1 or DNMT3.

In some embodiments, the engineered nucleases described herein may be coupled to a partner (e.g., a protein, or functional domain or functional fragment thereof) that has demethylase activity (e.g., can remove methyl groups from nucleic acids, proteins, or other molecules). In some cases, the partner (e.g., a protein, or functional domain or functional fragment thereof) that has demethylase activity is a histone lysine demethylase, such as, but not limited to KDM1, KDM2, KDM3, KDM4, KDM5, and KDM6.

In some embodiments, the engineered nucleases described herein may be coupled to a partner (e.g., a protein, or functional domain or functional fragment thereof) that has dismutase activity. In some cases, the partner (e.g., a protein, or functional domain or functional fragment thereof) that has dismutase activity is superoxide dismutase, formaldehyde dismutase, or chlorite dismutase.

In some embodiments, the engineered nucleases described herein may be coupled to a partner (e.g., a protein, or functional domain or functional fragment thereof) that has alkylation activity. In some cases, the partner (e.g., a protein, or functional domain or functional fragment thereof) that has alkylation activity is a prenyltransferase, a terpene cyclase, or a terpene synthase.

In some embodiments, the engineered nucleases described herein may be coupled to a partner (e.g., a protein, or functional domain or functional fragment thereof) that has depurination activity. In some cases, the partner (e.g., a protein, or functional domain or functional fragment thereof) that has depurination activity is DNA glycosylase.

In some embodiments, the engineered nucleases described herein may be coupled to a partner (e.g., a protein, or functional domain or functional fragment thereof) that has oxidation activity. In some cases, the partner (e.g., a protein, or functional domain or functional fragment thereof) that has oxidation activity is a peroxidase or an oxidase.

In some embodiments, the engineered nucleases described herein may be coupled to a partner (e.g., a protein, or functional domain or functional fragment thereof) that has pyrimidine dimer forming activity.

In some embodiments, the engineered nucleases described herein may be coupled to a partner (e.g., a protein, or functional domain or functional fragment thereof) that has integrase activity. In some cases, the partner (e.g., a protein, or functional domain or functional fragment thereof) that has integrase activity is retroviral integrase or HIV integrase.

In some embodiments, the engineered nucleases described herein may be coupled to a partner (e.g., a protein, or functional domain or functional fragment thereof) that has transposase activity. In some cases, the partner (e.g., a protein, or functional domain or functional fragment thereof) that has transposase activity is ty1, Mariner transposase, Tn3, transposase (Tnp) Tn5, or Tn7 transposon.

In some embodiments, the engineered nucleases described herein may be coupled to a partner e.g., a protein, or functional domain or functional fragment thereof) that has recombinase activity. In some cases, the partner (e.g., a protein, or functional domain or functional fragment thereof) that has recombinase activity is tyrosine recombinase, Rad51 recombinase, RecA recombinase, or Dmc 1 recombinase.

In some embodiments, the engineered nucleases described herein may be coupled to a partner (e.g., a protein, or functional domain or functional fragment thereof) that has polymerase activity. In some cases, the partner (e.g., a protein, or functional domain or functional fragment thereof) that has polymerase activity is DNA polymerase, RNA polymerase, reverse transcriptase, or RdRp replicase.

In some embodiments, the engineered nucleases described herein may be coupled to a partner (e.g., a protein, or functional domain or functional fragment thereof) that has ligase activity. In some cases, the partner (e.g., a protein, or functional domain or functional fragment thereof) that has ligase activity is ubiquitin ligases, glutamate-cysteine ligase, aminoacyl tRNA synthetase, succinyl coenzyme A synthetase, acetyl-CoA synthetase, pyruvate carboxylase, acetyl-CoA carboxylase, propionyl-CoA carboxylase, methylcrotonyl-CoA carboxylase, DNA ligase, magnesium chelatase, cobalt chelatase, or DNA synthetase.

In some embodiments, the engineered nucleases described herein may be coupled to a partner (e.g., a protein, or functional domain or functional fragment thereof) that has helicase activity. In some cases, the partner (e.g., a protein, or functional domain or functional fragment thereof) that has helicase activity is a DNA helicase, an RNA helicase, chromodomain helicase, or DEAD box/DEAD/DEAH box helicase.

In some embodiments, the engineered nucleases described herein may be coupled to a partner (e.g., a protein, or functional domain or functional fragment thereof) that has photolyase activity. In some cases, the partner (e.g., a protein, or functional domain or functional fragment thereof) that has photolyase activity is photoreactivating enzyme, DNA photolyase, DNA-photoreactivating enzyme, DNA cyclobutane dipyrimidine photolyase, DNA photolyase, deoxyribonucleic photolyase, deoxyribodipyrimidine photolyase, photolyase, PRE, PhrB photolyase, deoxyribonucleic cyclobutane dipyrimidine photolyase, phr A photolyase, dipyrimidine photolyase (photosensitive), or deoxyribonucleate pyrimidine dimer lyase (photosensitive).

In some embodiments, the engineered nucleases described herein may be coupled to a partner (e.g., a protein, or functional domain or functional fragment thereof) that has glycosylase activity. In some cases, the partner (e.g., a protein, or functional domain or functional fragment thereof) that has glycosylase activity is N-methylpurine DNA glycosylase, UNG, hOGG1, hNTH1, hNEIL1, hMYH, hSMUG1, TDG, MBD4, Mag1, Ung1, Ogg1, Ntg1, AlkE, Ntg2, hNEIL2, hNEIL3, AlkC, AlkD, MutY, Nei, Nth, Fpg, or UDG.

In some embodiments, the engineered nucleases described herein may be coupled to a partner (e.g., a protein, or functional domain or functional fragment thereof) that has acetyltransferase activity. In some cases, the partner (e.g., a protein, or functional domain or functional fragment thereof) that has acetyltransferase activity is CBP histone acetyltransferase, choline acetyltransferase, chloramphenicol acetyltransferase, serotonin N-acetyltransferase, NatA Acetyltransferase, or NatB acetyltransferase.

In some embodiments, the engineered nucleases described herein may be coupled to a partner (e.g., a protein, or functional domain or functional fragment thereof) that has deacetylase activity. In some cases, the partner (e.g., a protein, or functional domain or functional fragment thereof) that has deacetylase activity is HDAC-1, HDAC-2, HDAC-3, HDAC-4, HDAC-5, HDAC-6, HDAC-7, or HDAC-8.

In some embodiments, the engineered nucleases described herein may be coupled to a partner (e.g., a protein, or functional domain or functional fragment thereof) that has kinase activity. In some cases, the partner (e.g., a protein, or functional domain or functional fragment thereof) that has kinase activity is Ca2+/calmodulin-dependent protein kinase, cyclin-dependent kinase, nucleoside-diphosphate kinase, a phosphatidylinositol phosphate kinase, thymidine kinase, thymidylate kinase, or wall-associated kinase.

In some embodiments, the engineered nucleases described herein may be coupled to a partner (e.g., a protein, or functional domain or functional fragment thereof) that has phosphatase activity. In some cases, the partner (e.g., a protein, or functional domain or functional fragment thereof) that has phosphatase activity is acid phosphatase, alkaline phosphatase, endonuclease/exonuclease/phosphatase family, kinase, phosphatome, phosphotransferase, protein phosphatase, or protein phosphatase 2.

In some embodiments, the engineered nucleases described herein may be coupled to a partner (e.g., a protein, or functional domain or functional fragment thereof) that has ubiquitin ligase activity. In some cases, the partner (e.g., a protein, or functional domain or functional fragment thereof) that has ubiquitin ligase activity is E3A, mdm2, Anaphase-promoting complex (APC), UBR5 (EDD1), SOCS/ BC-box/ eloBC/ CUL5/ RING, LNXp80, CBX4, CBLL1, HACE1, HECTD1, HECTD2, HECTD3, HECTD4, HECW1, HECW2, HERC1, HERC2, HERC3, HERC4, HERC5, HERC6, HUWE1, ITCH, NEDD4, NEDD4L, PPIL2, PRPF19, PIAS1, PIAS2, PIAS3, PIAS4, RANBP2, RNF4, RBX1, SMURF1, SMURF2, STUB1, TOPORS, TRIP12, UBE3A, UBE3B, UBE3C, UBE3D, UBE4A, UBE4B, UBOX5, UBR5, VHL, WWP1, WWP2, Parkin, or MKRN1.

In some embodiments, the engineered nucleases described herein may be coupled to a partner (e.g., a protein, or functional domain or functional fragment thereof) that has deubiquitinating activity. In some cases, the partner (e.g., a protein, or functional domain or functional fragment thereof) that has deubiquitinating activity is a deubiquitinating peptidase, a deubiquitinating isopeptidase, a deubiquitinase, a ubiquitin protease, a ubiquitin hydrolase, or a ubiquitin isopeptidase.

In some embodiments, the engineered nucleases described herein may be coupled to a partner (e.g., a protein, or functional domain or functional fragment thereof) that has adenylation activity. In some cases, the partner (e.g., a protein, or functional domain or functional fragment thereof) that has adenylation activity is carboxylic acid reductase.

In some embodiments, the engineered nucleases described herein may be coupled to a partner (e.g., a protein, or functional domain or functional fragment thereof) that has deadenylation activity. In some cases, the partner (e.g., a protein, or functional domain or functional fragment thereof) that has deadenylation activity is 5'-deadenylase, CNOT6 deadenylase, CNOT6L deadenylase, or CCR4-NOT deadenylase,

In some embodiments, the engineered nucleases described herein may be coupled to a partner (e.g., a protein, or functional domain or functional fragment thereof) that has SUMOylating activity. In some cases, the partner (e.g., a protein, or functional domain or functional fragment thereof) that has SUMOylating activity is small ubiquitin-related modifier (SUMO-1), SUMO-2, or SUMO-3.

In some embodiments, the engineered nucleases described herein may be coupled to a partner (e.g., a protein, or functional domain or functional fragment thereof) that has deSUMOylating activity. In some cases, the partner (e.g., a protein, or functional domain or functional fragment thereof) that has deSUMOylating activity is SENP1, SENP2, SENP3, or SENP5.

In some embodiments, the engineered nucleases described herein may be coupled to a partner (e.g., a protein, or functional domain or functional fragment thereof) that has ribosylation activity. In some cases, the partner (e.g., a protein, or functional domain or functional fragment thereof) that has ribosylation activity is a mono(ADP-ribosyl)transferase, a poly(ADP-ribose)polymerase, or histone ribosylase.

In some embodiments, the engineered nucleases described herein may be coupled to a partner (e.g., a protein, or functional domain or functional fragment thereof) that has deribosylation activity. In some cases, the partner (e.g., a protein, or functional domain or functional fragment thereof) that has deribosylation activity is histone lysine deribosylase or ADP-ribose deribosylase.

In some embodiments, the engineered nucleases described herein may be coupled to a partner (e.g., a protein, or functional domain or functional fragment thereof) that has myristoylation activity. In some cases, the partner (e.g., a protein, or functional domain or functional fragment thereof) that has myristoylation activity is N-myristoltransferase (NMT) 1, N-myristoltransferase (NMT) 2, or glycylpeptide N-tetradecanoyltransferase.

In some embodiments, the engineered nucleases described herein may be coupled to a partner (e.g., a protein, or functional domain or functional fragment thereof) that has remodeling activity. In some cases, the partner (e.g., a protein, or functional domain or functional fragment thereof) that has remodeling activity is a histone acetyltransferase (HAT), a deacetylase, or a methyltransferase.

In some embodiments, the engineered nucleases described herein may be coupled to a partner (e.g., a protein, or functional domain or functional fragment thereof) that has protease activity. In some cases, the partner (e.g., a protein, or functional domain or functional fragment thereof) that has protease activity is trypsin, chymotrypsin, elastase, papain, bromelain, a serine protease, a cysteine protease, a threonine protease, an aspartic protease, a glutamic protease, a metalloprotease, or an asparagine peptide lyase.

In some embodiments, the engineered nucleases described herein may be coupled to a partner (e.g., a protein, or functional domain or functional fragment thereof) that has oxidoreductase activity. In some cases, the partner (e.g., a protein, or functional domain or functional fragment thereof) that has oxidoreductase activity is donor dehydrogenase, peroxidase, reductase, dehydrogenase, oxidase, oxygenase, hydroxylase, luciferase, DMSO reductase, glucose oxidase, L-gulonolactone oxidase, thiamine oxidase, xanthine oxidase, acetaldehyde dehydrogenase, pyruvate dehydrogenase, oxoglutarate dehydrogenase, monoamine oxidase, biliverdin reductase, dihydrofolate reductase, methylenetetrahydrofolate reductase, sarcosine oxidase, or dihydrobenzophenanthridine oxidase.

In some embodiments, the engineered nucleases described herein may be coupled to a partner (e.g., a protein, or functional domain or functional fragment thereof) that has transferase activity. In some cases, the partner (e.g., a protein, or functional domain or functional fragment thereof) that has transferase activity is coenzyme A transferase, acyl transferase, peptidyl transferase, N-acetyltransferase, or pyruvate dehydrogenase.

In some embodiments, the engineered nucleases described herein may be coupled to a partner (e.g., a protein, or functional domain or functional fragment thereof) that has hydrolase activity. In some cases, the partner (e.g., a protein, or functional domain or functional fragment thereof) that has hydrolase activity is an esterase, a protease, a glycosidase, or a lipase.

In some embodiments, the engineered nucleases described herein may be coupled to a partner (e.g., a protein, or functional domain or functional fragment thereof) that has lyase activity. In some cases, the partner (e.g., a protein, or functional domain or functional fragment thereof) that has lyase activity is phenylalanine ammonia-lyase, citrate lyase, isocitrate lyase, hydroxynitrile, pectate lyase, argininosuccinate lyase, pyruvate formate lyase, alginate lyase, or pectin lyase.

In some embodiments, the engineered nucleases described herein may be coupled to a partner (e.g., a protein, or functional domain or functional fragment thereof) that has isomerase activity. In some cases, the partner (e.g., a protein, or functional domain or functional fragment thereof) that has isomerase activity is ribose phosphate isomerase, bisphosphoglycerate mutase, or photoisomerase.

In some embodiments, the engineered nucleases described herein may be coupled to a partner (e.g., a protein, or functional domain or functional fragment thereof) that has synthase activity. In some cases, the partner (e.g., a protein, or functional domain or functional fragment thereof) that has synthase activity is ATP synthase, citrate synthase, tryptophan synthase, pseudouridine synthase, or fatty acid synthase.

In some embodiments, the engineered nucleases described herein may be coupled to a partner (e.g., a protein, or functional domain or functional fragment thereof) that has demyristoylation activity. In some cases, the partner (e.g., a protein, or functional domain or functional fragment thereof) that has demyristoylation activity is T3SS effector protein.

In some embodiments, the engineered nucleases described herein may be coupled to a partner (e.g., a protein, or functional domain or functional fragment thereof) that has transposition activity. In some cases, the partner (e.g., a protein, or functional domain or functional fragment thereof) that has transposition activity is transposase Tn5 or Sleeping Beauty transposase.

The engineered nuclease as disclosed herein can be provided in any form. For example, the engineered nuclease can be provided in the form of a protein, such as the engineered nuclease alone or complexed with a guide nucleic acid as a ribonucleoprotein. The engineered nuclease can be provided in a complex, for example, complexed with a guide nucleic acid and/or one or more heterologous gene effectors of the disclosure. The engineered nuclease can be provided in the form of a nucleic acid encoding at least the engineered nuclease, such as an RNA (e.g., messenger RNA (mRNA), or DNA. The nucleic acid encoding at least the engineered nuclease can be codon optimized for efficient translation into protein in a particular cell or organism (e.g., human codon optimized).

Nucleic acids encoding at least the engineered nuclease as disclosed herein (not part of the present invention), fragments, or derivatives thereof can be stably integrated in the genome of a cell. Nucleic acids encoding at least the engineered nuclease can be operably linked to a promoter, for example, a promoter that is constitutively or inducibly active in the cell. Nucleic acids encoding at least the engineered nuclease can be operably linked to a promoter in an expression construct. Expression constructs can include any nucleic acid constructs capable of directing expression of a gene or other nucleic acid sequence of interest (e.g., at least the engineered nuclease) and which can transfer such a nucleic acid sequence of interest to a target cell.

The engineered nuclease as disclosed herein may associate with a single guide RNA (sgRNA) to activate or repress transcription of a target gene (e.g., target endogenous gene), for example, in combination with heterologous gene effector(s) disclosed herein. sgRNAs can be introduced into cells expressing the engineered nuclease or variant thereof, as provided herein. In some cases, such cells can contain one or more different sgRNAs that target the same target gene (e.g., target endogenous gene) or target gene regulatory sequence. In other cases, the sgRNAs target different nucleic acids in the cell (e.g., different target genes, different target gene regulatory sequences, or different sequences within the same target gene or target gene regulatory sequence).

Enzymatically inactive (e.g., nuclease deficient) can refer to a nuclease that can bind to a nucleic acid sequence in a polynucleotide in a sequence-specific manner, but may not cleave a target polynucleotide or will cleave it at a substantially reduced frequency. An enzymatically inactive guide moiety can comprise an enzymatically inactive domain (e.g., nuclease domain). Enzymatically inactive can refer to no activity. Enzymatically inactive can refer to substantially no activity. Enzymatically inactive can refer to essentially no activity. Enzymatically inactive can refer to an activity no more than 1%, no more than 2%, no more than 3%, no more than 4%, no more than 5%, no more than 6%, no more than 7%, no more than 8%, no more than 9%, or no more than 10% activity compared to a comparable wild-type activity (e.g., nucleic acid cleaving activity, wild-type Cas activity).

The target nucleic acid of the engineered nuclease may be dsDNA. In such case, dsDNA-targeting specificity is determined, at least in part, by two parameters: the gRNA spacer targeting a protospacer in the target dsDNA (the sequence in the target dsDNA corresponding to the gRNA spacer on the non-complementary DNA strand) and a short sequence, the protospacer-adjacent motif (PAM), located immediately 5' (upstream) of the protospacer on the non-complementary DNA strand. The PAM may be 5'-TTTG-3', 5'-TTTA-3', or 5'-TTTR-3'.

The target nucleic acid of the engineered nuclease as disclosed herein may be RNA. In such case, RNA-targeting specificity is determined, at least in part, by the gRNA spacer targeting a protospacer-like sequence in the target RNA (the sequence in the target RNA complementary to the gRNA spacer), and is independent of the sequence located immediately 5' (upstream) of the protospacer-like sequence. The engineered nuclease may be further capable of targeting a dsDNA molecule, wherein the gRNA spacer is selected such that it targets a protospacer in the target dsDNA molecule having a PAM selected from 5'-TTTG-3', 5'-TTTA-3', and 5'-TTTR-3'. In other cases, the engineered nuclease is incapable of targeting a dsDNA molecule, wherein the gRNA spacer is selected such that any protospacers in the dsDNA molecule targeted by the gRNA spacer do not have a PAM selected from 5'-TTTG-3', 5'-TTTA-3', and 5'-TTTR-3'.

In some embodiments, the heterologous polypeptide comprising the engineered nuclease (e.g., and/or a complex comprising the heterologous polypeptide) can regulate expression and/or activity of a target gene (e.g., target endogenous gene). In some embodiments, the heterologous polypeptide and/or a complex thereof can edit the sequence of a nucleic acid (e.g., a gene and/or gene product). A nuclease-active variant of the engineered nuclease can edit a nucleic acid sequence by generating a double-stranded break or single-stranded break in a target polynucleotide.

In some embodiments, the heterologous polypeptide comprising the engineered nuclease (e.g., and/or a complex comprising the heterologous polypeptide) can generate a double-strand break in a target polynucleotide, such as DNA. A double-strand break in DNA can result in DNA break repair which allows for the introduction of gene modification(s) (e.g., nucleic acid editing). In some embodiments, a nuclease induces site-specific single-strand DNA breaks or nicks, thus resulting in HDR.

A double-strand break in DNA can result in DNA break repair which allows for the introduction of gene modification(s) (e.g., nucleic acid editing). DNA break repair can occur via non-homologous end joining (NHEJ) or homology-directed repair (HDR). In HDR, a donor DNA repair template or template polynucleotide that contains homology arms flanking sites of the target DNA can be provided.

In some embodiments, the heterologous polypeptide comprising the engineered nuclease (e.g., and/or a complex comprising the heterologous polypeptide) does not generate a double-strand break in a target polynucleotide, such as DNA. Binding of the heterologous polypeptide or the complex comprising the heterologous polypeptide (e.g., a complex comprising a nuclease deficient variant of the engineered nuclease and a guide RNA) without a nucleic acid break can be sufficient to regulate expression (e.g., enhance or suppress) of a target gene (e.g., endogenous target gene).

### Target gene

Disclosed are further compositions, methods, and systems for modulating expression of one or more target genes (not part of the present invention). The target gene(s) can be one or more endogenous target genes, such as (i) a disease causing allele, e.g., a mutant allele, and/or (ii) a non-disease causing allele, e.g., a wild type allele. For example, disclosed herein are one or more complexes that comprise a guide moiety and one or more heterologous polypeptides comprising the engineered polypeptide (e.g., comprising the engineered nuclease) as disclosed herein that can modulate (e.g., increase or decrease) an activity or expression level of a target gene (e.g., in a cell). Such complex comprising a guide moiety (e.g., small guide RNA) and an engineered nuclease can effect the modulation of expression of target gene(s) via cleavage of the target gene(s). Alternatively or in addition to, such complex comprise a gene modulator operatively coupled to (e.g., fused to) the engineered nuclease, such that the complex can effect the modulation of expression of target gene(s) without cleaving the target gene(s). In some cases, the gene modulator may effect an increase in expression of the target gene. In some cases, the gene modulator may effect a decrease in expression of the target gene. In some cases, the gene modulator (e.g., a gene editing moiety, as described herein) may effect editing of a target gene, for example, to correct an undesirable mutation in a target gene such that expression of the mutated gene is decreased, and expression of the corrected gene is increased.

A target gene or regulatory sequence thereof may be endogenous to a cell, for example, present in the cell's genome, or endogenous to a subject, for example, present in the subject's genome. In some cases, a target gene or regulatory sequence thereof is not part of an engineered reporter system.

A target gene may be exogenous to a host subject, for example, a pathogen target gene or an exogenous gene expressed as a result of a therapeutic intervention, such as a gene therapy and/or cell therapy. In some cases, a target gene is an exogenous reporter gene. In some cases, a target gene is an exogenous synthetic gene.

In some cases, the systems and methods as disclosed herein can modulate (e.g., increase or decrease) expression of a target gene (e.g., upon introducing a complex comprising the heterologous polypeptide into a cell or population of cells) or a duration thereof. An expression level may be an RNA expression level can be measured by, for example, RNAseq, qPCR, microarray, gene array, FISH, etc. In some cases, an expression level is a protein expression level can be measured by, for example, Western Blot, ELISA, multiplex immunoassay, mass spectrometry, NMR, proteomics, flow cytometry, mass cytometry, etc.

In some cases, the systems and methods as disclosed herein can modulate (e.g., increase or decrease) expression of a target gene (e.g., upon introducing a complex comprising the heterologous polypeptide into a cell or population of cells) or a duration thereof by at least about 10%, at least about 20%, at least about 30%, at least about 40%, at least about 50%, at least about 60%, at least about 70%, at least about 80%, at least about 90%, at least about 2-fold, at least about 3 fold, at least about 4 fold, at least about 5 fold, at least about 6 fold, at least about 7 fold, at least about 8 fold, at least about 9 fold, at least about 10 fold, at least about 11 fold, at least about 12 fold, at least about 13 fold, at least about 14, at least fold about 15 fold, at least about 20 fold, at least about 30 fold, at least about 40 fold, at least about 50 fold, at least about 60 fold, at least about 70 fold, at least about 80 fold, at least about 90 fold, at least about 100 fold, at least about 150 fold, at least about 200 fold, at least about 250 fold, at least about 300 fold, at least about 350 fold, at least about 400 fold, at least about 500 fold, at least about 600 fold, at least about 700 fold, at least about 800 fold, at least about 900 fold, at least about 1000 fold, at least about 1500 fold, at least about 2000 fold, or at least about 3000 fold.

In some cases, the systems and methods as disclosed herein can modulate (e.g., increase or decrease) expression of a target gene (e.g., upon introducing a complex comprising the heterologous polypeptide into a cell or population of cells) or a duration thereof by at most about 50%, at most about 60%, at most about 70%, at most about 80%, at most about 90%, at most about 2-fold, at most about 3 fold, at most about 4 fold, at most about 5 fold, at most about 6 fold, at most about 7 fold, at most about 8 fold, at most about 9 fold, at most about 10 fold, at most about 11 fold, at most about 12 fold, at most about 13 fold, at most about 14, at most fold about 15 fold, at most about 20 fold, at most about 30 fold, at most about 40 fold, at most about 50 fold, at most about 60 fold, at most about 70 fold, at most about 80 fold, at most about 90 fold, at most about 100 fold, at most about 150 fold, at most about 200 fold, at most about 250 fold, at most about 300 fold, at most about 350 fold, at most about 400 fold, at most about 500 fold, at most about 600 fold, at most about 700 fold, at most about 800 fold, at most about 900 fold, at most about 1000 fold, at most about 1500 fold, at most about 2000 fold, at most about 3000 fold, at most about 5000 fold, or at most about 10000 fold.

In some cases, the systems and methods as disclosed herein can modulate (e.g., increase or decrease) expression of a target gene (e.g., upon introducing a complex comprising the heterologous polypeptide into a cell or population of cells) or a duration thereof by about 10%, about 20%, about 30%, about 40%, about 50%, about 60%, about 70%, about 80%, about 90%, about 2-fold, about 3 fold, about 4 fold, about 5 fold, about 6 fold, about 7 fold, about 8 fold, about 9 fold, about 10 fold, about 11 fold, about 12 fold, about 13 fold, about 14, about 15 fold, about 20 fold, about 30 fold, about 40 fold, about 50 fold, about 60 fold, about 70 fold, about 80 fold, about 90 fold, about 100 fold, about 150 fold, about 200 fold, about 250 fold, about 300 fold, about 350 fold, about 400 fold, about 500 fold, about 600 fold, about 700 fold, about 800 fold, about 900 fold, about 1000 fold, about 1500 fold, about 2000 fold, about 3000 fold, about 5000 fold, or about 10000 fold.

In some cases, the systems and methods as disclosed herein can modulate (e.g., increase or decrease) expression of a target gene (e.g., upon introducing a complex comprising the heterologous polypeptide into a cell or population of cells) or a duration thereof from below a limit of detection to a detectable level.

In some cases, the degree in change of expression or duration thereof is relative to before introducing the system of the present disclosure (e.g., a complex comprising the heterologous polypeptide) into the cell or population of cells. In some embodiments, the degree in change of expression or duration thereof is relative to a corresponding control cell or population of cells that are not treated with the system of the present disclosure. In some embodiments, the degree in change of expression or duration thereof is relative to a corresponding control cell or population of cells that are treated with an alternative to the system of the present disclosure.

In some cases, the degree in change of expression or duration thereof is relative to a control nuclease. The control nuclease can comprise a naturally occurring nuclease (e.g., (i) the polypeptide sequence of SEQ ID NO: 1 or (ii) the polypeptide sequence of any one of the Cas proteins selected from TABLE 2) or any modification thereof (e.g., a variant thereof with reduced nuclease activity and operatively coupled to a gene modulator). For example, the control nuclease can be dCasMINI that is coupled to (e.g., fused to) the same gene modulator, which same gene modulator is coupled to the engineered nuclease as disclosed herein.

In some cases, the systems and methods as disclosed herein can modulate (e.g., increase or decrease) an activity level of a target gene (e.g., upon introducing a complex comprising the heterologous polypeptide comprising the engineered nuclease as disclosed herein into a cell or population of cells) or a duration thereof. An activity level can be determined by a suitable functional assay for the target gene in question depending on the functional characteristics of the target gene. For example, an activity level of a target gene that is a mitogen could be determined by measuring cell proliferation; an activity level of a target gene that induces apoptosis could be measured by an annexin V assay or other suitable cell death assay; an activity level of an anti-inflammatory cytokine could be measured by an LPS-induced cytokine release assay.

The systems and methods of the present disclosure can, in some cases, elicit changes in expression and/or activity level of a target gene (e.g., target endogenous gene) that persists for longer than can be achieved with alternative compositions and methods (e.g., suppression via RNAi, e.g., using siRNA). In some cases, persistent modulation of gene expression is advantageous as compared to transient modulation.

In some cases, the systems and methods as disclosed herein can modulate (e.g., increase or decrease) expression and/or activity level of a target gene for at least about 1 hour, at least about 2 hours, at least about 3 hours, at least about 4 hours, at least about 5 hours, at least about 6 hours, at least about 7 hours, at least about 8 hours, at least about 9 hours, at least about 10 hours, at least about 12 hours, at least about 14 hours, at least about 18 hours, at least about 20 hours, at least about 1 day, at least about 2 days, at least about 3 days, at least about 4 days, at least about 5 days, at least about 6 days, at least about 7 days, at least about 8 days, at least about 9 days, at least about 10 days, at least about 14 days, at least about 21 days, at least about 28 days, at least about 5 weeks, at least about 6 weeks, at least about 7 weeks, at least about 8 weeks, at least about 9 weeks, at least about 10 weeks, at least about 12 weeks, at least about 14 weeks, at least about 18 weeks, at least about 20 weeks, at least about 26 weeks, or at least about 5 months, at least about 6 months, at least about 9 months, at least about 12 months, or more.

In some cases the systems and methods as disclosed herein can modulate (e.g., increase or decrease) expression and/or activity level of a target gene (e.g., target endogenous gene) to above a certain threshold for at least or up to about 1 hour, at least or up to about 2 hours, at least or up to about 3 hours, at least or up to about 4 hours, at least or up to about 5 hours, at least or up to about 6 hours, at least or up to about 7 hours, at least or up to about 8 hours, at least or up to about 9 hours, at least or up to about 10 hours, at least or up to about 12 hours, at least or up to about 14 hours, at least or up to about 18 hours, at least or up to about 20 hours, at least or up to about 1 day, at least or up to about 2 days, at least or up to about 3 days, at least or up to about 4 days, at least or up to about 5 days, at least or up to about 6 days, at least or up to about 7 days, at least or up to about 8 days, at least or up to about 9 days, at least or up to about 10 days, at least or up to about 14 days, at least or up to about 21 days, at least or up to about 28 days, at least or up to about 5 weeks, at least or up to about 6 weeks, at least or up to about 7 weeks, at least or up to about 8 weeks, at least or up to about 9 weeks, at least or up to about 10 weeks, at least or up to about 12 weeks, at least or up to about 14 weeks, at least or up to about 18 weeks, at least or up to about 20 weeks, at least or up to about 26 weeks, or at least or up to about 5 months, at least or up to about 6 months, at least or up to about 9 months, or at least or up to about 12 months.

In some cases, the systems and methods as disclosed herein can modulate (e.g., increase or decrease) expression and/or activity level of a target gene (e.g., target endogenous gene) to above a certain threshold for about 1 hour, about 2 hours, about 3 hours, about 4 hours, about 5 hours, about 6 hours, about 7 hours, about 8 hours, about 9 hours, about 10 hours, about 12 hours, about 14 hours, about 18 hours, about 20 hours, about 1 day, about 2 days, about 3 days, about 4 days, about 5 days, about 6 days, about 7 days, about 8 days, about 9 days, about 10 days, about 14 days, about 21 days, about 28 days, about 5 weeks, about 6 weeks, about 7 weeks, about 8 weeks, about 9 weeks, about 10 weeks, about 12 weeks, about 14 weeks, about 18 weeks, about 20 weeks, about 26 weeks, about 5 months, about 6 months, about 9 months, or about 12 months.

In some cases, the engineered polypeptide as disclosed herein (e.g., an engineered nuclease operatively coupled to a gene modulator) can be capable of or can effect enhanced modulation of a target gene, as compared to modulation of the target gene by a control polypeptide (e.g., a control nuclease operatively coupled to the same gene modulator). The control nuclease can be a naturally occurring nuclease (e.g., (i) the polypeptide sequence of SEQ ID NO: 1 or (ii) the polypeptide sequence of any one of the Cas proteins selected from TABLE 2) or any modification thereof (e.g., dCasMINI as disclosed herein).

In some cases, the enhanced modulation of the target gene can be characterized by a change in expression level of the target gene that is greater than that by the control polypeptide. In some examples, such change can be increased expression level of the target gene. The increased expression level of the target gene by the engineered polypeptide as disclosed herein can be greater than that by the control polypeptide, by at least or up to about 1%, at least or up to about 2%, at least or up to about 3%, at least or up to about 4%, at least or up to about 5%, at least or up to about 10%, at least or up to about 15%, at least or up to about 20%, at least or up to about 30%, at least or up to about 40%, at least or up to about 50%, at least or up to about 60%, at least or up to about 70%, at least or up to about 80%, at least or up to about 90%, at least or up to about 100%, at least or up to about 110%, at least or up to about 120%, at least or up to about 150%, at least or up to about 200%, at least or up to about 300%, at least or up to about 400%, or at least or up to about 500%. The increased expression level of the target gene by the engineered polypeptide as disclosed herein can be greater than that by the control polypeptide, by at least or up to about 0.1-fold, at least or up to about 0.2-fold, at least or up to about 0.5-fold, at least or up to about 1-fold, at least or up to about 1.5-fold, at least or up to about 2-fold, at least or up to about 3-fold, at least or up to about 4-fold, at least or up to about 5-fold, at least or up to about 6-fold, at least or up to about 7-fold, at least or up to about 8-fold, at least or up to about 9-fold, at least or up to about 10-fold, at least or up to about 15-fold, at least or up to about 20-fold, at least or up to about 30-fold, at least or up to about 40-fold, or at least or up to about 100-fold. In some examples, such change can be decreased (or reduced) expression level of the target gene. The decreased expression level of the target gene by the engineered polypeptide as disclosed herein can be less than that by the control polypeptide, by at least or up to about 1%, at least or up to about 2%, at least or up to about 3%, at least or up to about 4%, at least or up to about 5%, at least or up to about 10%, at least or up to about 15%, at least or up to about 20%, at least or up to about 30%, at least or up to about 40%, at least or up to about 50%, at least or up to about 60%, at least or up to about 70%, at least or up to about 80%, at least or up to about 90%, at least or up to about 100%, at least or up to about 110%, at least or up to about 120%, at least or up to about 150%, at least or up to about 200%, at least or up to about 300%, at least or up to about 400%, or at least or up to about 500%. The decreased (or reduced) expression level of the target gene by the engineered polypeptide as disclosed herein can be less than that by the control polypeptide, by at least or up to about 0.1-fold, at least or up to about 0.2-fold, at least or up to about 0.5-fold, at least or up to about 1-fold, at least or up to about 1.5-fold, at least or up to about 2-fold, at least or up to about 3-fold, at least or up to about 4-fold, at least or up to about 5-fold, at least or up to about 6-fold, at least or up to about 7-fold, at least or up to about 8-fold, at least or up to about 9-fold, at least or up to about 10-fold, at least or up to about 15-fold, at least or up to about 20-fold, at least or up to about 30-fold, at least or up to about 40-fold, or at least or up to about 100-fold.

In some cases, the enhanced modulation of the target gene can be characterized by a prolonged change in expression level of the target gene (e.g., increased expression or decreased expression above certain threshold as disclosed herein) that is longer than that by the control polypeptide. The prolonged change in the expression level of the target gene by the engineered polypeptide as disclosed herein can be longer than that by the control polypeptide, by at least or up to about 1%, at least or up to about 2%, at least or up to about 3%, at least or up to about 4%, at least or up to about 5%, at least or up to about 10%, at least or up to about 15%, at least or up to about 20%, at least or up to about 30%, at least or up to about 40%, at least or up to about 50%, at least or up to about 60%, at least or up to about 70%, at least or up to about 80%, at least or up to about 90%, at least or up to about 100%, at least or up to about 110%, at least or up to about 120%, at least or up to about 150%, at least or up to about 200%, at least or up to about 300%, at least or up to about 400%, or at least or up to about 500%. The prolonged change in the expression level of the target gene by the engineered polypeptide as disclosed herein can be longer than that by the control polypeptide, by at least or up to about 0.1-fold, at least or up to about 0.2-fold, at least or up to about 0.5-fold, at least or up to about 1-fold, at least or up to about 1.5-fold, at least or up to about 2-fold, at least or up to about 3-fold, at least or up to about 4-fold, at least or up to about 5-fold, at least or up to about 6-fold, at least or up to about 7-fold, at least or up to about 8-fold, at least or up to about 9-fold, at least or up to about 10-fold, at least or up to about 15-fold, at least or up to about 20-fold, at least or up to about 30-fold, at least or up to about 40-fold, or at least or up to about 100-fold. For example, the threshold level can be relative to (i) expression level of the target gene prior to the enhanced modulation, (ii) the greatest increase in the expression level of the target gene for activating the target gene or a portion thereof (e.g., at least 50%, at least 60%, at least 70%, at least 80%, or at least 90% thereof), or (iii) the greatest decrease in the expression level of the target gene for repressing the target gene or a portion thereof (e.g., at least 50%, at least 60%, at least 70%, at least 80%, or at least 90% thereof)In some embodiments, the target gene (e.g., endogenous target gene) can be a disease-causing allele, such as a mutant variant of a wild type allele. The disease can be a genetic disease, such as a hereditary disorder. Non-limiting examples of the genetic disorder can include Duchenne muscular dystrophy (DMD), hemophilia, cystic fibrosis, Huntington's chorea, familial hypercholesterolemia (LDL receptor defect), hepatoblastoma, Wilson's disease, congenital hepatic porphyria, inherited disorders of hepatic metabolism, Lesch Nyhan syndrome, sickle cell anemia, thalassaemias, xeroderma pigmentosum, Fanconi's anemia, retinitis pigmentosa, ataxia telangiectasia, Bloom's syndrome, retinoblastoma, and Tay-Sachs disease. In some cases, the target gene can be a gene encoding a protein. In some cases, the target gene can be a gene regulatory sequence (e.g., promoters, enhancers, repressors, silencers, insulators, cis-regulatory elements, trans-regulatory elements, epigenetic modification (e.g., DNA methylation) sites, etc.) that can influence expression of a gene encoding a protein of interest as provided herein. For example, target gene regulatory sequences can be physically located outside of the transcriptional unit or open reading frame that encodes a product of the target gene.

In some cases, a target gene regulatory sequence does not contain a nucleotide sequence that is exogenous to the subject or host cell. In some embodiments, a target gene regulatory sequence does not contain an engineered or artificially generated or introduced nucleotide sequence.

In some cases, a target gene (e.g., target endogenous gene) is a gene that is over-expressed or under-expressed in a disease or condition. In some embodiments, a target gene is a gene that is over-expressed or under-expressed in a heritable genetic disease.

In some cases, a target gene (e.g., target endogenous gene) is a gene that is over-expressed or under-expressed in a cancer, for example, acute leukemia, astrocytomas, biliary cancer (cholangiocarcinoma), bone cancer, breast cancer, brain stem glioma, bronchioloalveolar cell lung cancer, cancer of the adrenal gland, cancer of the anal region, cancer of the bladder, cancer of the endocrine system, cancer of the esophagus, cancer of the head or neck, cancer of the kidney, cancer of the parathyroid gland, cancer of the penis, cancer of the pleural/peritoneal membranes, cancer of the salivary gland, cancer of the small intestine, cancer of the thyroid gland, cancer of the ureter, cancer of the urethra, carcinoma of the cervix, carcinoma of the endometrium, carcinoma of the fallopian tubes, carcinoma of the renal pelvis, carcinoma of the vagina, carcinoma of the vulva, cervical cancer, chronic leukemia, colon cancer, colorectal cancer, cutaneous melanoma, ependymoma, epidermoid tumors, Ewings sarcoma, gastric cancer, glioblastoma, glioblastoma multiforme, glioma, hematologic malignancies, hepatocellular (liver) carcinoma, hepatoma, Hodgkin's Disease, intraocular melanoma, Kaposi sarcoma, lung cancer, lymphomas, medulloblastoma, melanoma, meningioma, mesothelioma, multiple myeloma, muscle cancer, neoplasms of the central nervous system (CNS), neuronal cancer, small cell lung cancer, non-small cell lung cancer, osteosarcoma, ovarian cancer, pancreatic cancer, pediatric malignancies, pituitary adenoma, prostate cancer, rectal cancer, renal cell carcinoma, sarcoma of soft tissue, schwanoma, skin cancer, spinal axis tumors, squamous cell carcinomas, stomach cancer, synovial sarcoma, testicular cancer, uterine cancer, or tumors and their metastases, including refractory versions of any of the above cancers, or a combination thereof.

Non-limiting examples of a target gene or a gene encoding a protein of interest, as disclosed herein, are included in TABLE 1.

### Guide nucleic acid molecule

Disclosed are guide nucleic acid molecule (e.g., an engineered guide nucleic acid molecule) configured to form a complex with a Cas protein (not part of the present invention). The Cas protein can be a naturally occurring protein. The Cas protein can be an engineered nuclease variant as provided herein. The guide nucleic acid molecule can comprise a spacer sequence exhibiting specific binding to a target polynucleotide sequence operatively coupled to a target gene (e.g., in a cell). The target polynucleotide sequence can be part of the target gene. Alternatively, the target polynucleotide sequence can be upstream (e.g., part of or adjacent to a promoter sequence of the target gene) or downstream of the target gene (e.g., part of or adjacent to a termination sequence of the target gene). The guide nucleic acid molecule can comprise a scaffold sequence for forming the complex with the Cas protein. The spacer sequence and the scaffold sequence can be part of a single polynucleotide sequence (e.g., a single guide nucleic acid molecule, such as sgRNA). Alternatively, the spacer sequence and the scaffold sequence can be separate molecules that are hybridize for forming the complex with the Cas protein.

Without wishing to be bound by theory, the guide nucleic acid molecule as disclosed herein can be operatively coupled to (e.g., can form a functional complex with) one or more Cas proteins, including, but not limited to, Un1Cas12f1, a selected from TABLE 2, or any of the engineered nuclease variant provided throughout the present disclosure (e.g., the polypeptide of SEQ ID NO: 12).

The scaffold sequence as disclosed herein may not be identical to the polynucleotide sequence of SEQ ID NO: 500. The scaffold sequence can comprise at least one deletion, as compared to (e.g., when aligned to) the polynucleotide sequence of SEQ ID NO: 500. Without wishing to be bound by theory, the at least one deletion of the scaffold sequence can be determined by performing a deletion landscape study (e.g., iterative and/or comprehensive deletion) of the control scaffold sequence of SEQ ID NO: 500. The scaffold sequence can comprise at least one mutation, as compared to (e.g., when aligned to) the polynucleotide sequence of SEQ ID NO: 500. Without wishing to be bound by theory, the at least one mutation of the scaffold sequence can be determined by performing a mutation landscape study (e.g., iterative and/or comprehensive mutation) of the control scaffold sequence of SEQ ID NO: 500. The at least one deletion as disclosed herein can be removal of a nucleotide. Alternatively, the at least one deletion can be replacement of a nucleotide with a different nucleotide (e.g., mutation).

In some cases, the scaffold sequence can comprise one or more nucleotide deletions when aligned to (or compared to) the control polynucleotide sequence of SEQ ID NO: 500. The one or more nucleotide deletions can comprise a single deletion. The one or more nucleotide deletions can comprise a plurality of nucleotide deletions, such as at least or up to about 2 deletions, at least or up to about 3 deletions, at least or up to about 4 deletions, at least or up to about 5 deletions, at least or up to about 6 deletions, at least or up to about 7 deletions, at least or up to about 8 deletions, at least or up to about 9 deletions, at least or up to about 10 deletions, at least or up to about 11 deletions, at least or up to about 12 deletions, at least or up to about 13 deletions, at least or up to about 14 deletions, at least or up to about 15 deletions, at least or up to about 16 deletions, at least or up to about 17 deletions, at least or up to about 18 deletions, at least or up to about 19 deletions, at least or up to about 20 deletions, at least or up to about 22 deletions, at least or up to about 24 deletions, at least or up to about 25 deletions, at least or up to about 26 deletions, at least or up to about 28 deletions, at least or up to about 30 deletions, at least or up to about 32 deletions, at least or up to about 34 deletions, at least or up to about 35 deletions, at least or up to about 36 deletions, at least or up to about 38 deletions, at least or up to about 40 deletions, at least or up to about 42 deletions, at least or up to about 44 deletions, at least or up to about 45 deletions, at least or up to about 46 deletions, at least or up to about 48 deletions, at least or up to about 50 deletions, at least or up to about 52 deletions, at least or up to about 54 deletions, at least or up to about 55 deletions, at least or up to about 56 deletions, at least or up to about 58 deletions, at least or up to about 60 deletions, at least or up to about 70 deletions, or at least or up to about 80 deletions. The plurality of nucleotide deletions can be adjacent to each other (e.g., consecutive), when aligned to the polynucleotide sequence of SEQ ID NO: 500. The scaffold sequence can comprise a single consecutive deletion. The scaffold sequence can comprise a plurality of consecutive deletions, in which one consecutive deletion is not directly adjacent to another consecutive deletion when aligned to the polynucleotide sequence of SEQ ID NO: 500.

In some cases, when aligned to the control polynucleotide sequence of SEQ ID NO: 500, the scaffold sequence as disclosed herein can comprise one or more nucleotide deletions in one or more members from the nucleotides 1-10, the nucleotides 11-20, the nucleotides 21-30, the nucleotides 31-40, the nucleotides 41-50, the nucleotides 51-60, the nucleotides 61-70, the nucleotides 71-80, the nucleotides 81-90, the nucleotides 91-100, the nucleotides 101-110, the nucleotides 111-120, the nucleotides 121-130, the nucleotides 131-140, the nucleotides 141-150, and/or the nucleotides 151-159 of SEQ ID NO: 500.

In some cases, when aligned to the control polynucleotide sequence of SEQ ID NO: 500, the scaffold sequence as disclosed herein can comprise one or more nucleotide deletions in the nucleotides 1-25 of the polynucleotide sequence of SEQ ID NO: 500, such as the nucleotides 1-23, the nucleotides 3-23, the nucleotides 5-23, the nucleotides 7-23, the nucleotides 9-23, the nucleotides 11-23, the nucleotides 13-23, the nucleotides 15-23, the nucleotides 17-23, the nucleotides 19-23, and/or the nucleotides 21-23 of the polynucleotide sequence of SEQ ID NO: 500. When aligned to the control polynucleotide sequence of SEQ ID NO: 500, the scaffold sequence as disclosed herein can comprise one or more nucleotide deletions in the nucleotides 1-23, the nucleotides 1-21, the nucleotides 1-19, the nucleotides 1-17, the nucleotides 1-15, the nucleotides 1-13, the nucleotides 1-11, the nucleotides 1-9, the nucleotides 1-7, the nucleotides 1-5, and/or the nucleotides 1-3 of the polynucleotide sequence of SEQ ID NO: 500. When aligned to the control polynucleotide sequence of SEQ ID NO: 500, the scaffold sequence as disclosed herein can comprise one or more nucleotide deletions in the nucleotides 1-5, the nucleotides 6-10, the nucleotides 11-15, the nucleotides 16-20, and/or the nucleotides 21-23 of the polynucleotide sequence of SEQ ID NO: 500. When aligned to the control polynucleotide sequence of SEQ ID NO: 500, the scaffold sequence as disclosed herein can comprise one or more nucleotide deletions in the nucleotide 1, nucleotide 2, nucleotide 3, nucleotide 4, nucleotide 5, nucleotide 6, nucleotide 7, nucleotide 8, nucleotide 9, nucleotide 10, nucleotide 11, nucleotide 12, nucleotide 13, nucleotide 14, nucleotide 15, nucleotide 16, nucleotide 17, nucleotide 18, nucleotide 19, nucleotide 20, nucleotide 21, nucleotide 22, nucleotide 23, nucleotide 24, and/or nucleotide 25 of the polynucleotide sequence of SEQ ID NO: 500.

In some cases, when aligned to the control polynucleotide sequence of SEQ ID NO: 500, the scaffold sequence as disclosed herein can comprise one or more nucleotide deletions in the nucleotides 35-65 of the polynucleotide sequence of SEQ ID NO: 500, such as the nucleotides 35-61, the nucleotides 37-61, the nucleotides 39-61, the nucleotides 41-61, the nucleotides 43-61, the nucleotides 45-61, the nucleotides 47-61, the nucleotides 49-61, the nucleotides 51-61, the nucleotides 53-61, the nucleotides 55-61, the nucleotides 57-61, and/or the nucleotides 59-61 of the polynucleotide sequence of SEQ ID NO: 500. When aligned to the control polynucleotide sequence of SEQ ID NO: 500, the scaffold sequence as disclosed herein can comprise one or more nucleotide deletions in the nucleotides 35-61, the nucleotides 35-59, the nucleotides 35-57, the nucleotides 35-55, the nucleotides 35-53, the nucleotides 35-51, the nucleotides 35-49, the nucleotides 35-47, the nucleotides 35-45, the nucleotides 35-43, the nucleotides 35-41, the nucleotides 35-39, and/or the nucleotides 35-37 of the polynucleotide sequence of SEQ ID NO: 500. When aligned to the control polynucleotide sequence of SEQ ID NO: 500, the scaffold sequence as disclosed herein can comprise one or more nucleotide deletions in the nucleotide 35, nucleotide 36, nucleotide 37, nucleotide 38, nucleotide 39, nucleotide 40, nucleotide 41, nucleotide 42, nucleotide 43, nucleotide 44, nucleotide 45, nucleotide 46, nucleotide 47, nucleotide 48, nucleotide 49, nucleotide 50, nucleotide 51, nucleotide 52, nucleotide 53, nucleotide 54, nucleotide 55, nucleotide 56, nucleotide 57, nucleotide 58, nucleotide 59, nucleotide 60, nucleotide 61, nucleotide 62, nucleotide 63, nucleotide 64, and/or nucleotide 65 of the polynucleotide sequence of SEQ ID NO: 500.

In some cases, when aligned to the control polynucleotide sequence of SEQ ID NO: 500, the scaffold sequence as disclosed herein can comprise one or more nucleotide deletions in the nucleotides 135-150 of the polynucleotide sequence of SEQ ID NO: 500, such as the nucleotides 136-149, the nucleotides 137-149, the nucleotides 139-149, the nucleotides 141-149, the nucleotides 143-149, the nucleotides 145-149, and/or the nucleotides 147-149 nucleotides of the polynucleotide sequence of SEQ ID NO: 500. When aligned to the control polynucleotide sequence of SEQ ID NO: 500, the scaffold sequence as disclosed herein can comprise one or more nucleotide deletions in the nucleotides 136-149, the nucleotides 136-147, the nucleotides 136-145, the nucleotides 136-143, the nucleotides 136-141, the nucleotides 136-139, and/or the nucleotides 136-137 of the polynucleotide sequence of SEQ ID NO: 500. When aligned to the control polynucleotide sequence of SEQ ID NO: 500, the scaffold sequence as disclosed herein can comprise one or more nucleotide deletions in the nucleotide 135, nucleotide 136, nucleotide 137, nucleotide 138, nucleotide 139, nucleotide 140, nucleotide 141, nucleotide 142, nucleotide 143, nucleotide 144, nucleotide 145, nucleotide 146, nucleotide 147, nucleotide 148, nucleotide 149, and/or nucleotide 150 of the polynucleotide sequence of SEQ ID NO: 500.

In some cases, when aligned to the control polynucleotide sequence of SEQ ID NO: 500, the scaffold sequence as disclosed herein can comprise one or more nucleotide deletions in the nucleotides 136-151 of the polynucleotide sequence of SEQ ID NO: 500. In some cases, the scaffold sequence as disclosed herein can comprise one or more nucleotide deletions in the nucleotide T136, T137, C138, A139, T140, T141, T142, G143, A144, A145, T146, G147, A148, A149, G150, and/or G151 of the polynucleotide sequence of SEQ ID NO: 500. In some cases, the scaffold sequence as disclosed herein can comprise one or more nucleotide deletions (e.g., at least 1, at least 2, at least 3, at least 4, at least 5, at least 6, at least 7, at least 8, at least 9, at least 10, at least 11, or all 12 of) in the nucleotide T136, T137, C138, A139, T140, T141, T142, A144, A145, T146, A148, and/or A149 of the polynucleotide sequence of SEQ ID NO: 500. In some cases, the scaffold sequence as disclosed herein can comprise at least or up to about 1 nucleotide, at least or up to about 2 nucleotides, at least or up to about 3 nucleotides, or all 4 nucleotides selected from the group consisting of G143, G147, G150, and G151, when aligned to the polynucleotide sequence of SEQ ID NO: 500.

In some cases, the scaffold sequence as disclosed herein is not identical to the polynucleotide sequence of a combination of SEQ ID NO: 549 and SEQ ID NO: 550. For example, the polynucleotides of SEQ ID NO: 549 and SEQ ID NO: 550 may be coupled to the 5' end the 3' end of a spacer sequence, respectively, to be used as a control sgRNA molecule to compare the activity of any of the scaffold sequence provided herein. In some cases, the scaffold sequence as disclosed herein is not identical to the polynucleotide sequence of a combination of SEQ ID NO: 551 and SEQ ID NO: 552. For example, the polynucleotides of SEQ ID NO: 551 and SEQ ID NO: 552 may be coupled to the 5' end the 3' end of a spacer sequence, respectively, to be used as a control sgRNA molecule to compare the activity of any of the scaffold sequence provided herein.

In some cases, the scaffold sequence (e.g., a consecutive polynucleotide sequence of the scaffold sequence) can be characterized by exhibiting at least or up to about 60%, at least or up to about 65%, at least or up to about 70%, at least or up to about 71%, at least or up to about 72%, at least or up to about 73%, at least or up to about 74%, at least or up to about 75%, at least or up to about 76%, at least or up to about 77%, at least or up to about 78%, at least or up to about 79%, at least or up to about 80%, at least or up to about 81%, at least or up to about 82%, at least or up to about 83%, at least or up to about 84%, at least or up to about 85%, at least or up to about 86%, at least or up to about 87%, at least or up to about 88%, at least or up to about 89%, at least or up to about 90%, at least or up to about 91%, at least or up to about 92%, at least or up to about 93%, at least or up to about 94%, at least or up to about 95%, at least or up to about 96%, at least or up to about 97%, at least or up to about 98%, at least or up to about 99, or substantially 100% sequence identity (or complementarity) to the polynucleotide sequence of a member selected from TABLE 6B, TABLE 7B, and TABLE 8B.

In some cases the member can be selected from the group consisting of SEQ ID NOs: 503-152, 519, 524, 528, and 553. In some cases, the member can be selected from the group consisting of SEQ ID NOs: 555, 557, 558, 568, 569, 578, and 580. In some cases, the member can be selected from the group consisting of SEQ ID NOs: 555, 557, 568, 569, 576, 577, 578, 580, 593, 519, and 528.

In some cases, the length of the scaffold sequence can be at least or up to about 80 nucleotides, at least or up to about 85 nucleotides, at least or up to about 90 nucleotides, at least or up to about 91 nucleotides, at least or up to about 92 nucleotides, at least or up to about 93 nucleotides, at least or up to about 94 nucleotides, at least or up to about 95 nucleotides, at least or up to about 96 nucleotides, at least or up to about 97 nucleotides, at least or up to about 98 nucleotides, at least or up to about 99 nucleotides, at least or up to about 100 nucleotides, at least or up to about 101 nucleotides, at least or up to about 102 nucleotides, at least or up to about 103 nucleotides, at least or up to about 104 nucleotides, at least or up to about 105 nucleotides, at least or up to about 106 nucleotides, at least or up to about 107 nucleotides, at least or up to about 108 nucleotides, at least or up to about 109 nucleotides, at least or up to about 110 nucleotides, at least or up to about 112 nucleotides, at least or up to about 114 nucleotides, at least or up to about 115 nucleotides, at least or up to about 116 nucleotides, at least or up to about 118 nucleotides, at least or up to about 120 nucleotides, at least or up to about 122 nucleotides, at least or up to about 124 nucleotides, at least or up to about 125 nucleotides, at least or up to about 126 nucleotides, at least or up to about 128 nucleotides, at least or up to about 130 nucleotides, at least or up to about 135 nucleotides, at least or up to about 140 nucleotides, at least or up to about 145 nucleotides, at least or up to about 150 nucleotides, at least or up to about 155 nucleotides, or at least or up to about 160 nucleotides.

In some cases, the scaffold sequence can comprise a consecutive polynucleotide sequence exhibiting at least or up to about 60%, at least or up to about 65%, at least or up to about 70%, at least or up to about 71%, at least or up to about 72%, at least or up to about 73%, at least or up to about 74%, at least or up to about 75%, at least or up to about 76%, at least or up to about 77%, at least or up to about 78%, at least or up to about 79%, at least or up to about 80%, at least or up to about 81%, at least or up to about 82%, at least or up to about 83%, at least or up to about 84%, at least or up to about 85%, at least or up to about 86%, at least or up to about 87%, at least or up to about 88%, at least or up to about 89%, at least or up to about 90%, at least or up to about 91%, at least or up to about 92%, at least or up to about 93%, at least or up to about 94%, at least or up to about 95%, at least or up to about 96%, at least or up to about 97%, at least or up to about 98%, at least or up to about 99, or substantially 100% sequence identity (or complementarity) to the polynucleotide sequence of SEQ ID NO: 597 or SEQ ID NO: 598. The consecutive polynucleotide sequence of the scaffold sequence can have a length of at least or up to about 15 nucleotides, at least or up to about 16 nucleotides, at least or up to about 17 nucleotides, at least or up to about 18 nucleotides, at least or up to about 19 nucleotides, at least or up to about 20 nucleotides, at least or up to about 21 nucleotides, at least or up to about 22 nucleotides, at least or up to about 23 nucleotides, at least or up to about 24 nucleotides, at least or up to about 25 nucleotides, at least or up to about 26 nucleotides, at least or up to about 27 nucleotides, at least or up to about 28 nucleotides, at least or up to about 29 nucleotides, at least or up to about 30 nucleotides, at least or up to about 31 nucleotides, at least or up to about 32 nucleotides, at least or up to about 33 nucleotides, at least or up to about 34 nucleotides, at least or up to about 35 nucleotides, at least or up to about 36 nucleotides, at least or up to about 37 nucleotides, at least or up to about 38 nucleotides, at least or up to about 39 nucleotides, or at least or up to about 40 nucleotides. The consecutive polynucleotide sequence can be disposed at the N-terminus or at the C-terminus of the scaffold sequence. The consecutive polynucleotide sequence can be disposed at the N-terminal 50%, at the N-terminal 45%, at the N-terminal 40%, at the N-terminal 35%, at the N-terminal 30%, at the N-terminal 25%, at the N-terminal 20%, at the N-terminal 15%, or at the N-terminal 10% of the scaffold sequence. Alternatively, the consecutive polynucleotide sequence can be disposed at the C-terminal 50%, at the C-terminal 45%, at the C-terminal 40%, at the C-terminal 35%, at the C-terminal 30%, at the C-terminal 25%, at the C-terminal 20%, at the C-terminal 15%, or at the C-terminal 10% of the scaffold sequence. The consecutive polynucleotide sequence can be disposed between the scaffold sequence.

In some cases, the spacer sequence of the guide nucleic acid molecule can have a length of at least or up to about 12 nucleotides, at least or up to about 13 nucleotides, at least or up to about 14 nucleotides, at least or up to about 15 nucleotides, at least or up to about 16 nucleotides, at least or up to about 17 nucleotides, at least or up to about 18 nucleotides, at least or up to about 19 nucleotides, at least or up to about 20 nucleotides, at least or up to about 21 nucleotides, or at least or up to about 22 nucleotides.

In some cases, the guide nucleic acid molecule can have a length of at least or up to about 80 nucleotides, at least or up to about 85 nucleotides, at least or up to about 90 nucleotides, at least or up to about 95 nucleotides, at least or up to about 96 nucleotides, at least or up to about 97 nucleotides, at least or up to about 98 nucleotides, at least or up to about 99 nucleotides, at least or up to about 100 nucleotides, at least or up to about 101 nucleotides, at least or up to about 102 nucleotides, at least or up to about 103 nucleotides, at least or up to about 104 nucleotides, at least or up to about 105 nucleotides, at least or up to about 106 nucleotides, at least or up to about 107 nucleotides, at least or up to about 108 nucleotides, at least or up to about 109 nucleotides, at least or up to about 110 nucleotides, at least or up to about 111 nucleotides, at least or up to about 112 nucleotides, at least or up to about 113 nucleotides, at least or up to about 114 nucleotides, at least or up to about 115 nucleotides, at least or up to about 116 nucleotides, at least or up to about 117 nucleotides, at least or up to about 118 nucleotides, at least or up to about 119 nucleotides, at least or up to about 120 nucleotides, at least or up to about 121 nucleotides, at least or up to about 122 nucleotides, at least or up to about 123 nucleotides, at least or up to about 124 nucleotides, at least or up to about 125 nucleotides, at least or up to about 130 nucleotides, at least or up to about 135 nucleotides, at least or up to about 140 nucleotides, at least or up to about 145 nucleotides, at least or up to about 150 nucleotides, at least or up to about 155 nucleotides, or at least or up to about 160 nucleotides.

In some examples, the consecutive polynucleotide sequence of the scaffold sequence can be at least about 20, at least about 25, or at least about 30 nucleotides long, and such consecutive polynucleotide sequence can exhibit at least or up to about 60%, at least or up to about 65%, at least or up to about 70%, at least or up to about 71%, at least or up to about 72%, at least or up to about 73%, at least or up to about 74%, at least or up to about 75%, at least or up to about 76%, at least or up to about 77%, at least or up to about 78%, at least or up to about 79%, at least or up to about 80%, at least or up to about 81%, at least or up to about 82%, at least or up to about 83%, at least or up to about 84%, at least or up to about 85%, at least or up to about 86%, at least or up to about 87%, at least or up to about 88%, at least or up to about 89%, at least or up to about 90%, at least or up to about 91%, at least or up to about 92%, at least or up to about 93%, at least or up to about 94%, at least or up to about 95%, at least or up to about 96%, at least or up to about 97%, at least or up to about 98%, at least or up to about 99, or substantially 100% sequence identity (or complementarity) to the polynucleotide sequence of (i) the N-terminal 30 nucleotide sequence or (ii) the C-terminal 30 nucleotide sequence of a member selected from TABLE 6B, TABLE 7B, and TABLE 8B (e.g., one or more members from SEQ ID NOs: 555, 557*,* 568, 569, 576, 577, 578, 580, 593, 519, and 528)

### Heterologous polynucleotide

In some cases, a target gene (not part of the invention) can be targeted by the systems of the present disclosure (e.g., comprising a variant of the engineered nuclease that retains at least a portion of its nuclease activity) to edit the target gene. In some cases, a complex comprising (i) the heterologous polypeptide that comprises the engineered nuclease as disclosed herein and (ii) a guide nucleic acid (e.g., sgRNA) can recognize, bind to, and create a nick (one strand) or a break (two strands) in the target gene, e.g., at or near a target sequence of complex within the target gene. In some cases, the nick or break can be repaired via Non-Homologous End Joining (NHEJ). In some cases, the nick or break can be repaired via Homology-Directed Repair (HDR) or via Homologous Recombination (HR), with a polynucleotide modification template (e.g., a donor template, such as a donor DNA template). In some examples, a heterologous polynucleotide modification template encoding a gene of interest can be provided to the cell, such that the gene of interest can be inserted into the target gene, e.g., for a gene replacement therapy.

In some cases, the systems and compositions of the present disclosure a heterologous polynucleotide (e.g., encoding a gene of interest, such as one or more genes selected from TABLE 1) that is introduced to the cell without being interested into a genome of the cell via action of the engineered nuclease of the present disclosure. In some cases, such heterologous polynucleotide encoding the gene of interest can be interested into the genome of the cell via other means, e.g., via adeno-associated virus vectors (e.g., AAV2 or AAV8). Alternatively, such heterologous polynucleotide encoding the gene of interest may be introduced to the intracellular portion of the cell and remain achromosomal (e.g., as an achromosomal plasmid).

Thus, the systems and compositions can comprise the non-disease causing wild type or variant of the target gene, as abovementioned. Alternatively or in addition to, the systems and compositions can comprise a heterologous polynucleotide sequence encoding (or comprising) at least the non-disease causing wild type or variant of the target gene (e.g., that of the endogenous target gene) as disclosed herein.

### Composition

Disclosed is a composition comprising at least a portion of the system as described, e.g., (i) the heterologous polypeptide comprising the engineered nuclease or a heterologous polynucleotide encoding the heterologous polypeptide and/or (ii) the guide nucleic acid or a heterologous polynucleotide encoding the guide nucleic acid, as disclosed herein, for use in any of the methods as disclosed herein (not part of the invention). The subject composition can be usable for modifying a cell in vitro, ex vivo, or in vivo. The subject composition can be usable for treating or enhancing a condition of a subject, as disclosed herein.

The composition as disclosed herein can comprise an active ingredient (e.g., the heterologous polypeptide comprising the engineered nuclease, the guide nucleic acid, etc.) and optionally an additional ingredient (e.g., excipient). If necessary and/or desirable, the composition can be divided, shaped and/or packaged into a desired single- or multi-dose unit or single-or multi-implantation unit.

In some cases, the composition can comprise one or more heterologous polynucleotides encoding the active ingredients as disclosed herein. When there are different members within the active ingredients, each member can be encoded by a different heterologous polynucleotide. Alternatively, two or more (e.g., all of) the ingredients can be encoded by a single heterologous polynucleotide. In some cases, a single heterologous polynucleotide an encode (i) the heterologous polypeptide comprising the engineered nuclease (e.g., dCas-transcriptional effector fusion protein, such as dCas-KRAB, dCas-DNMT, dCas-ADA) and (ii) one or more guide nucleic acids (e.g., at least 1, at least 2, at least 3, at least 4, at least 5, or more guide nucleic acids) for targeting specific region(s) or sequence(s) of the target gene.

The one or more heterologous polynucleotides can further comprise one or more promoters (or one or more transcriptional control elements, as used interchangeably herein). Different active ingredients encoded by the one or more heterologous polynucleotides can be under the control of the same promoter or different promoters. A promoter as disclosed herein can be active in a eukaryotic, mammalian, non-human mammalian or human cell. The promoter can be an inducible or constitutively active promoter. Alternatively or additionally, the promoter can be tissue or cell specific. Non-limiting examples of suitable eukaryotic promoters (i.e. promoters functional in a eukaryotic cell) can include those from cytomegalovirus (CMV) immediate early, herpes simplex virus (HSV) thymidine kinase, early and late SV40, long terminal repeats (LTRs) from retrovirus, human elongation factor-1 promoter (EF1), a hybrid construct comprising the cytomegalovirus (CMV) enhancer fused to the chicken beta-active promoter (CAG), murine stem cell virus promoter (MSCV), phosphoglycerate kinase-1 locus promoter (PGK) and mouse metallothionein-I. The promoter can be a fungi promoter. The promoter can be a plant promoter. A database of plant promoters can be found (e.g., PlantProm). The expression vector may also contain a ribosome binding site for translation initiation and a transcription terminator. The expression vector may also include appropriate sequences for amplifying expression. In some cases, a promoter as disclosed herein can be a promoter specific for any of the tissues provided herein, or a promoter specific for any of the cell types provided herein.

A heterologous polynucleotide of the one or more heterologous polynucleotides (e.g., the single heterologous polynucleotide) can have a size of at least or up to about 2.5 kilobases, at least or up to about 2.6 kilobases, at least or up to about 2.7 kilobases, at least or up to about 2.8 kilobases, at least or up to about 2.9 kilobases, at least or up to about 3.0 kilobases, at least or up to about 3.1 kilobases, at least or up to about 3.2 kilobases, at least or up to about 3.3 kilobases, at least or up to about 3.4 kilobases, at least or up to about 3.5 kilobases, at least or up to about 3.6 kilobases, at least or up to about 3.7 kilobases, at least or up to about 3.8 kilobases, at least or up to about 3.9 kilobases, at least or up to about 4.0 kilobases, at least or up to about 4.1 kilobases, at least or up to about 4.2 kilobases, at least or up to about 4.3 kilobases, at least or up to about 4.4 kilobases, at least or up to about 4.5 kilobases, at least or up to about 4.6 kilobases, at least or up to about 4.7 kilobases, at least or up to about 4.8 kilobases, at least or up to about 4.9 kilobases, at least or up to about 5.0 kilobases, at least or up to about 5.5 kilobases, at least or up to about 6.0 kilobases, at least or up to about 6.5 kilobases, at least or up to about 7.0 kilobases, at least or up to about 7.5 kilobases, at least or up to about 8.0 kilobases, at least or up to about 9.0 kilobases, or at least or up to about 10 kilobases. In some cases, the heterologous polynucleotide of the one or more heterologous polynucleotides (e.g., the single heterologous polynucleotide) can have a size of between about 3 kilobases and about 5 kilobases, between about 3 kilobases and about 4.8 kilobases, between about 3 kilobases and about 4.6 kilobases, between about 3 kilobases and about 4.4 kilobases, between about 3 kilobases and about 4.2 kilobases, between about 3 kilobases and about 4.0 kilobases, between about 3 kilobases and about 3.5 kilobases, between about 3.5 kilobases and about 5 kilobases, between about 3.5 kilobases and about 4.8 kilobases, between about 3.5 kilobases and about 4.6 kilobases, between about 3.5 kilobases and about 4.4 kilobases, between about 3.5 kilobases and about 4.2 kilobases, between about 3.5 kilobases and about 4 kilobases, between about 4 kilobases and about 5 kilobases, between about 4 kilobases and about 4.9 kilobases, between about 4 kilobases and about 4.8 kilobases, between about 4 kilobases and about 4.7 kilobases, between about 4 kilobases and about 4.6 kilobases, between about 4 kilobases and about 4.5 kilobases, between about 4 kilobases and about 4.4 kilobases, between about 4 kilobases and about 4.3 kilobases, between about 4 kilobases and about 4.2 kilobases, or between about 4 kilobases and about 4.1 kilobases.

A vector (or an expression cassette) can encode at least (i) a Cas protein and (ii) a guide nucleic acid molecule comprising a spacer sequence and a scaffold sequence, as provided herein. The vector can comprise a first polynucleotide sequence encoding the Cas protein, a second polynucleotide sequencing encoding the scaffold sequence, and/or a third polynucleotide sequence encoding the scaffold sequence. A sum of a length of the first polynucleotide sequence and a length of the second polynucleotide sequence combined can be at least or up to about 1400 nucleotide, at least or up to about 1420 nucleotide, at least or up to about 1440 nucleotide, at least or up to about 1450 nucleotide, at least or up to about 1460 nucleotide, at least or up to about 1480 nucleotide, at least or up to about 1500 nucleotide, at least or up to about 1520 nucleotide, at least or up to about 1540 nucleotide, at least or up to about 1550 nucleotide, at least or up to about 1560 nucleotide, at least or up to about 1580 nucleotide, at least or up to about 1600 nucleotide, at least or up to about 1620 nucleotide, at least or up to about 1640 nucleotide, at least or up to about 1650 nucleotide, at least or up to about 1660 nucleotide, at least or up to about 1680 nucleotide, at least or up to about 1700 nucleotide, at least or up to about 1720 nucleotide, at least or up to about 1740 nucleotide, or at least or up to about 1750 nucleotides. In some embodiments, the sum of the length of the first polynucleotide sequence and the length of the second polynucleotide sequence combined can be less than 1746 nucleotides, less than 1737 nucleotides, or less than 1720 nucleotides.

In some cases, the length of the first polynucleotide sequence can be at least or up to about 1400 nucleotides, at least or up to about 1420 nucleotides, at least or up to about 1440 nucleotides, at least or up to about 1450 nucleotides, at least or up to about 1460 nucleotides, at least or up to about 1480 nucleotides, at least or up to about 1500 nucleotides, at least or up to about 1520 nucleotides, at least or up to about 1540 nucleotides, at least or up to about 1550 nucleotides, at least or up to about 1560 nucleotides, at least or up to about 1580 nucleotides, at least or up to about 1600 nucleotides, at least or up to about 1620 nucleotides, at least or up to about 1640 nucleotides, at least or up to about 1650 nucleotides, at least or up to about 1660 nucleotides, at least or up to about 1680 nucleotides, or at least or up to about 1700 nucleotides.

In some cases, the length of the second polynucleotide sequence can be at least or up to about 80 nucleotides, at least or up to about 85 nucleotides, at least or up to about 90 nucleotides, at least or up to about 91 nucleotides, at least or up to about 92 nucleotides, at least or up to about 93 nucleotides, at least or up to about 94 nucleotides, at least or up to about 95 nucleotides, at least or up to about 96 nucleotides, at least or up to about 97 nucleotides, at least or up to about 98 nucleotides, at least or up to about 99 nucleotides, at least or up to about 100 nucleotides, at least or up to about 101 nucleotides, at least or up to about 102 nucleotides, at least or up to about 103 nucleotides, at least or up to about 104 nucleotides, at least or up to about 105 nucleotides, at least or up to about 106 nucleotides, at least or up to about 107 nucleotides, at least or up to about 108 nucleotides, at least or up to about 109 nucleotides, at least or up to about 110 nucleotides, at least or up to about 112 nucleotides, at least or up to about 114 nucleotides, at least or up to about 115 nucleotides, at least or up to about 116 nucleotides, at least or up to about 118 nucleotides, at least or up to about 120 nucleotides, at least or up to about 122 nucleotides, at least or up to about 124 nucleotides, at least or up to about 125 nucleotides, at least or up to about 126 nucleotides, at least or up to about 128 nucleotides, at least or up to about 130 nucleotides, at least or up to about 135 nucleotides, at least or up to about 140 nucleotides, at least or up to about 145 nucleotides, at least or up to about 150 nucleotides, at least or up to about 155 nucleotides, or at least or up to about 160 nucleotides.

In some cases, sum of the length of the first polynucleotide sequence and the length of the second polynucleotide sequence combined may be sufficiently small, such that the vector encoding at least the Cas protein and the guide nucleic acid molecule can be (i) small/compact and/or (ii) have enough room for additional cargo (e.g., gene modulator(s) operatively coupled to the Cas protein, or the heterologous polynucleotide as provided herein). Even with the small/compact size of the vector, a complex comprising the Cas protein and the guide nucleic acid molecule encoded by the vector may be functional. In some cases, the complex encoded by the vector can be functionally active to bind a target polynucleotide sequence and edit (e.g., cleave, delete nucleotide(s), add nucleotide(s), edit base(s), etc.) at least a portion of the target polynucleotide sequence. In some cases, the complex encoded by the vector can be functionally active to effect modulated expression level of the target gene in the cell. Accordingly, (A1) the modulated expression level of the target gene (alternatively or in addition to, the activity level thereof) by the complex can be comparable to or superior than (A2) that by a control complex comprising the Cas protein and a control guide nucleic acid molecule.

In some cases, (A1) the modulated expression level of the target gene by the complex can be comparable to (A2), such that (A1) does not differ from (A2) by no more than 50%, no more than 45%, no more than 40%, no more than 35%, no more than 30%, no more than 25%, no more than 20%, no more than 15%, no more than 10%, no more than 8%, no more than 6%, no more than 5%, no more than 4%, no more than 3%, no more than 2%, or no more than 1% of (A2).

In some cases, the expression level of the target gene can be activated by the engineered nuclease variant and/or guide nucleic acid molecule disclosed herein, and (A1) the modulated expression level of the target gene by the complex can be superior than (A2), such that (A1) is greater than (A2) by at least or up to about 1%, at least or up to about 2%, at least or up to about 5%, at least or up to about 10%, at least or up to about 15%, at least or up to about 20%, at least or up to about 30%, at least or up to about 40%, at least or up to about 50%, at least or up to about 60%, at least or up to about 70%, at least or up to about 80%, at least or up to about 90%, at least or up to about 100%, at least or up to about 150%, at least or up to about 200%, at least or up to about 250%, at least or up to about 300%, at least or up to about 350%, at least or up to about 400%, at least or up to about 450%, or at least or up to about 500% of (A2), or such that (A1) is greater than (A2) by at least or up to about 0.1-fold, at least or up to about 0.2-fold, at least or up to about 0.3-fold, at least or up to about 0.4-fold, at least or up to about 0.5-fold, at least or up to about 1-fold, at least or up to about 1.5-fold, at least or up to about 2-fold, at least or up to about 3-fold, at least or up to about 4-fold, at least or up to about 5-fold, at least or up to about 6-fold, at least or up to about 7-fold, at least or up to about 8-fold, at least or up to about 9-fold, or at least or up to about 10-fold as compared to (A2).

In some cases, the expression level of the target gene can be reduced (e.g., repressed) by the engineered nuclease variant and/or guide nucleic acid molecule disclosed herein, and (A1) the modulated expression level of the target gene by the complex can be superior than (A2), such that (A1) is less than (A2) by at least or up to about 1%, at least or up to about 2%, at least or up to about 5%, at least or up to about 10%, at least or up to about 15%, at least or up to about 20%, at least or up to about 30%, at least or up to about 40%, at least or up to about 50%, at least or up to about 60%, at least or up to about 70%, at least or up to about 80%, at least or up to about 90%, at least or up to about 100%, at least or up to about 150%, at least or up to about 200%, at least or up to about 250%, at least or up to about 300%, at least or up to about 350%, at least or up to about 400%, at least or up to about 450%, or at least or up to about 500% of (A2), or such that (A1) is less than (A2) by at least or up to about 0.1-fold, at least or up to about 0.2-fold, at least or up to about 0.3-fold, at least or up to about 0.4-fold, at least or up to about 0.5-fold, at least or up to about 1-fold, at least or up to about 1.5-fold, at least or up to about 2-fold, at least or up to about 3-fold, at least or up to about 4-fold, at least or up to about 5-fold, at least or up to about 6-fold, at least or up to about 7-fold, at least or up to about 8-fold, at least or up to about 9-fold, or at least or up to about 10-fold as compared to (A2).

In some cases, the control guide nucleic acid molecule can be longer than the guide nucleic acid molecule encoded by the vector disclosed herein. A control scaffold sequence of the control guide nucleic acid molecule can be longer than the scaffold sequence of the guide nucleic acid molecule encoded by the vector, by at least or up to about 1 nucleotide, at least or up to about 2 nucleotides, at least or up to about 5 nucleotides, at least or up to about 10 nucleotides, at least or up to about 15 nucleotides, at least or up to about 20 nucleotides, at least or up to about 25 nucleotides, at least or up to about 30 nucleotides, at least or up to about 35 nucleotides, at least or up to about 40 nucleotides, at least or up to about 45 nucleotides, at least or up to about 50 nucleotides, at least or up to about 55 nucleotides, at least or up to about 60 nucleotides, at least or up to about 65 nucleotides, at least or up to about 70 nucleotides, at least or up to about 75 nucleotides, or at least or up to about 80 nucleotides. For example, the control guide nucleic acid molecule can comprise the polypeptide sequence of SEQ ID NO: 10.

A method of delivery of the one or more heterologous polynucleotides provided herein to the cell can involve viral delivery methods or non-viral delivery methods. Thus, the one or more heterologous polynucleotides can be one or more viral vectors (e.g., one or more AAV vectors). Alternatively, the one or more heterologous polynucleotides can be non-viral vectors that are complexed with or encapsulated by non-viral delivery moieties, such as cationic lipids and/or lipid particles (e.g., lipid nanoparticles (LNP)).

Methods of non-viral delivery of nucleic acids can include lipofection, nucleofection, microinjection, biolistics, virosomes, liposomes, immunoliposomes, polycation or lipid:nucleic acid conjugates, naked DNA, artificial virions, and agent-enhanced uptake of DNA. Cationic and neutral lipids that are suitable for efficient receptor-recognition lipofection of polynucleotides can be used. Delivery can be to cells (e.g., in vitro or ex vivo administration) or target tissues (e.g., in vivo administration).

RNA or DNA viral based systems can be used to target specific cells in the body and trafficking the viral payload to the nucleus of the cell. Viral vectors can be administered directly (in vivo), or they can be used to treat cells in vitro, and the modified cells can optionally be administered (ex vivo). Viral based systems can include retroviral, lentivirus, adenoviral, adeno-associated and herpes simplex virus vectors for gene transfer. Integration in the host genome can occur with the retrovirus, lentivirus, and adeno-associated virus gene transfer methods, which can result in long term expression of the inserted transgene. High transduction efficiencies can be observed in many different cell types and target tissues.

The tropism of a retrovirus can be altered by incorporating foreign envelope proteins, expanding the potential target population of target cells. Lentiviral vectors are retroviral vectors that can transduce or infect non-dividing cells and produce high viral titers. Selection of a retroviral gene transfer system can depend on the target tissue. Retroviral vectors can comprise cis-acting long terminal repeats with packaging capacity for up to 6-10 kb of foreign sequence. The minimum cis-acting LTRs can be sufficient for replication and packaging of the vectors, which can be used to integrate the therapeutic gene into the target cell to provide permanent transgene expression. Retroviral vectors can include those based upon murine leukemia virus (MuLV), gibbon ape leukemia virus (GaLV), Simian Immuno deficiency virus (SIV), human immuno deficiency virus (HIV), and combinations thereof.

An adenoviral-based systems can be used. Adenoviral-based systems can lead to transient expression of the transgene. Adenoviral based vectors can have high transduction efficiency in cells and may not require cell division. High titer and levels of expression can be obtained with adenoviral based vectors. Adeno-associated virus ("AAV") vectors can be used to transduce cells with target nucleic acids, e.g., in the in vitro production of nucleic acids and peptides, and for in vivo and ex vivo gene therapy procedures.

Packaging cells can be used to form virus particles capable of infecting a host cell. Such cells can include 293 cells, (e.g., for packaging adenovirus), and Psi2 cells or PA317 cells (e.g., for packaging retrovirus). Viral vectors can be generated by producing a cell line that packages a nucleic acid vector into a viral particle. The vectors can contain the minimal viral sequences required for packaging and subsequent integration into a host. The vectors can contain other viral sequences being replaced by an expression cassette for the polynucleotide(s) to be expressed. The missing viral functions can be supplied in trans by the packaging cell line. For example, AAV vectors can comprise ITR sequences from the AAV genome which are required for packaging and integration into the host genome. Viral DNA can be packaged in a cell line, which can contain a helper plasmid encoding the other AAV genes, namely rep and cap, while lacking ITR sequences. The cell line can also be infected with adenovirus as a helper. The helper virus can promote replication of the AAV vector and expression of AAV genes from the helper plasmid. Contamination with adenovirus can be reduced by, e.g., heat treatment to which adenovirus is more sensitive than AAV.

A host cell can be transiently or non-transiently transfected with one or more vectors described herein. A cell can be transfected as it naturally occurs in a subject. A cell can be taken or derived from a subject and transfected. A cell can be derived from cells taken from a subject, such as a cell line. In some embodiments, a cell transfected with one or more vectors described herein is used to establish a new cell line comprising one or more vector-derived sequences. In some embodiments, a cell transiently transfected with the compositions of the disclosure (such as by transient transfection of one or more vectors, or transfection with RNA), and modified through the activity of the heterologous polypeptide comprising the engineered nuclease as disclosed herein, is used to establish a new cell line comprising cells containing the modification but lacking any other exogenous sequence.

Any suitable vector compatible with the host cell can be used with the methods of the disclosure. Non-limiting examples of vectors for eukaryotic host cells include pXT1, pSG5 (Stratagene^{™}), pSVK3, pBPV, pMSG, and pSVLSV40 (Pharmacia^{™}).

The additional ingredient of the composition as disclosed herein may comprise an excipient. Non-limiting examples of the excipient can include solvents, dispersion media, diluents, or other liquid vehicles, dispersion or suspension aids, surface active agents, isotonic agents, thickening or emulsifying agents, preservatives, lipidoids, liposomes, lipid nanoparticles, polymers, lipoplexes, core-shell nanoparticles, peptides, proteins, hyaluronidase, nanoparticle mimics, inert diluents, buffering agents, lubricating agents, oils, and combinations thereof. In some examples, the composition as disclosed herein can include one or more excipients, each in an amount that together increases the stability of (i) the heterologous polypeptide or the heterologous gene encoding thereof and/or (ii) cells or modified cells.

Disclosed is a kit comprising such composition and instructions directing (i) contacting the cell with the composition (e.g., in vitro, ex vivo, or in vivo), or (ii) administration of cells comprising any one of the compositions disclosed herein to a subject (not part of the invention). The subject may have or may be suspected of having a condition, such as a hereditary disease.

In some cases, any of the compositions as disclosed herein, can be administered to the subject via orally, intraperitoneally, intravenously, intraarterially, transdermally, intramuscularly, liposomally, via local delivery by catheter or stent, subcutaneously, intraadiposally, or intrathecally.

The compositions (e.g., pharmaceutical compositions) as disclosed herein can be suitable for administration to humans. In addition, such compositions can be suitable for administration to any other animal, e.g., to non-human animals, e.g., non-human mammals. Modification of pharmaceutical compositions suitable for administration to humans in order to render the compositions suitable for administration to various animals is well understood, and the ordinarily skilled veterinary pharmacologist can design and/or perform such modification with merely ordinary, if any, experimentation. Subjects to which administration of the pharmaceutical compositions is contemplated include, but are not limited to, humans and/or other primates; mammals, including commercially relevant mammals such as cattle, pigs, horses, sheep, cats, dogs, mice, and/or rats; and/or birds, including commercially relevant birds such as poultry, chickens, ducks, geese, and/or turkeys.

### Cells

A cell as provided herein may be referred to as a target cell (not part of the invention). In some cases, the systems, compositions, and methods as provided herein can be applied to modify a target cell (e.g., modify expression profile of a target gene of the target cell, such as one or genes in TABLE 1). A target cell can include a wide variety of cell types. A target cell can be in vitro. A target cell can be in vivo. A target cell can be ex vivo. A target cell can be an isolated cell. A target cell can be a cell inside of an organism. A target cell can be an organism. A target cell can be a cell in a cell culture. A target cell can be one of a collection of cells. A target cell can be a mammalian cell or derived from a mammalian cell. A target cell can be a rodent cell or derived from a rodent cell. A target cell can be a human cell or derived from a human cell. A target cell can be a prokaryotic cell or derived from a prokaryotic cell. A target cell can be a bacterial cell or can be derived from a bacterial cell. A target cell can be an archaeal cell or derived from an archaeal cell. A target cell can be a eukaryotic cell or derived from a eukaryotic cell. A target cell can be a pluripotent stem cell. A target cell can be a plant cell or derived from a plant cell. A target cell can be an animal cell or derived from an animal cell. A target cell can be an invertebrate cell or derived from an invertebrate cell. A target cell can be a vertebrate cell or derived from a vertebrate cell. A target cell can be a microbe cell or derived from a microbe cell. A target cell can be a fungi cell or derived from a fungi cell. A target cell can be from a specific organ or tissue.

A target cell can be a stem cell or progenitor cell. Target cells can include stem cells (e.g., adult stem cells, embryonic stem cells, induced pluripotent stem (iPS) cells) and progenitor cells (e.g., cardiac progenitor cells, neural progenitor cells, etc.). Target cells can include mammalian stem cells and progenitor cells, including rodent stem cells, rodent progenitor cells, human stem cells, human progenitor cells, etc. Clonal cells can comprise the progeny of a cell. A target cell can comprise a target nucleic acid. A target cell can be in a living organism. A target cell can be a genetically modified cell. A target cell can be a host cell.

A target cell can be a primary cell. For example, cultures of primary cells can be passaged 0 times, 1 time, 2 times, 4 times, 5 times, 10 times, 15 times or more. Cells can be unicellular organisms. Cells can be grown in culture.

A target cell can be a diseased cell. A diseased cell can have altered metabolic, gene expression, and/or morphologic features. A diseased cell can be a cancer cell, a diabetic cell, and a apoptotic cell. A diseased cell can be a cell from a diseased subject. Exemplary diseases can include blood disorders, cancers, metabolic disorders, eye disorders, organ disorders, musculoskeletal disorders, cardiac disease, and the like.

If the target cells are primary cells, they may be harvested from an individual by any method. For example, leukocytes may be harvested by apheresis, leukocytapheresis, density gradient separation, etc. Cells from tissues such as skin, muscle, bone marrow, spleen, liver, pancreas, lung, intestine, stomach, etc. can be harvested by biopsy.

Non-limiting examples of cells which can be target cells include, but are not limited to, lymphoid cells, such as B cell, T cell (Cytotoxic T cell, Natural Killer T cell, Regulatory T cell, T helper cell), Natural killer cell, cytokine induced killer (CIK) cells; myeloid cells, such as granulocytes (Basophil granulocyte, Eosinophil granulocyte, Neutrophil granulocyte/Hypersegmented neutrophil), Monocyte/Macrophage, Red blood cell (Reticulocyte), Mast cell, Thrombocyte/Megakaryocyte, Dendritic cell; cells from the endocrine system, including thyroid (Thyroid epithelial cell, Parafollicular cell), parathyroid (Parathyroid chief cell, Oxyphil cell), adrenal (Chromaffin cell), pineal (Pinealocyte) cells; cells of the nervous system, including glial cells (Astrocyte, Microglia), Magnocellular neurosecretory cell, Stellate cell, Boettcher cell, and pituitary (Gonadotrope, Corticotrope, Thyrotrope, Somatotrope, Lactotroph); cells of the Respiratory system, including Pneumocyte (Type I pneumocyte, Type II pneumocyte), Clara cell, Goblet cell, Dust cell; cells of the circulatory system, including Myocardiocyte, Pericyte; cells of the digestive system, including stomach (Gastric chief cell, Parietal cell), Goblet cell, Paneth cell, G cells, D cells, ECL cells, I cells, K cells, S cells; enteroendocrine cells, including enterochromaffm cell, APUD cell, liver (Hepatocyte, Kupffer cell), Cartilage/bone/muscle; bone cells, including Osteoblast, Osteocyte, Osteoclast, teeth (Cementoblast, Ameloblast); cartilage cells, including Chondroblast, Chondrocyte; skin cells, including Trichocyte, Keratinocyte, Melanocyte (Nevus cell); muscle cells, including Myocyte; urinary system cells, including Podocyte, Juxtaglomerular cell, Intraglomerular mesangial cell/Extraglomerular mesangial cell, Kidney proximal tubule brush border cell, Macula densa cell; reproductive system cells, including Spermatozoon, Sertoli cell, Leydig cell, Ovum; and other cells, including Adipocyte, Fibroblast, Tendon cell, Epidermal keratinocyte (differentiating epidermal cell), Epidermal basal cell (stem cell), Keratinocyte of fingernails and toenails, Nail bed basal cell (stem cell), Medullary hair shaft cell, Cortical hair shaft cell, Cuticular hair shaft cell, Cuticular hair root sheath cell, Hair root sheath cell of Huxley's layer, Hair root sheath cell of Henle's layer, External hair root sheath cell, Hair matrix cell (stem cell), Wet stratified barrier epithelial cells, Surface epithelial cell of stratified squamous epithelium of cornea, tongue, oral cavity, esophagus, anal canal, distal urethra and vagina, basal cell (stem cell) of epithelia of cornea, tongue, oral cavity, esophagus, anal canal, distal urethra and vagina, Urinary epithelium cell (lining urinary bladder and urinary ducts), Exocrine secretory epithelial cells, Salivary gland mucous cell (polysaccharide-rich secretion), Salivary gland serous cell (glycoprotein enzyme-rich secretion), Von Ebner's gland cell in tongue (washes taste buds), Mammary gland cell (milk secretion), Lacrimal gland cell (tear secretion), Ceruminous gland cell in ear (wax secretion), Eccrine sweat gland dark cell (glycoprotein secretion), Eccrine sweat gland clear cell (small molecule secretion). Apocrine sweat gland cell (odoriferous secretion, sex-hormone sensitive), Gland of Moll cell in eyelid (specialized sweat gland), Sebaceous gland cell (lipid-rich sebum secretion), Bowman's gland cell in nose (washes olfactory epithelium), Brunner's gland cell in duodenum (enzymes and alkaline mucus), Seminal vesicle cell (secretes seminal fluid components, including fructose for swimming sperm), Prostate gland cell (secretes seminal fluid components), Bulbourethral gland cell (mucus secretion), Bartholin's gland cell (vaginal lubricant secretion), Gland of Littre cell (mucus secretion), Uterus endometrium cell (carbohydrate secretion), Isolated goblet cell of respiratory and digestive tracts (mucus secretion), Stomach lining mucous cell (mucus secretion), Gastric gland zymogenic cell (pepsinogen secretion), Gastric gland oxyntic cell (hydrochloric acid secretion), Pancreatic acinar cell (bicarbonate and digestive enzyme secretion), Paneth cell of small intestine (lysozyme secretion), Type II pneumocyte of lung (surfactant secretion), Clara cell of lung, Hormone secreting cells, Anterior pituitary cells, Somatotropes, Lactotropes, Thyrotropes, Gonadotropes, Corticotropes, Intermediate pituitary cell, Magnocellular neurosecretory cells, Gut and respiratory tract cells, Thyroid gland cells, thyroid epithelial cell, parafollicular cell, Parathyroid gland cells, Parathyroid chief cell, Oxyphil cell, Adrenal gland cells, chromaffin cells, Ley dig cell of testes, Theca interna cell of ovarian follicle, Corpus luteum cell of ruptured ovarian follicle, Granulosa lutein cells, Theca lutein cells, Juxtaglomerular cell (renin secretion), Macula densa cell of kidney, Metabolism and storage cells, Barrier function cells (Lung, Gut, Exocrine Glands and Urogenital Tract), Kidney, Type I pneumocyte (lining air space of lung), Pancreatic duct cell (centroacinar cell), Nonstriated duct cell (of sweat gland, salivary gland, mammary gland, etc.), Duct cell (of seminal vesicle, prostate gland, etc.), Epithelial cells lining closed internal body cavities, Ciliated cells with propulsive function, Extracellular matrix secretion cells, Contractile cells; Skeletal muscle cells, stem cell, Heart muscle cells, Blood and immune system cells, Erythrocyte (red blood cell), Megakaryocyte (platelet precursor), Monocyte, Connective tissue macrophage (various types), Epidermal Langerhans cell, Osteoclast (in bone), Dendritic cell (in lymphoid tissues), Microglial cell (in central nervous system), Neutrophil granulocyte, Eosinophil granulocyte, Basophil granulocyte, Mast cell, Helper T cell, Suppressor T cell, Cytotoxic T cell, Natural Killer T cell, B cell, Natural killer cell, Reticulocyte, Stem cells and committed progenitors for the blood and immune system (various types), Pluripotent stem cells, Totipotent stem cells, Induced pluripotent stem cells, adult stem cells, Sensory transducer cells, Autonomic neuron cells, Sense organ and peripheral neuron supporting cells, Central nervous system neurons and glial cells, Lens cells, Pigment cells, Melanocyte, Retinal pigmented epithelial cell, Germ cells, Oogonium/Oocyte, Spermatid, Spermatocyte, Spermatogonium cell (stem cell for spermatocyte), Spermatozoon, Nurse cells, Ovarian follicle cell, Sertoli cell (in testis), Thymus epithelial cell, Interstitial cells, and Interstitial kidney cells.

The cell (or target cell) can be engineered to comprise (or exhibit) any one of the systems or compositions as disclosed herein or can be treated by any one of the methods disclosed herein in vitro or ex vivo, then administered to the subject, e.g., to treat a condition of the subject. For example, any subject modified cell product can be administered to the subject to treat a condition of a bodily tissue of the subject. In some cases, the cell can be resident inside the subject's body, and any of the systems or compositions thereof can be administered to the subject, to contact the cell by the systems/compositions (e.g., to engineer the cell with the systems/compositions).

### EXAMPLES

### Example 1: Engineered nuclease

The Cas protein encoded by the polypeptide sequence of SEQ ID NO: 1 has a size of 529 amino acid residues. When delivering a gene encoding the Cas protein in a vector, e.g., in a viral vector such as AAV vector, reducing the size of such Cas protein can provide more cargo space within the vector (e.g., the viral vector that has a cargo size or length limitation). The increased cargo space within the vector can be used to deliver (e.g., encode) at least one additional component (e.g., one or more heterologous gene effector(s), one or more guide nucleic acid molecules, one or more cDNAs for therapeutic gene delivery, etc.), to effect a desired outcome (e.g., therapeutic effect). Alternatively or in addition to, when delivering a recombinant version of the Cas protein as disclosed herein in a delivery vehicle (e.g., lipid nanoparticles, viral capsids, etc.), reducing the size of the Cas protein can provide more cargo space to fit in, e.g., the at least one additional component, to effect he desired outcome. Without wishing to be bound by theory, use of the engineered nuclease as disclose herein, along with the at least one additional component, can enhance its activity (e.g., targeted gene binding, cleaving, editing, and/or regulation thereof), as compared to a control nuclease that is different.

In some cases, throughout the Examples of the present disclosure, one or more engineered nucleases of the present disclosure can be assessed (e.g., in vitro) to assess each of the one or more engineered nucleases activity in binding, cleaving, and/or editing a target polynucleotide sequence, e.g., to regulate expression and/or activity level of the target polynucleotide sequence of a polypeptide (e.g., a protein) encoded by the target polynucleotide sequence or operatively coupled to the target polynucleotide sequence. In some examples, a heterologous polypeptide comprising the engineered nuclease and a heterologous polynucleotide comprising a guide nucleic acid (e.g., sgRNA) against a target polynucleotide can be tested in a cell (e.g., in vitro) to assess the gene knockout efficiency at the target polynucleotide. In some examples, a heterologous polypeptide comprising a nuclease deficient variant of the engineered nuclease that is coupled to (e.g., fused to) a gene effector that is heterologous to the engineered nuclease (e.g., gene activator, gene repressor) and a heterologous polynucleotide comprising a guide nucleic acid (e.g., sgRNA) against a target polynucleotide can be tested in a cell (e.g., in vitro) to assess the ability to regulate expression and/or activity level of a gene coupled to (or comprising) the target polynucleotide.

In some cases, throughout the Examples of the present disclosure, a library of a plurality of engineered nuclease candidates can be generated by using full-plasmid amplification via opposite-facing primers spanning the deletion region.

### Example 2: Engineered nuclease based on structural comparison

### A. Approach

In some cases, the size (e.g., the number of amino acid residues) of CasMini or deactivated CasMini (dCasMini) (e.g., mutated variant of the polypeptide sequence of SEQ ID NO: 1) can be further reduced while maintaining or enhancing its activity (e.g., overall epigenetic gene regulatory activity). For example, additional Cas proteins (e.g., naturally occurring Cas12f proteins, such as Un2Cas12f1 (SEQ ID NO: 2) or AsCas12f (SEQ ID NO: 3)) that are smaller than that of SEQ ID NO: 1 can serve as a reference point to determine at least one amino acid residue and/or at least one tertiary structure of the Cas protein of SEQ ID NO: 1 that can be modified (e.g., deleted), e.g., with minimal or no comprise of its activity.

In some cases, the native Un1Cas12f1 nuclease encoded by SEQ ID NO: 1 can be engineered by creating at least one deletion, to generate the engineered nuclease as disclosed herein, e.g., for one or more reasons described in Example 1.

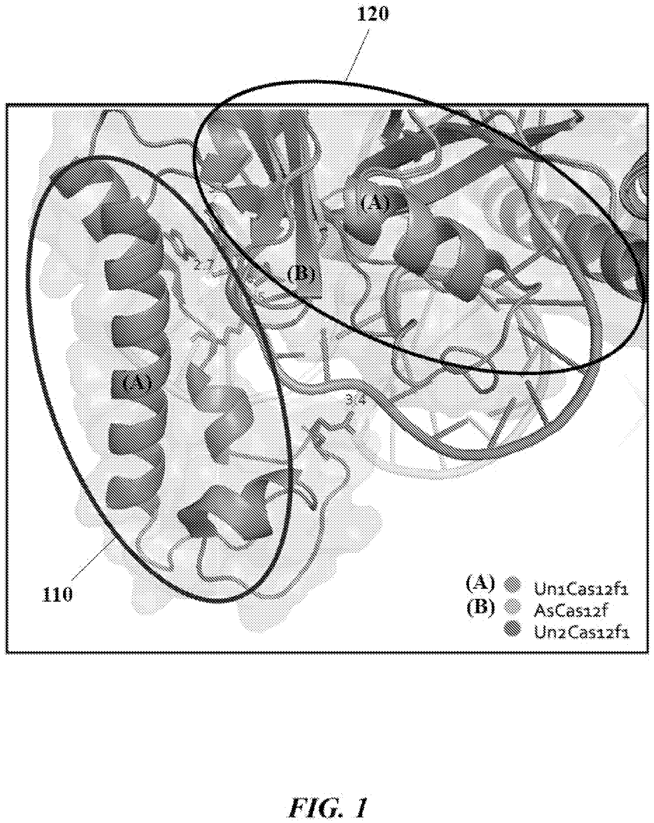

In some cases, the at least one deletion of the amino acid sequence of the engineered nuclease can be found in one or more regions of the native Un1Cas12f1 nuclease that do not structurally align to a AsCas12f (SEQ ID NO: 3). When compared to AsCas12f, Un1Cas12f1 comprises the additional amino acid residues 1-71 domain, and at least a portion of the domain and/or one or more amino acid residues near the domain may be removed, e.g., with minimal or substantially no reduction of the engineered nuclease's activity (e.g., the engineered nuclease's interaction with the guide nucleic acid molecule (e.g., crRNA). As shown in FIG. 1, Un1Cas12f1 comprises domain 110 (e.g., comprising at least a portion of the additional amino acid residues 1-71 domain) that may not be conserved in AsCas12f. On the other hand, Un1Cas12f1 comprises domain 120 that may be conserved in AsCas12f.

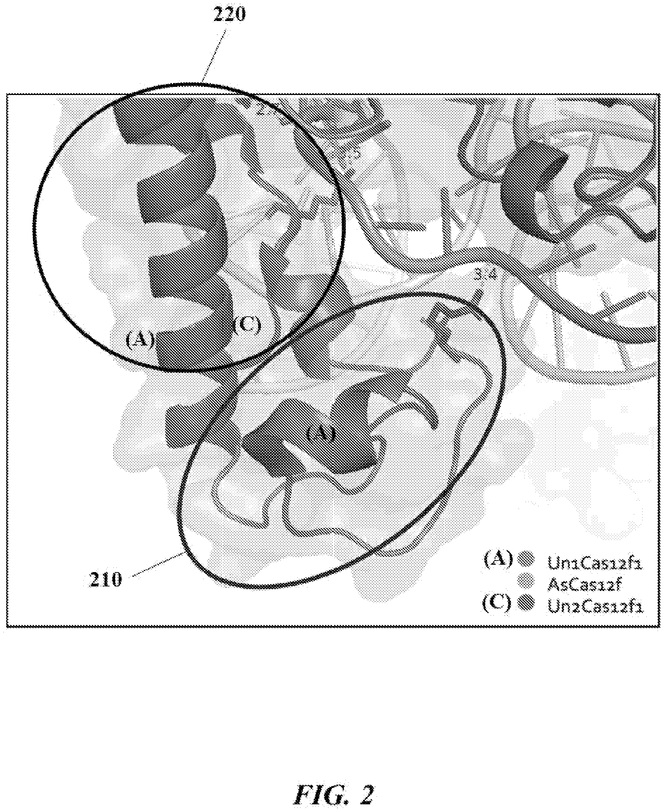

In some cases, the at least one deletion of the amino acid sequence of the engineered nuclease can be found in one or more regions of the native Un1Cas12f1 nuclease that do not structurally align to a Un2Cas12f1 (SEQ ID NO: 2). When compared to Un2Cas12f1, Un1Cas12f1 comprises the additional amino acid residues 41-71 domain, and at least a portion of the domain and/or one or more amino acid residues near the domain may be removed, e.g., with minimal or substantially no reduction of the engineered nuclease's activity (e.g., the engineered nuclease's interaction with the guide nucleic acid molecule (e.g., crRNA). As shown in FIG. 2, Un1Cas12f1 comprises domain 210 (e.g., comprising at least a portion of the additional amino acid residues 41-71 domain) that may not be conserved in Un2Cas12f1. On the other hand, Un1Cas12f1 comprises domain 220 that may be conserved in Un2Cas12f1.

### B. Example library designs

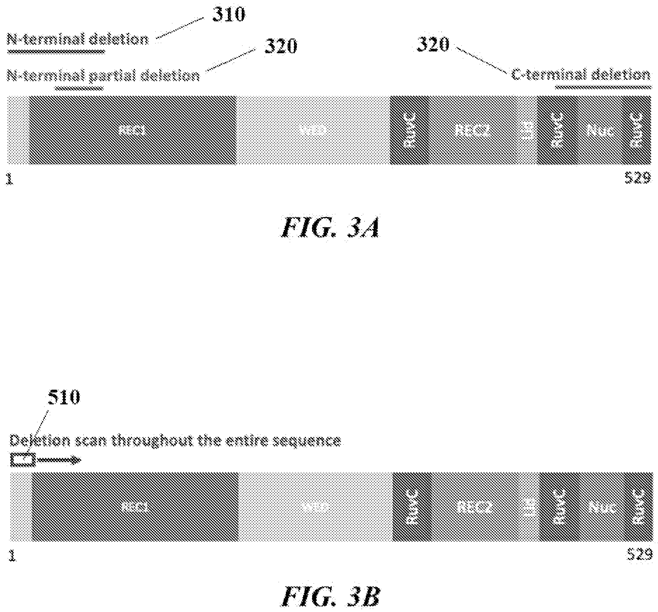

FIG. 3A schematically illustrates different regions of Un1Cas12f1, as encoded by SEQ ID NO: 1, and example domains (310, 320, and 330) that can be at least partially deleted to generate one or engineered nucleases as disclosed herein. For example, the domain 310 can refer to N-terminal deletion (e.g., the amino acid residues 2-76 when aligned to SEQ ID NO: 1) can be evaluated by generating 25 variants via incremental removal of 3 amino acids at a time from the N-terminus of the protein. In another example, the domain 320 can refer to a N-terminal partial deletion (e.g., the amino acid residues 41-71 when aligned to SEQ ID NO: 1) can be evaluated by generating 16 variants via incremental removal of additional 2 amino acids at a time from the middle of this region (e.g., del55-56, del54-57, del54-58, del53-59, del52-60, del51-61, del50-62, del49-63, del48-64, del47-65, del46-66, del45-67, del44-68, del43-69, del42-70, del41-71, etc.). In a different example, the domain 330 can refer to C-terminal deletion (e.g., the last 75 amino acid residues when aligned to SEQ ID NO: 1) can be evaluated by generating 25 variants by incremental removal of 3 amino acids at a time from the C-terminus.

Examples of engineered nucleases with reduced nuclease activity, as generated in accordance with the present disclosure, can include SEQ ID NOs: 4-9, as provided herein.
**SEQ ID NO: 4** (comprising N-terminal deletion, e.g., the amino acid residues 2-21 (AKNTITKTLKLRIVRPYNSA), when aligned to SEQ ID NO: 1) 
1 MEVEKIVADE KNNREKIALE KNKDKVKEAC SKHLKVAAYC TTQVERNACL
51 FCKARKLDDK FYQKLRGQFP DAVFWQEISE IFRQLQKQAA EIYNQSLIEL
101 YYEIFIKGKG IANASSVEHY LSRVCYRRAA ELFKNAAIAS GLRSKIKSNF
151 RLKELKNMKS GLPTTKSDNF PIPLVKQKGG QYTGFEISNH NSDFIIKIPF
201 GRWQVKKEID KYRPWEKFDF EQVQKSPKPI SLLLSTQRRK RNKGWSKDEG
251 TEAEIKKVMN GDYQTSYIEV KRGSKICEKS AWMLNLSIDV PKIDKGVDPS
301 IIGGIAVGVR SPLVCAINNA FSRYSISDND LFHFNKKMFA RRRILLKKNR
351 HKRAGHGAKN KLKPITILTE KSERFRKKLI ERWACEIADF FIKNKVGTVQ
 
401 MENLESMKRK EDSYFNIRLR GFWPYAEMQN KIEFKLKQYG IEIRKVAPNN

451 TSKTCSKCGH LNNYFNFEYR KKNKFPHFKC EKCNFKENAA YNAALNISNP

501 KLKSTKERP
**SEQ ID NO: 5** (comprising N-terminal deletion, e.g., the amino acid residues 2-31 (AKNTITKTLKLRIVRPYNSAEVEKIVADEK), when aligned to SEQ ID NO: 1) 
1 MNNREKIALE KNKDKVKEAC SKHLKVAAYC TTQVERNACL FCKARKLDDK
51 FYQKLRGQFP DAVFWQEISE IFRQLQKQAA EIYNQSLIEL YYEIFIKGKG
101 IANASSVEHY LSRVCYRRAA ELFKNAAIAS GLRSKIKSNF RLKELKNMKS
151 GLPTTKSDNF PIPLVKQKGG QYTGFEISNH NSDFIIKIPF GRWQVKKEID
201 KYRPWEKFDF EQVQKSPKPI SLLLSTQRRK RNKGWSKDEG TEAEIKKVMN
251 GDYQTSYIEV KRGSKICEKS AWMLNLSIDV PKIDKGVDPS IIGGIAVGVR
301 SPLVCAINNA FSRYSISDND LFHFNKKMFA RRRILLKKNR HKRAGHGAKN
351 KLKPITILTE KSERFRKKLI ERWACEIADF FIKNKVGTVQ MENLESMKRK
401 EDSYFNIRLR GFWPYAEMQN KIEFKLKQYG IEIRKVAPNN TSKTCSKCGH
451 LNNYFNFEYR KKNKFPHFKC EKCNFKENAA YNAALNISNP KLKSTKERP
**SEQ ID NO: 6** (comprising C-terminal deletion, e.g., the amino acid residues 510-529 (DYNAALNISNPKLKSTKEEP), when aligned to SEQ ID NO: 1) 
1 MAKNTITKTL KLRIVRPYNS AEVEKIVADE KNNREKIALE KNKDKVKEAC
51 SKHLKVAAYC TTQVERNACL FCKARKLDDK FYQKLRGQFP DAVFWQEISE
101 IFRQLQKQAA EIYNQSLIEL YYEIFIKGKG IANASSVEHY LSRVCYRRAA
151 ELFKNAAIAS GLRSKIKSNF RLKELKNMKS GLPTTKSDNF PIPLVKQKGG
201 QYTGFEISNH NSDFIIKIPF GRWQVKKEID KYRPWEKFDF EQVQKSPKPI
251 SLLLSTQRRK RNKGWSKDEG TEAEIKKVMN GDYQTSYIEV KRGSKICEKS
301 AWMLNLSIDV PKIDKGVDPS IIGGIAVGVR SPLVCAINNA FSRYSISDND
351 LFHFNKKMFA RRRILLKKNR HKRAGHGAKN KLKPITILTE KSERFRKKLI
401 ERWACEIADF FIKNKVGTVQ MENLESMKRK EDSYFNIRLR GFWPYAEMQN
451 KIEFKLKQYG IEIRKVAPNN TSKTCSKCGH LNNYFNFEYR KKNKFPHFKC
501 EKCNFKENA
**SEQ ID NO: 7** (comprising C-terminal deletion, e.g., the amino acid residues 500-529 (CEKCNFKENADYNAALNISNPKLKSTKEEP), when aligned to SEQ ID NO: 1) 
1 MAKNTITKTL KLRIVRPYNS AEVEKIVADE KNNREKIALE KNKDKVKEAC
51 SKHLKVAAYC TTQVERNACL FCKARKLDDK FYQKLRGQFP DAVFWQEISE
101 IFRQLQKQAA EIYNQSLIEL YYEIFIKGKG IANASSVEHY LSRVCYRRAA
151 ELFKNAAIAS GLRSKIKSNF RLKELKNMKS GLPTTKSDNF PIPLVKQKGG
201 QYTGFEISNH NSDFIIKIPF GRWQVKKEID KYRPWEKFDF EQVQKSPKPI
251 SLLLSTQRRK RNKGWSKDEG TEAEIKKVMN GDYQTSYIEV KRGSKICEKS
301 AWMLNLSIDV PKIDKGVDPS IIGGIAVGVR SPLVCAINNA FSRYSISDND
351 LFHFNKKMFA RRRILLKKNR HKRAGHGAKN KLKPITILTE KSERFRKKLI
401 ERWACEIADF FIKNKVGTVQ MENLESMKRK EDSYFNIRLR GFWPYAEMQN
 451 KIEFKLKQYG IEIRKVAPNN TSKTCSKCGH LNNYFNFEYR KKNKFPHFK
**SEQ ID NO: 8** (comprising partial N-terminal deletion, e.g., the amino acid residues 47-66 (KEACSKHLKVAAYCTTQVER), when aligned to SEQ ID NO: 1) 
1 MAKNTITKTL KLRIVRPYNS AEVEKIVADE KNNREKIALE KNKDKVNACL
51 FCKARKLDDK FYQKLRGQFP DAVFWQEISE IFRQLQKQAA EIYNQSLIEL
101 YYEIFIKGKG IANASSVEHY LSRVCYRRAA ELFKNAAIAS GLRSKIKSNF
151 RLKELKNMKS GLPTTKSDNF PIPLVKQKGG QYTGFEISNH NSDFIIKIPF
201 GRWQVKKEID KYRPWEKFDF EQVQKSPKPI SLLLSTQRRK RNKGWSKDEG
251 TEAEIKKVMN GDYQTSYIEV KRGSKICEKS AWMLNLSIDV PKIDKGVDPS
301 IIGGIAVGVR SPLVCAINNA FSRYSISDND LFHFNKKMFA RRRILLKKNR
351 HKRAGHGAKN KLKPITILTE KSERFRKKLI ERWACEIADF FIKNKVGTVQ
401 MENLESMKRK EDSYFNIRLR GFWPYAEMQN KIEFKLKQYG IEIRKVAPNN
451 TSKTCSKCGH LNNYFNFEYR KKNKFPHFKC EKCNFKENAA YNAALNISNP
501 KLKSTKERP
**SEQ ID NO:** 9 (comprising partial N-terminal deletion, e.g., the amino acid residues 41-71 (KNKDKVKEACSKHLKVAAYCTTQVERNACLF), when aligned to SEQ ID NO: 1)

1 MAKNTITKTL KLRIVRPYNS AEVEKIVADE KNNREKIALE CKARKLDDKF
51 YQKLRGQFPD AVFWQEISEI FRQLQKQAAE IYNQSLIELY YEIFIKGKGI
101 ANASSVEHYL SRVCYRRAAE LFKNAAIASG LRSKIKSNFR LKELKNMKSG
151 LPTTKSDNFP IPLVKQKGGQ YTGFEISNHN SDFIIKIPFG RWQVKKEIDK
201 YRPWEKFDFE QVQKSPKPIS LLLSTQRRKR NKGWSKDEGT EAEIKKVMNG
251 DYQTSYIEVK RGSKICEKSA WMLNLSIDVP KIDKGVDPSI IGGIAVGVRS
301 PLVCAINNAF SRYSISDNDL FHFNKKMFAR RRILLKKNRH KRAGHGAKNK
351 LKPITILTEK SERFRKKLIE RWACEIADFF IKNKVGTVQM ENLESMKRKE
401 DSYFNIRLRG FWPYAEMQNK IEFKLKQYGI EIRKVAPNNT SKTCSKCGHL
451 NNYFNFEYRK KNKFPHFKCE KCNFKENAAY NAALNISNPK LKSTKERP

### Example 3: Engineered nuclease based on deletion landscape

The native Un1Cas12f1 nuclease encoded by SEQ ID NO: 1 can be engineered by creating at least one deletion, to generate the engineered nuclease of the present disclosure, e.g., for one or more reasons described in Example 1. The at least one deletion of the amino acid sequence of the engineered nuclease, as disclosed herein, can be determined based on deletion (or truncation) landscape of the Cas protein (or the nuclease-deficient variant thereof).

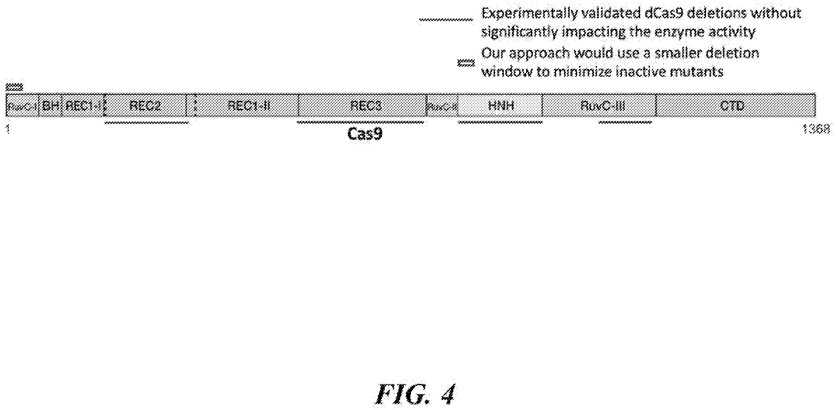

For example, as illustrated in FIG. 4, mapping of the truncation landscape of dCas9 suggests tolerance for significant deletions, e.g., deletions of the amino acid residues 167-316 of the dCas9. Thus, a similar deletion landscape can be utilized to determine one or more amino acid residues (e.g., a plurality of consecutive amino acid residues) of the native Un1Cas12f1 (or the nuclease-deficient variant thereof), to generate deletion mutants of the Un1Cas12f1 protein without significantly impacting activity (e.g., without impacting activity). In some cases, a deletion of about 20 amino acid residues (e.g., from one consecutive region, or from two non-consecutive regions) can be determined to generate a mutant variant of Un1Cas12f1 that still exhibits comparable (or enhanced) activity as compared to the wild type Un1Cas12f1. In some cases, a small deletion window (e.g., a window of between about 3 and about 5 amino acids) can be assessed throughout the native Un1Cas12f1 protein for the deletion landscape.

FIG. 3B schematically illustrates deletion landscape approach to generate engineered nuclease variants of Un1Cas12f1. A deletion tile 510 of a plurality of amino acid residues (e.g., a plurality of consecutive amino acid residues, such as 5 consecutive amino acid residues) can be scanned throughout at least a portion of Un1Cas12f1 (e.g., the entire Un1Cas12f1, except for the dimerization domain and/or the PAM domain) to generate, e.g., up to 95 variants for individual transfection screening.

### Example 4: Engineered nuclease based on deletion landscape

In addition to deletion of one or more amino acid residues from the native Un1Cas12f1 nuclease encoded by SEQ ID NO: 1 to generate the engineered nuclease, one or more amino acid residues can be mutated as compared to the native Un1Cas12f1 nuclease, to further modify the engineered nuclease of the present disclosure, e.g., for one or more reasons described in Example 1. The at least one deletion of the amino acid sequence of the engineered nuclease, as disclosed herein, can be determined based on Site Saturation Mutagenesis (SSM). For example, a single codon or set of codons of a polynucleotide encoding the native Un1Cas12f1 protein (or the nuclease-deficient variant thereof) can be substituted with one or more possible amino acids at the position for enhanced activity (e.g., for epigenetic regulation improvement, such as enhanced activation/repression, different PAM recognition, etc.), stability, expression, binding to the respective guide nucleic acid molecule, etc.

### Example 5: Assessment of engineered nuclease(s)

Nuclease-deficient variants of the engineered nucleases as disclosed herein (e.g., generated in accordance with Examples 1-4) can be coupled to (e.g., fused to) heterologous gene effectors (e.g., VP16, VP64, p65, Rta, VPR, etc.), and one or more heterologous polypeptides encoding such fusion protein and/or a guide RNA can be transfected into a cell to assess activation of expression/activity level of a target gene. For example, engineered HEK93T cells bearing a synthetic reporter can be used, in which fluorescence activation may be measured as a readout. When a plurality of engineered nucleases are identified, the screening method (e.g., via using the engineered HEK93T cells) can be repeated to (i) confirm the prior screening results and/or (ii) identify top hits.

Depending on the result from the first round of engineering, a plurality of deletions of amino acid residues (e.g., when aligned to the polypeptide sequence of SEQ ID NO: 1) can be combined to generate one or more additional engineered nucleases. Alternatively or in addition to, one or more additional engineered nucleases can be generated by assessing one or more granular deletions around one or more leads from the first round of engineering.

### A. First round of engineering

Multiple sequence alignments of dCasMINI (SEQ ID NO: 10) with one or more naturally occurring Cas12f protein orthologs with reported nuclease activity in bacteria (e.g., Un1Cas12f1, Un2Cas12f1, AsCas12f, and other orthologs as provided in TABLE 2), to, for example, identify one or more potentially beneficial mutations in generating engineered nuclease variants as disclosed herein. Homology modeling of the Cas12f orthologs was also performed to identify structural conservation (e.g., not entirely based on amino acid based conservation), to generate the engineered nuclease variants. Combining these two approaches (e.g., sequence alignment and structural conservation analysis), stretches of amino acid sequences as well as combinations of individual residue mutations were identified as promising candidates to generate the engineered nuclease variants. Based on the identified information, chimeric protein variants were designed via sequence swapping, and mutation variants were designed via one or more residue mutations. In some cases, the sequence swapping variants also resulted in overall reduced protein size.

In the first round of engineering, engineered nuclease variants (e.g., truncation variants, chimeric protein variants, and/or mutation variants) were generated, tested, and screened to identify lead hits. For example, various chimeric engineered nuclease variants with reduced nuclease activity were designed (as provided in TABLE 3B) and tested in combination with (e.g., fused to) a gene modulator such as a gene activator or a gene repressor, to test their efficacy in gene modulation as compared to a control dCasMINI (SEQ ID NO: 10).

Gene activation was assessed by fusing each engineered nuclease variant with a gene activator. Each engineered nuclease variant with reduced nuclease activity was individually cloned into a dCas plasmid in frame with a gene activator (e.g., VPR) for transcriptional activation. HEK293T cells were transfected with identical sgRNA plasmid and individual dCas variant plasmid as triplicate or quadruplicate repeats in 96-well plate format. After several days post-transfection (e.g., after 2 days, after 3 days, or after 4 days), CD2 protein levels were quantified by cell surface antibody staining of live cells followed by flow cytometry, while secreted IFN gamma (IFNγ) protein levels were measured using ELISA on cell culture supernatants. The level of gene activation of each engineered nuclease variant was compared to the activity of a dCasMINI fused to the same gene activator as a control.

Gene repression was assessed by fusing each engineered nuclease variant with a gene repressor. Each engineered nuclease variant with reduced nuclease activity was individually cloned into the dCas plasmid in frame with a gene repressor for transcriptional inhibition. In 96-well plate format, HEK293T GFP reporter cells (e.g., ESR221) were transfected with identical sgRNA plasmid and individual dCas variant plasmid as triplicate or quadruplicate repeats. After several days post-transfection (e.g., 5 to 7 days), suppression of GFP expression was measured by flow cytometry. The level of gene repression of each engineered nuclease variant was compared to the activity of a dCasMINI fused to the same gene activator as a control.

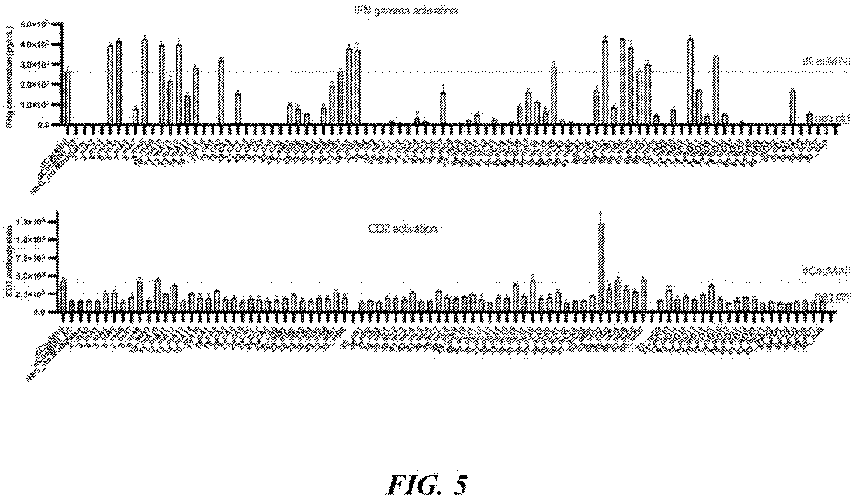

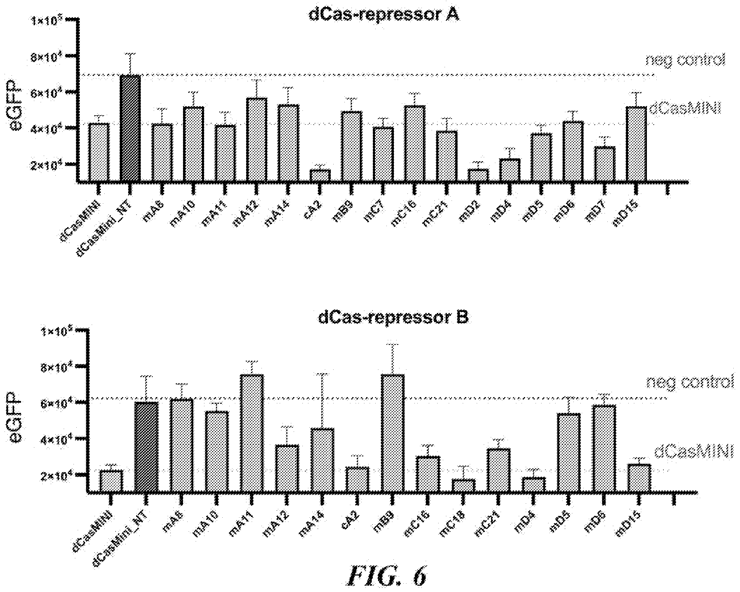

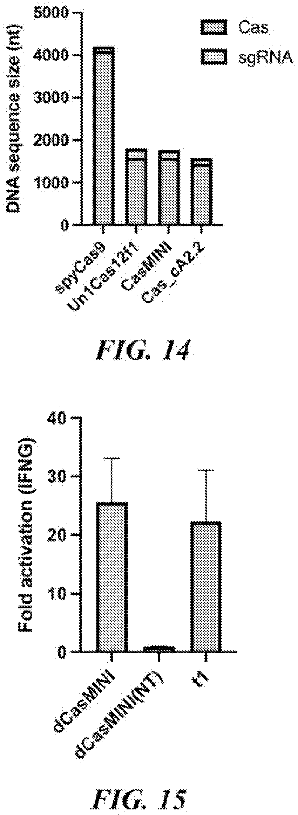

As shown in FIG. 5, out of the chimeric engineered nuclease variants cA1 through cA9, the chimeric engineered nuclease variant cA2 resulted in the greatest activation of IFN gamma expression (e.g., greater than that by the dCasMINI control) and also of CD2 activation (as summarized in TABLE 3A). Additional engineered nuclease variants were designed with one or more mutations relative to Un1Cas12f1, without significantly changing the overall size of the engineered nuclease variant as compared to dCasMINI. See TABLE 4B for the amino acid sequences of such engineered nuclease variants. As shown in FIG. 5, when fused to a gene activator, various engineered nuclease (e.g., mD2, mD4) exhibited comparable or greater efficacy in activating target genes (e.g., IFN gamma or CD2) as compared to the control dCasMINI (as summarized in TABLE 4A). As shown in FIG. 6, some of the various engineered nuclease (e.g., mD2, mD4) exhibited comparable or greater efficacy in repressing target genes (e.g., eGFP) as compared to the control dCasMINI (as summarized in TABLE 4A). Another engineered nuclease variant (t1) comprising deletion on the C-terminal region as compared to SEQ ID NO: 1 was also identified without sacrificing the gene modulation activity. As shown in FIG. 15, when fused to a gene activator, the engineered nuclease variant t1 exhibited comparable efficacy in activating a target gene (e.g., IFN gamma) as compared to the control dCasMINI.

The chimeric engineered nuclease variant cA2 was designed with sequence deletions at the zinc-binding motif. Without wishing to be bound by theory, such deletions at the particular zinc-binding motif resulted in a smaller nuclease variant that maintained or even improved the gene modulation activity (e.g., epigenetic modification activity) when operatively linked to (e.g., fused with) one or more gene modulators.

TABLE 5A summarizes each set of modifications relative to the amino acid sequence of Un1Cas12f1 (SEQ ID NO: 1) that is embedded in each engineered nuclease variant, to yield enhanced gene modulation activity as compared to dCasMINI (SEQ ID NO: 10).

Without wishing to be bound by theory, one or more additional modification can be made to any of the engineered nuclease variants from TABLE 3A, TABLE 4A, TABLE 5A, and TABLE 5B to enhance desired activity (e.g., forming a complex with guide nucleic acid molecule, target sequence nuclease activity, increasing expression of a target gene, decreasing expression of a target gene, etc.)

### B. Second round of engineering

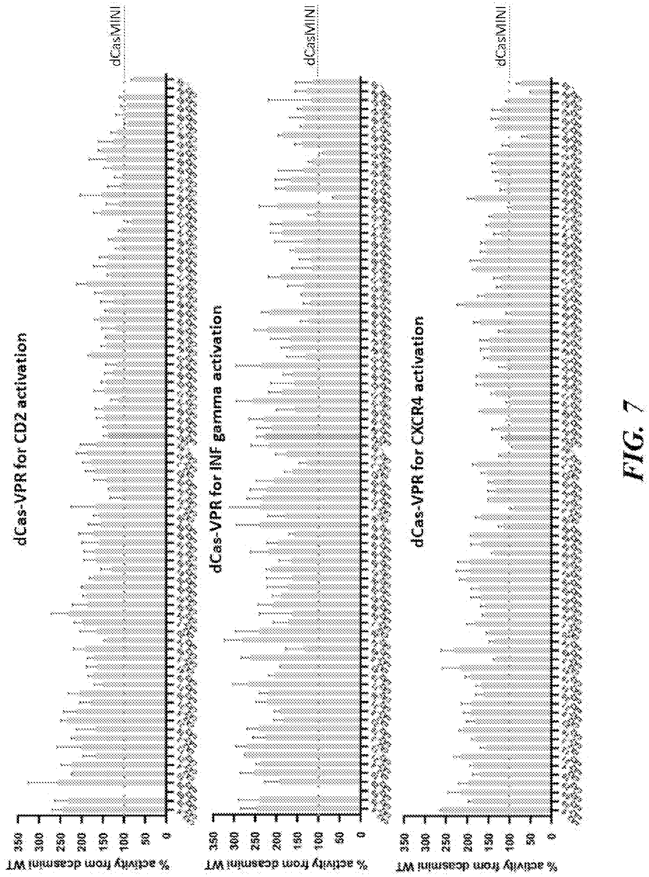

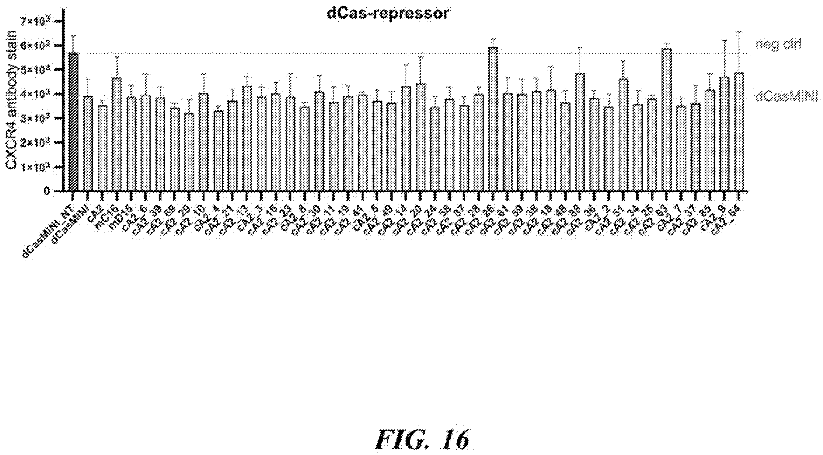

In the second round of engineering, the engineered nuclease variant "cA2" (SEQ ID NO: 12) was selected as scaffold, to generate a new library of engineered nuclease variants by grafting, onto the cA2 scaffold, one or more combinations of the mutation and/or truncations identified and listed in TABLE 5A. TABLE 5B shows a list of the engineered nuclease variants in the new library, indicating the combination of modification that has been grafted onto each of the engineered nuclease variant. TABLE 5C shows the respective amino acid sequence of each of the engineered nuclease variants listed in TABLE 5B. As shown in FIG. 7, upon testing for their ability to activate target genes (e.g., CD2, IFN gamma, and CXCR4) when in combination with a gene activator (e.g., VPR), various engineered nuclease variants exhibited comparable or enhanced gene activation than the control dCasMINI, some outperforming the starting scaffold cA2 (e.g., cA2.6, cA2.39, cA2.69, cA2.29, cA2.10, cA2.4, cA2.21, cA2.13, cA2.3, cA2.16, cA2.23, cA2.8, cA2.31, cA2.30, cA2.11, cA2.5, cA2.41, cA2.49, cA2.26, cA2.14, cA2.20, cA2.1, cA2.24, cA2.58, cA2.61, cA2.38, cA2.88, cA2.2, cA2.51, cA2.34, cA2.25, cA2.85, cA2.54, cA2.15, cA2.75, cA2.32, cA2.90, cA2.89, or cA2.46). In contrast, some newly engineered nuclease variants did not exhibit enhanced activity as compared to the control dCasMINI (e.g., cA2.55, cA2.84). As shown in FIG. 16, upon testing for their ability to suppress a target gene (e.g., CXCR4) when in combination with a gene repressing modulator (e.g., ZNF10-KRAB-hDNMT3L), various engineered nuclease variants exhibited comparable or enhanced gene suppression than the control dCasMINI, with some engineered nuclease variants performing comparable to the starting scaffold cA2 (e.g., cA2.69, cA.2.29, cA2.4, cA2.2, cA2.34, or cA2.7). In contrast, some newly engineered nuclease did not exhibit enhanced gene suppression activity as compared to the control dCasMINI (e.g., cA2.26, cA2.63).

In sum, two regions of sequence deletions were identified herein as a compact Cas variant. Additional point mutations were also identified that contributed to the improved gene modulation activity when used in conjunction with a gene modulator and a guide nucleic acid molecule. Combining these sequence modifications, the cA2 protein (SEQ ID NO: 12) and variants thereof (with additional point mutations and sequence deletions, see TABLE 5C) outperformed the control dCasMINI, in terms of both transcriptional activation and suppression activity over multiple endogenous loci.

Without wishing to be bound by theory, any amino acid deletion, structural deletion, or amino acid modification (e.g., mutation/substitution) identified herein to enhance gene modulating efficacy of the engineered nuclease variants can be "grafted" back into any other naturally or non-naturally occurring Cas proteins (e.g., a naturally occurring Cas protein selected from TABLE 2 or a deactivated nuclease variant thereof) to generate one or more additional engineered nuclease variants that my yield greater gene modulation efficacy than a control Cas protein (e.g., dCasMINI as disclosed herein).

### Example 6: Assessment of engineered nuclease(s)

An engineered nuclease variant of the engineered nucleases as disclosed herein (e.g., generated in accordance with Examples 1-4) that maintains at least a portion of the nuclease activity (e.g., as compared to SEQ ID NO: 1) can be tested in a cell for its nuclease activity. A polypeptide comprising the engineered nuclease variant and/or a guide RNA can be transfected into a cell to assess the ability of a complex comprising the engineered nuclease and the guide RNA to create a break in a target polynucleotide sequence (e.g., create a double-strand break in, or adjacent to, the target polynucleotide sequence that comprises an appropriate PAM). Such nuclease activity can be observed by in vitro enzymatic assay (e.g., with purified enzyme or cell lysate), or in vivo (e.g., in E. coli or in eukaryotic cell).

### Example 7: Guide nucleic acid scaffold engineering

A Cas protein can comprise a naturally occurring Cas protein (e.g., (i) the polypeptide sequence of SEQ ID NO: 1 or (ii) the polypeptide sequence of any one of the Cas proteins selected from TABLE 2) or any modification thereof (e.g., an engineered polypeptide comprising an engineered nuclease variant as disclosed herein). The Cas protein can form a complex (e.g., a ribonucleoprotein (RNP) complex) with a guide nucleic acid molecule (e.g., sgRNA), which complex can bind a target polynucleotide sequence to modulate a target gene. The guide nucleic acid molecule can comprise (i) a scaffold sequence to at least form a complex with the Cas protein and (ii) a spacer sequence that exhibits at least partial sequence complementarity to the target polynucleotide sequence. Without wishing to be bound by theory, generating a smaller and more compact guide nucleic acid scaffold sequence (e.g., as compared to that for dCasMINI) can enhance the bioactivity of the complex overall (e.g., enhanced modulation of target gene., reduced off-target effects, etc.).

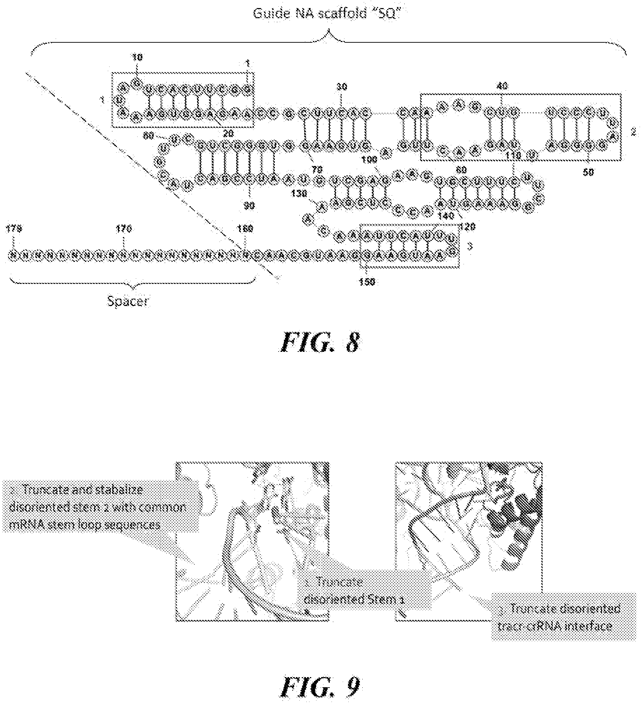

A control guide nucleic acid used throughout this Example is denoted as "SQ" (see schematic in FIG. 8 and its scaffold polynucleotide sequence in TABLE 6B). The control guide nucleic acid SQ is 179 nucleotide (nt) long, including a 20 nt spacer sequence.

Without wishing to be bound by theory, one or more of the following regions from a guide nucleic acid sequence (e.g., SQ) can be modified (e.g., mutated or deleted) while either maintaining the bioactivity of the resulting RNP complex or improving such bioactivity. As shown in FIG. 9, such regions can include structurally disoriented and/or solvent exposed loops (e.g., stem loops) within the scaffold sequence. For the SQ guide nucleic acid sequence, non-limiting examples of such region can include region 1 (e.g., 1-23 nt), region 2 (e.g., 35-61 nt), and region 3 (e.g., 138-143 nt), each nt position relative to the polynucleotide sequence of SQ.

### A. Truncated guide nucleic acid molecules

Guide RNA scaffold variant were generated to comprise combinations of stepwise deletions in the three regions discussed above. For region 1, stepwise base pair trimming was performed. For region 2, in addition to removing the disoriented stem loop, we installed stable hairpin structures commonly found in RNA (e.g., cUUCGg). For region 3 and its adjacent sequences, one or more deletions was performed (e.g., deletion of 136-149 nt), and additional stepwise truncations of other sequence(s) was performed to further reduce the scaffold size near region 3.

Gene repression was assessed by testing the gRNA scaffold variants with an engineered nuclease variant that is fused with a gene repressor. Each gRNA scaffold variant was individually cloned into the sgRNA plasmid with identical spacer sequence targeting the gene of interest. In 96-well plate format, HEK293T GFP reporter cells ESR221 (in each well) were transfected with identical dCas plasmid (e.g., encoding a gene suppression modulator, e.g., a dCas (such as dCasMINI as disclosed herein) that is fused with a gene repressor, such as KRAB), and individual sgRNA variant plasmid as triplicate or quadruplicate repeats. After five to seven days post-transfection, suppression of GFP expression was measured by flow cytometry. The level of GFP suppression of each gRNA scaffold variant was compared to the activity of the SQ control gRNA.

Gene activation was assessed by testing the gRNA scaffold variants with an engineered nuclease variant that is fused with a gene activator. Each gRNA scaffold variant was individually cloned into the sgRNA plasmid with identical spacer sequence targeting the gene of interest. HEK293T cells were transfected with identical dCas plasmid (e.g., encoding a gene activation modulator, e.g., a dCas (such as dCasMINI as disclosed herein) that is fused with a gene activator, such as VPR), and individual sgRNA variant plasmid as triplicate or quadruplicate repeats in 96-well plate format. Two to four days post-transfection, target gene activation was measured (e.g., CD2 protein levels were quantified by cell surface antibody staining of live cells followed by flow cytometry, while secreted IFNγ protein levels were measured using ELISA on cell culture supernatants). The level of gene activation of each gRNA scaffold variant was compared to the activity of the SQ control gRNA.

### B. First round

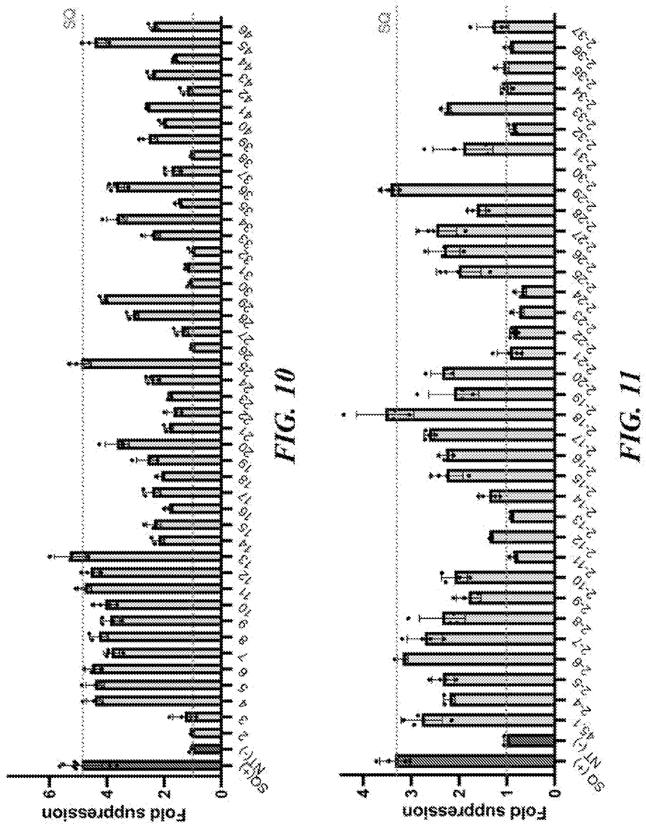

In the first round, gRNA scaffold variants provided in TABLE 6B (e.g., SEQ ID NOs: 501-554 and 600) and the control gRNA scaffold sequence SQ (SEQ ID NO: 500) were tested with identical spacer sequence targeting synthetic GFP promoter region and with an identical gene suppression modulator. On day 5 post-transfection, GFP expression levels were quantified by flow cytometry and normalized to the negative control (SQ scaffold with non-targeting spacer sequence). As shown in FIG. 10 and summarized in TABLE 6A, various gRNA scaffold variants that were smaller than the SQ positive control exhibited either (i) comparable gene repression to the SQ positive control or (ii) greater gene repression than the SQ positive control (e.g., SEQ ID NOs: 503-152, 519, 524, 528, 553, etc.).

### C. Second round

In the second round, gRNA scaffold variants provided in TABLE 7B (e.g., SEQ ID NOs: 555-588) and the control gRNA scaffold sequence SQ (SEQ ID NO: 500) were tested with identical spacer sequence targeting synthetic GFP promoter region and with an identical gene suppression modulator. On day 5 post-transfection, eGFP expression levels were quantified by flow cytometry and normalized to the negative control (SQ scaffold with non-targeting spacer sequence). As shown in FIG. 11 and summarized in TABLE 7A, various gRNA scaffold variants that were smaller than the SQ positive control exhibited either (i) comparable gene repression to the SQ positive control or (ii) greater gene repression than the SQ positive control (e.g., SEQ ID NOs: 555, 557, 558, 568, 569, 578, 580, etc.).

### D. Third round

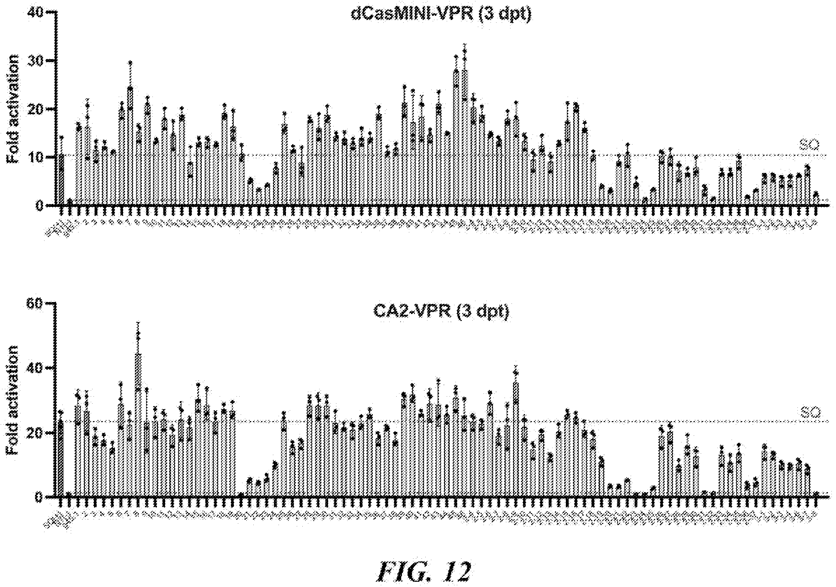

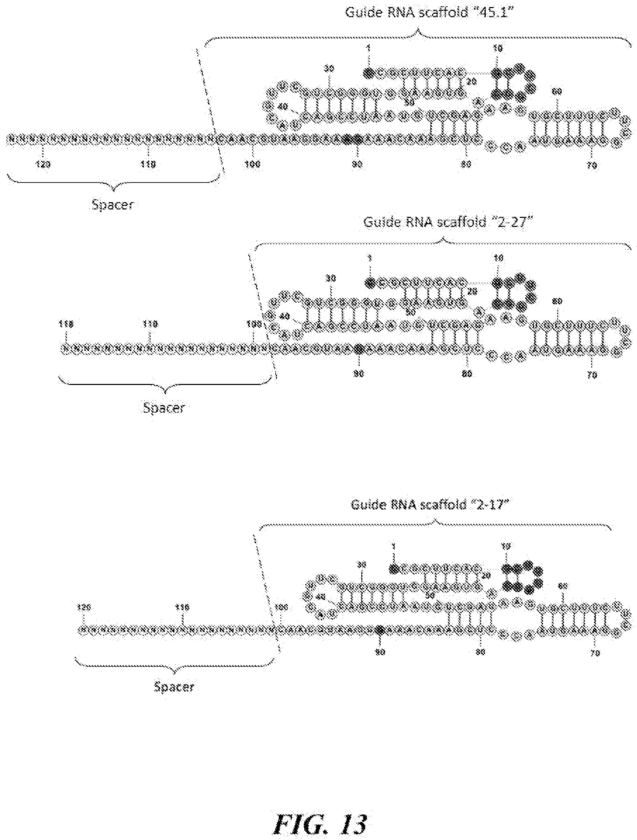

In the third round, lead gRNA scaffold variants (e.g., provided throughout TABLE 6B, TABLE 7B, and TABLE 8B) from the first and second rounds and the control gRNA scaffold sequence SQ (SEQ ID NO: 500) were tested with identical spacer sequence targeting upstream of CD2 locus (e.g., targeting endogenous DNA sequence) and with an identical gene activation modulator. On day 2 post-transfection, CD2 expression levels were quantified by antibody staining and flow cytometry and normalized to the negative control (SQ scaffold with non-targeting spacer sequence). As shown in FIG. 12 and summarized in TABLE 8A, various gRNA scaffold variants that were smaller than the SQ positive control exhibited either (i) comparable gene activation to the SQ positive control or (ii) greater gene activation than the SQ positive control (e.g., SEQ ID NOs: 555, 557, 568, 569, 576, 577, 578, 580, 593, 519, 528, etc.). For example, the gRNA scaffold variants such as SEQ ID NO: 555 (gRNA scaffold variant "45.1"), SEQ ID NO: 557 (gRNA scaffold variant "2-6"), SEQ ID NO: 556 (gRNA scaffold variant "2-17"), and SEQ ID NO: 578 (gRNA scaffold variant "2-27") resulted in greater CD2 activation than that by the SQ positive control (e.g., greater than 0.5-fold, greater than 1-fold, or greater than 1.5-fold increase in the degree of gene activation as compared to the SQ positive control). Single guide RNA molecules comprising the gRNA scaffold variants 45.1 and 2-27, and 2-17 are schematically illustrated in FIG. 13.

The gRNA scaffold variant disclosed herein (e.g., 45.1, 2-17 or 2-27) can be characterized to comprise at least a portion of its N-terminal sequence, e.g., the polynucleotide sequence of SEQ ID NO: 597 from TABLE 9.

The gRNA scaffold variant disclosed herein (e.g., 2-6) can be characterized to comprise at least a portion of its N-terminal sequence, e.g., the polynucleotide sequence of SEQ ID NO: 598 from TABLE 9.

### E. RNP complex comprising a gRNA scaffold variant and an engineered nuclease variant

A gRNA scaffold variant disclosed herein (e.g., 45.1 or SEQ ID NO: 555) can be used to generate a sgRNA, which can be used in conjunction with any of the engineered nuclease variants disclosed herein (e.g., SEQ ID NO: 12) to (i) reduce the size of a vector encoding the sgRNA and the engineered nuclease variant, as illustrated in FIG. 14, and/or (ii) further improve target gene modulation (e.g., greater degree or modulation, longer duration of modulation, etc.).

### TABLES

**TABLE 1. List of examples of target genes (e.g., encoding a protein of interest)**

| **List of target genes/proteins of interest** |
|---|
| A1BG, CCND3, FAM163A, KCNK10, NRG4, REM1, TECTB, A1CF, CCNDBP1, FAM163B, KCNK12, NRGN, REM2, TEDDM1, A2M, CCNE1, FAM166A, KCNK13, NRIP1, REN, TEF, A2ML1, CCNE2, FAM166B, KCNK15, NRIP2, RENBP, TEFM, A3GALT2, CCNF, FAM167A, KCNK16, NRIP3, REP15, TEK, A4GALT, CCNG1, FAM167B, KCNK17, NRK, REPIN1, TEKT1, A4GNT, CCNG2, FAM168A, KCNK18, NRL, REPS1, TEKT2, AAAS, CCNH, FAM168B, KCNK2, NRM, REPS2, TEKT3, AACS, CCNI, FAM169A, KCNK3, NRN1, RER1, TEKT4, AADAC, CCNI2, FAM169B, KCNK4, NRN1L, RERE, TEKT5, AADACL2, CCNJ, FAM170A, KCNK5, NRP1, RERG, TELO2, AADACL3, CCNJL, FAM170B, KCNK6, NRP2, RERGL, TEN1, AADACL4, CCNK, FAM171A1, KCNK7, NRROS, RESP18, TENC1, AADAT, CCNL1, FAM171A2, KCNK9, NRSN1, REST, TENM1, AAED1, CCNL2, FAM171B, KCNMA1, NRSN2, RET, TENM2, AAGAB, CCNO, FAM172A, KCNMB1, NRTN, RETN, TENM3, AAK1, CCNT1, FAM173A, KCNMB2, NRXN1, RETNLB, TENM4, AAMDC, CCNT2, FAM173B, KCNMB3, NRXN2, RETSAT, TEP1, AAMP, CCNY, FAM174A, KCNMB4, NRXN3, REV1, TEPP, AANAT, CCNYL1, FAM174B, KCNN1, NSA2, REV3L, TERF1, AAR2, CCP110, FAM175A, KCNN2, NSD1, REXO1, TERF2, AARD, CCPG1, FAM175B, KCNN3, NSDHL, REXO2, TERF2IP, AARS, CCR1, FAM177A1, KCNN4, NSF, REXO4, TERT, AARS2, CCR10, FAM177B, KCNQ1, NSFL1C, RFC1, TES, AARSD1, CCR2, FAM178A, KCNQ2, NSG1, RFC2, TESC, AASDH, CCR3, FAM178B, KCNQ3, NSL1, RFC3, TESK1, AASDHPPT, CCR4, FAM179A, KCNQ4, NSMAF, RFC4, TESK2, AASS, CCR5, FAM179B, KCNQ5, NSMCE1, RFC5, TESPA1, AATF, CCR6, FAM180A, KCNRG, NSMCE2, RFESD, TET1, AATK, CCR7, FAM180B, KCNS1, NSMCE4A, RFFL, TET2, ABAT, CCR8, FAM181A, KCNS2, NSMF, RFK, TET3, ABCA1, CCR9, FAM181B, KCNS3, NSRP1, RFNG, TEX10, ABCA10, CCRL2, FAM183A, KCNT1, NSUN2, RFPL1, TEX101, ABCA12, CCRN4L, FAM184A, KCNT2, NSUN3, RFPL2, TEX11, ABCA13, CCS, FAM184B, |
| KCNU1, NSUN4, RFPL3, TEX12, ABCA2, CCSAP, FAM185A, KCNV1, NSUN5, RFPL4A, TEX13A, ABCA3, CCSER1, FAM186A, KCNV2, NSUN6, RFPL4AL1, TEX13B, ABCA4, CCSER2, FAM186B, KCP, NSUN7, RFPL4B, TEX14, ABCA5, CCT2, FAM187B, KCTD1, NT5C, RFT1, TEX15, ABCA6, CCT3, FAM188A, KCTD10, NT5C1A, RFTN1, TEX19, ABCA7, CCT4, FAM188B, KCTD11, NT5C1B, RFTN2, TEX2, ABCA8, CCT5, FAM189A1, KCTD12, NT5C1B-RDH14, RFWD2, TEX22, ABCA9, CCT6A, FAM189A2, KCTD13, NT5C2, RFWD3, TEX26, ABCB1, CCT6B, FAM189B, KCTD14, NT5C3A, RFX1, TEX261, ABCB10, CCT7, FAM192A, KCTD15, NT5C3B, RFX2, TEX264, ABCB11, CCT8, FAM193A, KCTD16, NT5DC1, RFX3, TEX28, ABCB4, CCT8L2, FAM193B, KCTD17, NT5DC2, RFX4, TEX29, ABCB5, CCZ1, FAM194A, KCTD18, NT5DC3, RFX5, TEX30, ABCB6, CCZ1B, FAM194B, KCTD19, NT5E, RFX6, TEX33, ABCB7, CD101, FAM195A, KCTD2, NT5M, RFX7, TEX35, ABCB8, CD109, FAM195B, KCTD20, NTAN1, RFX8, TEX36, ABCB9, CD14, FAM196A, KCTD21, NTF3, RFXANK, TEX37, ABCC1, CD151, FAM196B, KCTD3, NTF4, RFXAP, TEX38, ABCC10, CD160, FAM198A, KCTD4, NTHL1, RGAG1, TEX40, ABCC11, CD163, FAM198B, KCTD5, NTM, RGAG4, TEX9, ABCC12, CD163L1, FAM199X, KCTD6, NTMT1, RGCC, TF, ABCC2, CD164, FAM19A1, KCTD7, NTN1, RGL1, TFAM, ABCC3, CD164L2, FAM19A2, KCTD8, NTN3, RGL2, TFAP2A, ABCC4, CD177, FAM19A3, KCTD9, NTN4, RGL3, TFAP2B, ABCC5, CD180, FAM19A4, KDELC1, NTN5, RGL4, TFAP2C, ABCC6, CD19, FAM19A5, KDELC2, NTNG1, RGMA, TFAP2D, ABCC8, CD1A, FAM200A, KDELR1, NTNG2, RGMB, TFAP2E, ABCC9, CD1B, FAM203A, KDELR2, NTPCR, RGN, TFAP4, ABCD1, CD1C, FAM203B, KDELR3, NTRK1, RGP1, TFB1M, ABCD2, CD1D, FAM204A, KDM1A, NTRK2, RGPD1, TFB2M, ABCD3, CD1E, FAM205A, KDM1B, NTRK3, RGPD2, TFCP2, ABCD4, CD2, FAM206A, KDM2A, NTS, RGPD3, TFCP2L1, ABCE1, CD200, FAM207A, KDM2B, NTSR1, RGPD4, TFDP1, ABCF1, CD200R1, FAM208A, KDM3A, NTSR2, RGPD5, TFDP2, ABCF2, CD200R1L, FAM208B, KDM3B, NUAK1, RGPD6, TFDP3, ABCF3, CD207, FAM209A, KDM4A, NUAK2, RGPD8, TFE3, ABCG1, CD209, FAM209B, KDM4B, NUB1, RGR, TFEB, ABCG2, CD22, FAM20A, KDM4C, NUBP1, RGS1, TFEC, ABCG4, CD226, FAM20B, KDM4D, NUBP2, RGS10, TFF1, ABCG5, CD24, FAM20C, KDM4E, NUBPL, RGS11, TFF2, ABCG8, CD244, FAM210A, KDM5A, NUCB1, RGS12, TFF3, ABHD1, CD247, FAM210B, KDM5B, NUCB2, RGS13, TFG, ABHD10, CD248, FAM211A, KDM5C, NUCKS1, RGS14, TFIP11, ABHD11, CD27, FAM211B, KDM5D, NUDC, RGS16, TFPI, ABHD12, CD274, FAM212A, KDM6A, NUDCD1, RGS17, TFPI2, ABHD12B, CD276, FAM212B, KDM6B, NUDCD2, RGS18, TFPT, ABHD13, CD28, FAM213A, KDM8, NUDCD3, RGS19, TFR2, ABHD14A, CD2AP, FAM213B, KDR, NUDT1, RGS2, TFRC, ABHD14B, CD2BP2, FAM214A, KDSR, NUDT10, RGS20, TG, ABHD15, CD300A, FAM214B, KEAP1, NUDT11, RGS21, TGDS, ABHD16A, CD300C, FAM216A, KEL, NUDT12, RGS22, TGFA, ABHD16B, CD300E, FAM216B, KERA, NUDT13, RGS3, TGFB1, ABHD17A, CD300LB, FAM217A, KHDC1, NUDT14, RGS4, TGFB1I1, ABHD17B, CD300LD, FAM217B, KHDC1L, NUDT15, RGS5, TGFB2, ABHD17C, CD300LF, FAM218A, KHDC3L, NUDT16, RGS6, TGFB3, |
| ABHD2, CD300LG, FAM219A, KHDRBS1, NUDT16L1, RGS7, TGFBI, ABHD3, CD302, FAM219B, KHDRBS2, NUDT17, RGS7BP, TGFBR1, ABHD4, CD320, FAM21A, KHDRBS3, NUDT18, RGS8, TGFBR2, ABHD5, CD33, FAM21B, KHK, NUDT19, RGS9, TGFBR3, ABHD6, CD34, FAM21C, KHNYN, NUDT2, RGS9BP, TGFBR3L, ABHD8, CD36, FAM220A, KHSRP, NUDT21, RGSL1, TGFBRAP1, ABI1, CD37, FAM221A, KIAA0020, NUDT22, RHAG, TGIF1, ABI2, CD38, FAM221B, KIAA0040, NUDT3, RHBDD1, TGIF2, ABI3, CD3D, FAM222A, KIAA0100, NUDT4, RHBDD2, TGIF2-C20orf24, ABI3BP, CD3E, FAM222B, KIAA0101, NUDT5, RHBDD3, TGIF2LX, ABL1, CD3EAP, FAM227A, HIAA0141, NUDT6, RHBDF1, TGIF2LY, ABL2, CD3G, FAM227B, KIAA0195, NUDT7, RHBDF2, TGM1, ABLIM1, CD4, FAM228A, KIAA0196, NUDT8, RHBDL1, TGM2, ABLIM2, CD40, FAM228B, KIAA0226, NUDT9, RHBDL2, TGM3, ABLIM3, CD40LG, FAM229A, KIAA0226L, NUF2, RHBDL3, TGM4, ABO, CD44, FAM229B, KIAA0232, NUFIP1, RHBG, TGM5, ABR, CD46, FAM230A, KIAA0247, NUFIP2, RHCE, TGM6, ABRA, CD47, FAM24A, KIAA0319, NUGGC, RHCG, TGM7, ABRACL, CD48, FAM24B, KIAA0319L, NUMA1, RHD, TGOLN2, ABT1, CD5, FAM25A, KIAA0355, NUMB, RHEB, TGS1, ABTB1, CD52, FAM25C, KIAA0368, NUMBL, RHEBL1, TH, ABTB2, CD53, FAM25G, KIAA0391, NUP107, RHNO1, THADA, ACAA1, CD55, FAM26D, KIAA0408, NUP133, RHO, THAP1, ACAA2, CD58, FAM26E, KIAA0430, NUP153, RHOA, THAP10, ACACA, CD59, FAM26F, KIAA0513, NUP155, RHOB, THAP11, ACACB, CD5L, FAM32A, KIAA0556, NUP160, RHOBTB1, THAP2, ACAD10, CD6, FAM35A, KIAA0586, NUP188, RHOBTB2, THAP3, ACAD11, CD63, FAM3A, KIAA0753, NUP205, RHOBTB3, THAP4, ACAD8, CD68, FAM3B, KIAA0754, NUP210, RHOC, THAP5, ACAD9, CD69, FAM3C, KIAA0825, NUP210L, RHOD, THAP6, ACADL, CD7, FAM3D, KIAA0895, NUP214, RHOF, THAP7, ACADM, CD70, FAM43A, KIAA0895L, NUP35, RHOG, THAP8, ACADS, CD72, FAM43B, KIAA0907, NUP37, RHOH, THAP9, ACADSB, CD74, FAM45A, KIAA0922, NUP43, RHOJ, THBD, ACADVL, CD79A, FAM46A, KIAA0930, NUP50, RHOQ, THBS1, ACAN, CD79B, FAM46B, KIAA0947, NUP54, RHOT1, THBS2, ACAP1, CD80, FAM46C, KIAA1009, NUP62, RHOT2, THBS3, ACAP2, CD81, FAM46D, KIAA1024, NUP62CL, RHOU, THBS4, ACAP3, CD82, FAM47A, KIAA1024L, NUP85, RHOV, THEG, ACAT1, CD83, FAM47B, KIAA1033, NUP88, RHOXF1, THEG5, ACAT2, CD84, FAM47C, KIAA1045, NUP93, RHOXF2, THEGL, ACBD3, CD86, FAM47E, KIAA1107, NUP98, RHOXF2B, THEM4, ACBD4, CD8A, FAM47E-STBD1, KIAA1109, NUPL1, RHPN1, THEM5, ACBD5, CD8B, FAM49A, KIAA1143, NUPL2, RHPN2, THEM6, ACBD6, CD9, FAM49B, KIAA1147, NUPR1, RIBC1, THEMIS, ACBD7, CD93, FAM50A, KIAA1161, NUPR1L, RIBC2, THEMIS2, ACCS, CD96, FAM50B, KIAA1191, NUS1, RIC3, THG1L, ACCSL, CD97, FAM53A, KIAA1199, NUSAP1, RIC8A, THNSL1, ACD, CD99, FAM53B, KIAA1210, NUTF2, RIC8B, THNSL2, ACE, CD99L2, FAM53C, KIAA1211, NUTM1, RICTOR, THOC1, ACE2, CDA, FAM57A, KIAA1211L, NUTM2A, RIF1, THOC2, ACER1, CDADC1, FAM57B, KIAA1217, NUTM2B, RIIAD1, THOC3, ACER2, CDAN1, FAM58A, KIAA1239, NUTM2F, RILP, THOC5, ACER3, CDC123, FAM60A, KIAA1244, NUTM2G, |
| RILPL1, THOC6, ACHE, CDC14A, FAM63A, KIAA1257, NVL, RILPL2, THOC7, ACIN1, CDC14B, FAM63B, KIAA1279, NWD1, RIMBP2, THOP1, ACKR1, CDC16, FAM64A, KIAA1324, NXF1, RIMBP3, THPO, ACKR2, CDC20, FAM65A, KIAA1324L, NXF2, RIMBP3B, THRA, ACKR3, CDC20B, FAM65B, KIAA1328, NXF2B, RIMBP3C, THRAP3, ACKR4, CDC23, FAM65C, KIAA1377, NXF3, RIMKLA, THRB, ACLY, CDC25A, FAM69A, KIAA1407, NXF5, RIMKLB, THRSP, ACMSD, CDC25B, FAM69B, KIAA1429, NXN, RIMS1, THSD1, ACN9, CDC25C, FAM69C, KIAA1430, NXNL1, RIMS2, THSD4, ACO1, CDC26, FAM71A, KIAA1432, NXNL2, RIMS3, THSD7A, ACO2, CDC27, FAM71B, KIAA1456, NXPE1, RIMS4, THSD7B, ACOT1, CDC34, FAM71C, KIAA1462, NXPE2, RIN1, THTPA, ACOT11, CDC37, FAM71D, KIAA1467, NXPE3, RIN2, THUMPD1, ACOT12, CDC37L1, FAM71E1, KIAA1468, NXPE4, RIN3, THUMPD2, ACOT13, CDC40, FAM71E2, KIAA1522, NXPH1, RING1, THUMPD3, ACOT2, CDC42, FAM71F1, KIAA1524, NXPH2, RINL, THY1, ACOT4, CDC42BPA, FAM71F2, KIAA1549, NXPH3, RINT1, THYN1, ACOT6, CDC42BPB, FAM72A, KIAA1549L, NXPH4, RIOK1, TIA1, ACOT7, CDC42BPG, FAM72B, KIAA1551, NXT1, RIOK2, TIAF1, ACOT8, CDC42EP1, FAM72D, KIAA1586, NXT2, RIOK3, TIAL1, ACOT9, CDC42EP2, FAM73A, KIAA1598, NYAP1, RIPK1, TIAM1, ACOX1, CDC42EP3, FAM73B, KIAA1614, NYAP2, RIPK2, TIAM2, ACOX2, CDC42EP4, FAM76A, KIAA1644, NYNRIN, RIPK3, TICAM1, ACOX3, CDC42EP5, FAM76B, KIAA1671, NYX, RIPK4, TICAM2, ACOXL, CDC42SE1, FAM78A, KIAA1683, OAF, RIPPLY1, TICRR, ACP1, CDC42SE2, FAM78B, KIAA1715, OARD1, RIPPLY2, TIE1, ACP2, CDC45, FAM81A, KIAA1731, OAS1, RIPPLY3, TIFA, ACP5, CDC5L, FAM81B, KIAA1737, OAS2, RIT1, TIFAB, ACP6, CDC6, FAM83A, KIAA1751, OAS3, RIT2, TIGD2, ACPL2, CDC7, FAM83B, KIAA1755, OASL, RLBP1, TIGD3, ACPP, CDC73, FAM83C, KIAA1804, OAT, RLF, TIGD4, ACPT, CDCA2, FAM83D, KIAA1841, OAZ1, RLIM, TIGD5, ACR, CDCA3, FAM83E, KIAA1919, OAZ2, RLN1, TIGD6, ACRBP, CDCA4, FAM83F, KIAA1958, OAZ3, RLN2, TIGD7, ACRC, CDCA5, FAM83G, KIAA1984, OBFC1, RLN3, TIGIT, ACRV1, CDCA7, FAM83H, KIAA2013, OBP2A, RLTPR, TIMD4, ACSBG1, CDCA7L, FAM84A, KIAA2018, OBP2B, RMDN1, TIMELESS, ACSBG2, CDCA8, FAM84B, KIAA2022, OBSCN, RMDN2, TIMM10, ACSF2, CDCP1, FAM86A, KIAA2026, OBSL1, RMDN3, TIMM10B, ACSF3, CDCP2, FAM86B1, KIDINS220, OC90, RMI1, TIMM13, ACSL1, CDH1, FAM86B2, KIF11, OCA2, RMI2, TIMM17A, ACSL3, CDH10, FAM86C1, KIF12, OCEL1, RMND1, TIMM17B, ACSL4, CDH11, FAM86KP, KIF13A, OCIAD1, RMND5A, TIMM21, ACSL5, CDH12, FAM89A, KIF13B, OCIAD2, RMND5B, TIMM22, ACSL6, CDH13, FAM89B, KIF14, OCLM, RNASE1, TIMM23, ACSM1, CDH15, FAM8A1, KIF15, OCLN, RNASE10, TIMM23B, ACSM2A, CDH16, FAM90A1, KIF16B, OCM, RNASE11, TIMM44, ACSM2B, CDH17, FAM91A1, KIF17, OCM2, RNASE12, TIMM50, ACSM3, CDH18, FAM92A1, KIF18A, OCRL, RNASE13, TIMM8A, ACSM4, CDH19, FAM92B, KIF18B, OCSTAMP, RNASE2, TIMM8B, ACSM5, CDH2, FAM96A, KIF19, ODAM, RNASE3, TIMM9, ACSS1, CDH20, FAM96B, KIF1A, ODC1, RNASE4, TIMMDC1, ACSS2, CDH22, FAM98A, KIF1B, ODF1, RNASE6, TIMP1, ACSS3, CDH23, FAM98B, KIF1C, |
| ODF2, RNASE7, TIMP2, ACTA1, CDH24, FAM98C, KIF20A, ODF2L, RNASE8, TIMP3, ACTA2, CDH26, FAM9A, KIF20B, ODF3, RNASE9, TIMP4, ACTB, CDH3, FAM9B, KIF21A, ODF3B, RNASEH1, TINAG, ACTBL2, CDH4, FAM9C, KIF21B, ODF3L1, RNASEH2A, TINAGL1, ACTC1, CDH5, FAN1, KIF22, ODF3L2, RNASEH2B, TINF2, ACTG1, CDH6, FANCA, KIF23, ODF4, RNASEH2C, TIPARP, ACTG2, CDH7, FANCB, KIF24, OFCC1, RNASEK, TIPIN, ACTL10, CDH8, FANCC, KIF25, OFD1, RNASEL, TIPRL, ACTL6A, CDH9, FANCD2, KIF26A, OGDH, RNASET2, TIRAP, ACTL6B, CDHR1, FANCD2OS, KIF26B, OGDHL, RND1, TJAP1, ACTL7A, CDHR2, FANCE, KIF27, OGFOD1, RND2, TJP1, ACTL7B, CDHR3, FANCF, KIF28P, OGFOD2, RND3, TJP2, ACTL8, CDHR4, FANCG, KIF2A, OGFOD3, RNF10, TJP3, ACTL9, CDHR5, FANCI, KIF2B, OGFR, RNF103, TK1, ACTN1, CDIP1, FANCL, KIF2C, OGFRL1, RNF103-CHMP3, TK2, ACTN2, CDIPT, FANCM, KIF3A, OGG1, RNF11, TKT, ACTN3, CDK1, FANK1, KIF3B, OGN, RNF111, TKTL1, ACTN4, CDK10, FAP, KIF3C, OGT, RNF112, TKTL2, ACTR10, CDK11A, FAR1, KIF4A, OIP5, RNF113A, TLCD1, ACTR1A, CDK11B, FAR2, KIF4B, OIT3, RNF113B, TLCD2, ACTR1B, CDK12, FARP1, KIF5A, OLA1, RNF114, TLDC1, ACTR2, CDK13, FARP2, KIF5B, OLAH, RNF115, TLDC2, ACTR3, CDK14, FARS2, KIF5C, OLFM1, RNF121, TLE1, ACTR3B, CDK15, FARSA, KIF6, OLFM2, RNF122, TLE2, ACTR3C, CDK16, FARSB, KIF7, OLFM3, RNF123, TLE3, ACTR5, CDK17, FAS, KIF9, OLFM4, RNF125, TLE4, ACTR6, CDK18, FASLG, KIFAP3, OLFML1, RNF126, TLE6, ACTR8, CDK19, FASN, KIFC1, OLFML2A, RNF128, TLK1, ACTRT1, CDK2, FASTK, KIFC2, OLFML2B, RNF13, TLK2, ACTRT2, CDK20, FASTKD1, KIFC3, OLFML3, RNF130, TLL1, ACTRT3, CDK2AP1, FASTKD2, KIN, OLIG1, RNF133, TLL2, ACVR1, CDK2AP2, FASTKD3, KIR2DL1, OLIG2, RNF135, TLN1, ACVR1B, CDK3, FASTKD5, KIR2DL3, OLIG3, RNF138, TLN2, ACVR1C, CDK4, FAT1, KIR2DL4, OLR1, RNF139, TLR1, ACVR2A, CDK5, FAT2, KIR2DS4, OMA1, RNF14, TLR10, ACVR2B, CDK5R1, FAT3, HIR3DL1, OMD, RNF141, TLR2, ACVRL1, CDK5R2, FAT4, KIR3DL2, OMG, RNF144A, TLR3, ACY1, CDK5RAP1, FATE1, KIR3DL3, OMP, RNF144B, TLR4, ACY3, CDK5RAP2, FAU, KIRREL, ONECUT1, RNF145, TLR5, ACYP1, CDK5RAP3, FAXC, KIRREL2, ONECUT2, RNF146, TLR6, ACYP2, CDK6, FAXDC2, KIRREL3, ONECUT3, RNF148, TLR7, ADA, CDK7, FBF1, KISS1, OOEP, RNF149, TLR8, ADAD1, CDK8, FBL, KISS1R, OOSP2, RNF150, TLR9, ADAD2, CDK9, FBLIM1, KIT, OPA1, RNF151, TLX1, ADAL, CDKAL1, FBLN1, KITLG, OPA3, RNF152, TLX1NB, ADAM10, CDKL1, FBLN2, KL, OPALIN, RNF157, TLX2, ADAM11, CDKL2, FBLN5, KLB, OPCML, RNF165, TLX3, ADAM12, CDKL3, FBLN7, KLC1, OPHN1, RNF166, TM2D1, ADAM15, CDKL4, FBN1, KLC2, OPLAH, RNF167, TM2D2, ADAM17, CDKL5, FBN2, KLC3, OPN1LW, RNF168, TM2D3, ADAM18, CDKN1A, FBN3, KLC4, OPN1MW, RNF169, TM4SF1, ADAM19, CDKN1B, FBP1, KLF1, OPN1MW2, RNF17, TM4SF18, ADAM2, CDKN1C, FBP2, KLF10, OPN1SW, RNF170, TM4SF19, ADAM20, CDKN2A, FBRS, KLF11, OPN3, RNF175, TM4SF20, ADAM21, CDKN2AIP, FBRSL1, KLF12, OPN4, RNF180, TM4SF4, ADAM22, CDKN2AIPNL, FBXL12, KLF13, OPN5, RNF181, TM4SF5, ADAM23, CDKN2B, FBXL13, KLF14, OPRD1, RNF182, TM6SF1, ADAM28, CDKN2C, FBXL14, |
| KLF15, OPRK1, RNF183, TM6SF2, ADAM29, CDKN2D, FBXL15, KLF16, OPRL1, RNF185, TM7SF2, ADAM30, CDKN3, FBXL16, KLF17, OPRM1, RNF186, TM7SF3, ADAM32, CDNF, FBXL17, KLF2, OPTC, RNF187, TM9SF1, ADAM33, CDO1, FBXL18, KLF3, OPTN, RNF19A, TM9SF2, ADAM7, CDON, FBXL19, KLF4, OR10A2, RNF19B, TM9SF3, ADAM8, CDPF1, FBXL2, KLF5, OR10A3, RNF2, TM9SF4, ADAM9, CDR1, FBXL20, KLF6, OR10A4, RNF20, TMA16, ADAMDEC1, CDR2, FBXL21, KLF7, OR10A5, RNF207, TMA7, ADAMTS1, CDR2L, FBXL22, KLF8, OR10A6, RNF208, TMBIM1, ADAMTS10, CDRT1, FBXL3, KLF9, OR10A7, RNF212, TMBIM4, ADAMTS12, CDRT15, FBXL4, KLHDC1, OR10AG1, RNF213, TMBIM6, ADAMTS13, CDRT15L2, FBXL5, KLHDC10, OR10C1, RNF214, TMC1, ADAMTS14, CDRT4, FBXL6, KLHDC2, OR10G2, RNF215, TMC2, ADAMTS15, CDS1, FBXL7, KLHDC3, OR10G3, RNF216, TMC3, ADAMTS16, CDS2, FBXL8, KLHDC4, OR10G4, RNF217, TMC4, ADAMTS17, CDSN, FBXO10, KLHDC7A, OR10G7, RNF219, TMC5, ADAMTS18, CDT1, FBXO11, KLHDC7B, OR10G8, RNF220, TMC6, ADAMTS19, CDV3, FBXO15, KLHDC8A, OR10G9, RNF222, TMC7, ADAMTS2, CDX1, FBXO16, KLHDC8B, OR10H1, RNF223, TMC8, ADAMTS20, CDX2, FBXO17, KLHDC9, OR10H2, RNF224, TMCC1, ADAMTS3, CDX4, FBXO18, KLHL1, OR10H3, RNF24, TMCC2, ADAMTS4, CDY1, FBXO2, KLHL10, OR10H4, RNF25, TMCC3, ADAMTS5, CDY1B, FBXO21, KLHL11, OR10H5, RNF26, TMCO1, ADAMTS6, CDY2A, FBXO22, KLHL12, OR10J1, RNF31, TMCO2, ADAMTS7, CDY2B, FBXO24, KLHL13, OR10J3, RNF32, TMCO3, ADAMTS8, CDYL, FBXO25, KLHL14, OR10J5, RNF34, TMCO4, ADAMTS9, CDYL2, FBXO27, KLHL15, OR10K1, RNF38, TMCO5A, ADAMTSL1, CEACAM1, FBXO28, KLHL17, OR10K2, RNF39, TMCO6, ADAMTSL2, CEACAM16, FBXO3, KLHL18, OR10P1, RNF4, TMED1, ADAMTSL3, CEACAM18, FBXO30, KLHL2, OR10Q1, RNF40, TMED10, ADAMTSL4, CEACAM19, FBXO31, KLHL20, OR10R2, RNF41, TMED2, ADAMTSL5, CEACAM20, FBXO32, KLHL21, OR10S1, RNF43, TMED3, ADAP1, CEACAM21, FBXO33, KLHL22, OR10T2, RNF44, TMED4, ADAP2, CEACAM3, FBXO34, KLHL23, OR10V1, RNF5, TMED5, ADAR, CEACAM4, FBXO36, KLHL24, OR10W1, RNF6, TMED6, ADARB1, CEACAM5, FBXO38, KLHL25, OR10X1, RNF7, TMED7, ADARB2, CEACAM6, FBXO39, KLHL26, OR10Z1, RNF8, TMED7-TICAM2, ADAT1, CEACAM7, FBXO4, KLHL28, OR11A1, RNFT1, TMED8, ADAT2, CEACAM8, FBXO40, KLHL29, OR11G2, RNFT2, TMED9, ADAT3, CEBPA, FBXO41, KLHL3, OR11H1, RNGTT, TMEFF1, ADC, CEBPB, FBXO42, KLHL30, OR11H12, RNH1, TMEFF2, ADCK1, CEBPD, FBXO43, KLHL31, OR11H2, RNLS, TMEM100, ADCK2, CEBPE, FBXO44, KLHL32, OR11H4, RNMT, TMEM101, ADCK3, CEBPG, FBXO45, KLHL33, OR11H6, RNMTL1, TMEM102, ADCK4, CEBPZ, FBXO46, KLHL34, OR11L1, RNPC3, TMEM104, ADCK5, CECR1, FBXO47, KLHL35, OR12D2, RNPEP, TMEM105, ADCY1, CECR2, FBXO48, KLHL36, OR12D3, RNPEPL1, TMEM106A, ADCY10, CECR5, FBXO5, KLHL38, OR13A1, RNPS1, TMEM106B, ADCY2, CECR6, FBXO6, KLHL4, OR13C2, ROBO1, TMEM106C, ADCY3, CEL, FBXO7, KLHL40, OR13C3, ROBO2, TMEM107, ADCY4, CELA1, FBXO8, KLHL41, OR13C4, ROBO3, TMEM108, ADCY5, CELA2A, FBXO9, KLHL42, OR13C5, |
| ROBO4, TMEM109, ADCY6, CELA2B, FBXW10, KLHL5, OR13C8, ROCK1, TMEM11, ADCY7, CELA3A, FBXW11, KLHL6, OR13C9, ROCK2, TMEM110, ADCY8, CELA3B, FBXW12, KLHL7, OR13D1, ROGDI, TMEM110-MUSTN1, ADCY9, CELF1, FBXW2, KLHL8, OR13F1, ROM1, TMEM114, ADCYAP1, CELF2, FBXW4, KLHL9, OR13G1, ROMO1, TMEM115, ADCYAP1R1, CELF3, FBXW5, KLK1, OR13H1, ROPN1, TMEM116, ADD1, CELF4, FBXW7, KLK10, OR13J1, ROPN1B, TMEM117, ADD2, CELF5, FBXW8, KLK11, OR14A16, ROPN1L, TMEM119, ADD3, CELF6, FBXW9, KLK12, OR14C36, ROR1, TMEM120A, ADGB, CELSR1, FCAMR, KLK13, OR14J1, ROR2, TMEM120B, ADH1A, CELSR2, FCAR, KLK14, OR1A1, RORA, TMEM121, ADH1B, CELSR3, FCER1A, KLK15, OR1A2, RORB, TMEM123, ADH1C, CEMP1, FCER1G, KLK2, OR1B1, RORC, TMEM125, ADH4, CEND1, FCER2, KLK3, OR1C1, ROS1, TMEM126A, ADH5, CENPA, FCF1, KLK4, OR1D2, RP1, TMEM126B, ADH6, CENPB, FCGBP, KLK5, OR1D5, RP1L1, TMEM127, ADH7, CENPBD1, FCGR1A, KLK6, OR1E1, RP2, TMEM128, ADHFE1, CENPC, FCGR1B, KLK7, OR1E2, RP9, TMEM129, ADI1, CENPE, FCGR2A, KLK8, OR1F1, RPA1, TMEM130, ADIG, CENPF, FCGR2B, KLK9, OR1G1, RPA2, TMEM131, ADIPOQ, CENPH, FCGR2C, KLKB1, OR1I1, RPA3, TMEM132A, ADIPOR1, CENPI, FCGR3A, KLLN, OR1J1, RPA4, TMEM132B, ADIPOR2, CENPJ, FCGR3B, KLRB1, OR1J4, RPAIN, TMEM132C, ADIRF, CENPK, FCGRT, KLRC1, OR1K1, RPAP1, TMEM132D, ADK, CENPL, FCHO1, KLRC2, OR1L1, RPAP2, TMEM132E, ADM, CENPM, FCHO2, KLRC3, OR1L3, RPAP3, TMEM133, ADM2, CENPN, FCHSD1, KLRC4, OR1L4, RPE, TMEM134, ADM5, CENPO, FCHSD2, KLRC4-KLRK1, OR1L6, RPE65, TMEM135, ADNP, CENPP, FCN1, KLRD1, OR1L8, RPF1, TMEM136, ADNP2, CENPQ, FCN2, KLRF1, OR1M1, RPF2, TMEM138, ADO, CENPT, FCN3, KLRF2, OR1N1, RPGR, TMEM139, ADORA1, CENPU, FCRL1, KLRG1, OR1N2, RPGRIP1, TMEM140, ADORA2A, CENPV, FCRL2, KLRG2, OR1Q1, RPGRIP1L, TMEM141, ADORA2B, CENPW, FCRL3, KLRK1, OR1S1, RPH3A, TMEM143, ADORA3, CEP104, FCRL4, KMO, OR1S2, RPH3AL, TMEM144, ADPGK, CEP112, FCRL5, KMT2A, OR2A12, RPIA, TMEM145, ADPRH, CEP120, FCRL6, KMT2B, OR2A14, RPL10, TMEM147, ADPRHL1, CEP128, FCRLA, KMT2C, OR2A2, RPL10A, TMEM14A, ADPRHL2, CEP135, FCRLB, KMT2D, OR2A25, RPL10L, TMEM14B, ADPRM, CEP152, FDCSP, KMT2E, OR2A4, RPL11, TMEM14C, ADRA1A, CEP164, FDFT1, KNCN, OR2A5, RPL12, TMEM14E, ADRA1B, CEP170, FDPS, KNDC1, OR2A7, RPL13, TMEM150A, ADRA1D, CEP170B, FDX1, KNG1, OR2AE1, RPL13A, TMEM150B, ADRA2A, CEP19, FDX1L, KNOP1, OR2AG1, RPL14, TMEM150C, ADRA2B, CEP192, FDXACB1, KNSTRN, OR2AG2, RPL15, TMEM151A, ADRA2C, CEP250, FDXR, KNTC1, OR2AK2, RPL17, TMEM151B, ADRB1, CEP290, FECH, KPNA1, OR2AP1, RPL17-C18orf32, TMEM154, ADRB2, CEP350, FEM1A, KPNA2, OR2AT4, RPL18, TMEM155, ADRB3, CEP41, FEM1B, KPNA3, OR2B11, RPL18A, TMEM156, ADRBK1, CEP44, FEM1C, KPNA4, OR2B2, RPL19, TMEM158, ADRBK2, CEP55, FEN1, KPNA5, OR2B3, RPL21, TMEM159, ADRM1, CEP57, FER, KPNA6, OR2B6, RPL22, TMEM160, ADSL, CEP57L1, FER1L5, KPNA7, OR2C1, RPL22L1, TMEM161A, ADSS, CEP63, FER1L6, KPNB1, OR2C3, RPL23, TMEM161B, ADSSL1, CEP68, FERD3L, KPRP, |
| OR2D2, RPL23A, TMEM163, ADTRP, CEP70, FERMT1, KPTN, OR2D3, RPL24, TMEM164, AEBP1, CEP72, FERMT2, KRAS, OR2F1, RPL26, TMEM165, AEBP2, CEP76, FERMT3, KRBA1, OR2F2, RPL26L1, TMEM167A, AEN, CEP78, FES, KRBA2, OR2G2, RPL27, TMEM167B, AES, CEP85, FETUB, KRBOX1, OR2G3, RPL27A, TMEM168, AFAP1, CEP85L, FEV, KRBOX4, OR2G6, RPL28, TMEM169, AFAP1L1, CEP89, FEZ1, KRCC1, OR2H1, RPL29, TMEM17, AFAP1L2, CEP95, FEZ2, KREMEN1, OR2J2, RPL3, TMEM170A, AFF1, CEP97, FEZF1, KREMEN2, OR2J3, RPL30, TMEM170B, AFF2, CEPT1, FEZF2, KRI1, OR2K2, RPL31, TMEM171, AFF3, CER1, FFAR1, KRIT1, OR2L13, RPL32, TMEM173, AFF4, CERCAM, FFAR2, KRR1, OR2L2, RPL34, TMEM174, AFG3L2, CERK, FFAR3, KRT1, OR2L3, RPL35, TMEM175, AFM, CERKL, FFAR4, KRT10, OR2L5, RPL35A, TMEM176A, AFMID, CERS1, FGA, KRT12, OR2L8, RPL36, TMEM176B, AFP, CERS2, FGB, KRT13, OR2M2, RPL36A, TMEM177, AFTPH, CERS3, FGD1, KRT14, OR2M3, RPL36A-HNRNPH2, TMEM178A, AGA, CERS4, FGD2, KRT15, OR2M4, RPL36AL, TMEM178B, AGAP1, CERS5, FGD3, KRT16, OR2M5, RPL37, TMEM179, AGAP10, CERS6, FGD4, KRT17, OR2M7, RPL37A, TMEM179B, AGAP11, CES1, FGD5, KRT18, OR2S2, RPL38, TMEM18, AGAP2, CES2, FGD6, KRT19, OR2T1, RPL39, TMEM180, AGAP3, CES3, FGF1, KRT2, OR2T10, RPL39L, TMEM181, AGAP4, CES4A, FGF10, KRT20, OR2T11, RPL3L, TMEM182, AGAP5, CES5A, FGF11, KRT222, OR2T12, RPL4, TMEM183A, AGAP6, CETN1, FGF12, KRT23, OR2T2, RPL41, TMEM183B, AGAP7, CETN2, FGF13, KRT24, OR2T27, RPL5, TMEM184A, AGAP8, CETN3, FGF14, KRT25, OR2T29, RPL6, TMEM184B, AGAP9, CETP, FGF16, KRT26, OR2T3, RPL7, TMEM184C, AGBL1, CFB, FGF17, KRT27, OR2T33, RPL7A, TMEM185A, AGBL2, CFC1, FGF18, KRT28, OR2T34, RPL7L1, TMEM185B, AGBL3, CFC1B, FGF19, KRT3, OR2T35, RPL8, TMEM186, AGBL4, CFD, FGF2, KRT31, OR2T4, RPL9, TMEM187, AGBL5, CFDP1, FGF20, KRT32, OR2T5, RPLP0, TMEM189, AGER, CFH, FGF21, KRT33A, OR2T6, RPLP1, TMEM189-UBE2V1, AGFG1, CFHR1, FGF22, KRT33B, OR2T8, RPLP2, TMEM19, AGFG2, CFHR2, FGF23, KRT34, OR2V1, RPN1, TMEM190, AGGF1, CFHR3, FGF3, KRT35, OR2V2, RPN2, TMEM191B, AGK, CFHR4, FGF4, KRT36, OR2W1, RPP14, TMEM191C, AGL, CFHR5, FGF5, KRT37, OR2W3, RPP21, TMEM192, AGMAT, CFI, FGF6, KRT38, OR2W5, RPP25, TMEM194A, AGMO, CFL1, FGF7, KRT39, OR2Y1, RPP25L, TMEM194B, AGO1, CFL2, FGF8, KRT4, OR2Z1, RPP30, TMEM196, AGO2, CFLAR, FGF9, KRT40, OR3A1, RPP38, TMEM198, AGO3, CFP, FGFBP1, KRT5, OR3A2, RPP40, TMEM199, AGO4, CFTR, FGFBP2, KRT6A, OR3A3, RPRD1A, TMEM2, AGPAT1, CGA, FGFBP3, KRT6B, OR4A15, RPRD1B, TMEM200A, AGPAT2, CGB, FGFR1, KRT6C, OR4A16, RPRD2, TMEM200B, AGPAT3, CGB1, FGFR1OP, KRT7, OR4A47, RPRM, TMEM201, AGPAT4, CGB2, FGFR1OP2, KRT71, OR4A5, RPRML, TMEM202, AGPAT5, CGB5, FGFR2, KRT72, OR4B1, RPS10, TMEM203, AGPAT6, CGB7, FGFR3, KRT73, OR4C11, RPS10-NUDT3, TMEM204, AGPAT9, CGB8, FGFR4, KRT74, OR4C12, RPS11, TMEM205, AGPS, CGGBP1, FGFRL1, KRT75, OR4C13, RPS12, TMEM206, AGR2, CGN, FGG, KRT76, OR4C15, RPS13, TMEM207, AGR3, CGNL1, FGGY, KRT77, OR4C16, RPS14, TMEM208, AGRN, CGREF1, FGL1, KRT78, |
| OR4C3, RPS15, TMEM209, AGRP, CGRRF1, FGL2, KRT79, OR4C45, RPS15A, TMEM210, AGT, CH25H, FGR, KRT8, OR4C46, RPS16, TMEM211, AGTPBP1, CHAC1, FH, KRT80, OR4C6, RPS17, TMEM212, AGTR1, CHAC2, FHAD1, KRT81, OR4D1, RPS17L, TMEM213, AGTR2, CHAD, FHDC1, KRT82, OR4D10, RPS18, TMEM214, AGTRAP, CHADL, FHIT, KRT83, OR4D11, RPS19, TMEM215, AGXT, CHAF1A, FHL1, KRT84, OR4D2, RPS19BP1, TMEM216, AGXT2, CHAF1B, FHL2, KRT85, OR4D5, RPS2, TMEM217, AHCTF1, CHAMP1, FHL3, KRT86, OR4D6, RPS20, TMEM218, AHCY, CHAT, FHL5, KRT9, OR4D9, RPS21, TMEM219, AHCYL1, CHCHD1, FHOD1, KRTAP10-1, OR4E2, RPS23, TMEM220, AHCYL2, CHCHD10, FHOD3, KRTAP10-10, OR4F15, RPS24, TMEM221, AHDC1, CHCHD2, FIBCD1, KRTAP10-11, OR4F16, RPS25, TMEM222, AHI1, CHCHD3, FIBIN, KRTAP10-12, OR4F21, RPS26, TMEM223, AHNAK, CHCHD4, FIBP, KRTAP10-2, OR4F29, RPS27, TMEM225, AHNAK2, CHCHD5, FICD, KRTAP10-3, OR4F3, RPS27A, TMEM229A, AHR, CHCHD6, FIG4, KRTAP10-4, OR4F4, RPS27L, TMEM229B, AHRR, CHCHD7, FIGF, KRTAP10-5, OR4F5, RPS28, TMEM230, AHSA1, CHD1, FIGLA, KRTAP10-6, OR4F6, RPS29, TMEM231, AHSA2, CHD1L, FIGN, KRTAP10-7, OR4K1, RPS3, TMEM232, AHSG, CHD2, FIGNL1, KRTAP10-8, OR4K13, RPS3A, TMEM233, AHSP, CHD3, FIGNL2, KRTAP10-9, OR4K14, RPS4X, TMEM234, AICDA, CHD4, FILIP1, KRTAP1-1, OR4K15, RPS4Y1, TMEM235, AIDA, CHD5, FILIP1L, KRTAP11-1, OR4K17, RPS4Y2, TMEM236, AIF1, CHD6, FIP1L1, KRTAP12-1, OR4K2, RPS5, TMEM237, AIF1L, CHD7, FIS1, KRTAP12-2, OR4K5, RPS6, TMEM238, AIFM1, CHD8, FITM1, KRTAP12-3, OR4L1, RPS6KA1, TMEM239, AIFM2, CHD9, FITM2, KRTAP12-4, OR4M1, RPS6KA2, TMEM240, AIFM3, CHDC2, FIZ1, KRTAP1-3, OR4M2, RPS6KA3, TMEM241, AIG1, CHDH, FJX1, KRTAP13-1, OR4N2, RPS6KA4, TMEM242, AIM1, CHEK1, FKBP10, KRTAP13-2, OR4N4, RPS6KA5, TMEM243, AIM1L, CHEK2, FKBP11, KRTAP13-3, OR4N5, RPS6KA6, TMEM244, AIM2, CHERP, FKBP14, KRTAP13-4, OR4P4, RPS6KB1, TMEM245, AIMP1, CHFR, FKBP15, KRTAP1-4, OR4Q3, RPS6KB2, TMEM246, AIMP2, CHGA, FKBP1A, KRTAP1-5, OR4S1, RPS6KC1, TMEM247, AIP, CHGB, FKBP1B, KRTAP15-1, OR4S2, RPS6KL1, TMEM248, AIPL1, CHI3L1, FKBP2, KRTAP16-1, OR4X1, RPS7, TMEM249, AIRE, CHI3L2, FKBP3, KRTAP17-1, OR4X2, RPS8, TMEM25, AJAP1, CHIA, FKBP4, KRTAP19-1, OR51A2, RPS9, TMEM251, AJUBA, CHIC1, FKBP5, KRTAP19-2, OR51A4, RPSA, TMEM252, AK1, CHIC2, FKBP6, KRTAP19-3, OR51A7, RPTN, TMEM253, AK2, CHID1, FKBP7, KRTAP19-4, OR51B2, RPTOR, TMEM254, AK3, CHIT1, FKBP8, KRTAP19-5, OR51B4, RPUSD1, TMEM255A, AK4, CHKA, FKBP9, KRTAP19-6, OR51B5, RPUSD2, TMEM255B, AK5, CHKB, FKBPL, KRTAP19-7, OR51B6, RPUSD3, TMEM256, AK6, CHL1, FKRP, KRTAP19-8, OR51D1, RPUSD4, TMEM257, AK7, CHM, FKTN, KRTAP20-1, OR51E1, RQCD1, TMEM258, AK8, CHML, FLAD1, KRTAP20-2, OR51E2, RRAD, TMEM259, AK9, CHMP1A, FLCN, KRTAP20-3, OR51F1, RRAGA, TMEM26, AKAP1, CHMP1B, FLG, KRTAP2-1, OR51F2, RRAGB, TMEM260, AKAP10, CHMP2A, FLG2, KRTAP21-1, OR51G1, RRAGC, TMEM261, AKAP11, CHMP2B, FLI1, KRTAP21-2, OR51G2, RRAGD, TMEM27, AKAP12, CHMP3, FLII, KRTAP21-3, OR51I1, RRAS, TMEM30A, AKAP13, |
| CHMP4A, FLJ22184, KRTAP2-2, OR51I2, RRAS2, TMEM30B, AKAP14, CHMP4B, FLJ25363, KRTAP22-1, OR51L1, RRBP1, TMEM31, AKAP17A, CHMP4C, FLJ44313, KRTAP22-2, OR51M1, RREB1, TMEM33, AKAP2, CHMP5, FLJ44635, KRTAP2-3, OR51Q1, RRH, TMEM35, AKAP3, CHMP6, FLJ45513, KRTAP23-1, OR51S1, RRM1, TMEM37, AKAP4, CHMP7, FLNA, KRTAP2-4, OR51T1, RRM2, TMEM38A, AKAP5, CHN1, FLNB, KRTAP24-1, OR51V1, RRM2B, TMEM38B, AKAP6, CHN2, FLNC, KRTAP25-1, OR52A1, RRN3, TMEM39A, AKAP7, CHODL, FLOT1, KRTAP26-1, OR52A5, RRNAD1, TMEM39B, AKAP8, CHORDC1, FLOT2, KRTAP27-1, OR52B2, RRP1, TMEM40, AKAP8L, CHP1, FLRT1, KRTAP29-1, OR52B4, RRP12, TMEM41A, AKAP9, CHP2, FLRT2, KRTAP3-1, OR52B6, RRP15, TMEM41B, AKIP1, CHPF, FLRT3, KRTAP3-2, OR52D1, RRP1B, TMEM42, AKIRIN1, CHPF2, FLT1, KRTAP3-3, OR52E2, RRP36, TMEM43, AKIRIN2, CHPT1, FLT3, KRTAP4-1, OR52E4, RRP7A, TMEM44, AKNA, CHRAC1, FLT3LG, KRTAP4-11, OR52E6, RRP8, TMEM45A, AKNAD1, CHRD, FLT4, KRTAP4-12, OR52E8, RRP9, TMEM45B, AKR1A1, CHRDL1, FLVCR1, KRTAP4-2, OR52H1, RRS1, TMEM47, AKR1B1, CHRDL2, FLVCR2, KRTAP4-3, OR52I1, RS1, TMEM5, AKR1B10, CHRFAM7A, FLYWCH1, KRTAP4-4, OR5212, RSAD1, TMEM50A, AKR1B15, CHRM1, FLYWCH2, KRTAP4-5, OR52J3, RSAD2, TMEM50B, AKR1C1, CHRM2, FMN1, KRTAP4-6, OR52K1, RSBN1, TMEM51, AKR1C2, CHRM3, FMN2, KRTAP4-7, OR52K2, RSBN1L, TMEM52, AKR1C3, CHRM4, FMNL1, KRTAP4-8, OR52L1, RSC1A1, TMEM52B, AKR1C4, CHRM5, FMNL2, KRTAP4-9, OR52M1, RSF1, TMEM53, AKR1D1, CHRNA1, FMNL3, KRTAP5-1, OR52N1, RSG1, TMEM54, AKR1E2, CHRNA10, FMO1, KRTAP5-10, OR52N4, RSL1D1, TMEM55A, AKR7A2, CHRNA2, FMO2, KRTAP5-11, OR52N5, RSL24D1, TMEM55B, AKR7A3, CHRNA3, FMO3, KRTAP5-2, OR52R1, RSPH1, TMEM56, AKT1, CHRNA4, FMO4, KRTAP5-3, OR52W1, RSPH10B, TMEM56-RWDD3, AKT1S1, CHRNA5, FMOS, KRTAP5-4, OR56A1, RSPH10B2, TMEM57, AKT2, CHRNA6, FMOD, KRTAP5-5, OR56A3, RSPH3, TMEM59, AKT3, CHRNA7, FMR1, KRTAP5- 6, OR56A4, RSPH4A, TMEM59L, AKTIP, CHRNA9, FMR1NB, KRTAP5-7, OR56A5, RSPH6A, TMEM60, ALAD, CHRNB1, FN1, KRTAP5-8, OR56B1, RSPH9, TMEM61, ALAS1, CHRNB2, FN3K, KRTAP5-9, OR56B4, RSPO1, TMEM62, ALAS2, CHRNB3, FN3KRP, KRTAP6-1, OR5A1, RSPO2, TMEM63A, ALB, CHRNB4, FNBP1, KRTAP6-2, OR5A2, RSPO3, TMEM63B, ALCAM, CHRND, FNBP1L, KRTAP6-3, OR5AC2, RSPO4, TMEM63C, ALDH16A1, CHRNE, FNBP4, KRTAP7-1, OR5AK2, RSPRY1, TMEM64, ALDH18A1, CHRNG, FNDC1, KRTAP8-1, OR5AN1, RSRC1, TMEM65, ALDH1A1, CHST1, FNDC3A, KRTAP9-1, OR5AP2, RSRC2, TMEM66, ALDH1A2, CHST10, FNDC3B, KRTAP9-2, OR5AR1, RSU1, TMEM67, ALDH1A3, CHST11, FNDC4, KRTAP9-3, OR5AS1, RTBDN, TMEM68, ALDH1B1, CHST12, FNDC5, KRTAP9-4, OR5AU1, RTCA, TMEM69, ALDH1L1, CHST13, FNDC7, KRTAP9-6, OR5B12, RTCB, TMEM70, ALDH1L2, CHST14, FNDC8, KRTAP9-7, OR5B17, RTDR1, TMEM71, ALDH2, CHST15, FNDC9, KRTAP9- 8, OR5B2, RTEL1, TMEM72, ALDH3A1, CHST2, FNIP1, KRTAP9-9, OR5B21, RTF1, TMEM74, ALDH3A2, CHST3, FNIP2, KRTCAP2, OR5B3, RTFDC1, TMEM74B, ALDH3B1, CHST4, FNTA, KRTCAP3, OR5C1, RTKN, |
| TMEM79, ALDH3B2, CHST5, FNTB, KRTDAP, OR5D13, RTKN2, TMEM80, ALDH4A1, CHST6, FOCAD, KSR1, OR5D14, RTL1, TMEM81, ALDH5A1, CHST7, FOLH1, KSR2, OR5D16, RTN1, TMEM82, ALDH6A1, CHST8, FOLH1B, KTI12, OR5D18, RTN2, TMEM86A, ALDH7A1, CHST9, FOLR1, KTN1, OR5F1, RTN3, TMEM86B, ALDH8A1, CHSY1, FOLR2, KXD1, ORSH1, RTN4, TMEM87A, ALDH9A1, CHSY3, FOLR3, KY, OR5H14, RTN4IP1, TMEM87B, ALDOA, CHTF18, FOLR4, KYNU, OR5H15, RTN4R, TMEM88, ALDOB, CHTF8, FOPNL, L1CAM, OR5H2, RTN4RL1, TMEM88B, ALDOC, CHTOP, FOS, L1TD1, OR5H6, RTN4RL2, TMEM89, ALG1, CHUK, FOSB, L2HGDH, OR5I1, RTP1, TMEM8A, ALG10, CHURC1, FOSL1, L3HYPDH, OR5J2, RTP2, TMEM8B, ALG10B, CHURC1-FNTB, FOSL2, L3MBTL1, OR5K1, RTP3, TMEM8C, ALG11, CIAO1, FOXA1, L3MBTL2, ORSK2, RTP4, TMEM9, ALG12, CIAPIN1, FOXA2, L3MBTL3, OR5K3, RTTN, TMEM91, ALG13, CIB1, FOXA3, L3MBTL4, OR5K4, RUFY1, TMEM92, ALG14, CIB2, FOXB1, LACC1, OR5L1, RUFY2, TMEM95, ALG1L, CIB3, FOXB2, LACE1, OR5L2, RUFY3, TMEM97, ALG1L2, CIB4, FOXC1, LACRT, OR5M1, RUFY4, TMEM98, ALG2, CIC, FOXC2, LACTB, OR5M10, RUNDC1, TMEM99, ALG3, CIDEA, FOXD1, LACTB2, OR5M11, RUNDC3A, TMEM9B, ALG5, CIDEB, FOXD2, LACTBL1, OR5M3, RUNDC3B, TMF1, ALG6, CIDEC, FOXD3, LAD1, OR5M8, RUNX1, TMIE, ALG8, CIITA, FOXD4, LAG3, OR5M9, RUNX1T1, TMIGD1, ALG9, CILP, FOXD4L1, LAGE3, OR5P2, RUNX2, TMIGD2, ALK, CILP2, FOXD4L2, LAIR1, OR5P3, RUNX3, TMLHE, ALKBH1, CINP, FOXD4L3, LAIR2, OR5R1, RUSC1, TMOD1, ALKBH2, CIR1, FOXD4L4, LALBA, OR5T1, RUSC1-AS1, TMOD2, ALKBH3, CIRBP, FOXD4L5, LAMA1, OR5T2, RUSC2, TMOD3, ALKBH4, CIRH1A, FOXD4L6, LAMA2, OR5T3, RUVBL1, TMOD4, ALKBH5, CISD1, FOXE1, LAMA3, OR5V1, RUVBL2, TMPO, ALKBH6, CISD2, FOXE3, LAMA4, OR5W2, RWDD1, TMPPE, ALKBH7, CISD3, FOXF1, LAMA5, OR6A2, RWDD2A, TMPRSS11A, ALKBH8, CISH, FOXF2, LAMB1, OR6B1, RWDD2B, TMPRSS11B, ALLC, CIT, FOXG1, LAMB2, OR6B3, RWDD3, TMPRSS11D, ALMS1, CITED1, FOXH1, LAMB3, OR6C1, RWDD4, TMPRSS11E, ALOX12, CITED2, FOXI1, LAMB4, OR6C2, RXFP1, TMPRSS11F, ALOX12B, CITED4, FOXI2, LAMC1, OR6C3, RXFP2, TMPRSS12, ALOX15, CIZ1, FOXI3, LAMC2, OR6C4, RXFP3, TMPRSS13, ALOX15B, CKAP2, FOXJ1, LAMC3, OR6C6, RXFP4, TMPRSS15, ALOX5, CKAP2L, FOXJ2, LAMP1, OR6C65, RXRA, TMPRSS2, ALOX5AP, CKAP4, FOXJ3, LAMP2, OR6C68, RXRB, TMPRSS3, ALOXE3, CKAP5, FOXK1, LAMP3, OR6C70, RXRG, TMPRSS4, ALPI, CKB, FOXK2, LAMP5, OR6C74, RYBP, TMPRSS5, ALPK1, CKLF, FOXL1, LAMTOR1, OR6C75, RYK, TMPRSS6, ALPK2, CKLF-CMTM1, FOXL2, LAMTOR2, OR6C76, RYR1, TMPRSS7, ALPK3, CKM, FOXM1, LAMTOR3, OR6F1, RYR2, TMPRSS9, ALPL, CKMT1A, FOXN1, LAMTOR4, OR6K2, RYR3, TMSB10, ALPP, CKMT1B, FOXN2, LAMTOR5, OR6K3, S100A1, TMSB15A, ALPPL2, CKMT2, FOXN3, LANCL1, OR6K6, S100A10, TMSB15B, ALS2, CKS1B, FOXN4, LANCL2, OR6M1, S100A11, TMSB4X, ALS2CL, CKS2, FOXO1, LANCL3, OR6N1, S100A12, TMSB4Y, ALS2CR11, CLASP1, FOXO3, LAP3, OR6N2, S100A13, TMTC1, ALS2CR12, CLASP2, FOXO4, LAPTM4A, OR6P1, S100A14, TMTC2, ALX1, CLASRP, FOXO6, |
| LAPTM4B, OR6Q1, S100A16, TMTC3, ALX3, CLC, FOXP1, LAPTM5, OR6S1, S100A2, TMTC4, ALX4, CLCA1, FOXP2, LARGE, OR6T1, S100A3, TMUB1, ALYREF, CLCA2, FOXP3, LARP1, OR6V1, S100A4, TMUB2, AMACR, CLCA4, FOXP4, LARP1B, OR6X1, S100A5, TMX1, AMBN, CLCC1, FOXQ1, LARP4, OR6Y1, S100A6, TMX2, AMBP, CLCF1, FOXR1, LARP4B, OR7A10, S100A7, TMX3, AMBRA1, CLCN1, FOXR2, LARP6, OR7A17, S100A7A, TMX4, AMD1, CLCN2, FOXRED1, LARP7, OR7A5, S100A7L2, TNC, AMDHD1, CLCN3, FOXRED2, LARS, OR7C1, S100A8, TNF, AMDHD2, CLCN4, FOXS1, LARS2, OR7C2, S100A9, TNFAIP1, AMELX, CLCN5, FPGS, LAS1L, OR7D2, S100B, TNFAIP2, AMELY, CLCN6, FPGT, LASP1, OR7D4, S100G, TNFAIP3, AMER1, CLCN7, FPGT-TNNI3K, LAT, OR7E24, S100P, TNFAIP6, AMER2, CLCNKA, FPR1, LAT2, OR7G1, S100PBP, TNFAIP8, AMER3, CLCNKB, FPR2, LATS1, OR7G2, S100Z, TNFAIP8L1, AMFR, CLDN1, FPR3, LATS2, OR7G3, S1PR1, TNFAIP8L2, AMH, CLDN10, FRA10AC1, LAX1, OR8A1, S1PR2, TNFAIP8L2-SCNM1, AMHR2, CLDN11, FRAS1, LAYN, OR8B12, S1PR3, TNFAIP8L3, AMICA1, CLDN12, FRAT1, LBH, OR8B2, S1PR4, TNFRSF10A, AMIGO1, CLDN14, FRAT2, LBP, OR8B3, S1PR5, TNFRSF10B, AMIGO2, CLDN15, FREM1, LBR, OR8B4, SAA1, TNFRSF10C, AMIGO3, CLDN16, FREM2, LBX1, OR8B8, SAA2, TNFRSF10D, AMMECR1, CLDN17, FREM3, LBX2, OR8D1, SAA2-SAA4, TNFRSF11A, AMMECR1L, CLDN18, FRG1, LCA5, OR8D2, SAA4, TNFRSF11B, AMN, CLDN19, FRG2, LCA5L, OR8D4, SAAL1, TNFRSF12A, AMN1, CLDN2, FRG2B, LCAT, OR8G1, SAC3D1, TNFRSF13B, AMOT, CLDN20, FRG2C, LCE1A, OR8G2, SACM1L, TNFRSF13C, AMOTL1, CLDN22, FRK, LCE1B, OR8G5, SACS, TNFRSF14, AMOTL2, CLDN23, FRMD1, LCE1C, OR8H1, SAE1, TNFRSF17, AMPD1, CLDN24, FRMD3, LCE1D, OR8H2, SAFB, TNFRSF18, AMPD2, CLDN25, FRMD4A, LCE1E, OR8H3, SAFB2, TNFRSF19, AMPD3, CLDN3, FRMD4B, LCE1F, OR8I2, SAG, TNFRSF1A, AMPH, CLDN4, FRMD5, LCE2A, OR8J1, SAGE1, TNFRSF1B, AMT, CLDN5, FRMD6, LCE2B, OR8J3, SALL1, TNFRSF21, AMTN, CLDN6, FRMD7, LCE2C, OR8K1, SALL2, TNFRSF25, AMY1A, CLDN7, FRMD8, LCE2D, OR8K3, SALL3, TNFRSF4, AMY1B, CLDN8, FRMPD1, LCE3A, OR8K5, SALL4, TNFRSF6B, AMY1C, CLDN9, FRMPD2, LCE3B, OR8S1, SAMD1, TNFRSF8, AMY2A, CLDND1, FRMPD3, LCE3C, OR8U1, SAMD10, TNFRSF9, AMY2B, CLDND2, FRMPD4, LCE3D, OR8U8, SAMD11, TNFSF10, AMZ1, CLEC10A, FRRS1, LCE3E, OR9A2, SAMD12, TNFSF11, AMZ2, CLEC11A, FRRS1L, LCE4A, OR9A4, SAMD13, TNFSF12, ANAPC1, CLEC12A, FRS2, LCE5A, OR9G1, SAMD14, TNFSF12-TNFSF13, ANAPC10, CLEC12B, FRS3, LCE6A, OR9G4, SAMD15, TNFSF13, ANAPC11, CLEC14A, FRY, LCK, OR9G9, SAMD3, TNFSF13B, ANAPC13, CLEC16A, FRYL, LCLAT1, OR9I1, SAMD4A, TNFSF14, ANAPC15, CLEC17A, FRZB, LCMT1, OR9K2, SAMD4B, TNFSF15, ANAPC16, CLEC18A, FSBP, LCMT2, OR9Q1, SAMD5, TNFSF18, ANAPC2, CLEC18B, FSCB, LCN1, OR9Q2, SAMD7, TNFSF4, ANAPC4, CLEC18C, FSCN1, LCN10, ORAI1, SAMD8, TNFSF8, ANAPC5, CLEC19A, FSCN2, LCN12, ORAI2, SAMD9, TNFSF9, ANAPC7, CLEC1A, FSCN3, LCN15, ORAI3, SAMD9L, TNIK, ANG, CLEC1B, FSD1, LCN2, ORAOV1, SAMHD1, TNIP1, ANGEL1, CLEC2A, FSD1L, LCN6, ORC1, SAMM50, TNIP2, |
| ANGEL2, CLEC2B, FSD2, LCN8, ORC2, SAMSN1, TNIP3, ANGPT1, CLEC2D, FSHB, LCN9, ORC3, SAP130, TNK1, ANGPT2, CLEC2L, FSHR, LCNL1, ORC4, SAP18, TNK2, ANGPT4, CLEC3A, FSIP1, LCOR, ORC5, SAP25, TNKS, ANGPTL1, CLEC3B, FSIP2, LCORL, ORC6, SAP30, TNKS1BP1, ANGPTL2, CLEC4A, FST, LCP1, ORM1, SAP30BP, TNKS2, ANGPTL3, CLEC4C, FSTL1, LCP2, ORM2, SAP30L, TNMD, ANGPTL4, CLEC4D, FSTL3, LCT, ORMDL1, SAPCD1, TNN, ANGPTL5, CLEC4E, FSTL4, LCTL, ORMDL2, SAPCD2, TNNC1, ANGPTL6, CLEC4F, FSTL5, LDB1, ORMDL3, SAR1A, TNNC2, ANGPTL7, CLEC4G, FTCD, LDB2, OS9, SAR1B, TNNI1, ANHX, CLEC4M, FTH1, LDB3, OSBP, SARDH, TNNI2, ANK1, CLEC5A, FTH1P18, LDHA, OSBP2, SARM1, TNNI3, ANK2, CLEC6A, FTHL17, LDHAL6A, OSBPL10, SARNP, TNNI3K, ANK3, CLEC7A, FTL, LDHAL6B, OSBPL11, SARS, TNNT1, ANKAR, CLEC9A, FTMT, LDHB, OSBPL1A, SARS2, TNNT2, ANKDD1A, CLECL1, FTO, LDHC, OSBPL2, SART1, TNNT3, ANKDD1B, CLGN, FTSJ1, LDHD, OSBPL3, SART3, TNP1, ANKEF1, CLHC1, FTSJ2, LDLR, OSBPL5, SASH1, TNP2, ANKFN1, CLIC1, FTSJ3, LDLRAD1, OSBPL6, SASH3, TNPO1, ANKFY1, CLIC2, FUBP1, LDLRAD2, OSBPL7, SASS6, TNPO2, ANKH, CLIC3, FUBP3, LDLRAD3, OSBPL8, SAT1, TNPO3, ANKHD1, CLIC4, FUCA1, LDLRAD4, OSBPL9, SAT2, TNR, ANKHD1-EIF4EBP3, CLIC5, FUCA2, LDLRAP1, OSCAR, SATB1, TNRC18, ANKIB1, CLIC6, FUK, LDOC1, OSCP1, SATB2, TNRC6A, ANKK1, CLINT1, FUNDC1, LDOC1L, OSER1, SATL1, TNRC6B, ANKLE1, CLIP1, FUNDC2, LEAP2, OSGEP, SAV1, TNRC6C, ANKLE2, CLIP2, FUOM, LECT1, OSGEPL1, SAYSD1, TNS1, ANKMY1, CLIP3, FURIN, LECT2, OSGIN1, SBDS, TNS3, ANKMY2, CLIP4, FUS, LEF1, OSGIN2, SBF1, TNS4, ANKRA2, CLK1, FUT1, LEFTY1, OSM, SBF2, TNXB, ANKRD1, CLK2, FUT10, LEFTY2, OSMR, SBK1, TOB1, ANKRD10, CLK3, FUT11, LEKR1, OSR1, SBK2, TOB2, ANKRD11, CLK4, FUT2, LELP1, OSR2, SBK3, TOE1, ANKRD12, CLLU1, FUT3, LEMD1, OST4, SBNO1, TOLLIP, ANKRD13A, CLLU1OS, FUT4, LEMD2, OSTC, SBNO2, TOM1, ANKRD13B, CLMN, FUT5, LEMD3, OSTF1, SBSN, TOM1L1, ANKRD13C, CLMP, FUT6, LENEP, OSTM1, SBSPON, TOM1L2, ANKRD13D, CLN3, FUT7, LENG1, OSTN, SC5D, TOMM20, ANKRD16, CLN5, FUT8, LENG8, OTC, SCAF1, TOMM20L, ANKRD17, CLN6, FUT9, LENG9, OTOA, SCAF11, TOMM22, ANKRD18A, CLN8, FUZ, LEO1, OTOF, SCAF4, TOMM34, ANKRD18B, CLNK, FXN, LEP, OTOG, SCAF8, TOMM40, ANKRD2, CLNS1A, FXR1, LEPR, OTOGL, SCAI, TOMM40L, ANKRD20A1, CLOCK, FXR2, LEPRE1, OTOL1, SCAMP1, TOMM5, ANKRD20A2, CLP1, FXYD1, LEPREL1, OTOP1, SCAMP2, TOMM6, ANKRD20A3, CLPB, FXYD2, LEPREL2, OTOP2, SCAMP3, TOMM7, ANKRD20A4, CLPP, FXYD3, LEPREL4, OTOP3, SCAMP4, TOMM70A, ANKRD22, CLPS, FXYD4, LEPROT, OTOR, SCAMP5, TONSL, ANKRD23, CLPSL1, FXYD5, LEPROTL1, OTOS, SCAND1, TOP1, ANKRD24, CLPSL2, FXYD6, LETM1, OTP, SCAND3, TOP1MT, ANKRD26, CLPTM1, FXYD6-FXYD2, LETM2, OTUB1, SCAP, TOP2A, ANKRD27, CLPTM1L, FXYD7, LETMD1, OTUB2, SCAPER, TOP2B, ANKRD28, CLPX, FYB, LEUTX, OTUD1, SCARA3, TOP3A, ANKRD29, CLRN1, FYCO1, LFNG, OTUD3, SCARA5, TOP3B, ANKRD30A, CLRN2, FYN, LGALS1, OTUD4, SCARB1, TOPAZ1, |
| ANKRD30B, CLRN3, FYTTD1, LGALS12, OTUD5, SCARB2, TOPBP1, ANKRD31, CLSPN, FZD1, LGALS13, OTUD6A, SCARF1, TOPORS, ANKRD32, CLSTN1, FZD10, LGALS14, OTUD6B, SCARF2, TOR1A, ANKRD33, CLSTN2, FZD2, LGALS16, OTUD7A, SCCPDH, TOR1AIP1, ANKRD33B, CLSTN3, FZD3, LGALS2, OTUD7B, SCD, TOR1AIP2, ANKRD34A, CLTA, FZD4, LGALS3, OTX1, SCD5, TOR1B, ANKRD34B, CLTB, FZD5, LGALS3BP, OTX2, SCEL, TOR2A, ANKRD34C, CLTC, FZD6, LGALS4, OVCA2, SCFD1, TOR3A, ANKRD35, CLTCL1, FZD7, LGALS7, OVCH1, SCFD2, TOR4A, ANKRD36, CLU, FZD8, LGALS7B, OVCH2, SCG2, TOX, ANKRD36B, CLUAP1, FZD9, LGALS8, OVGP1, SCG3, TOX2, ANKRD36C, CLUH, FZR1, LGALS9, OVOL1, SCG5, TOX3, ANKRD37, CLUL1, G0S2, LGALS9B, OVOL2, SCGB1A1, TOX4, ANKRD39, CLVS1, G2E3, LGALS9C, OVOL3, SCGB1C1, TP53, ANKRD40, CLVS2, G3BP1, LGALSL, OVOS, SCGB1D1, TP53AIP1, ANKRD42, CLYBL, G3BP2, LGI1, OVOS2, SCGB1D2, TP53BP1, ANKRD44, CMA1, G6PC, LGI2, OXA1L, SCGB1D4, TP53BP2, ANKRD45, CMAS, G6PC2, LGI3, OXCT1, SCGB2A1, TP53I11, ANKRD46, CMBL, G6PC3, LGI4, OXCT2, SCGB2A2, TP53I13, ANKRD49, CMC1, G6PD, LGMN, OXER1, SCGB2B2, TP53I3, ANKRD50, CMC2, GAA, LGR4, OXGR1, SCGB3A1, TP53INP1, ANKRD52, CMC4, GAB1, LGR5, OXLD1, SCGB3A2, TP53INP2, ANKRD53, CMIP, GAB2, LGR6, OXNAD1, SCGN, TP53RK, ANKRD54, CMKLR1, GAB3, LGSN, OXR1, SCHIP1, TP53TG3, ANKRD55, CMPK1, GAB4, LHB, OXSM, SCIMP, TP53TG3B, ANKRD6, CMPK2, GABARAP, LHCGR, OXSR1, SCIN, TP53TG3C, ANKRD60, CMSS1, GABARAPL1, LHFP, OXT, SCLT1, TP53TG3D, ANKRD61, CMTM1, GABARAPL2, LHFPL1, OXTR, SCLY, TP53TG5, ANKRD62, CMTM2, GABBR1, LHFPL2, P2RX1, SCMH1, TP63, ANKRD63, CMTM3, GABBR2, LHFPL3, P2RX2, SCML1, TP73, ANKRD65, CMTM4, GABPA, LHFPL4, P2RX3, SCML2, TPBG, ANKRD66, CMTM5, GABPB1, LHFPL5, P2RX4, SCML4, TPBGL, ANKRD7, CMTM6, GABPB2, LHPP, P2RX5, SCN10A, TPCN1, ANKRD9, CMTM7, GABRA1, LHX1, P2RX6, SCN11A, TPCN2, ANKS1A, CMTM8, GABRA2, LHX2, P2RX7, SCN1A, TPD52, ANKS1B, CMTR1, GABRA3, LHX3, P2RY1, SCN1B, TPD52L1, ANKS3, CMTR2, GABRA4, LHX4, P2RY10, SCN2A, TPD52L2, ANKS4B, CMYA5, GABRA5, LHX5, P2RY11, SCN2B, TPD52L3, ANKS6, CNBD1, GABRA6, LHX6, P2RY12, SCN3A, TPGS1, ANKUB1, CNBD2, GABRB1, LHX8, P2RY13, SCN3B, TPGS2, ANKZF1, CNBP, GABRB2, LHX9, P2RY14, SCN4A, TPH1, ANLN, CNDP1, GABRB3, LIAS, P2RY2, SCN4B, TPH2, ANO1, CNDP2, GABRD, LIF, P2RY4, SCN5A, TPI1, ANO10, CNEP1R1, GABRE, LIFR, P2RY6, SCN7A, TPK1, ANO2, CNFN, GABRG1, LIG1, P2RY8, SCN8A, TPM1, ANO3, CNGA1, GABRG2, LIG3, P4HA1, SCN9A, TPM2, ANO4, CNGA2, GABRG3, LIG4, P4HA2, SCNM1, TPM3, ANO5, CNGA3, GABRP, LILRA1, P4HA3, SCNN1A, TPM4, ANO6, CNGA4, GABRQ, LILRA2, P4HB, SCNN1B, TPMT, ANO7, CNGB1, GABRR1, LILRA3, P4HTM, SCNN1D, TPO, ANO8, CNGB3, GABRR2, LILRA4, PA2G4, SCNN1G, TPP1, ANO9, CNIH1, GABRR3, LILRA5, PAAF1, SCO1, TPP2, ANP32A, CNIH2, GAD1, LILRA6, PABPC1, SCO2, TPPP, ANP32B, CNIH3, GAD2, LILRB1, PABPC1L, SCOC, TPPP2, ANP32C, CNIH4, GADD45A, LILRB2, PABPC1L2A, SCP2, TPPP3, ANP32D, CNKSR1, |
| GADD45B, LILRB3, PABPC1L2B, SCP2D1, TPR, ANP32E, CNKSR2, GADD45G, LILRB4, PABPC3, SCPEP1, TPRA1, ANPEP, CNKSR3, GADD45GIP1, LILRB5, PABPC4, SCRG1, TPRG1, ANTXR1, CNN1, GADL1, LIM2, PABPC4L, SCRIB, TPRG1L, ANTXR2, CNN2, GAGE1, LIMA1, PABPC5, SCRN1, TPRKB, ANTXRL, CNN3, GAGE10, LIMCH1, PABPN1, SCRN2, TPRN, ANXA1, CNNM1, GAGE12B, LIMD1, PABPN1L, SCRN3, TPRX1, ANXA10, CNNM2, GAGE12C, LIMD2, PACRG, SCRT1, TPSAB1, ANXA11, CNNM3, GAGE12D, LIME1, PACRGL, SCRT2, TPSB2, ANXA13, CNNM4, GAGE12E, LIMK1, PACS1, SCT, TPSD1, ANXA2, CNOT1, GAGE12F, LIMK2, PACS2, SCTR, TPSG1, ANXA2R, CNOT10, GAGE12G, LIMS1, PACSIN1, SCUBE1, TPST1, ANXA3, CNOT11, GAGE12H, LIMS2, PACSIN2, SCUBE2, TPST2, ANXA4, CNOT2, GAGE12I, LIMS3, PACSIN3, SCUBE3, TPT1, ANXA5, CNOT3, GAGE12J, LIN28A, PADI1, SCXA, TPTE, ANXA6, CNOT4, GAGE13, LIN28B, PADI2, SCXB, TPTE2, ANXA7, CNOT6, GAGE2A, LIN37, PADI3, SCYL1, TPX2, ANXA8, CNOT6L, GAGE2B, LIN52, PADI4, SCYL2, TRA2A, ANXA8L1, CNOT7, GAGE2C, LIN54, PADI6, SCYL3, TRA2B, ANXA8L2, CNOT8, GAGE2D, LIN7A, PAEP, SDAD1, TRABD, ANXA9, CNP, GAGE2E, LIN7B, PAF1, SDC1, TRABD2A, AOAH, CNPPD1, GAGE4, LIN7C, PAFAH1B1, SDC2, TRABD2B, AOC1, CNPY1, GAGE5, LIN9, PAFAH1B2, SDC3, TRADD, AOC2, CNPY2, GAGE6, LINC00452, PAFAH1B3, SDC4, TRAF1, AOC3, CNPY3, GAGE7, LINC00984, PAFAH2, SDCBP, TRAF2, AOX1, CNPY4, GAGE8, LINGO1, PAG1, SDCBP2, TRAF3, AP1AR, CNR1, GAK, LINGO2, PAGE1, SDCCAG3, TRAF3IP1, AP1B1, CNR2, GAL, LINGO3, PAGE2, SDCCAG8, TRAF3IP2, AP1G1, CNRIP1, GAL3ST1, LINGO4, PAGE2B, SDE2, TRAF3IP3, AP1G2, CNST, GAL3ST2, LINS, PAGE4, SDF2, TRAF4, AP1M1, CNTD1, GAL3ST3, LIPA, PAGE5, SDF2L1, TRAF5, AP1M2, CNTD2, GAL3ST4, LIPC, PAGR1, SDF4, TRAF6, AP1S1, CNTF, GALC, LIPE, PAH, SDHA, TRAF7, AP1S2, CNTFR, GALE, LIPF, PAICS, SDHAF1, TRAFD1, AP1S3, CNTLN, GALK1, LIPG, PAIP1, SDHAF2, TRAIP, AP2A1, CNTN1, GALK2, LIPH, PAIP2, SDHB, TRAK1, AP2A2, CNTN2, GALM, LIPI, PAIP2B, SDHC, TRAK2, AP2B1, CNTN3, GALNS, LIPJ, PAK1, SDHD, TRAM1, AP2M1, CNTN4, GALNT1, LIPK, PAK1IP1, SDK1, TRAM1L1, AP2S1, CNTN5, GALNT10, LIPM, PAK2, SDK2, TRAM2, AP3B1, CNTN6, GALNT11, LIPN, PAK3, SDPR, TRANK1, AP3B2, CNTNAP1, GALNT12, LIPT1, PAK4, SDR16C5, TRAP1, AP3D1, CNTNAP2, GALNT13, LIPT2, PAK6, SDR39U1, TRAPPC1, AP3M1, CNTNAP3, GALNT14, LITAF, PAK7, SDR42E1, TRAPPC10, AP3M2, CNTNAP3B, GALNT15, LIX1, PALB2, SDR9C7, TRAPPC11, AP3S1, CNTNAP4, GALNT16, LIX1L, PALD1, SDS, TRAPPC12, AP3S2, CNTNAP5, GALNT18, LLGL1, PALLD, SDSL, TRAPPC13, AP4B1, CNTRL, GALNT2, LLGL2, PALM, SEBOX, TRAPPC2, AP4E1, CNTROB, GALNT3, LLPH, PALM2, SEC11A, TRAPPC2L, AP4M1, COA1, GALNT4, LMAN1, PALM2-AKAP2, SEC11C, TRAPPC3, AP4S1, COA3, GALNT5, LMAN1L, PALM3, SEC13, TRAPPC3L, AP5B1, COA4, GALNT6, LMAN2, PALMD, SEC14L1, TRAPPC4, AP5M1, COA5, GALNT7, LMAN2L, PAM, SEC14L2, TRAPPC5, AP5S1, COA6, GALNT8, LMBR1, PAM16, SEC14L3, TRAPPC6A, AP5Z1, COASY, GALNT9, LMBR1L, PAMR1, SEC14L4, TRAPPC6B, APAF1, COBL, GALNTL5, LMBRD1, PAN2, SEC14L5, TRAPPC8, |
| APBA1, COBLL1, GALNTL6, LMBRD2, PAN3, SEC14L6, TRAPPC9, APBA2, COCH, GALP, LMCD1, PANK1, SEC16A, TRAT1, APBA3, COG1, GALR1, LMF1, PANK2, SEC16B, TRDMT1, APBB1, COG2, GALR2, LMF2, PANK3, SEC22A, TRDN, APBB1IP, COG3, GALR3, LMLN, PANK4, SEC22B, TREH, APBB2, COG4, GALT, LMNA, PANX1, SEC22C, TREM1, APBB3, COG5, GAMT, LMNB1, PANX2, SEC23A, TREM2, APC, COG6, GAN, LMNB2, PANX3, SEC23B, TREML1, APC2, COG7, GANAB, LMO1, PAOX, SEC23IP, TREML2, APCDD1, COG8, GANC, LMO2, PAPD4, SEC24A, TREML4, APCDD1L, COIL, GAP43, LMO3, PAPD5, SEC24B, TRERF1, APCS, COL10A1, GAPDH, LMO4, PAPD7, SEC24C, TREX1, APEH, COL11A1, GAPDHS, LMO7, PAPL, SEC24D, TREX2, APEX1, COL11A2, GAPT, LMOD1, PAPLN, SEC31A, TRH, APEX2, COL12A1, GAPVD1, LMOD2, PAPOLA, SEC31B, TRHDE, APH1A, COL13A1, GAR1, LMOD3, PAPOLB, SEC61A1, TRHR, APH1B, COL14A1, GAREM, LMTK2, PAPOLG, SEC61A2, TRIAP1, API5, COL15A1, GAREML, LMTK3, PAPPA, SEC61B, TRIB1, APIP, COL16A1, GARNL3, LMX1A, PAPPA2, SEC61G, TRIB2, APITD1, COL17A1, GARS, LMX1B, PAPSS1, SEC62, TRIB3, APITD1-CORT, COL18A1, GART, LNP1, PAPSS2, SEC63, TRIL, APLF, COL19A1, GAS1, LNPEP, PAQR3, SECISBP2, TRIM10, APLN, COL1A1, GAS2, LNX1, PAQR4, SECISBP2L, TRIM11, APLNR, COL1A2, GAS2L1, LNX2, PAQR5, SECTM1, TRIM13, APLP1, COL20A1, GAS2L2, LOH12CR1, PAQR6, SEH1L, TRIM14, APLP2, COL21A1, GAS2L3, LONP1, PAQR7, SEL1L, TRIM15, APMAP, COL22A1, GAS6, LONP2, PAQR8, SEL1L2, TRIM16, APOA1, COL23A1, GAS7, LONRF1, PAQR9, SEL1L3, TRIM16L, APOA1BP, COL24A1, GAS8, LONRF2, PARD3, SELE, TRIM17, APOA2, COL25A1, GAST, LONRF3, PARD3B, SELENBP1, TRIM2, APOA4, COL26A1, GATA1, LOR, PARD6A, SELK, TRIM21, APOA5, COL27A1, GATA2, LOX, PARD6B, SELL, TRIM22, APOB, COL28A1, GATA3, LOXHD1, PARD6G, SELM, TRIM23, APOBEC1, COL2A1, GATA4, LOXL1, PARG, SELO, TRIM24, APOBEC2, COL3A1, GATA5, LOXL2, PARK2, SELP, TRIM25, APOBEC3A, COL4A1, GATA6, LOXL3, PARK7, SELPLG, TRIM26, APOBEC3B, COL4A2, GATAD1, LOXL4, PARL, SELRC1, TRIM27, APOBEC3C, COL4A3, GATAD2A, LPA, PARM1, SELT, TRIM28, APOBEC3D, COL4A3BP, GATAD2B, LPAR1, PARN, SELV, TRIM29, APOBEC3F, COL4A4, GATC, LPAR2, PARP1, SEMA3A, TRIM3, APOBEC3G, COL4A5, GATM, LPAR3, PARP10, SEMA3B, TRIM31, APOBEC3H, COL4A6, GATS, LPAR4, PARP11, SEMA3C, TRIM32, APOBEC4, COL5A1, GATSL1, LPAR5, PARP12, SEMA3D, TRIM33, APOBR, COL5A2, GATSL2, LPAR6, PARP14, SEMA3E, TRIM34, APOC1, COL5A3, GATSL3, LPCAT1, PARP15, SEMA3F, TRIM35, APOC2, COL6A1, GBA, LPCAT2, PARP16, SEMA3G, TRIM36, APOC3, COL6A2, GBA2, LPCAT3, PARP2, SEMA4A, TRIM37, APOC4, COL6A3, GBA3, LPCAT4, PARP3, SEMA4B, TRIM38, APOD, COL6A5, GBAS, LPGAT1, PARP4, SEMA4C, TRIM39, APOE, COL6A6, GBE1, LPHN1, PARP6, SEMA4D, TRIM39-RPP21, APOF, COL7A1, GBF1, LPHN2, PARP8, SEMA4F, TRIM4, APOH, COL8A1, GBGT1, LPHN3, PARP9, SEMA4G, TRIM40, APOL1, COL8A2, GBP1, LPIN1, PARPBP, SEMA5A, TRIM41, APOL2, COL9A1, GBP2, LPIN2, PARS2, SEMA5B, TRIM42, APOL3, COL9A2, GBP3, LPIN3, PARVA, SEMA6A, |
| TRIM43, APOL4, COL9A3, GBP4, LPL, PARVB, SEMA6B, TRIM43B, APOL5, COLCA2, GBP5, LPO, PARVG, SEMA6C, TRIM44, APOL6, COLEC10, GBP6, LPP, PASD1, SEMA6D, TRIM45, APOLD1, COLEC11, GBP7, LPPR1, PASK, SEMA7A, TRIM46, APOM, COLEC12, GBX1, LPPR2, PATE1, SEMG1, TRIM47, APOO, COLGALT1, GBX2, LPPR3, PATE2, SEMG2, TRIM48, APOOL, COLGALT2, GC, LPPR4, PATE3, SENP1, TRIM49, APOPT1, COLQ, GCA, LPPR5, PATE4, SENP2, TRIM49B, APP, COMMD1, GCAT, LPXN, PATL1, SENP3, TRIM49C, APPBP2, COMMD10, GCC1, LRAT, PATL2, SENP5, TRIM49D1, APPL1, COMMD2, GCC2, LRBA, PATZ1, SENP6, TRIM49D2P, APPL2, COMMD3, GCDH, LRCH1, PAWR, SENP7, TRIM5, APRT, COMMD3-BMI1, GCFC2, LRCH2, PAX1, SENP8, TRIM50, APTX, COMMD4, GCG, LRCH3, PAX2, SEPHS1, TRIM51, AQP1, COMMD5, GCGR, LRCH4, PAX3, SEPHS2, TRIM52, AQP10, COMMD6, GCH1, LRCOL1, PAX4, SEPN1, TRIM54, AQP11, COMMD7, GCHFR, LRFN1, PAX5, SEPP1, TRIM55, AQP12A, COMMD8, GCK, LRFN2, PAX6, SEPSECS, TRIM56, AQP12B, COMMD9, GCKR, LRFN3, PAX7, SEPT1, TRIM58, AQP2, COMP, GCLC, LRFN4, PAX8, SEPT10, TRIM59, AQP3, COMT, GCLM, LRFN5, PAX9, SEPT11, TRIM6, AQP4, COMTD1, GCM1, LRG1, PAXBP1, SEPT12, TRIM60, AQP5, COPA, GCM2, LRGUK, PAXIP1, SEPT14, TRIM61, AQP6, COPB1, GCN1L1, LRIF1, PBDC1, SEPT15, TRIM62, AQP7, COPB2, GCNT1, LRIG1, PBK, SEPT2, TRIM63, AQP8, COPE, GCNT2, LRIG2, PBLD, SEPT3, TRIM64, AQP9, COPG1, GCNT3, LRIG3, PBOV1, SEPT4, TRIM64B, AQPEP, COPG2, GCNT4, LRIT1, PBRM1, SEPT5, TRIM64C, AQR, COPRS, GCNT7, LRIT2, PBX1, SEPT6, TRIM65, AR, COPS2, GCOM1, LRIT3, PBX2, SEPT7, TRIM66, ARAF, COPS3, GCSAM, LRMP, PBX3, SEPT8, TRIM67, ARAP1, COPS4, GCSAML, LRP1, PBX4, SEPT9, TRIM68, ARAP2, COPS5, GCSH, LRP10, PBXIP1, SEPW1, TRIM69, ARAP3, COPS6, GDA, LRP11, PC, SERAC1, TRIM6-TRIM34, ARC, COPS7A, GDAP1, LRP12, PCBD1, SERBP1, TRIM7, ARCN1, COPS7B, GDAP1L1, LRP1B, PCBD2, SERF1A, TRIM71, AREG, COPS8, GDAP2, LRP2, PCBP1, SERF1B, TRIM72, AREGB, COPZ1, GDE1, LRP2BP, PCBP2, SERF2, TRIM73, AREL1, COPZ2, GDF1, LRP3, PCBP3, SERGEF, TRIM74, ARF1, COQ10A, GDF10, LRP4, PCBP4, SERHL2, TRIM77, ARF3, COQ10B, GDF11, LRP5, PCCA, SERINC1, TRIM8, ARF4, COQ2, GDF15, LRP5L, PCCB, SERINC2, TRIM9, ARF5, COQ3, GDF2, LRP6, PCDH1, SERINC3, TRIML1, ARF6, COQ4, GDF3, LRP8, PCDH10, SERINC4, TRIML2, ARFGAP1, COQ5, GDF5, LRPAP1, PCDH11X, SERINC5, TRIO, ARFGAP2, COQ6, GDF6, LRPPRC, PCDH11Y, SERP1, TRIOBP, ARFGAP3, COQ7, GDF7, LRR1, PCDH12, SERP2, TRIP10, ARFGEF1, COQ9, GDF9, LRRC1, PCDH15, SERPINA1, TRIP11, ARFGEF2, CORIN, GDI1, LRRC10, PCDH17, SERPINA10, TRIP12, ARFIP1, CORO1A, GDI2, LRRC10B, PCDH18, SERPINA11, TRIP13, ARFIP2, CORO1B, GDNF, LRRC14, PCDH19, SERPINA12, TRIP4, ARFRP1, CORO1C, GDNF-AS1, LRRC14B, PCDH20, SERPINA3, TRIP6, ARG1, CORO2A, GDPD1, LRRC15, PCDH7, SERPINA4, TRIQK, ARG2, CORO2B, GDPD2, LRRC16A, PCDH8, SERPINA5, TRIT1, ARGFX, CORO6, GDPD3, LRRC16B, PCDH9, SERPINA6, TRMT1, ARGLU1, CORO7, GDPD4, LRRC17, PCDHA1, SERPINA7, TRMT10A, ARHGAP1, CORO7-PAM16, GDPD5, LRRC18, PCDHA10, SERPINA9, TRMT10B, ARHGAP10, |
| CORT, GDPGP1, LRRC19, PCDHA11, SERPINB1, TRMT10C, ARHGAP11A, COTL1, GEM, LRRC2, PCDHA12, SERPINB10, TRMT11, ARHGAP11B, COX10, GEMIN2, LRRC20, PCDHA13, SERPINB11, TRMT112, ARHGAP12, COX11, GEMIN4, LRRC23, PCDHA2, SERPINB12, TRMT12, ARHGAP15, COX14, GEMIN5, LRRC24, PCDHA3, SERPINB13, TRMT13, ARHGAP17, COX15, GEMIN6, LRRC25, PCDHA4, SERPINB2, TRMT1L, ARHGAP18, COX16, GEMIN7, LRRC26, PCDHA5, SERPINB3, TRMT2A, ARHGAP19, COX17, GEMIN8, LRRC27, PCDHA6, SERPINB4, TRMT2B, ARHGAP20, COX18, GEN1, LRRC28, PCDHA7, SERPINB5, TRMT44, ARHGAP21, COX19, GET4, LRRC29, PCDHA8, SERPINB6, TRMT5, ARHGAP22, COX20, GFAP, LRRC3, PCDHA9, SERPINB7, TRMT6, ARHGAP23, COX4I1, GFER, LRRC30, PCDHAC1, SERPINB8, TRMT61A, ARHGAP24, COX4I2, GFI1, LRRC31, PCDHAC2, SERPINB9, TRMT61B, ARHGAP25, COX5A, GFI1B, LRRC32, PCDHB1, SERPINC1, TRMU, ARHGAP26, COX5B, GFM1, LRRC34, PCDHB10, SERPIND1, TRNAU1AP, ARHGAP27, COX6A1, GFM2, LRRC36, PCDHB11, SERPINE1, TRNP1, ARHGAP28, COX6A2, GFOD1, LRRC37A2, PCDHB12, SERPINE2, TRNT1, ARHGAP29, COX6B1, GFOD2, LRRC37A3, PCDHB13, SERPINE3, TRO, ARHGAP30, COX6B2, GFPT1, LRRC37B, PCDHB14, SERPINF1, TROAP, ARHGAP31, COX6C, GFPT2, LRRC38, PCDHB15, SERPINF2, TROVE2, ARHGAP32, COX7A1, GFRA1, LRRC39, PCDHB16, SERPING1, TRPA1, ARHGAP33, COX7A2, GFRA2, LRRC3B, PCDHB2, SERPINH1, TRPC1, ARHGAP35, COX7A2L, GFRA3, LRRC3C, PCDHB3, SERPINI1, TRPC3, ARHGAP36, COX7B, GFRA4, LRRC4, PCDHB4, SERPINI2, TRPC4, ARHGAP39, COX7B2, GFRAL, LRRC40, PCDHB5, SERTAD1, TRPC4AP, ARHGAP4, COX7C, GGA1, LRRC41, PCDHB6, SERTAD2, TRPC5, ARHGAP40, COX8A, GGA2, LRRC42, PCDHB7, SERTAD3, TRPC5OS, ARHGAP42, COX8C, GGA3, LRRC43, PCDHB8, SERTAD4, TRPC6, ARHGAP44, CP, GGACT, LRRC45, PCDHB9, SERTM1, TRPC7, ARHGAP5, CPA1, GGCT, LRRC46, PCDHGA1, SESN1, TRPM1, ARHGAP6, CPA2, GGCX, LRRC47, PCDHGA10, SESN2, TRPM2, ARHGAP8, CPA3, GGH, LRRC48, PCDHGA11, SESN3, TRPM3, ARHGAP9, CPA4, GGN, LRRC49, PCDHGA12, SESTD1, TRPM4, ARHGDIA, CPA5, GGNBP2, LRRC4B, PCDHGA2, SET, TRPM5, ARHGDIB, CPA6, GGPS1, LRRC4C, PCDHGA3, SETBP1, TRPM6, ARHGDIG, CPAMD8, GGT1, LRRC52, PCDHGA4, SETD1A, TRPM7, ARHGEF1, CPB1, GGT2, LRRC55, PCDHGA5, SETD1B, TRPM8, ARHGEF10, CPB2, GGT5, LRRC56, PCDHGA6, SETD2, TRPS1, ARHGEF10L, CPD, GGT6, LRRC57, PCDHGA7, SETD3, TRPT1, ARHGEF11, CPE, GGT7, LRRC58, PCDHGA8, SETD4, TRPV1, ARHGEF12, CPEB1, GGTLC1, LRRC59, PCDHGA9, SETD5, TRPV2, ARHGEF15, CPEB2, GGTLC2, LRRC6, PCDHGB1, SETD6, TRPV3, ARHGEF16, CPEB3, GH1, LRRC61, PCDHGB2, SETD7, TRPV4, ARHGEF17, CPEB4, GH2, LRRC63, PCDHGB3, SETD8, TRPV5, ARHGEF18, CPED1, GHDC, LRRC66, PCDHGB4, SETD9, TRPV6, ARHGEF19, CPLX1, GHITM, LRRC69, PCDHGB5, SETDB1, TRRAP, ARHGEF2, CPLX2, GHR, LRRC7, PCDHGB6, SETDB2, TRUB1, ARHGEF25, CPLX3, GHRH, LRRC70, PCDHGB7, SETMAR, TRUB2, ARHGEF26, CPLX4, GHRHR, LRRC71, PCDHGC3, SETSIP, TSACC, ARHGEF28, CPM, GHRL, LRRC72, PCDHGC4, SETX, TSC1, ARHGEF3, CPN1, GHSR, |
| LRRC73, PCDHGC5, SEZ6, TSC2, ARHGEF33, CPN2, GID4, LRRC8A, PCDP1, SEZ6L, TSC22D1, ARHGEF35, CPNE1, GID8, LRRC8B, PCED1A, SEZ6L2, TSC22D2, ARHGEF37, CPNE2, GIF, LRRC8C, PCED1B, SF1, TSC22D3, ARHGEF38, CPNE3, GIGYF1, LRRC8D, PCF11, SF3A1, TSC22D4, ARHGEF39, CPNE4, GIGYF2, LRRC8E, PCGF1, SF3A2, TSEN15, ARHGEF4, CPNE5, GIMAP1, LRRCC1, PCGF2, SF3A3, TSEN2, ARHGEF40, CPNE6, GIMAP1-GIMAP5, LRRD1, PCGF3, SF3B1, TSEN34, ARHGEF5, CPNE7, GIMAP2, LRRFIP1, PCGF5, SF3B14, TSEN54, ARHGEF6, CPNE8, GIMAP4, LRRFIP2, PCGF6, SF3B2, TSFM, ARHGEF7, CPNE9, GIMAP5, LRRIQ1, PCID2, SF3B3, TSG101, ARHGEF9, CPO, GIMAP6, LRRIQ3, PCIF1, SF3B4, TSGA10, ARID1A, CPOX, GIMAP7, LRRIQ4, PCK1, SF3B5, TSGA10IP, ARID1B, CPPED1, GIMAP8, LRRK1, PCK2, SFI1, TSGA13, ARID2, CPQ, GIMD1, LRRK2, PCLO, SFMBT1, TSHB, ARID3A, CPS1, GIN1, LRRN1, PCM1, SFMBT2, TSHR, ARID3B, CPSF1, GINM1, LRRN2, PCMT1, SFN, TSHZ1, ARID3C, CPSF2, GINS1, LRRN3, PCMTD1, SFPQ, TSHZ2, ARID4A, CPSF3, GINS2, LRRN4, PCMTD2, SFR1, TSHZ3, ARID4B, CPSF3L, GINS3, LRRN4CL, PCNA, SFRP1, TSKS, ARID5A, CPSF4, GINS4, LRRTM1, PCNP, SFRP2, TSKU, ARID5B, CPSF4L, GIP, LRRTM2, PCNT, SFRP4, TSLP, ARIH1, CPSF6, GIPC1, LRRTM3, PCNX, SFRP5, TSN, ARIH2, CPSF7, GIPC2, LRRTM4, PCNXL2, SFSWAP, TSNARE1, ARIH2OS, CPT1A, GIPC3, LRSAM1, PCNXL3, SFT2D1, TSNAX, ARL1, CPT1B, GIPR, LRTM1, PCNXL4, SFT2D2, TSNAXIP1, ARL10, CPT1C, GIT1, LRTM2, PCOLCE, SFT2D3, TSPAN1, ARL11, CPT2, GIT2, LRTOMT, PCOLCE2, SFTA2, TSPAN10, ARL13A, CPVL, GJA1, LRWD1, PCP2, SFTA3, TSPAN11, ARL13B, CPXCR1, GJA10, LSAMP, PCP4, SFTPA1, TSPAN12, ARL14, CPXM1, GJA3, LSG1, PCP4L1, SFTPA2, TSPAN13, ARL14EP, CPXM2, GJA4, LSM1, PCSK1, SFTPB, TSPAN14, ARL14EPL, CPZ, GJA5, LSM10, PCSK1N, SFTPC, TSPAN15, ARL15, CR1, GJA8, LSM11, PCSK2, SFTPD, TSPAN16, ARL16, CR1L, GJA9, LSM12, PCSK4, SFXN1, TSPAN17, ARL17A, CR2, GJB1, LSM14A, PCSK5, SFXN2, TSPAN18, ARL17B, CRABP1, GJB2, LSM14B, PCSK6, SFXN3, TSPAN19, ARL2, CRABP2, GJB3, LSM2, PCSK7, SFXN4, TSPAN2, ARL2BP, CRADD, GJB4, LSM3, PCSK9, SFXN5, TSPAN3, ARL3, CRAMP1L, GJB5, LSM4, PCTP, SGCA, TSPAN31, ARL4A, CRAT, GJB6, LSM5, PCYOX1, SGCB, TSPAN32, ARL4C, CRB1, GJB7, LSM6, PCYOX1L, SGCD, TSPAN33, ARL4D, CRB2, GJC1, LSM7, PCYT1A, SGCE, TSPAN4, ARL5A, CRB3, GJC2, LSMD1, PCYT1B, SGCG, TSPAN5, ARL5B, CRBN, GJC3, LSMEM1, PCYT2, SGCZ, TSPAN6, ARL5C, CRCP, GJD2, LSMEM2, PDAP1, SGIP1, TSPAN7, ARL6, CRCT1, GJD3, LSP1, PDC, SGK1, TSPAN8, ARL6IP1, CREB1, GJD4, LSR, PDCD1, SGK2, TSPAN9, ARL6IP4, CREB3, GK, LSS, PDCD10, SGK223, TSPEAR, ARL6IP5, CREB3L1, GK2, LST1, PDCD11, SGK3, TSPO, ARL6IP6, CREB3L2, GK5, LTA, PDCD1LG2, SGK494, TSPO2, ARL8A, CREB3L3, GKAP1, LTA4H, PDCD2, SGMS1, TSPY1, ARL8B, CREB3L4, GKN1, LTB, PDCD2L, SGMS2, TSPY10, ARL9, CREB5, GKN2, LTB4R, PDCD4, SGOL1, TSPY2, ARMC1, CREBBP, GLA, LTB4R2, PDCD5, SGOL2, TSPY3, ARMC10, CREBL2, GLB1, LTBP1, PDCD6, SGPL1, TSPY4, ARMC12, CREBRF, GLB1L, LTBP2, PDCD6IP, SGPP1, TSPY8, ARMC2, CREBZF, GLB1L2, LTBP3, PDCD7, SGPP2, TSPYL1, |
| ARMC3, CREG1, GLB1L3, LTBP4, PDCL, SGSH, TSPYL2, ARMC4, CREG2, GLCCI1, LTBR, PDCL2, SGSM1, TSPYL4, ARMC5, CRELD1, GLCE, LTC4S, PDCL3, SGSM2, TSPYL5, ARMC6, CRELD2, GLDC, LTF, PDDC1, SGSM3, TSPYL6, ARMC7, CREM, GLDN, LTK, PDE10A, SGTA, TSR1, ARMC8, CRH, GLE1, LTN1, PDE11A, SGTB, TSR2, ARMC9, CRHBP, GLG1, LTV1, PDE12, SH2B1, TSR3, ARMCX1, CRHR1, GLI1, LUC7L, PDE1A, SH2B2, TSSC1, ARMCX2, CRHR2, GLI2, LUC7L2, PDE1B, SH2B3, TSSC4, ARMCX3, CRIM1, GLI3, LUC7L3, PDE1C, SH2D1A, TSSK1B, ARMCX4, CRIP1, GLI4, LUM, PDE2A, SH2D1B, TSSK2, ARMCX5, CRIP2, GLIPR1, LURAP1, PDE3A, SH2D2A, TSSK3, ARMCX5-GPRASP2, CRIP3, GLIPR1L1, LURAP1L, PDE3B, SH2D3A, TSSK4, ARMCX6, CRIPAK, GLIPR1L2, LUZP1, PDE4A, SH2D3C, TSSK6, ARMS2, CRIPT, GLIPR2, LUZP2, PDE4B, SH2D4A, TST, ARNT, CRISP1, GLIS1, LUZP4, PDE4C, SH2D4B, TSTA3, ARNT2, CRISP2, GLIS2, LUZP6, PDE4D, SH2D5, TSTD1, ARNTL, CRISP3, GLIS3, LXN, PDE4DIP, SH2D6, TSTD2, ARNTL2, CRISPLD1, GLMN, LY6D, PDE5A, SH2D7, TSTD3, ARPC1A, CRISPLD2, GLO1, LY6E, PDE6A, SH3BGR, TTBK1, ARPC1B, CRK, GLOD4, LY6G5B, PDE6B, SH3BGRL, TTBK2, ARPC2, CRKL, GLOD5, LY6G5C, PDE6C, SH3BGRL2, TTC1, ARPC3, CRLF1, GLP1R, LY6G6C, PDE6D, SH3BGRL3, TTC12, ARPC4, CRLF2, GLP2R, LY6G6D, PDE6G, SH3BP1, TTC13, ARPC4-TTLL3, CRLF3, GLRA1, LY6G6F, PDE6H, SH3BP2, TTC14, ARPC5, CRLS1, GLRA2, LY6H, PDE7A, SH3BP4, TTC16, ARPC5L, CRMP1, GLRA3, LY6K, PDE7B, SH3BP5, TTC17, ARPP19, CRNKL1, GLRA4, LY75, PDE8A, SH3BP5L, TTC18, ARPP21, CRNN, GLRB, LY75-CD302, PDE8B, SH3D19, TTC19, ARR3, CROCC, GLRX, LY86, PDE9A, SH3D21, TTC21A, ARRB1, CROT, GLRX2, LY9, PDF, SH3GL1, TTC21B, ARRB2, CRP, GLRX3, LY96, PDGFA, SH3GL2, TTC22, ARRDC1, CRTAC1, GLRX5, LYAR, PDGFB, SH3GL3, TTC23, ARRDC2, CRTAM, GLS, LYG1, PDGFC, SH3GLB1, TTC23L, ARRDC3, CRTAP, GLS2, LYG2, PDGFD, SH3GLB2, TTC24, ARRDC4, CRTC1, GLT1D1, LYL1, PDGFRA, SH3KBP1, TTC25, ARRDC5, CRTC2, GLT6D1, LYN, PDGFRB, SH3PXD2A, TTC26, ARSA, CRTC3, GLT8D1, LYNX1, PDGFRL, SH3PXD2B, TTC27, ARSB, CRX, GLT8D2, LYPD1, PDHA1, SH3RF1, TTC28, ARSD, CRY1, GLTP, LYPD2, PDHA2, SH3RF2, TTC29, ARSE, CRY2, GLTPD1, LYPD3, PDHB, SH3RF3, TTC3, ARSF, CRYAA, GLTPD2, LYPD4, PDHX, SH3TC1, TTC30A, ARSG, CRYAB, GLTSCR1, LYPD5, PDIA2, SH3TC2, TTC30B, ARSH, CRYBA1, GLTSCR1L, LYPD6, PDIA3, SH3YL1, TTC31, ARSI, CRYBA2, GLTSCR2, LYPD6B, PDIA4, SHANK1, TTC32, ARSJ, CRYBA4, GLUD1, LYPD8, PDIA5, SHANK2, TTC33, ARSK, CRYBB1, GLUD2, LYPLA1, PDIA6, SHANK3, TTC34, ART1, CRYBB2, GLUL, LYPLA2, PDIK1L, SHARPIN, TTC36, ART3, CRYBB3, GLYAT, LYPLAL1, PDILT, SHB, TTC37, ART4, CRYBG3, GLYATL1, LYRM1, PDK1, SHBG, TTC38, ART5, CRYGA, GLYATL2, LYRM2, PDK2, SHC1, TTC39A, ARTN, CRYGB, GLYATL3, LYRM4, PDK3, SHC2, TTC39B, ARV1, CRYGC, GLYCTK, LYRM5, PDK4, SHC3, TTC39C, ARVCF, CRYGD, GLYR1, LYRM7, PDLIM1, SHC4, TTC4, ARX, CRYGN, GM2A, LYRM9, PDLIM2, SHCBP1, TTC40, AS3MT, CRYGS, GMCL1, LYSMD1, PDLIM3, SHCBP1L, TTC5, ASAH1, CRYL1, GMDS, LYSMD2, PDLIM4, SHD, TTC6, ASAH2, CRYM, GMEB1, LYSMD3, PDLIM5, |
| SHE, TTC7A, ASAH2B, CRYZ, GMEB2, LYSMD4, PDLIM7, SHF, TTC7B, ASAP1, CRYZL1, GMFB, LYST, PDP1, SHFM1, TTC8, ASAP2, CS, GMFG, LYVE1, PDP2, SHH, TTC9, ASAP3, CSAD, GMIP, LYZ, PDPK1, SHISA2, TTC9B, ASB1, CSAG1, GML, LYZL1, PDPN, SHISA3, TTC9C, ASB10, CSAG2, GMNC, LYZL2, PDPR, SHISA4, TTF1, ASB11, CSAG3, GMNN, LYZL4, PDRG1, SHISA5, TTF2, ASB12, CSDC2, GMPPA, LYZL6, PDS5A, SHISA6, TTI1, ASB13, CSDE1, GMPPB, LZIC, PDS5B, SHISA7, TTI2, ASB14, CSE1L, GMPR, LZTFL1, PDSS1, SHISA8, TTK, ASB15, CSF1, GMPR2, LZTR1, PDSS2, SHISA9, TTL, ASB16, CSF1R, GMPS, LZTS1, PDX1, SHKBP1, TTLL1, ASB17, CSF2, GNA11, LZTS2, PDXDC1, SHMT1, TTLL10, ASB18, CSF2RA, GNA12, LZTS3, PDXK, SHMT2, TTLL11, ASB2, CSF2RB, GNA13, M1AP, PDXP, SHOC2, TTLL12, ASB3, CSF3, GNA14, M6PR, PDYN, SHOX, TTLL13, ASB4, CSF3R, GNA15, MAATS1, PDZD11, SHOX2, TTLL2, ASB5, CSGALNACT1, GNAI1, MAB21L1, PDZD2, SHPK, TTLL3, ASB6, CSGALNACT2, GNAI2, MAB21L2, PDZD3, SHPRH, TTLL4, ASB7, CSH1, GNAI3, MAB21L3, PDZD4, SHQ1, TTLL5, ASB8, CSH2, GNAL, MACC1, PDZD7, SHROOM1, TTLL6, ASB9, CSHL1, GNAO1, MACF1, PDZD8, SHROOM2, TTLL7, ASCC1, CSK, GNAQ, MACROD1, PDZD9, SHROOM3, TTLL8, ASCC2, CSMD1, GNAS, MACROD2, PDZK1, SHROOM4, TTLL9, ASCC3, CSMD2, GNAT1, MAD1L1, PDZK1IP1, SI, TTN, ASCL1, CSMD3, GNAT2, MAD2L1, PDZRN3, SIAE, TTPA, ASCL2, CSN1S1, GNAT3, MAD2L1BP, PDZRN4, SIAH1, TTPAL, ASCL3, CSN2, GNAZ, MAD2L2, PEA15, SIAH2, TTR, ASCL4, CSN3, GNB1, MADCAM1, PEAK1, SIAH3, TTYH1, ASCL5, CSNK1A1, GNB1L, MADD, PEAR1, SIDT1, TTYH2, ASF1A, CSNK1A1L, GNB2, MAEA, PEBP1, SIDT2, TTYH3, ASF1B, CSNK1D, GNB2L1, MAEL, PEBP4, SIGIRR, TUB, ASGR1, CSNK1E, GNB3, MAF, PECAM1, SIGLEC1, TUBA1A, ASGR2, CSNK1G1, GNB4, MAF1, PECR, SIGLEC10, TUBA1B, ASH1L, CSNK1G2, GNB5, MAFA, PEF1, SIGLEC11, TUBA1C, ASH2L, CSNK1G3, GNE, MAFB, PEG10, SIGLEC12, TUBA3C, ASIC1, CSNK2A1, GNG10, MAFF, PEG3, SIGLEC14, TUBA3D, ASIC2, CSNK2A2, GNG11, MAFG, PELI1, SIGLEC15, TUBA3E, ASIC3, CSNK2A3, GNG12, MAFK, PELI2, SIGLEC5, TUBA4A, ASIC4, CSNK2B, GNG13, MAG, PELI3, SIGLEC6, TUBA8, ASIC5, CSPG4, GNG2, MAGEA1, PELO, SIGLEC7, TUBAL3, ASIP, CSPG5, GNG3, MAGEA10, PELP1, SIGLEC8, TUBB, ASL, CSPP1, GNG4, MAGEA10-MAGEA5, PEMT, SIGLEC9, TUBB1, ASMT, CSRNP1, GNG5, MAGEA11, PENK, SIGLECL1, TUBB2A, ASMTL, CSRNP2, GNG7, MAGEA12, PEPD, SIGMAR1, TUBB2B, ASNA1, CSRNP3, GNG8, MAGEA3, PER1, SIK1, TUBB3, ASNS, CSRP1, GNGT1, MAGEA4, PER2, SIK2, TUBB4A, ASNSD1, CSRP2, GNGT2, MAGEA5, PER3, SIK3, TUBB4B, ASPA, CSRP2BP, GNL1, MAGEA6, PERM1, SIKE1, TUBB6, ASPDH, CSRP3, GNL2, MAGEA8, PERP, SIL1, TUBB8, ASPG, CST1, GNL3, MAGEB1, PES1, SIM1, TUBD1, ASPH, CST11, GNL3L, MAGEB10, PET100, SIM2, TUBE1, ASPHD1, CST2, GNLY, MAGEB16, PET112, SIMC1, TUBG1, ASPHD2, CST3, GNMT, MAGEB17, PET117, SIN3A, TUBG2, ASPM, CST4, GNPAT, MAGEB18, PEX1, SIN3B, TUBGCP2, ASPN, CST5, GNPDA1, MAGEB2, PEX10, SIPA1, TUBGCP3, ASPRV1, CST6, GNPDA2, MAGEB3, PEX11A, SIPA1L1, TUBGCP4, ASPSCR1, CST7, GNPNAT1, MAGEB4, PEX11B, SIPA1L2, TUBGCP5, |
| ASRGL1, CST8, GNPTAB, MAGEB5, PEX11G, SIPA1L3, TUBGCP6, ASS1, CST9, GNPTG, MAGEB6, PEX12, SIRPA, TUFM, ASTE1, CST9L, GNRH1, MAGEC1, PEX13, SIRPB1, TUFT1, ASTL, CSTA, GNRH2, MAGEC2, PEX14, SIRPB2, TULP1, ASTN1, CSTB, GNRHR, MAGEC3, PEX16, SIRPD, TULP2, ASTN2, CSTF1, GNS, MAGED1, PEX19, SIRPG, TULP3, ASUN, CSTF2, GOLGA1, MAGED2, PEX2, SIRT1, TULP4, ASXL1, CSTF2T, GOLGA2, MAGEE1, PEX26, SIRT2, TUSC1, ASXL2, CSTF3, GOLGA3, MAGEE2, PEX3, SIRT3, TUSC2, ASXL3, CSTL1, GOLGA4, MAGEF1, PEX5, SIRT4, TUSC3, ASZ1, CT45A1, GOLGA5, MAGEH1, PEX5L, SIRT5, TUSC5, ATAD1, CT45A2, GOLGA6A, MAGEL2, PEX6, SIRT6, TUT1, ATAD2, CT45A3, GOLGA6B, MAGI1, PEX7, SIRT7, TVP23A, ATAD2B, CT45A4, GOLGA6C, MAGI2, PF4, SIT1, TVP23B, ATAD3A, CT45A5, GOLGA6D, MAGI3, PF4V1, SIVA1, TVP23C, ATAD3B, CT45A6, GOLGA6L1, MAGIX, PFAS, SIX1, TVP23C-CDRT4, ATAD3C, CT47A1, GOLGA6L10, MAGOH, PFDN1, SIX2, TWF1, ATAD5, CT47A10, GOLGA6L2, MAGOHB, PFDN2, SIX3, TWF2, ATAT1, CT47A11, GOLGA6L3, MAGT1, PFDN4, SIX4, TWIST1, ATCAY, CT47A12, GOLGA6L4, MAK, PFDN5, SIX5, TWIST2, ATE1, CT47A2, GOLGA6L6, MAK16, PFDN6, SIX6, TWISTNB, ATF1, CT47A3, GOLGA6L9, MAL, PFKFB1, SKA1, TWSG1, ATF2, CT47A4, GOLGA7, MAL2, PFKFB2, SKA2, TXK, ATF3, CT47A5, GOLGA7B, MALL, PFKFB3, SKA3, TXLNA, ATF4, CT47A6, GOLGA8A, MALSU1, PFKFB4, SKAP1, TXLNB, ATF5, CT47A7, GOLGA8B, MALT1, PFKL, SKAP2, TXLNG, ATF6, CT47A8, GOLGA8H, MAMDC2, PFKM, SKI, TXN, ATF6B, CT47A9, GOLGA8J, MAMDC4, PFKP, SKIDA1, TXN2, ATF7, CT47B1, GOLGA8K, MAML1, PFN1, SKIL, TXNDC11, ATF7IP, CT62, GOLGA8M, MAML2, PFN2, SKIV2L, TXNDC12, ATF7IP2, CTAG1A, GOLGA8O, MAML3, PFN3, SKIV2L2, TXNDC15, ATG10, CTAG1B, GOLGA8R, MAMLD1, PFN4, SKOR1, TXNDC16, ATG12, CTAG2, GOLGB1, MAMSTR, PGA3, SKOR2, TXNDC17, ATG13, CTAGE1, GOLIM4, MAN1A1, PGA4, SKP1, TXNDC2, ATG14, CTAGE15, GOLM1, MAN1A2, PGA5, SKP2, TXNDC5, ATG16L1, CTAGE4, GOLPH3, MAN1B1, PGAM1, SLA, TXNDC8, ATG16L2, CTAGE5, GOLPH3L, MAN1C1, PGAM2, SLA2, TXNDC9, ATG2A, CTAGE6, GOLT1A, MAN2A1, PGAM4, SLAIN1, TXNIP, ATG2B, CTAGE8, GOLT1B, MAN2A2, PGAM5, SLAIN2, TXNL1, ATG3, CTAGE9, GON4L, MAN2B1, PGAP1, SLAMF1, TXNL4A, ATG4A, CTBP1, GOPC, MAN2B2, PGAP2, SLAMF6, TXNL4B, ATG4B, CTBP2, GORAB, MAN2C1, PGAP3, SLAMF7, TXNRD1, ATG4C, CTBS, GORASP1, MANBA, PGBD1, SLAMF8, TXNRD2, ATG4D, CTC1, GORASP2, MANBAL, PGBD2, SLAMF9, TXNRD3, ATG5, CTCF, GOSR1, MANEA, PGBD3, SLBP, TXNRD3NB, ATG7, CTCFL, GOSR2, MANEAL, PGBD4, SLC10A1, TYK2, ATG9A, CTDNEP1, GOT1, MANF, PGBD5, SLC10A2, TYMP, ATG9B, CTDP1, GOT1L1, MANSC1, PGC, SLC10A3, TYMS, ATHL1, CTDSP1, GOT2, MANSC4, PGD, SLC10A4, TYR, ATIC, CTDSP2, GP1BA, MAOA, PGF, SLC10A5, TYRO3, ATL1, CTDSPL, GP1BB, MAOB, PGGT1B, SLC10A6, TYROBP, ATL2, CTDSPL2, GP2, MAP10, PGK1, SLC10A7, TYRP1, ATL3, CTF1, GP5, MAP1A, PGK2, SLC11A1, TYSND1, ATM, CTGF, GP6, MAP1B, PGLS, SLC11A2, TYW1, ATMIN, CTH, GP9, MAP1LC3A, PGLYRP1, SLC12A1, TYW1B, ATN1, CTHRC1, GPA33, MAP1LC3B, PGLYRP2, SLC12A2, TYW3, ATOH1, CTIF, |
| GPAA1, MAP1LC3B2, PGLYRP3, SLC12A3, TYW5, ATOH7, CTLA4, GPALPP1, MAP1LC3C, PGLYRP4, SLC12A4, U2AF1, ATOH8, CTNNA1, GPAM, MAP1S, PGM1, SLC12A5, U2AF1L4, ATOX1, CTNNA2, GPANK1, MAP2, PGM2, SLC12A6, U2AF2, ATP10A, CTNNA3, GPAT2, MAP2K1, PGM2L1, SLC12A7, U2SURP, ATP10B, CTNNAL1, GPATCH1, MAP2K2, PGM3, SLC12A8, UACA, ATP10D, CTNNB1, GPATCH11, MAP2K3, PGM5, SLC12A9, UAP1, ATP11A, CTNNBIP1, GPATCH2, MAP2K4, PGP, SLC13A1, UAP1L1, ATP11B, CTNNBL1, GPATCH2L, MAP2K5, PGPEP1, SLC13A2, UBA1, ATP11C, CTNND1, GPATCH3, MAP2K6, PGPEP1L, SLC13A3, UBA2, ATP12A, CTNND2, GPATCH4, MAP2K7, PGR, SLC13A4, UBA3, ATP13A1, CTNS, GPATCH8, MAP3K1, PGRMC1, SLC13A5, UBA5, ATP13A2, CTPS1, GPBAR1, MAP3K10, PGRMC2, SLC14A1, UBA52, ATP13A3, CTPS2, GPBP1, MAP3K11, PGS1, SLC14A2, UBA6, ATP13A4, CTR9, GPBP1L1, MAP3K12, PHACTR1, SLC15A1, UBA7, ATP13A5, CTRB1, GPC1, MAP3K13, PHACTR2, SLC15A2, UBAC1, ATP1A1, CTRB2, GPC2, MAP3K14, PHACTR3, SLC15A3, UBAC2, ATP1A2, CTRC, GPC3, MAP3K15, PHACTR4, SLC15A4, UBALD1, ATP1A3, CTRL, GPC4, MAP3K19, PHAX, SLC15A5, UBALD2, ATP1A4, CTSA, GPC5, MAP3K2, PHB, SLC16A1, UBAP1, ATP1B1, CTSB, GPC6, MAP3K3, PHB2, SLC16A10, UBAP1L, ATP1B2, CTSC, GPCPD1, MAP3K4, PHC1, SLC16A11, UBAP2, ATP1B3, CTSD, GPD1, MAP3K5, PHC2, SLC16A12, UBAP2L, ATP1B4, CTSE, GPD1L, MAP3K6, PHC3, SLC16A13, UBASH3A, ATP2A1, CTSF, GPD2, MAP3K7, PHEX, SLC16A14, UBASH3B, ATP2A2, CTSG, GPER1, MAP3K7CL, PHF1, SLC16A2, UBB, ATP2A3, CTSH, GPHA2, MAP3K8, PHF10, SLC16A3, UBC, ATP2B1, CTSK, GPHB5, MAP3K9, PHF11, SLC16A4, UBD, ATP2B2, CTSL, GPHN, MAP4, PHF12, SLC16A5, UBE2A, ATP2B3, CTSO, GPI, MAP4K1, PHF13, SLC16A6, UBE2B, ATP2B4, CTSS, GPIHBP1, MAP4K2, PHF14, SLC16A7, UBE2C, ATP2C1, CTSV, GPKOW, MAP4K3, PHF19, SLC16A8, UBE2D1, ATP2C2, CTSW, GPLD1, MAP4K4, PHF2, SLC16A9, UBE2D2, ATP4A, CTSZ, GPM6A, MAP4K5, PHF20, SLC17A1, UBE2D3, ATP4B, CTTN, GPM6B, MAP6, PHF20L1, SLC17A2, UBE2D4, ATP5A1, CTTNBP2, GPN1, MAP6D1, PHF21A, SLC17A3, UBE2E1, ATP5B, CTTNBP2NL, GPN2, MAP7, PHF21B, SLC17A4, UBE2E2, ATP5C1, CTU1, GPN3, MAP7D1, PHF23, SLC17A5, UBE2E2-AS1, ATP5D, CTU2, GPNMB, MAP7D2, PHF3, SLC17A6, UBE2E3, ATP5E, CTXN1, GPR1, MAP7D3, PHF5A, SLC17A7, UBE2F, ATP5F1, CTXN2, GPR101, MAP9, PHF6, SLC17A8, UBE2G1, ATP5G1, CTXN3, GPR107, MAPK1, PHF7, SLC17A9, UBE2G2, ATP5G2, CUBN, GPR108, MAPK10, PHF8, SLC18A1, UBE2H, ATP5G3, CUEDC1, GPR110, MAPK11, PHGDH, SLC18A2, UBE2I, ATP5H, CUEDC2, GPR111, MAPK12, PHGR1, SLC18A3, UBE2J1, ATP5I, CUL1, GPR112, MAPK13, PHIP, SLC18B1, UBE2J2, ATP5J, CUL2, GPR113, MAPK14, PHKA1, SLC19A1, UBE2K, ATP5J2, CUL3, GPR114, MAPK15, PHKA2, SLC19A2, UBE2L3, ATP5J2-PTCD1, CUL4A, GPR115, MAPK1IP1L, PHKB, SLC19A3, UBE2L6, ATP5L, CUL4B, GPR116, MAPK3, PHKG1, SLC1A1, UBE2M, ATP5L2, CUL5, GPR119, MAPK4, PHKG2, SLC1A2, UBE2N, ATP5O, CUL7, GPR12, MAPK6, PHLDA1, SLC1A3, UBE2NL, ATP5S, CUL9, GPR123, MAPK7, PHLDA2, SLC1A4, UBE2O, ATP5SL, CUTA, GPR124, MAPK8, PHLDA3, SLC1A5, UBE2Q1, |
| ATP6AP1, CUTC, GPR125, MAPK8IP1, PHLDB1, SLC1A6, UBE2Q2, ATP6AP1L, CUX1, GPR126, MAPK8IP2, PHLDB2, SLC1A7, UBE2QL1, ATP6AP2, CUX2, GPR128, MAPK8IP3, PHLDB3, SLC20A1, UBE2R2, ATP6V0A1, CUZD1, GPR132, MAPK9, PHLPP1, SLC20A2, UBE2S, ATP6V0A2, CWC15, GPR133, MAPKAP1, PHLPP2, SLC22A1, UBE2T, ATP6V0A4, CWC22, GPR135, MAPKAPK2, PHOSPHO1, SLC22A10, UBE2U, ATP6V0B, CWC25, GPR137, MAPKAPK3, PHOSPHO2, SLC22A11, UBE2V1, ATP6V0C, CWC27, GPR137B, MAPKAPK5, PHOSPHO2-KLHL23, SLC22A12, UBE2V2, ATP6V0D1, CWF19L1, GPR137C, MAPKBP1, PHOX2A, SLC22A13, UBE2W, ATP6V0D2, CWF19L2, GPR139, MAPRE1, PHOX2B, SLC22A14, UBE2Z, ATP6V0E1, CWH43, GPR142, MAPRE2, PHPT1, SLC22A15, UBE3A, ATP6V0E2, CX3CL1, GPR143, MAPRE3, PHRF1, SLC22A16, UBE3B, ATP6V1A, CX3CR1, GPR148, MAPT, PHTF1, SLC22A17, UBE3C, ATP6V1B1, CXADR, GPR149, 1-Mar, PHTF2, SLC22A18, UBE3D, ATP6V1B2, CXCL1, GPR15, 10-Mar, PHYH, SLC22A18AS, UBE4A, ATP6V1C1, CXCL10, GPR150, 11- Mar, PHYHD1, SLC22A2, UBE4B, ATP6V1C2, CXCL11, GPR151, 2-Mar, PHYHIP, SLC22A20, UBFD1, ATP6V1D, CXCL12, GPR152, 3-Mar, PHYHIPL, SLC22A23, UBIAD1, ATP6V1E1, CXCL13, GPR153, 4- Mar, PHYKPL, SLC22A24, UBL3, ATP6V1E2, CXCL14, GPR155, 5-Mar, PI15, SLC22A25, UBL4A, ATP6V1F, CXCL16, GPR156, 6-Mar, PI16, SLC22A3, UBL4B, ATP6V1G1, CXCL17, GPR157, 7-Mar, PI3, SLC22A31, UBL5, ATP6V1G2, CXCL2, GPR158, 8-Mar, PI4K2A, SLC22A4, UBL7, ATP6V1G3, CXCL3, GPR160, 9-Mar, PI4K2B, SLC22A5, UBLCP1, ATP6V1H, CXCL5, GPR161, MARCKS, PI4KA, SLC22A6, UBN1, ATP7A, CXCL6, GPR162, MARCKSL1, PI4KB, SLC22A7, UBN2, ATP7B, CXCL9, GPR17, MARCO, PIANP, SLC22A8, UBOX5, ATP8A1, CXCR1, GPR171, MARK1, PIAS1, SLC22A9, UBP1, ATP8A2, CXCR2, GPR173, MARK2, PIAS2, SLC23A1, UBQLN1, ATP8B1, CXCR3, GPR174, MARK3, PIAS3, SLC23A2, UBQLN2, ATP8B2, CXCR4, GPR176, MARK4, PIAS4, SLC23A3, UBQLN3, ATP8B3, CXCR5, GPR179, MARS, PIBF1, SLC24A1, UBQLN4, ATP8B4, CXCR6, GPR18, MARS2, PICALM, SLC24A2, UBQLNL, ATP9A, CXorf21, GPR180, MARVELD1, PICK1, SLC24A3, UBR1, ATP9B, CXorf22, GPR182, MARVELD2, PID1, SLC24A4, UBR2, ATPAF1, CXorf23, GPR183, MARVELD3, PIDD, SLC24A5, UBR3, ATPAF2, CXorf27, GPR19, MAS1, PIEZO1, SLC25A1, UBR4, ATPIF1, CXorf30, GPR20, MAS1L, PIEZO2, SLC25A10, UBR5, ATR, CXorf36, GPR21, MASP1, PIF1, SLC25A11, UBR7, ATRAID, CXorf38, GPR22, MASP2, PIFO, SLC25A12, UBTD1, ATRIP, CXorf40A, GPR25, MAST1, PIGA, SLC25A13, UBTD2, ATRN, CXorf40B, GPR26, MAST2, PIGB, SLC25A14, UBTF, ATRNL1, CXorf48, GPR27, MAST3, PIGC, SLC25A15, UBTFL1, ATRX, CXorf49, GPR3, MAST4, PIGF, SLC25A16, UBXN1, ATXN1, CXorf49B, GPR31, MASTL, PIGG, SLC25A17, UBXN10, ATXN10, CXorf51A, GPR32, MAT1A, PIGH, SLC25A18, UBXN11, ATXN1L, CXorf51B, GPR33, MAT2A, PIGK, SLC25A19, UBXN2A, ATXN2, CXorf56, GPR34, MAT2B, PIGL, SLC25A2, UBXN2B, ATXN2L, CXorf57, GPR35, MATK, PIGM, SLC25A20, UBXN4, ATXN3, CXorf58, GPR37, MATN1, PIGN, SLC25A21, UBXN6, ATXN3L, CXorf61, GPR37L1, MATN2, PIGO, SLC25A22, UBXN7, ATXN7, CXorf64, GPR39, MATN3, PIGP, SLC25A23, UBXN8, ATXN7L1, CXorf65, GPR4, MATN4, |
| PIGQ, SLC25A24, UCHL1, ATXN7L2, CXorf66, GPR45, MATR3, PIGR, SLC25A25, UCHL3, ATXN7L3, CXXC1, GPR50, MAU2, PIGS, SLC25A26, UCHL5, ATXN7L3B, CXXC11, GPR52, MAVS, PIGT, SLC25A27, UCK1, AUH, CXXC4, GPR55, MAX, PIGU, SLC25A28, UCK2, AUNIP, CXXC5, GPR56, MAZ, PIGV, SLC25A29, UCKL1, AUP1, CYB561, GPR6, MB, PIGW, SLC25A3, UCMA, AURKA, CYB561A3, GPR61, MB21D1, PIGX, SLC25A30, UCN, AURKAIP1, CYB561D1, GPR62, MB21D2, PIGY, SLC25A31, UCN2, AURKB, CYB561D2, GPR63, MBD1, PIGZ, SLC25A32, UCN3, AURKC, CYB5A, GPR64, MBD2, PIH1D1, SLC25A33, UCP1, AUTS2, CYB5B, GPR65, MBD3, PIH1D2, SLC25A34, UCP2, AVEN, CYB5D1, GPR68, MBD3L1, PIH1D3, SLC25A35, UCP3, AVIL, CYB5D2, GPR75, MBD4, PIK3AP1, SLC25A36, UEVLD, AVL9, CYB5R1, GPR75-ASB3, MBD5, PIK3C2A, SLC25A37, UFC1, AVP, CYB5R2, GPR78, MBD6, PIK3C2B, SLC25A38, UFD1L, AVPI1, CYB5R3, GPR82, MBIP, PIK3C2G, SLC25A39, UFL1, AVPR1A, CYB5R4, GPR83, MBL2, PIK3C3, SLC25A4, UFM1, AVPR1B, CYB5RL, GPR84, MBLAC1, PIK3CA, SLC25A40, UFSP1, AVPR2, CYBA, GPR85, MBLAC2, PIK3CB, SLC25A41, UFSP2, AWAT1, CYBB, GPR87, MBNL1, PIK3CD, SLC25A42, UGCG, AWAT2, CYBRD1, GPR88, MBNL2, PIK3CG, SLC25A43, UGDH, AXDND1, CYC1, GPR89A, MBNL3, PIK3IP1, SLC25A44, UGGT1, AXIN1, CYCS, GPR89B, MBOAT1, PIK3R1, SLC25A45, UGGT2, AXIN2, CYFIP1, GPR89C, MBOAT2, PIK3R2, SLC25A46, UGP2, AXL, CYFIP2, GPR97, MBOAT4, PIK3R3, SLC25A47, UGT1A1, AZGP1, CYGB, GPR98, MBOAT7, PIK3R4, SLC25A48, UGT1A10, AZI1, CYHR1, GPRASP1, MBP, PIK3R5, SLC25A5, UGT1A3, AZI2, CYLC1, GPRASP2, MBTD1, PIK3R6, SLC25A51, UGT1A4, AZIN1, CYLC2, GPRC5A, MBTPS1, PIKFYVE, SLC25A52, UGT1A5, AZU1, CYLD, GPRC5B, MBTPS2, PILRA, SLC25A53, UGT1A6, B2M, CYorf17, GPRC5C, MC1R, PILRB, SLC25A6, UGT1A7, B3GALNT1, CYP11A1, GPRC5D, MC2R, PIM1, SLC26A1, UGT1A8, B3GALNT2, CYP11B1, GPRC6A, MC3R, PIM2, SLC26A10, UGT1A9, B3GALT1, CYP11B2, GPRIN1, MC4R, PIM3, SLC26A11, UGT2A1, B3GALT2, CYP17A1, GPRIN2, MC5R, PIN1, SLC26A2, UGT2A2, B3GALT4, CYP19A1, GPRIN3, MCAM, PIN4, SLC26A3, UGT2A3, B3GALT5, CYP1A1, GPS1, MCAT, PINK1, SLC26A4, UGT2B10, B3GALT6, CYP1A2, GPS2, MCC, PINLYP, SLC26A5, UGT2B11, B3GALTL, CYP1B1, GPSM1, MCCC1, PINX1, SLC26A6, UGT2B15, B3GAT1, CYP20A1, GPSM2, MCCC2, PIP, SLC26A7, UGT2B17, B3GAT2, CYP21A2, GPSM3, MCCD1, PIP4K2A, SLC26A8, UGT2B28, B3GAT3, CYP24A1, GPT, MCEE, PIP4K2B, SLC26A9, UGT2B4, B3GNT1, CYP26A1, GPT2, MCF2, PIP4K2C, SLC27A1, UGT2B7, B3GNT2, CYP26B1, GPX1, MCF2L, PIP5K1A, SLC27A2, UGT3A1, B3GNT3, CYP26C1, GPX2, MCF2L2, PIP5K1B, SLC27A3, UGT3A2, B3GNT4, CYP27A1, GPX3, MCFD2, PIP5K1C, SLC27A4, UGT8, B3GNT5, CYP27B1, GPX4, MCHR1, PIPSKL1, SLC27A5, UHMK1, B3GNT6, CYP27C1, GPX5, MCHR2, PIPOX, SLC27A6, UHRF1, B3GNT7, CYP2A13, GPX6, MCIDAS, PIR, SLC28A1, UHRF1BP1, B3GNT8, CYP2A6, GPX7, MCL1, PIRT, SLC28A2, UHRF1BP1L, B3GNT9, CYP2A7, GPX8, MCM10, PISD, SLC28A3, UHRF2, B3GNTL1, CYP2B6, GRAMD1A, MCM2, PITHD1, SLC29A1, UIMC1, B4GALNT1, CYP2C18, GRAMD1B, MCM3, PITPNA, SLC29A2, ULBP1, B4GALNT2, CYP2C19, |
| GRAMD1C, MCM3AP, PITPNB, SLC29A3, ULBP2, B4GALNT3, CYP2C8, GRAMD2, MCM4, PITPNC1, SLC29A4, ULBP3, B4GALNT4, CYP2C9, GRAMD3, MCM5, PITPNM1, SLC2A1, ULK1, B4GALT1, CYP2D6, GRAMD4, MCM6, PITPNM2, SLC2A10, ULK2, B4GALT2, CYP2E1, GRAP, MCM7, PITPNM3, SLC2A11, ULK3, B4GALT3, CYP2F1, GRAP2, MCM8, PITRM1, SLC2A12, ULK4, B4GALT4, CYP2J2, GRAPL, MCM9, PITX1, SLC2A13, UMOD, B4GALT5, CYP2R1, GRASP, MCMBP, PITX2, SLC2A14, UMODL1, B4GALT6, CYP2S1, GRB10, MCMDC2, PITX3, SLC2A2, UMPS, B4GALT7, CYP2U1, GRB14, MCOLN1, PIWIL1, SLC2A3, UNC119, B9D1, CYP2W1, GRB2, MCOLN2, PIWIL2, SLC2A4, UNC119B, B9D2, CYP39A1, GRB7, MCOLN3, PIWIL3, SLC2A4RG, UNC13A, BAALC, CYP3A4, GREB1, MCPH1, PIWIL4, SLC2A5, UNC13B, BAAT, CYP3A43, GREM1, MCRS1, PJA1, SLC2A6, UNC13C, BABAM1, CYP3A5, GREM2, MCTP1, PJA2, SLC2A7, UNC13D, BACE1, CYP3A7, GRHL1, MCTP2, PKD1, SLC2A8, UNC45A, BACE2, CYP3A7- CYP3AP1, GRHL2, MCTS1, PKD1L1, SLC2A9, UNC45B, BACH1, CYP46A1, GRHL3, MCU, PKD1L2, SLC30A1, UNC50, BACH2, CYP4A11, GRHPR, MCUR1, PKD1L3, SLC30A10, UNC5A, BAD, CYP4A22, GRIA1, MDC1, PKD2, SLC30A2, UNC5B, BAG1, CYP4B1, GRIA2, MDFI, PKD2L1, SLC30A3, UNC5C, BAG2, CYP4F11, GRIA3, MDFIC, PKD2L2, SLC30A4, UNC5CL, BAG3, CYP4F12, GRIA4, MDGA1, PKDCC, SLC30A5, UNC5D, BAG4, CYP4F2, GRID1, MDGA2, PKDREJ, SLC30A6, UNC79, BAG5, CYP4F22, GRID2, MDH1, PKHD1, SLC30A7, UNC80, BAG6, CYP4F3, GRID2IP, MDH1B, PKHD1L1, SLC30A8, UNC93A, BAGE, CYP4F8, GRIFIN, MDH2, PKIA, SLC30A9, UNC93B1, BAGE2, CYP4V2, GRIK1, MDK, PKIB, SLC31A1, UNCX, BAGE3, CYP4X1, GRIK2, MDM1, PKIG, SLC31A2, UNG, BAHCC1, CYP4Z1, GRIK3, MDM2, PKLR, SLC32A1, UNK, BAHD1, CYP51A1, GRIK4, MDM4, PKM, SLC33A1, UNKL, BAI1, CYP7A1, GRIK5, MDN1, PKMYT1, SLC34A1, UPB1, BAI2, CYP7B1, GRIN1, MDP1, PKN1, SLC34A2, UPF1, BAI3, CYP8B1, GRIN2A, ME1, PKN2, SLC34A3, UPF2, BAIAP2, CYR61, GRIN2B, ME2, PKN3, SLC35A1, UPF3A, BAIAP2L1, CYS1, GRIN2C, ME3, PKNOX1, SLC35A2, UPF3B, BAIAP2L2, CYSLTR1, GRIN2D, MEA1, PKNOX2, SLC35A3, UPK1A, BAIAP3, CYSLTR2, GRIN3A, MEAF6, PKP1, SLC35A4, UPK1B, BAK1, CYSTM1, GRIN3B, MECOM, PKP2, SLC35A5, UPK2, BAMBI, CYTH1, GRINA, MECP2, PKP3, SLC35B1, UPK3A, BANF1, CYTH2, GRIP1, MECR, PKP4, SLC35B2, UPK3B, BANF2, CYTH3, GRIP2, MED1, PLA1A, SLC35B3, UPK3BL, BANK1, CYTH4, GRIPAP1, MED10, PLA2G10, SLC35B4, UPP1, BANP, CYTIP, GRK1, MED11, PLA2G12A, SLC35C1, UPP2, BAP1, CYTL1, GRK4, MED12, PLA2G12B, SLC35C2, UPRT, BARD1, CYYR1, GRK5, MED12L, PLA2G15, SLC35D1, UQCC1, BARHL1, D2HGDH, GRK6, MED13, PLA2G16, SLC35D2, UQCC2, BARHL2, DAAM1, GRK7, MED13L, PLA2G1B, SLC35D3, UQCR10, BARX1, DAAM2, GRM1, MED14, PLA2G2A, SLC35E1, UQCR11, BARX2, DAB1, GRM2, MED15, PLA2G2C, SLC35E2, UQCRB, BASP1, DAB2, GRM3, MED16, PLA2G2D, SLC35E2B, UQCRC1, BATF, DAB2IP, GRM4, MED17, PLA2G2E, SLC35E3, UQCRC2, BATF2, DACH1, GRM5, MED18, PLA2G2F, SLC35E4, UQCRFS1, BATF3, DACH2, GRM6, MED19, PLA2G3, SLC35F1, UQCRH, BAX, DACT1, GRM7, MED20, PLA2G4A, |
| SLC35F2, UQCRHL, BAZ1A, DACT2, GRM8, MED21, PLA2G4B, SLC35F3, UQCRQ, BAZ1B, DACT3, GRN, MED22, PLA2G4C, SLC35F4, URAD, BAZ2A, DAD1, GRP, MED23, PLA2G4D, SLC35F5, URB1, BAZ2B, DAG1, GRPEL1, MED24, PLA2G4E, SLC35F6, URB2, BBC3, DAGLA, GRPEL2, MED25, PLA2G4F, SLC35G1, URGCP, BBIP1, DAGLB, GRPR, MED26, PLA2G5, SLC35G2, URGCP-MRPS24, BBOX1, DAK, GRSF1, MED27, PLA2G6, SLC35G3, URI1, BBS1, DALRD3, GRTP1, MED28, PLA2G7, SLC35G4, URM1, BBS10, DAND5, GRWD1, MED29, PLA2R1, SLC35G5, UROC1, BBS12, DAO, GRXCR1, MED30, PLAA, SLC35G6, UROD, BBS2, DAOA, GRXCR2, MED31, PLAC1, SLC36A1, UROS, BBS4, DAP, GSAP, MED4, PLAC4, SLC36A2, USB1, BBS5, DAP3, GSC, MED6, PLAC8, SLC36A3, USE1, BBS7, DAPK1, GSC2, MED7, PLAC8L1, SLC36A4, USF1, BBS9, DAPK2, GSDMA, MED8, PLAC9, SLC37A1, USF2, BBX, DAPK3, GSDMB, MED9, PLAG1, SLC37A2, USH1C, BCAM, DAPL1, GSDMC, MEDAG, PLAGL1, SLC37A3, USH1G, BCAN, DAPP1, GSDMD, MEF2A, PLAGL2, SLC37A4, USH2A, BCAP29, DARS, GSE1, MEF2B, PLAT, SLC38A1, USHBP1, BCAP31, DARS2, GSG1, MEF2BNB, PLAU, SLC38A10, USMG5, BCAR1, DAW1, GSG1L, MEF2BNB-MEF2B, PLAUR, SLC38A11, USO1, BCAR3, DAXX, GSG2, MEF2C, PLB1, SLC38A2, USP1, BCAS1, DAZ1, GSK3A, MEF2D, PLBD1, SLC38A3, USP10, BCAS2, DAZ2, GSK3B, MEFV, PLBD2, SLC38A4, USP11, BCAS3, DAZ3, GSKIP, MEGF10, PLCB1, SLC38A5, USP12, BCAS4, DAZ4, GSN, MEGF11, PLCB2, SLC38A6, USP13, BCAT1, DAZAP1, GSPT1, MEGF6, PLCB3, SLC38A7, USP14, BCAT2, DAZAP2, GSPT2, MEGF8, PLCB4, SLC38A8, USP15, BCCIP, DAZL, GSR, MEGF9, PLCD1, SLC38A9, USP16, BCDIN3D, DBF4, GSS, MEI1, PLCD3, SLC39A1, USP17L24, BCHE, DBF4B, GSTA1, MEI4, PLCD4, SLC39A10, USP17L25, BCKDHA, DBH, GSTA2, MEIG1, PLCE1, SLC39A11, USP17L26, BCKDHB, DBI, GSTA3, MEIOB, PLCG1, SLC39A12, USP17L28, BCKDK, DBN1, GSTA4, MEIS1, PLCG2, SLC39A13, USP17L29, BCL10, DBNDD1, GSTA5, MEIS2, PLCH1, SLC39A14, USP18, BCL11A, DBNDD2, GSTCD, MEIS3, PLCH2, SLC39A2, USP19, BCL11B, DBNL, GSTK1, MELK, PLCL1, SLC39A3, USP2, BCL2, DBP, GSTM1, MEMO1, PLCL2, SLC39A4, USP20, BCL2A1, DBR1, GSTM2, MEN1, PLCXD1, SLC39A5, USP21, BCL2L1, DBT, GSTM3, MEOX1, PLCXD2, SLC39A6, USP22, BCL2L10, DBX1, GSTM4, MEOX2, PLCXD3, SLC39A7, USP24, BCL2L11, DBX2, GSTM5, MEP1A, PLCZ1, SLC39A8, USP25, BCL2L12, DCAF10, GSTO1, MEP1B, PLD1, SLC39A9, USP26, BCL2L13, DCAF11, GSTO2, MEPCE, PLD2, SLC3A1, USP27X, BCL2L14, DCAF12, GSTP1, MEPE, PLD3, SLC3A2, USP28, BCL2L15, DCAF12L1, GSTT1, MERTK, PLD4, SLC40A1, USP29, BCL2L2, DCAF12L2, GSTT2, MESDC1, PLD5, SLC41A1, USP3, BCL2L2-PABPN1, DCAF13, GSTT2B, MESDC2, PLD6, SLC41A2, USP30, BCL3, DCAF15, GSTZ1, MESP1, PLEC, SLC41A3, USP31, BCL6, DCAF16, GSX1, MESP2, PLEK, SLC43A1, USP32, BCL6B, DCAF17, GSX2, MEST, PLEK2, SLC43A2, USP33, BCL7A, DCAF4, GTDC1, MET, PLEKHA1, SLC43A3, USP34, BCL7B, DCAF4L1, GTF2A1, METAP1, PLEKHA2, SLC44A1, USP35, BCL7C, DCAF4L2, GTF2A1L, METAP1D, PLEKHA3, SLC44A2, USP36, BCL9, DCAF5, GTF2A2, METAP2, PLEKHA4, SLC44A3, USP37, BCL9L, DCAF6, GTF2B, METRN, PLEKHA5, SLC44A4, USP38, BCLAF1, |
| DCAF7, GTF2E1, METRNL, PLEKHA6, SLC44A5, USP39, BCMO1, DCAF8, GTF2E2, METTL1, PLEKHA7, SLC45A1, USP4, BCO2, DCAF8L1, GTF2F1, METTL10, PLEKHA8, SLC45A2, USP40, BCOR, DCAKD, GTF2F2, METTL11B, PLEKHB1, SLC45A3, USP41, BCORL1, DCBLD1, GTF2H1, METTL12, PLEKHB2, SLC45A4, USP42, BCR, DCBLD2, GTF2H2, METTL13, PLEKHD1, SLC46A1, USP43, BCS1L, DCC, GTF2H2C, METTL14, PLEKHF1, SLC46A2, USP44, BDH1, DCD, GTF2H3, METTL15, PLEKHF2, SLC46A3, USP45, BDH2, DCDC1, GTF2H4, METTL16, PLEKHG1, SLC47A1, USP46, BDKRB1, DCDC2, GTF2H5, METTL17, PLEKHG2, SLC47A2, USP47, BDKRB2, DCDC2B, GTF2I, METTL18, PLEKHG3, SLC48A1, USP48, BDNF, DCDC2C, GTF2IRD1, METTL20, PLEKHG4, SLC4A1, USP49, BDP1, DCDC5, GTF2IRD2, METTL21A, PLEKHG4B, SLC4A10, USP5, BEAN1, DCHS1, GTF2IRD2B, METTL21B, PLEKHG5, SLC4A11, USP50, BECN1, DCHS2, GTF3A, METTL21C, PLEKHG6, SLC4A1AP, USP51, BECN1P1, DCK, GTF3C1, METTL22, PLEKHG7, SLC4A2, USP53, BEGAIN, DCLK1, GTF3C2, METTL23, PLEKHH1, SLC4A3, USP54, BEND2, DCLK2, GTF3C3, METTL24, PLEKHH2, SLC4A4, USP6, BEND3, DCLK3, GTF3C4, METTL25, PLEKHH3, SLC4A5, USP6NL, BEND4, DCLRE1A, GTF3C5, METTL2A, PLEKHJ1, SLC4A7, USP7, BEND5, DCLRE1B, GTF3C6, METTL2B, PLEKHM1, SLC4A8, USP8, BEND6, DCLRE1C, GTPBP1, METTL3, PLEKHM2, SLC4A9, USP9X, BEND7, DCN, GTPBP10, METTL4, PLEKHM3, SLC50A1, USP9Y, BEST1, DCP1A, GTPBP2, METTL5, PLEKHN1, SLC51A, USPL1, BEST2, DCP1B, GTPBP3, METTL6, PLEKHO1, SLC51B, UST, BEST3, DCP2, GTPBP4, METTL7A, PLEKHO2, SLC52A1, UTF1, BEST4, DCPS, GTPBP6, METTL7B, PLEKHS1, SLC52A2, UTP11L, BET1, DCST1, GTPBP8, METTL8, PLET1, SLC52A3, UTP14A, BET1L, DCST2, GTSCR1, METTL9, PLG, SLC5A1, UTP14C, BEX1, DCSTAMP, GTSE1, MEX3A, PLGLB1, SLC5A10, UTP15, BEX2, DCT, GTSF1, MEX3B, PLGLB2, SLC5A11, UTP18, BEX4, DCTD, GTSF1L, MEX3C, PLGRKT, SLC5A12, UTP20, BEX5, DCTN1, GUCA1A, MEX3D, PLIN1, SLC5A2, UTP23, BFAR, DCTN2, GUCA1B, MFAP1, PLIN2, SLC5A3, UTP3, BFSP1, DCTN3, GUCA1C, MFAP2, PLIN3, SLC5A4, UTP6, BFSP2, DCTN4, GUCA2A, MFAP3, PLIN4, SLC5A5, UTRN, BGLAP, DCTN5, GUCA2B, MFAP3L, PLIN5, SLC5A6, UTS2, BGN, DCTN6, GUCD1, MFAP4, PLK1, SLC5A7, UTS2B, BHLHA15, DCTPP1, GUCY1A2, MFAP5, PLK1S1, SLC5A8, UTY, BHLHA9, DCUN1D1, GUCY1A3, MFF, PLK2, SLC5A9, UVRAG, BHLHB9, DCUN1D2, GUCY1B3, MFGE8, PLK3, SLC6A1, UVSSA, BHLHE22, DCUN1D3, GUCY2C, MFHAS1, PLK4, SLC6A11, UXS1, BHLHE23, DCUN1D4, GUCY2D, MFI2, PLK5, SLC6A12, UXT, BHLHE40, DCUN1D5, GUCY2F, MFN1, PLLP, SLC6A13, VAC14, BHLHE41, DCX, GUF1, MFN2, PLN, SLC6A14, VAMP1, BHMT, DCXR, GUK1, MFNG, PLOD1, SLC6A15, VAMP2, BHMT2, DDA1, GULP1, MFRP, PLOD2, SLC6A16, VAMP3, BICC1, DDAH1, GUSB, MFSD1, PLOD3, SLC6A17, VAMP4, BICD1, DDAH2, GXYLT1, MFSD10, PLP1, SLC6A18, VAMP5, BICD2, DDB1, GXYLT2, MFSD11, PLP2, SLC6A19, VAMP7, BID, DDB2, GYG1, MFSD12, PLRG1, SLC6A2, VAMP8, BIK, DDC, GYG2, MFSD2A, PLS1, SLC6A20, VANGL1, BIN1, DDHD1, GYLTL1B, MFSD2B, PLS3, SLC6A3, VANGL2, BIN2, DDHD2, GYPA, MFSD3, PLSCR1, SLC6A4, VAPA, |
| BIN3, DDI1, GYPB, MFSD4, PLSCR2, SLC6A5, VAPB, BIRC2, DDI2, GYPC, MFSD5, PLSCR3, SLC6A6, VARS, BIRC3, DDIT3, GYPE, MFSD6, PLSCR4, SLC6A7, VARS2, BIRC5, DDIT4, GYS1, MFSD6L, PLSCR5, SLC6A8, VASH1, BIRC6, DDIT4L, GYS2, MFSD7, PLTP, SLC6A9, VASH2, BIRC7, DDN, GZF1, MFSD8, PLVAP, SLC7A1, VASN, BIRC8, DDO, GZMA, MFSD9, PLXDC1, SLC7A10, VASP, BIVM, DDOST, GZMB, MGA, PLXDC2, SLC7A11, VAT1, BIVM-ERCC5, DDR1, GZMH, MGAM, PLXNA1, SLC7A13, VAT1L, BLCAP, DDR2, GZMK, MGARP, PLXNA2, SLC7A14, VAV1, BLID, DDRGK1, GZMM, MGAT1, PLXNA3, SLC7A2, VAV2, BLK, DDT, H1F0, MGAT2, PLXNA4, SLC7A3, VAV3, BLM, DDTL, H1FNT, MGAT3, PLXNB1, SLC7A4, VAX1, BLMH, DDX1, H1FOO, MGAT4A, PLXNB2, SLC7A5, VAX2, BLNK, DDX10, H1FX, MGAT4B, PLXNB3, SLC7A6, VBP1, BLOC1S1, DDX11, H2AFB1, MGAT4C, PLXNC1, SLC7A6OS, VCAM1, BLOC1S2, DDX17, H2AFB2, MGAT5, PLXND1, SLC7A7, VCAN, BLOC1S3, DDX18, H2AFB3, MGAT5B, PM20D1, SLC7A8, VCL, BLOC1S4, DDX19A, H2AFJ, MGEA5, PM20D2, SLC7A9, VCP, BLOC1S5, DDX19B, H2AFV, MGLL, PMAIP1, SLC8A1, VCPIP1, BLOC1S6, DDX20, H2AFX, MGME1, PMCH, SLC8A2, VCPKMT, BLVRA, DDX21, H2AFY, MGMT, PMEL, SLC8A3, VCX, BLVRB, DDX23, H2AFY2, MGP, PMEPA1, SLC8B1, VCX2, BLZF1, DDX24, H2AFZ, MGRN1, PMF1, SLC9A1, VCX3A, BMF, DDX25, H2BFM, MGST1, PMF1-BGLAP, SLC9A2, VCX3B, BMI1, DDX26B, H2BFWT, MGST2, PMFBP1, SLC9A3, VCY, BMP1, DDX27, H3F3A, MGST3, PML, SLC9A3R1, VCY1B, BMP10, DDX28, H3F3B, MIA, PMM1, SLC9A3R2, VDAC1, BMP15, DDX31, H3F3C, MIA2, PMM2, SLC9A4, VDAC2, BMP2, DDX39A, H6PD, MIA3, PMP2, SLC9A5, VDAC3, BMP2K, DDX39B, HAAO, MIB1, PMP22, SLC9A6, VDR, BMP3, DDX3X, HABP2, MIB2, PMPCA, SLC9A7, VEGFA, BMP4, DDX3Y, HABP4, MICA, PMPCB, SLC9A8, VEGFB, BMP5, DDX4, HACE1, MICAL1, PMS1, SLC9A9, VEGFC, BMP6, DDX41, HACL1, MICAL2, PMS2, SLC9B1, VENTX, BMP7, DDX42, HADH, MICAL3, PMVK, SLC9B2, VEPH1, BMP8A, DDX43, HADHA, MICALCL, PNCK, SLC9C1, VEZF1, BMP8B, DDX46, HADHB, MICALL1, PNISR, SLC9C2, VEZT, BMPER, DDX47, HAGH, MICALL2, PNKD, SLCO1A2, VGF, BMPR1A, DDX49, HAGHL, MICB, PNKP, SLCO1B1, VGLL1, BMPR1B, DDX5, HAL, MICU1, PNLDC1, SLCO1B3, VGLL2, BMPR2, DDX50, HAMP, MICU2, PNLIP, SLCO1B7, VGLL3, BMS1, DDX51, HAND1, MICU3, PNLIPRP1, SLCO1C1, VGLL4, BMX, DDX52, HAND2, MID1, PNLIPRP2, SLCO2A1, VHL, BNC1, DDX53, HAO1, MID1IP1, PNLIPRP3, SLCO2B1, VHLL, BNC2, DDX54, HAO2, MID2, PNMA2, SLCO3A1, VIL1, BNIP1, DDX55, HAP1, MIDN, PNMA3, SLCO4A1, VILL, BNIP2, DDX56, HAPLN1, MIEF1, PNMA5, SLCO4C1, VIM, BNIP3, DDX58, HAPLN2, MIEF2, PNMA6A, SLCO5A1, VIMP, BNIP3L, DDX59, HAPLN3, MIEN1, PNMA6C, SLCO6A1, VIP, BNIPL, DDX6, HAPLN4, MIER1, PNMAL1, SLFN11, VIPAS39, BOC, DDX60, HARBI1, MIER2, PNMAL2, SLFN12, VIPR1, BOD1, DDX60L, HARS, MIER3, PNMT, SLFN12L, VIPR2, BOD1L1, DEAF1, HARS2, MIF, PNN, SLFN13, VIT, BOD1L2, 1-Dec, HAS1, MIF4GD, PNO1, SLFN14, VKORC1, BOK, DECR1, HAS2, MIIP, PNOC, SLFN5, VKORC1L1, BOLA1, DECR2, HAS3, MILR1, PNP, SLFNL1, VLDLR, BOLA2, DEDD, HAT1, MINA, PNPLA1, SLIRP, VMA21, |
| BOLA2B, DEDD2, HAUS1, MINK1, PNPLA2, SLIT1, VMAC, BOLA3, DEF6, HAUS2, MINOS1, PNPLA3, SLIT2, VMO1, BOLL, DEF8, HAUS3, MINOS1-NBL1, PNPLA4, SLIT3, VMP1, BOP1, DEFA1, HAUS4, MINPP1, PNPLA5, SLITRK1, VN1R1, BORA, DEFA1B, HAUS5, MIOS, PNPLA6, SLITRK2, VN1R2, BPGM, DEFA3, HAUS6, MIOX, PNPLA7, SLITRK3, VN1R4, BPHL, DEFA4, HAUS7, MIP, PNPLA8, SLITRK4, VN1R5, BPI, DEFA5, HAUS8, MIPEP, PNPO, SLITRK5, VNN1, BPIFA1, DEFA6, HAVCR1, MIPOL1, PNPT1, SLITRK6, VNN2, BPIFA2, DEFB1, HAVCR2, MIR205HG, PNRC1, SLK, VOPP1, BPIFA3, DEFB103A, HAX1, MIS12, PNRC2, SLMAP, VPRBP, BPIFB1, DEFB103B, HBA1, MIS18A, POC1A, SLMO1, VPREB1, BPIFB2, DEFB104A, HBA2, MIS18BP1, POC1B, SLMO2, VPREB3, BPIFB3, DEFB104B, HBB, MISP, POC1B-GALNT4, SLN, VPS11, BPIFB4, DEFB105A, HBD, MITD1, POC5, SLPI, VPS13A, BPIFB6, DEFB105B, HBE1, MITF, PODN, SLTM, VPS13B, BPIFC, DEFB106A, HBEGF, MIXL1, PODNL1, SLU7, VPS13C, BPNT1, DEFB106B, HBG1, MKI67, PODXL, SLURP1, VPS13D, BPTF, DEFB107A, HBG2, MKKS, PODXL2, SLX1A, VPS16, BPY2, DEFB107B, HBM, MKL1, POF1B, SLX1B, VPS18, BPY2B, DEFB108B, HBP1, MKL2, POFUT1, SLX4, VPS25, BPY2C, DEFB110, HBQ1, MKLN1, POFUT2, SLX4IP, VPS26A, BRAF, DEFB112, HBS1L, MKNK1, POGK, SMAD1, VPS26B, BRAP, DEFB113, HBZ, MKNK2, POGLUT1, SMAD2, VPS28, BRAT1, DEFB114, HCAR1, MKRN1, POGZ, SMAD3, VPS29, BRCA1, DEFB115, HCAR2, MKRN2, POLA1, SMAD4, VPS33A, BRCA2, DEFB116, HCAR3, MKRN3, POLA2, SMAD5, VPS33B, BRCC3, DEFB118, HCCS, MKS1, POLB, SMAD6, VPS35, BRD1, DEFB119, HCFC1, MKX, POLD1, SMAD7, VPS36, BRD2, DEFB121, HCFC1R1, MLANA, POLD2, SMAD9, VPS37A, BRD3, DEFB123, HCFC2, MLC1, POLD3, SMAGP, VPS37B, BRD4, DEFB124, HCK, MLEC, POLD4, SMAP1, VPS37C, BRD7, DEFB125, HCLS1, MLF1, POLDIP2, SMAP2, VPS37D, BRD8, DEFB126, HCN1, MLF2, POLDIP3, SMARCA1, VPS39, BRD9, DEFB127, HCN2, MLH1, POLE, SMARCA2, VPS41, BRDT, DEFB128, HCN3, MLH3, POLE2, SMARCA4, VPS45, BRE, DEFB129, HCN4, MLIP, POLE3, SMARCA5, VPS4A, BRF1, DEFB130, HCRT, MLKL, POLE4, SMARCAD1, VPS4B, BRF2, DEFB131, HCRTR1, MLLT1, POLG, SMARCAL1, VPS51, BRI3, DEFB132, HCRTR2, MLLT10, POLG2, SMARCB1, VPS52, BRI3BP, DEFB133, HCST, MLLT11, POLH, SMARCC1, VPS53, BRICD5, DEFB134, HDAC1, MLLT3, POLI, SMARCC2, VPS54, BRINP1, DEFB135, HDAC10, MLLT4, POLK, SMARCD1, VPS72, BRINP2, DEFB136, HDAC11, MLLT6, POLL, SMARCD2, VPS8, BRINP3, DEFB4A, HDAC2, MLN, POLM, SMARCD3, VPS9D1, BRIP1, DEFB4B, HDAC3, MLNR, POLN, SMARCE1, VRK1, BRIX1, DEGS1, HDAC4, MLPH, POLQ, SMC1A, VRK2, BRK1, DEGS2, HDAC5, MLST8, POLR1A, SMC1B, VRK3, BRMS1, DEK, HDAC6, MLX, POLR1B, SMC2, VRTN, BRMS1L, DENND1A, HDAC7, MLXIP, POLR1C, SMC3, VSIG1, BROX, DENND1B, HDAC8, MLXIPL, POLR1D, SMC4, VSIG10, BRPF1, DENND1C, HDAC9, MLYCD, POLR1E, SMC5, VSIG10L, BRPF3, DENND2A, HDC, MMAA, POLR2A, SMC6, VSIG2, BRS3, DENND2C, HDDC2, MMAB, POLR2B, SMCHD1, VSIG4, BRSK1, DENND2D, HDDC3, MMACHC, POLR2C, SMCO2, VSIG8, BRSK2, DENND3, HDGF, MMADHC, POLR2D, SMCO3, VSNL1, BRWD1, DENND4A, HDGFL1, MMD, POLR2E, |
| SMCO4, VSTM1, BRWD3, DENND4B, HDGFRP2, MMD2, POLR2F, SMCP, VSTM2A, BSCL2, DENND4C, HDGFRP3, MME, POLR2G, SMCR8, VSTM2B, BSDC1, DENND5A, HDHD1, MMEL1, POLR2H, SMCR9, VSTM2L, BSG, DENND5B, HDHD2, MMGT1, POLR2I, SMDT1, VSTM4, BSN, DENND6A, HDHD3, MMP1, POLR2J, SMEK1, VSTM5, BSND, DENND6B, HDLBP, MMP10, POLR2J2, SMEK2, VSX1, BSPH1, DENR, HDX, MMP11, POLR2J3, SMG1, VSX2, BSPRY, DEPDC1, HEATR1, MMP12, POLR2K, SMG5, VTA1, BST1, DEPDC1B, HEATR2, MMP13, POLR2L, SMG6, VTCN1, BST2, DEPDC4, HEATR3, MMP14, POLR2M, SMG7, VTI1A, BSX, DEPDC5, HEATR4, MMP15, POLR3A, SMG8, VTI1B, BTAF1, DEPDC7, HEATR5A, MMP16, POLR3B, SMG9, VTN, BTBD1, DEPTOR, HEATR5B, MMP17, POLR3C, SMIM1, VWA1, BTBD10, DERA, HEATR6, MMP19, POLR3D, SMIM10, VWA2, BTBD11, DERL1, HEBP1, MMP2, POLR3E, SMIM11, VWA3A, BTBD16, DERL2, HEBP2, MMP20, POLR3F, SMIM12, VWA3B, BTBD17, DERL3, HECA, MMP21, POLR3G, SMIM13, VWA5A, BTBD18, DES, HECTD1, MMP23B, POLR3GL, SMIM14, VWA5B1, BTBD19, DESI1, HECTD2, MMP24, POLR3H, SMIM15, VWA5B2, BTBD2, DESI2, HECTD3, MMP25, POLR3K, SMIM17, VWA7, BTBD3, DET1, HECTD4, MMP26, POLRMT, SMIM18, VWA8, BTBD6, DEXI, HECW1, MMP27, POM121, SMIM19, VWA9, BTBD7, DFFA, HECW2, MMP28, POM121C, SMIM2, VWC2, BTBD8, DFFB, HEG1, MMP3, POM121L12, SMIM20, VWC2L, BTBD9, DFNA5, HELB, MMP7, POM121L2, SMIM21, VWCE, BTC, DFNB31, HELLS, MMP8, POMC, SMIM22, VWDE, BTD, DFNB59, HELQ, MMP9, POMGNT1, SMIM3, VWF, BTF3, DGAT1, HELT, MMRN1, POMGNT2, SMIM4, WAC, BTF3L4, DGAT2, HELZ, MMRN2, POMK, SMIM5, WAPAL, BTG1, DGAT2L6, HELZ2, MMS19, POMP, SMIM6, WARS, BTG2, DGCR14, HEMGN, MMS22L, POMT1, SMIM7, WARS2, BTG3, DGCR2, HEMK1, MN1, POMT2, SMIM8, WAS, BTG4, DGCR6, HENMT1, MNAT1, POMZP3, SMIM9, WASF1, BTK, DGCR6L, HEPACAM, MND1, PON1, SMKR1, WASF2, BTLA, DGCR8, HEPACAM2, MNDA, PON2, SMLR1, WASF3, BTN1A1, DGKA, HEPH, MNS1, PON3, SMN1, WASH1, BTN2A1, DGKB, HEPHL1, MNT, POP1, SMN2, WASL, BTN2A2, DGKD, HEPN1, MNX1, POP4, SMNDC1, WBP1, BTN3A1, DGKE, HERC1, MOAP1, POP5, SMO, WBP11, BTN3A2, DGKG, HERC2, MOB1A, POP7, SMOC1, WBP1L, BTN3A3, DGKH, HERC3, MOB1B, POPDC2, SMOC2, WBP2, BTNL10, DGKI, HERC4, MOB2, POPDC3, SMOX, WBP2NL, BTNL2, DGKK, HERC5, MOB3A, POR, SMPD1, WBP4, BTNL3, DGKQ, HERC6, MOB3B, PORCN, SMPD2, WBP5, BTNL8, DGKZ, HERPUD1, MOB3C, POSTN, SMPD3, WBSCR16, BTNL9, DGUOK, HERPUD2, MOB4, POT1, SMPD4, WBSCR17, BTRC, DHCR24, HES1, MOBP, POTEA, SMPD5, WBSCR22, BUB1, DHCR7, HES2, MOCOS, POTEB, SMPDL3A, WBSCR27, BUB1B, DHDDS, HES3, MOCS1, POTEB2, SMPDL3B, WBSCR28, BUB3, DHDH, HES4, MOCS2, POTEC, SMPX, WDFY1, BUD13, DHFR, HES5, MOCS3, POTED, SMR3A, WDFY2, BUD31, DHFRL1, HES6, MOG, POTEE, SMR3B, WDFY3, BVES, DHH, HES7, MOGAT1, POTEF, SMS, WDFY4, BYSL, DHODH, HESX1, MOGAT2, POTEG, SMTN, WDHD1, BZRAP1, DHPS, HEXA, MOGAT3, POTEH, SMTNL1, WDPCP, BZW1, DHRS1, HEXB, MOGS, POTEI, SMTNL2, WDR1, BZW2, DHRS11, HEXDC, MOK, |
| POTEJ, SMU1, WDR11, C10orf10, DHRS12, HEXIM1, MON1A, POTEM, SMUG1, WDR12, C10orf105, DHRS13, HEXIM2, MON1B, POU1F1, SMURF1, WDR13, C10orf107, DHRS2, HEY1, MON2, POU2AF1, SMURF2, WDR16, C10orf11, DHRS3, HEY2, MORC1, POU2F1, SMYD1, WDR17, C10orf111, DHRS4, HEYL, MORC2, POU2F2, SMYD2, WDR18, C10orf112, DHRS4L1, HFE, MORC3, POU2F3, SMYD3, WDR19, C10orf113, DHRS4L2, HFE2, MORC4, POU3F1, SMYD4, WDR20, C10orf118, DHRS7, HFM1, MORF4L1, POU3F2, SMYD5, WDR24, C10orf12, DHRS7B, HGC6.3, MORF4L2, POU3F3, SNAI1, WDR25, C10orf120, DHRS7C, HGD, MORN1, POU3F4, SNAI2, WDR26, C10orf126, DHRS9, HGF, MORN2, POU4F1, SNAI3, WDR27, C10orf128, DHRSX, HGFAC, MORN3, POU4F2, SNAP23, WDR3, C10orf129, DHTKD1, HGS, MORN4, POU4F3, SNAP25, WDR31, C10orf131, DHX15, HGSNAT, MORN5, POU5F1, SNAP29, WDR33, C10orf137, DHX16, HHAT, MOS, POU5F1B, SNAP47, WDR34, C10orf2, DHX29, HHATL, MOSPD1, POU5F2, SNAP91, WDR35, C10orf25, DHX30, HHEX, MOSPD2, POU6F1, SNAPC1, WDR36, C10orf32, DHX32, HHIP, MOSPD3, POU6F2, SNAPC2, WDR37, C10orf35, DHX33, HHIPL1, MOV10, PP2D1, SNAPC3, WDR38, C10orf53, DHX34, HHIPL2, MOV10L1, PPA1, SNAPC4, WDR4, C10orf54, DHX35, HHLA1, MOXD1, PPA2, SNAPC5, WDR41, C10orf55, DHX36, HHLA2, MPC1, PPAN, SNAPIN, WDR43, C10orf62, DHX37, HHLA3, MPC1L, PPAN-P2RY11, SNCA, WDR44, C10orf67, DHX38, HIAT1, MPC2, PPAP2A, SNCAIP, WDR45, C10orf68, DHX40, HIATL1, MPDU1, PPAP2B, SNCB, WDR45B, C10orf71, DHX57, HIBADH, MPDZ, PPAP2C, SNCG, WDR46, C10orf76, DHX58, HIBCH, MPEG1, PPAPDC1A, SND1, WDR47, C10orfB2, DHX8, HIC1, MPG, PPAPDC1B, SNED1, WDR48, C10orf88, DHX9, HIC2, MPHOSPH10, PPAPDC2, SNF8, WDR49, C10orf90, DIABLO, HID1, MPHOSPH6, PPAPDC3, SNIP1, WDR5, C10orf91, DIAPH1, HIF1A, MPHOSPH8, PPARA, SNN, WDR52, C10orf95, DIAPH2, HIF1AN, MPHOSPH9, PPARD, SNPH, WDR53, C10orf99, DIAPH3, HIF3A, MPI, PPARG, SNRK, WDR54, C11orf1, DICER1, HIGD1A, MPL, PPARGC1A, SNRNP200, WDR55, C11orf16, DIDO1, HIGD1B, MPLKIP, PPARGC1B, SNRNP25, WDR59, C11orf21, DIEXF, HIGD1C, MPND, PPAT, SNRNP27, WDR5B, C11orf24, DIMT1, HIGD2A, MPO, PPBP, SNRNP35, WDR6, C11orf30, DIO1, HILPDA, MPP1, PPCDC, SNRNP40, WDR60, C11orf31, DIO2, HINFP, MPP2, PPCS, SNRNP48, WDR61, C11orf35, DIO3, HINT1, MPP3, PPDPF, SNRNP70, WDR62, C11orf40, DIP2A, HINT2, MPP4, PPEF1, SNRPA, WDR63, C11orf42, DIP2B, HINT3, MPP5, PPEF2, SNRPA1, WDR64, C11orf44, DIP2C, HIP1, MPP6, PPFIA1, SNRPB, WDR65, C11orf45, DIRAS1, HIP1R, MPP7, PPFIA2, SNRPB2, WDR66, C11orf48, DIRAS2, HIPK1, MPPE1, PPFIA3, SNRPC, WDR7, C11orf49, DIRAS3, HIPK2, MPPED1, PPFIA4, SNRPD1, WDR70, C11orf52, DIRC1, HIPK3, MPPED2, PPFIBP1, SNRPD2, WDR72, C11orf53, DIRC2, HIPK4, MPRIP, PPFIBP2, SNRPD3, WDR73, C11orf54, DIS3, HIRA, MPST, PPHLN1, SNRPE, WDR74, C11orf57, DIS3L, HIRIP3, MPV17, PPIA, SNRPF, WDR75, C11orf58, DIS3L2, HIST1H1A, MPV17L, PPIAL4A, SNRPG, WDR76, C11orf63, DISC1, HIST1H1B, MPV17L2, PPIAL4B, SNRPN, WDR77, C11orf65, DISP1, HIST1H1C, MPZ, PPIAL4C, SNTA1, WDR78, C11orf68, DISP2, HIST1H1D, MPZL1, PPIAL4D, SNTB1, WDR81, C11orf70, DIXDC1, |
| HIST1H1E, MPZL2, PPIAL4E, SNTB2, WDR82, C11orf71, DKC1, HIST1H1T, MPZL3, PPIAL4F, SNTG1, WDR83, C11orf73, DKK1, HIST1H2AA, MR1, PPIAL4G, SNTG2, WDR83OS, C11orf74, DKK2, HIST1H2AB, MRAP, PPIB, SNTN, WDR86, C11orf80, DKK3, HIST1H2AC, MRAP2, PPIC, SNUPN, WDR87, C11orf82, DKK4, HIST1H2AD, MRAS, PPID, SNURF, WDR88, C11orf83, DKKL1, HIST1H2AE, MRC2, PPIE, SNW1, WDR89, C11orf84, DLAT, HIST1H2AG, MRE11A, PPIF, SNX1, WDR90, C11orf85, DLC1, HIST1H2AH, MREG, PPIG, SNX10, WDR91, C11orf86, DLD, HIST1H2AI, MRFAP1, PPIH, SNX11, WDR92, C11orf87, DLEC1, HIST1H2AJ, MRFAP1L1, PPIL1, SNX12, WDR93, C11orf88, DLEU7, HIST1H2AK, MRGBP, PPIL2, SNX13, WDR96, C11orf91, DLG1, HIST1H2AL, MRGPRD, PPIL3, SNX14, WDSUB1, C11orf94, DLG2, HIST1H2AM, MRGPRE, PPIL4, SNX15, WDTC1, C11orf95, DLG3, HIST1H2BA, MRGPRF, PPIL6, SNX16, WDYHV1, C11orf96, DLG4, HIST1H2BB, MRGPRG, PPIP5K1, SNX17, WEE1, C12orf10, DLG5, HIST1H2BC, MRGPRX1, PPIP5K2, SNX18, WEE2, C12orf23, DLGAP1, HIST1H2BD, MRGPRX2, PPL, SNX19, WFDC1, C12orf29, DLGAP2, HIST1H2BE, MRGPRX3, PPM1A, SNX2, WFDC10A, C12orf39, DLGAP4, HIST1H2BF, MRGPRX4, PPM1B, SNX20, WFDC10B, C12orf4, DLGAP5, HIST1H2BG, MRI1, PPM1D, SNX21, WFDC11, C12orf40, DLK1, HIST1H2BH, MRM1, PPM1E, SNX22, WFDC12, C12orf42, DLK2, HIST1H2BI, MRO, PPM1F, SNX24, WFDC13, C12orf43, DLL1, HIST1H2BJ, MROH1, PPM1G, SNX25, WFDC2, C12orf44, DLL3, HIST1H2BK, MROH2A, PPM1H, SNX27, WFDC3, C12orf45, DLL4, HIST1H2BL, MROH2B, PPM1J, SNX29, WFDC5, C12orf49, DLST, HIST1H2BM, MROH5, PPM1K, SNX3, WFDC6, C12orf5, DLX1, HIST1H2BN, MROH6, PPM1L, SNX30, WFDC8, C12orf50, DLX2, HIST1H2BO, MROH7, PPM1M, SNX31, WFDC9, C12orf52, DLX3, HIST1H3A, MROH8, PPM1N, SNX32, WFIKKN1, C12orf54, DLX4, HIST1H3B, MROH9, PPME1, SNX33, WFIKKN2, C12orf55, DLX5, HIST1H3C, MRP63, PPOX, SNX4, WFS1, C12orf56, DLX6, HIST1H3D, MRPL1, PPP1CA, SNX5, WHAMM, C12orf57, DMAP1, HIST1H3E, MRPL10, PPP1CB, SNX6, WHSC1, C12orf60, DMBT1, HIST1H3F, MRPL11, PPP1CC, SNX7, WHSC1L1, C12orf61, DMBX1, HIST1H3G, MRPL12, PPP1R10, SNX8, WIBG, C12orf65, DMC1, HIST1H3H, MRPL13, PPP1R11, SNX9, WIF1, C12orf66, DMD, HIST1H3I, MRPL14, PPP1R12A, SOAT1, WIPF1, C12orf68, DMGDH, HIST1H3J, MRPL15, PPP1R12B, SOAT2, WIPF2, C12orf71, DMKN, HIST1H4A, MRPL16, PPP1R12C, SOBP, WIPF3, C12orf73, DMP1, HIST1H4B, MRPL17, PPP1R13B, SOCS1, WIPI1, C12orf74, DMPK, HIST1H4C, MRPL18, PPP1R13L, SOCS2, WIPI2, C12orf75, DMRT1, HIST1H4D, MRPL19, PPP1R14A, SOCS3, WISP1, C12orf76, DMRT2, HIST1H4E, MRPL2, PPP1R14B, SOCS4, WISP2, C12orf77, DMRT3, HIST1H4F, MRPL20, PPP1R14C, SOCS5, WISP3, C12orf79, DMRTA1, HIST1H4G, MRPL21, PPP1R14D, SOCS6, WIZ, C12orf80, DMRTA2, HIST1H4H, MRPL22, PPP1R15A, SOCS7, WLS, C13orf35, DMRTB1, HIST1H4I, MRPL23, PPP1R15B, SOD1, WNK1, C13orf45, DMRTC1, HIST1H4J, MRPL24, PPP1R16A, SOD2, WNK2, C14orf1, DMRTC1B, HIST1H4K, MRPL27, PPP1R16B, SOD3, WNK3, C14orf105, DMRTC2, HIST1H4L, MRPL28, PPP1R17, SOGA1, WNK4, C14orf119, DMTF1, HIST2H2AA3, MRPL3, PPP1R18, SOGA2, WNT1, C14orf132, DMTN, HIST2H2AA4, MRPL30, |
| PPP1R1A, SOGA3, WNT10A, C14orf142, DMWD, HIST2H2AB, MRPL32, PPP1R1B, SOHLH1, WNT10B, C14orf159, DMXL1, HIST2H2AC, MRPL33, PPP1R1C, SOHLH2, WNT11, C14orf164, DMXL2, HIST2H2BE, MRPL34, PPP1R2, SON, WNT16, C14orf166, DNA2, HIST2H2BF, MRPL35, PPP1R21, SORBS1, WNT2, C14orf166B, DNAAF1, HIST2H3A, MRPL36, PPP1R26, SORBS2, WNT2B, C14orf169, DNAAF2, HIST2H3C, MRPL37, PPP1R27, SORBS3, WNT3, C14orf177, DNAAF3, HIST2H3D, MRPL38, PPP1R32, SORCS1, WNT3A, C14orf178, DNAH1, HIST2H4A, MRPL39, PPP1R35, SORCS2, WNT4, C14orf180, DNAH10, HIST2H4B, MRPL4, PPP1R36, SORCS3, WNT5A, C14orf182, DNAH11, HIST3H2A, MRPL40, PPP1R37, SORD, WNT5B, C14orf183, DNAH12, HIST3H2BB, MRPL41, PPP1R3A, SORL1, WNT6, C14orf2, DNAH14, HIST3H3, MRPL42, PPP1R3B, SORT1, WNT7A, C14orf28, DNAH17, HIST4H4, MRPL43, PPP1R3C, SOS1, WNT7B, C14orf37, DNAH2, HIVEP1, MRPL44, PPP1R3D, SOS2, WNT8A, C14orf39, DNAH3, HIVEP2, MRPL45, PPP1R3E, SOST, WNT8B, C14orf79, DNAH5, HIVEP3, MRPL46, PPP1R3F, SOSTDC1, WNT9A, C14orf80, DNAH6, HJURP, MRPL47, PPP1R3G, SOWAHA, WNT9B, C14orf93, DNAH7, HK1, MRPL48, PPP1R42, SOWAHB, WRAP53, C15orf26, DNAH8, HK2, MRPL49, PPP1R7, SOWAHC, WRAP73, C15orf27, DNAH9, HK3, MRPL50, PPP1R8, SOWAHD, WRB, C15orf32, DNAI1, HKDC1, MRPL51, PPP1R9A, SOX1, WRN, C15orf38, DNAI2, HKR1, MRPL52, PPP1R9B, SOX10, WRNIP1, C15orf38-AP3S2, DNAJA1, HLA-A, MRPL53, PPP2CA, SOX11, WSB1, C15orf39, DNAJA2, HLA-B, MRPL54, PPP2CB, SOX12, WSB2, C15orf40, DNAJA3, HLA-C, MRPL55, PPP2R1A, SOX13, WSCD1, C15orf41, DNAJA4, HLA-DMA, MRPL9, PPP2R1B, SOX14, WSCD2, C15orf43, DNAJB1, HLA-DMB, MRPS10, PPP2R2A, SOX15, WT1, C15orf48, DNAJB11, HLA-DOA, MRPS11, PPP2R2B, SOX17, WTAP, C15orf52, DNAJB12, HLA-DOB, MRPS12, PPP2R2C, SOX18, WTH3DI, C15orf53, DNAJB13, HLA-DPA1, MRPS14, PPP2R2D, SOX2, WTIP, C15orf54, DNAJB14, HLA-DPB1, MRPS15, PPP2R3A, SOX21, WWC1, C15orf56, DNAJB2, HLA- DQA1, MRPS16, PPP2R3B, SOX3, WWC2, C15orf57, DNAJB3, HLA-DQA2, MRPS17, PPP2R3C, SOX30, WWC3, C15orf59, DNAJB4, HLA-DQB1, MRPS18A, PPP2R4, SOX4, WWOX, C15orf60, DNAJB5, HLA-DQB2, MRPS18B, PPP2R5A, SOX5, WWP1, C15orf61, DNAJB6, HLA-DRA, MRPS18C, PPP2R5B, SOX6, WWP2, C15orf62, DNAJB7, HLA-DRB1, MRPS2, PPP2R5C, SOX7, WWTR1, C15orf65, DNAJB8, HLA- DRB5, MRPS21, PPP2R5D, SOX8, XAB2, C16orf11, DNAJB9, HLA-E, MRPS22, PPP2R5E, SOX9, XAF1, C16orf13, DNAJC1, HLA-F, MRPS23, PPP3CA, SP1, XAGE1D, C16orf3, DNAJC10, HLA-G, MRPS24, PPP3CB, SP100, XAGE2, C16orf45, DNAJC11, HLCS, MRPS25, PPP3CC, SP110, XAGE3, C16orf46, DNAJC12, HLF, MRPS26, PPP3R1, SP140, XAGE5, C16orf47, DNAJC13, HLTF, MRPS27, PPP3R2, SP140L, XBP1, C16orf52, DNAJC14, HLX, MRPS28, PPP4C, SP2, XCL1, C16orf54, DNAJC15, HM13, MRPS30, PPP4R1, SP3, XCL2, C16orf58, DNAJC16, HMBOX1, MRPS31, PPP4R2, SP4, XCR1, C16orf59, DNAJC17, HMBS, MRPS33, PPP4R4, SP5, XDH, C16orf62, DNAJC18, HMCES, MRPS34, PPP5C, SP6, XG, C16orf70, DNAJC19, HMCN1, MRPS35, PPP5D1, SP7, XIAP, C16orf71, DNAJC2, HMCN2, MRPS36, PPP6C, SP8, XIRP1, C16orf72, DNAJC21, HMG20A, MRPS5, PPP6R1, SP9, XIRP2, |
| C16orf74, DNAJC22, HMG20B, MRPS6, PPP6R2, SPA17, XK, C16orf78, DNAJC24, HMGA1, MRPS7, PPP6R3, SPACA1, XKR3, C16orf80, DNAJC25, HMGA2, MRPS9, PPRC1, SPACA3, XKR4, C16orf82, DNAJC25-GNG10, HMGB1, MRRF, PPT1, SPACA4, XKR5, C16orf86, DNAJC27, HMGB2, MRS2, PPT2, SPACA5, XKR6, C16orf87, DNAJC28, HMGB3, MRTO4, PPTC7, SPACA5B, XKR7, C16orf89, DNAJC3, HMGB4, MRVI1, PPWD1, SPACA7, XKR8, C16orf90, DNAJC30, HMGCL, MS4A1, PPY, SPAG1, XKR9, C16orf91, DNAJC4, HMGCLL1, MS4A10, PQBP1, SPAG11A, XKRX, C16orf92, DNAJC5, HMGCR, MS4A12, PQLC1, SPAG11B, XKRY, C16orf93, DNAJC5B, HMGCS1, MS4A13, PQLC2, SPAG16, XKRY2, C16orf95, DNAJC5G, HMGCS2, MS4A14, PQLC3, SPAG17, XPA, C16orf96, DNAJC6, HMGN1, MS4A15, PRAC1, SPAG4, XPC, C16orf97, DNAJC7, HMGN2, MS4A2, PRAC2, SPAG5, XPNPEP1, C17orf100, DNAJC8, HMGN3, MS4A3, PRADC1, SPAG6, XPNPEP2, C17orf102, DNAJC9, HMGN4, MS4A4A, PRAF2, SPAG7, XPNPEP3, C17orf103, DNAL1, HMGN5, MS4A4E, PRAM1, SPAG8, XPO1, C17orf104, DNAL4, HMGXB3, MS4A5, PRAME, SPAG9, XPO4, C17orf105, DNALI1, HMGXB4, MS4A6A, PRAMEF1, SPAM1, XPO5, C17orf107, DNASE1, HMHA1, MS4A6E, PRAMEF10, SPANXA1, XPO6, C17orf112, DNASE1L1, HMHB1, MS4A7, PRAMEF11, SPANXA2, XPO7, C17orf47, DNASE1L2, HMMR, MS4A8, PRAMEF12, SPANXB1, XPOT, C17orf49, DNASE1L3, HMOX1, MSANTD1, PRAMEF13, SPANXB2, XPR1, C17orf50, DNASE2, HMOX2, MSANTD2, PRAMEF14, SPANXC, XRCC1, C17orf51, DNASE2B, HMP19, MSANTD3, PRAMEF15, SPANXD, XRCC2, C17orf53, DND1, HMSD, MSANTD3-TMEFF1, PRAMEF16, SPANXN1, XRCC3, C17orf58, DNER, HMX1, MSANTD4, PRAMEF17, SPANXN2, XRCC4, C17orf59, DNHD1, HMX2, MSC, PRAMEF18, SPANXN3, XRCC5, C17orf62, DNLZ, HMX3, MSGN1, PRAMEF19, SPANXN4, XRCC6, C17orf64, DNM1, HN1, MSH2, PRAMEF2, SPANXN5, XRCC6BP1, C17orf66, DNM1L, HN1L, MSH3, PRAMEF20, SPARC, XRN1, C17orf67, DNM2, HNF1A, MSH4, PRAMEF21, SPARCL1, XRN2, C17orf70, DNM3, HNF1B, MSH5, PRAMEF22, SPAST, XRRA1, C17orf72, DNMBP, HNF4A, MSH6, PRAMEF23, SPATA1, XXYLT1, C17orf74, DNMT1, HNF4G, MSI1, PRAMEF25, SPATA12, XYLB, C17orf75, DNMT3A, HNMT, MSI2, PRAMEF3, SPATA13, XYLT1, C17orf77, DNMT3B, HNRNPA0, MSL1, PRAMEF4, SPATA16, XYLT2, C17orf78, DNMT3L, HNRNPA1, MSL2, PRAMEF5, SPATA17, YAE1D1, C17orf80, DNPEP, HNRNPA1L2, MSL3, PRAMEF6, SPATA18, YAF2, C17orf82, DNPH1, HNRNPA2B1, MSLN, PRAMEF7, SPATA19, YAP1, C17orf85, DNTT, HNRNPA3, MSMB, PRAMEF8, SPATA2, YARS, C17orf89, DNTTIP1, HNRNPAB, MSMO1, PRAMEF9, SPATA20, YARS2, C17orf96, DNTTIP2, HNRNPC, MSMP, PRAP1, SPATA21, YBEY, C17orf97, DOC2A, HNRNPCL1, MSN, PRB1, SPATA22, YBX1, C17orf98, DOC2B, HNRNPCP5, MSR1, PRB2, SPATA24, YBX2, C17orf99, DOCK1, HNRNPD, MSRA, PRB3, SPATA25, YBX3, C18orf21, DOCK10, HNRNPDL, MSRB1, PRB4, SPATA2L, YDJC, C18orf25, DOCK11, HNRNPF, MSRB2, PRC1, SPATA3, YEATS2, C18orf32, DOCK2, HNRNPH1, MSRB3, PRCC, SPATA31A1, YEATS4, C18orf42, DOCK3, HNRNPH2, MSS51, PRCD, SPATA31A2, YES1, C18orf54, DOCK4, HNRNPH3, MST1, PRCP, SPATA31A3, YIF1A, C18orf56, DOCK5, HNRNPK, |
| MST1L, PRDM1, SPATA31A4, YIF1B, C18orf63, DOCK6, HNRNPL, MST1R, PRDM10, SPATA31A5, YIPF1, C18orf8, DOCK7, HNRNPLL, MST4, PRDM11, SPATA31A6, YIPF2, C19orf10, DOCK8, HNRNPM, MSTN, PRDM12, SPATA31A7, YIPF3, C19orf12, DOCK9, HNRNPR, MSTO1, PRDM13, SPATA31C1, YIPF4, C19orf18, DOHH, HNRNPU, MSX1, PRDM14, SPATA31C2, YIPF5, C19orf24, DOK1, HNRNPUL1, MSX2, PRDM15, SPATA31D1, YIPF6, C19orf25, DOK2, HNRNPUL2, MT1A, PRDM16, SPATA31D3, YIPF7, C19orf26, DOK3, HOGA1, MT1B, PRDM2, SPATA31D4, YJEFN3, C19orf33, DOK4, HOMER1, MT1E, PRDM4, SPATA31E1, YKT6, C19orf35, DOK5, HOMER2, MT1F, PRDM5, SPATA32, YLPM1, C19orf38, DOK6, HOMER3, MT1G, PRDM6, SPATA33, YME1L1, C19orf40, DOK7, HOMEZ, MT1H, PRDM7, SPATA4, YOD1, C19orf43, DOLK, HOOK1, MT1HL1, PRDM8, SPATA5, YPEL1, C19orf44, DOLPP1, HOOK2, MT1M, PRDM9, SPATA5L1, YPEL2, C19orf45, DONSON, HOOK3, MT1X, PRDX1, SPATA6, YPEL3, C19orf47, DOPEY1, HOPX, MT2A, PRDX2, SPATA6L, YPEL4, C19orf48, DOPEY2, HORMAD1, MT3, PRDX3, SPATA7, YPEL5, C19orf52, DOT1L, HORMAD2, MT4, PRDX4, SPATA8, YRDC, C19orf53, DPAGT1, HOXA1, MTA1, PRDX5, SPATA9, YTHDC1, C19orf54, DPCD, HOXA10, MTA2, PRDX6, SPATC1, YTHDC2, C19orf55, DPCR1, HOXA11, MTA3, PREB, SPATC1L, YTHDF1, C19orf57, DPEP1, HOXA13, MTAP, PRELID1, SPATS1, YTHDF2, C19orf59, DPEP2, HOXA2, MTBP, PRELID2, SPATS2, YTHDF3, C19orf60, DPEP3, HOXA3, MTCH1, PRELP, SPATS2L, YWHAB, C19orf66, DPF1, HOXA4, MTCH2, PREP, SPC24, YWHAE, C19orf67, DPF2, HOXA5, MTCP1, PREPL, SPC25, YWHAG, C19orf68, DPF3, HOXA6, MTDH, PREX1, SPCS1, YWHAH, C19orf69, DPH1, HOXA7, MTERF, PREX2, SPCS2, YWHAQ, C19orf70, DPH2, HOXA9, MTERFD1, PRF1, SPCS3, YWHAZ, C19orf71, DPH3, HOXB1, MTERFD2, PRG2, SPDEF, YY1, C19orf73, DPH3P1, HOXB13, MTERFD3, PRG3, SPDL1, YY1AP1, C19orf77, DPH5, HOXB2, MTF1, PRG4, SPDYA, YY2, C19orf80, DPH6, HOXB3, MTF2, PRH1, SPDYC, ZACN, C19orf81, DPH7, HOXB4, MTFMT, PRH2, SPDYE1, ZADH2, C19orf82, DPM1, HOXB5, MTFP1, PRICKLE1, SPDYE2, ZAK, C1D, DPM2, HOXB6, MTFR1, PRICKLE2, SPDYE2B, ZAN, C1GALT1, DPM3, HOXB7, MTFR1L, PRICKLE3, SPDYE3, ZAP70, C1GALT1C1, DPP10, HOXB8, MTFR2, PRICKLE4, SPDYE4, ZAR1, Clorf100, DPP3, HOXB9, MTG1, PRIM1, SPDYE5, ZAR1L, C1orf101, DPP4, HOXC10, MTG2, PRIM2, SPDYE6, ZBBX, C1orf105, DPP6, HOXC11, MTHFD1, PRIMA1, SPECC1, ZBED1, C1orf106, DPP7, HOXC12, MTHFD1L, PRIMPOL, SPECC1L, ZBED2, C1orf109, DPP8, HOXC13, MTHFD2, PRKAA1, SPEF1, ZBED3, C1orf110, DPP9, HOXC4, MTHFD2L, PRKAA2, SPEF2, ZBED4, C1orf111, DPPA2, HOXC5, MTHFR, PRKAB1, SPEG, ZBED5, C1orf112, DPPA3, HOXC6, MTHFS, PRKAB2, SPEM1, ZBED6, C1orf115, DPPA4, HOXC8, MTHFSD, PRKACA, SPEN, ZBED6CL, C1orf116, DPPA5, HOXC9, MTIF2, PRKACB, SPERT, ZBP1, C1orf122, DPRX, HOXD1, MTIF3, PRKACG, SPESP1, ZBTB1, C1orf123, DPT, HOXD10, MTL5, PRKAG1, SPG11, ZBTB10, C1orf127, DPY19L1, HOXD11, MTM1, PRKAG2, SPG20, ZBTB11, C1orf131, DPY19L2, HOXD12, MTMR1, PRKAG3, SPG21, ZBTB12, C1orf137, DPY19L3, HOXD13, MTMR10, PRKAR1A, SPG7, ZBTB14, C1orf141, DPY19L4, HOXD3, MTMR11, PRKAR1B, |
| SPHAR, ZBTB16, C1orf146, DPY30, HOXD4, MTMR12, PRKAR2A, SPHK1, ZBTB17, C1orf158, DPYD, HOXD8, MTMR14, PRKAR2B, SPHK2, ZBTB18, Clorf159, DPYS, HOXD9, MTMR2, PRKCA, SPHKAP, ZBTB2, C1orf162, DPYSL2, HP, MTMR3, PRKCB, SPI1, ZBTB20, C1orf167, DPYSL3, HP1BP3, MTMR4, PRKCD, SPIB, ZBTB21, C1orf168, DPYSL4, HPCA, MTMR6, PRKCDBP, SPIC, ZBTB22, C1orf172, DPYSL5, HPCAL1, MTMR7, PRKCE, SPICE1, ZBTB24, C1orf173, DQX1, HPCAL4, MTMR8, PRKCG, SPIDR, ZBTB25, C1orf174, DR1, HPD, MTMR9, PRKCH, SPIN1, ZBTB26, C1orf177, DRAM1, HPDL, MTNR1A, PRKCI, SPIN2A, ZBTB3, C1orf185, DRAM2, HPGD, MTNR1B, PRKCQ, SPIN2B, ZBTB32, C1orf186, DRAP1, HPGDS, MTO1, PRKCSH, SPIN3, ZBTB33, C1orf189, DRAXIN, HPN, MTOR, PRKCZ, SPIN4, ZBTB34, C1orf192, DRC1, HPR, MTPAP, PRKD1, SPINK1, ZBTB37, C1orf194, DRD1, HPRT1, MTPN, PRKD2, SPINK13, ZBTB38, C1orf195, DRD2, HPS1, MTR, PRKD3, SPINK14, ZBTB39, C1orf198, DRD3, HPS3, MTRF1, PRKDC, SPINK2, ZBTB4, C1orf204, DRD4, HPS4, MTRF1L, PRKG1, SPINK4, ZBTB40, C1orf21, DRD5, HPS5, MTRNR2L10, PRKG2, SPINK5, ZBTB41, C1orf210, DRG1, HPS6, MTRNR2L2, PRKRA, SPINK6, ZBTB42, C1orf216, DRG2, HPSE, MTRNR2L3, PRKRIP1, SPINK7, ZBTB43, C1orf226, DRGX, HPSE2, MTRNR2L4, PRKRIR, SPINK8, ZBTB44, C1orf227, DROSHA, HPX, MTRNR2L5, PRKX, SPINK9, ZBTB45, C1orf228, DRP2, HR, MTRNR2L6, PRL, SPINT1, ZBTB46, C1orf229, DSC1, HRAS, MTRNR2L7, PRLH, SPINT2, ZBTB47, C1orf233, DSC2, HRASLS, MTRNR2L9, PRLHR, SPINT3, ZBTB48, C1orf234, DSC3, HRASLS2, MTRR, PRLR, SPINT4, ZBTB49, C1orf27, DSCAM, HRASLS5, MTSS1, PRM1, SPIRE1, ZBTB5, C1orf35, DSCAML1, HRC, MTSS1L, PRM2, SPIRE2, ZBTB6, C1orf43, DSCC1, HRCT1, MTTP, PRM3, SPN, ZBTB7A, C1orf50, DSCR3, HRG, MTURN, PRMT1, SPNS1, ZBTB7B, C1orf51, DSCR4, HRH1, MTUS1, PRMT10, SPNS2, ZBTB7C, C1orf52, DSE, HRH2, MTUS2, PRMT2, SPNS3, ZBTB8A, C1orf53, DSEL, HRH3, MTX1, PRMT3, SPO11, ZBTB8B, C1orf54, DSG1, HRH4, MTX2, PRMT5, SPOCD1, ZBTB8OS, C1orf56, DSG2, HRK, MTX3, PRMT6, SPOCK1, ZBTB9, C1orf61, DSG3, HRNR, MUC1, PRMT7, SPOCK2, ZC2HC1A, C1orf63, DSG4, HRSP12, MUC12, PRMT8, SPOCK3, ZC2HC1B, C1orf64, DSN1, HS1BP3, MUC13, PRND, SPON1, ZC2HC1C, C1orf65, DSP, HS2ST1, MUC15, PRNP, SPON2, ZC3H10, C1orf68, DSPP, HS3ST1, MUC16, PROB1, SPOP, ZC3H11A, C1orf74, DST, HS3ST2, MUC17, PROC, SPOPL, ZC3H12A, C1orf85, DSTN, HS3ST3A1, MUC19, PROCA1, SPP1, ZC3H12B, C1orf86, DSTNP4, HS3ST3B1, MUC2, PROCR, SPP2, ZC3H12C, C1orf87, DSTYK, HS3ST4, MUC20, PRODH, SPPL2A, ZC3H12D, C1orf94, DTD1, HS3ST5, MUC21, PRODH2, SPPL2B, ZC3H13, C1orf95, DTD2, HS3ST6, MUC22, PROK1, SPPL2C, ZC3H14, C1QA, DTHD1, HS6ST1, MUC3A, PROK2, SPPL3, ZC3H15, C1QB, DTL, HS6ST2, MUC4, PROKR1, SPR, ZC3H18, C1QBP, DTNA, HS6ST3, MUC5AC, PROKR2, SPRED1, ZC3H3, C1QC, DTNB, HSBP1, MUC5B, PROL1, SPRED2, ZC3H4, C1QL1, DTNBP1, HSBP1L1, MUC6, PROM1, SPRED3, ZC3H6, C1QL2, DTWD1, HSCB, MUC7, PROM2, SPRN, ZC3H7A, C1QL3, DTWD2, HSD11B1, MUC8, PROP1, SPRR1A, ZC3H7B, C1QL4, DTX1, HSD11B1L, MUCL1, PROS1, SPRR1B, ZC3H8, C1QTNF1, DTX2, HSD11B2, MUL1, PROSC, SPRR2A, ZC3HAV1, C1QTNF2, DTX3, HSD17B1, |
| MUM1, PROSER1, SPRR2B, ZC3HAV1L, C1QTNF3, DTX3L, HSD17B10, MUM1L1, PROSER2, SPRR2D, ZC3HC1, C1QTNF4, DTX4, HSD17B11, MURC, PROX1, SPRR2E, ZC4H2, C1QTNF5, DTYMK, HSD17B12, MUS81, PROX2, SPRR2F, ZCCHC10, C1QTNF6, DUOX1, HSD17B13, MUSK, PROZ, SPRR2G, ZCCHC11, C1QTNF7, DUOX2, HSD17B14, MUSTN1, PRPF18, SPRR3, ZCCHC12, C1QTNF8, DUOXA1, HSD17B2, MUT, PRPF19, SPRR4, ZCCHC13, C1QTNF9, DUOXA2, HSD17B3, MUTYH, PRPF3, SPRTN, ZCCHC14, C1QTNF9B, DUPD1, HSD17B4, MVB12A, PRPF31, SPRY1, ZCCHC16, C1QTNF9B-AS1, DUS1L, HSD17B6, MVB12B, PRPF38A, SPRY2, ZCCHC17, C1R, DUS2, HSD17B7, MVD, PRPF38B, SPRY3, ZCCHC18, C1RL, DUS3L, HSD17B8, MVK, PRPF39, SPRY4, ZCCHC2, C1S, DUS4L, HSD3B1, MVP, PRPF4, SPRYD3, ZCCHC24, C2, DUSP1, HSD3B2, MX1, PRPF40A, SPRYD4, ZCCHC3, C20orf112, DUSP10, HSD3B7, MX2, PRPF40B, SPRYD7, ZCCHC4, C20orf141, DUSP11, HSDL1, MXD1, PRPF4B, SPSB1, ZCCHC5, C20orf144, DUSP12, HSDL2, MXD3, PRPF6, SPSB2, ZCCHC6, C20orf166, DUSP13, HSF1, MXD4, PRPF8, SPSB3, ZCCHC7, C20orf173, DUSP14, HSF2, MXI1, PRPH, SPSB4, ZCCHC8, C20orf194, DUSP15, HSF2BP, MXRA5, PRPH2, SPTA1, ZCCHC9, C20orf195, DUSP16, HSF4, MXRA7, PRPS1, SPTAN1, ZCRB1, C20orf196, DUSP18, HSF5, MXRA8, PRPS1L1, SPTB, ZCWPW1, C20orf197, DUSP19, HSFX1, MYADM, PRPS2, SPTBN1, ZCWPW2, C20orf201, DUSP2, HSFX2, MYADML2, PRPSAP1, SPTBN2, ZDBF2, C20orf202, DUSP21, HSFY1, MYB, PRPSAP2, SPTBN4, ZDHHC1, C20orf203, DUSP22, HSFY2, MYBBP1A, PRR11, SPTBN5, ZDHHC11, C20orf24, DUSP23, HSH2D, MYBL1, PRR12, SPTLC1, ZDHHC11B, C20orf26, DUSP26, HSP90AA1, MYBL2, PRR13, SPTLC2, ZDHHC12, C20orf27, DUSP27, HSP90AB1, MYBPC1, PRR14, SPTLC3, ZDHHC13, C20orf62, DUSP28, HSP90B1, MYBPC2, PRR14L, SPTSSA, ZDHHC14, C20orf78, DUSP3, HSPA12A, MYBPC3, PRR15, SPTSSB, ZDHHC15, C20orf85, DUSP4, HSPA12B, MYBPH, PRR15L, SPTY2D1, ZDHHC16, C20orf96, DUSP5, HSPA13, MYBPHL, PRR16, SPZ1, ZDHHC17, C21orf140, DUSP6, HSPA14, MYC, PRR18, SQLE, ZDHHC18, C21orf2, DUSP7, HSPA1A, MYCBP, PRR19, SQRDL, ZDHHC19, C21orf33, DUSP8, HSPA1B, MYCBP2, PRR20A, SQSTM1, ZDHHC2, C21orf58, DUSP9, HSPA1L, MYCBPAP, PRR20B, SRA1, ZDHHC20, C21orf59, DUT, HSPA2, MYCL, PRR20C, SRBD1, ZDHHC21, C21orf62, DUXA, HSPA4, MYCN, PRR20D, SRC, ZDHHC22, C21orf91, DVL1, HSPA4L, MYCT1, PRR20E, SRCAP, ZDHHC23, C22orf15, DVL2, HSPA5, MYD88, PRR21, SRCIN1, ZDHHC24, C22orf23, DVL3, HSPA6, MYEF2, PRR22, SRCRB4D, ZDHHC3, C22orf24, DXO, HSPA8, MYEOV, PRR23A, SRD5A1, ZDHHC4, C22orf26, DYDC1, HSPA9, MYEOV2, PRR23B, SRD5A2, ZDHHC5, C22orf29, DYDC2, HSPB1, MYF5, PRR23C, SRD5A3, ZDHHC6, C22orf31, DYM, HSPB11, MYF6, PRR24, SREBF1, ZDHHC7, C22orf39, DYNAP, HSPB2, MYH1, PRR25, SREBF2, ZDHHC8, C22orf42, DYNC1H1, HSPB3, MYH10, PRR3, SREK1, ZDHHC9, C22orf43, DYNC1I1, HSPB6, MYH11, PRR30, SREK1IP1, ZEB1, C22orf46, DYNC1I2, HSPB7, MYH13, PRR4, SRF, ZEB2, C2CD2, DYNC1LI1, HSPB8, MYH14, PRR5, SRFBP1, ZER1, C2CD2L, DYNC1LI2, HSPB9, MYH15, PRR5-ARHGAP8, SRGAP1, ZFAND1, C2CD3, DYNC2H1, HSPBAP1, MYH2, PRR5L, SRGAP2, ZFAND2A, C2CD4A, |
| DYNC2LI1, HSPBP1, MYH3, PRR7, SRGAP2B, ZFAND2B, C2CD4B, DYNLL1, HSPD1, MYH4, PRR9, SRGAP2C, ZFAND3, C2CD4C, DYNLL2, HSPE1, MYH6, PRRC1, SRGAP3, ZFAND4, C2CD4D, DYNLRB1, HSPE1-MOB4, MYH7, PRRC2A, SRGN, ZFAND5, C2CD5, DYNLRB2, HSPG2, MYH7B, PRRC2B, SRI, ZFAND6, C2orf15, DYNLT1, HSPH1, MYH8, PRRC2C, SRL, ZFAT, C2orf16, DYNLT3, HTATIP2, MYH9, PRRG1, SRM, ZFC3H1, C2orf27A, DYRK1A, HTATSF1, MYL1, PRRG2, SRMS, ZFHX2, C2orf27B, DYRK1B, HTN1, MYL10, PRRG3, SRP14, ZFHX3, C2orf40, DYRK2, HTN3, MYL12A, PRRG4, SRP19, ZFHX4, C2orf42, DYRK3, HTR1A, MYL12B, PRRT1, SRP54, ZFP1, C2orf43, DYRK4, HTR1B, MYL2, PRRT2, SRP68, ZFP14, C2orf44, DYSF, HTR1D, MYL3, PRRT3, SRP72, ZFP2, C2orf47, DYTN, HTR1E, MYL4, PRRT4, SRP9, ZFP28, C2orf48, DYX1C1, HTR1F, MYL5, PRRX1, SRPK1, ZFP3, C2orf49, DZANK1, HTR2A, MYL6, PRRX2, SRPK2, ZFP30, C2orf50, DZIP1, HTR2B, MYL6B, PRSS1, SRPK3, ZFP36, C2orf53, DZIP1L, HTR2C, MYL7, PRSS12, SRPR, ZFP36L1, C2orf54, DZIP3, HTR3A, MYL9, PRSS16, SRPRB, ZFP36L2, C2orf57, E2F1, HTR3B, MYLIP, PRSS2, SRPX, ZFP37, C2orf61, E2F2, HTR3C, MYLK, PRSS21, SRPX2, ZFP41, C2orf62, E2F3, HTR3D, MYLK2, PRSS22, SRR, ZFP42, C2orf66, E2F4, HTR3E, MYLK3, PRSS23, SRRD, ZFP57, C2orf68, E2F5, HTR4, MYLK4, PRSS27, SRRM1, ZFP62, C2orf69, E2F6, HTR5A, MYLPF, PRSS3, SRRM2, ZFP64, C2orf70, E2F7, HTR6, MYNN, PRSS33, SRRM3, ZFP69, C2orf71, E2F8, HTR7, MYO10, PRSS35, SRRM4, ZFP69B, C2orf72, E4F1, HTRA1, MYO15A, PRSS36, SRRM5, ZFP82, C2orf73, EAF1, HTRA2, MYO16, PRSS37, SRRT, ZFP90, C2orf74, EAF2, HTRA3, MYO18A, PRSS38, SRSF1, ZFP91, C2orf76, EAPP, HTRA4, MYO18B, PRSS41, SRSF10, ZFP92, C2orf78, EARS2, HTT, MYO19, PRSS42, SRSF11, ZFPL1, C2orf80, EBAG9, HUNK, MYO1A, PRSS44, SRSF12, ZFPM1, C2orf81, EBF1, HUS1, MYO1B, PRSS45, SRSF2, ZFPM2, C2orf82, EBF2, HUS1B, MYO1C, PRSS46, SRSF3, ZFR, C2orf83, EBF3, HUWE1, MYO1D, PRSS48, SRSF4, ZFR2, C2orf88, EBF4, HVCN1, MYO1E, PRSS50, SRSF5, ZFX, C2orf91, EBI3, HYAL1, MYO1F, PRSS53, SRSF6, ZFY, C3, EBLN1, HYAL2, MYO1G, PRSS54, SRSF7, ZFYVE1, C3AR1, EBLN2, HYAL3, MYO1H, PRSS55, SRSF8, ZFYVE16, C3orf14, EBNA1BP2, HYAL4, MYO3A, PRSS56, SRSF9, ZFYVE19, C3orf17, EBP, HYDIN, MYO3B, PRSS57, SRXN1, ZFYVE20, C3orf18, EBPL, HYI, MYO5A, PRSS58, SRY, ZFYVE21, C3orf20, ECD, HYKK, MYO5B, PRSS8, SS18, ZFYVE26, C3orf22, ECE1, HYLS1, MYO5C, PRTFDC1, SS18L1, ZFYVE27, C3orf27, ECE2, HYOU1, MYO6, PRTG, SS18L2, ZFYVE28, C3orf30, ECEL1, HYPK, MYO7A, PRTN3, SSB, ZFYVE9, C3orf33, ECH1, IAH1, MYO7B, PRUNE, SSBP1, ZG16, C3orf35, ECHDC1, IAPP, MYO9A, PRUNE2, SSBP2, ZG16B, C3orf36, ECHDC2, IARS, MYO9B, PRX, SSBP3, ZGLP1, C3orf38, ECHDC3, IARS2, MYOC, PRY, SSBP4, ZGPAT, C3orf43, ECHS1, IBA57, MYOCD, PRY2, SSC5D, ZHX1, C3orf52, ECI1, IBSP, MYOD1, PSAP, SSFA2, ZHX1-C8ORF76, C3orf55, ECI2, IBTK, MYOF, PSAPL1, SSH1, ZHX2, C3orf56, ECM1, ICA1, MYOG, PSAT1, SSH2, ZHX3, C3orf58, ECM2, ICA1L, MYOM1, PSCA, SSH3, ZIC1, C3orf62, ECSCR, ICAM1, MYOM2, PSD, SSMEM1, ZIC2, C3orf67, ECSIT, ICAM2, MYOM3, PSD2, SSNA1, ZIC3, C3orf70, ECT2, ICAM3, MYOT, PSD3, SSPN, ZIC4, C3orf72, ECT2L, ICAM4, MYOZ1, PSD4, SSPO, ZIC5, C3orf79, EDA, |
| ICAM5, MYOZ2, PSEN1, SSR1, ZIK1, C3orf80, EDA2R, ICK, MYOZ3, PSEN2, SSR2, ZIM2, C3orf83, EDAR, ICMT, MYPN, PSENEN, SSR3, ZIM3, C3orf84, EDARADD, ICOS, MYPOP, PSG1, SSR4, ZKSCAN1, C4A, EDC3, ICOSLG, MYRF, PSG11, SSRP1, ZKSCAN2, C4B, EDC4, ICT1, MYRFL, PSG2, SSSCA1, ZKSCAN3, C4B_2, EDDM3A, ID1, MYRIP, PSG3, SST, ZKSCAN4, C4BPA, EDDM3B, ID2, MYSM1, PSG4, SSTR1, ZKSCAN5, C4BPB, EDEM1, ID3, MYT1, PSG5, SSTR2, ZKSCAN7, C4orf17, EDEM2, ID4, MYT1L, PSG6, SSTR3, ZKSCAN8, C4orf19, EDEM3, IDE, MYZAP, PSG7, SSTR4, ZMAT1, C4orf21, EDF1, IDH1, MZB1, PSG8, SSTR5, ZMAT2, C4orf22, EDIL3, IDH2, MZF1, PSG9, SSU72, ZMAT3, C4orf26, EDN1, IDH3A, MZT1, PSIP1, SSUH2, ZMAT4, C4orf27, EDN2, IDH3B, MZT2A, PSKH1, SSX1, ZMAT5, C4orf29, EDN3, IDH3G, MZT2B, PSKH2, SSX2, ZMIZ1, C4orf3, EDNRA, IDI1, N4BP1, PSMA1, SSX2B, ZMIZ2, C4orf32, EDNRB, IDI2, N4BP2, PSMA2, SSX2IP, ZMPSTE24, C4orf33, EEA1, IDNK, N4BP2L1, PSMA3, SSX3, ZMYM1, C4orf36, EED, IDO1, N4BP2L2, PSMA4, SSX4, ZMYM2, C4orf40, EEF1A1, IDO2, N4BP3, PSMA5, SSX4B, ZMYM3, C4orf45, EEF1A2, IDS, N6AMT1, PSMA6, SSX5, ZMYM4, C4orf46, EEF1B2, IDUA, N6AMT2, PSMA7, SSX7, ZMYM5, C4orf47, EEF1D, IER2, NAA10, PSMA8, ST13, ZMYM6, C4orf48, EEF1E1, IER3, NAA11, PSMB1, ST14, ZMYM6NB, C4orf50, EEF1G, IER3IP1, NAA15, PSMB10, ST18, ZMYND10, C4orf51, EEF2, IER5, NAA16, PSMB11, ST20, ZMYND11, C4orf6, EEF2K, IER5L, NAA20, PSMB2, ST20-MTHFS, ZMYND12, C5, EEFSEC, IFFO1, NAA25, PSMB3, ST3GAL1, ZMYND15, C5AR1, EEPD1, IFFO2, NAA30, PSMB4, ST3GAL2, ZMYND19, C5AR2, EFCAB1, IFI16, NAA35, PSMB5, ST3GAL3, ZMYND8, C5orf15, EFCAB11, IFI27, NAA38, PSMB6, ST3GAL4, ZNF10, C5orf20, EFCAB12, IFI27L1, NAA40, PSMB7, ST3GAL5, ZNF100, C5orf22, EFCAB13, IFI27L2, NAA50, PSMB8, ST3GAL6, ZNF101, C5orf24, EFCAB14, IFI30, NAA60, PSMB9, ST5, ZNF106, C5orf28, EFCAB2, IFI35, NAAA, PSMC1, ST6GAL1, ZNF107, C5orf30, EFCAB3, IFI44, NAALAD2, PSMC2, ST6GAL2, ZNF112, C5orf34, EFCAB4A, IFI44L, NAALADL1, PSMC3, ST6GALNAC1, ZNF114, C5orf38, EFCAB4B, IFI6, NAALADL2, PSMC3IP, ST6GALNAC2, ZNF117, C5orf42, EFCAB5, IFIH1, NAB1, PSMC4, ST6GALNAC3, ZNF12, C5orf45, EFCAB6, IFIT1, NAB2, PSMC5, ST6GALNAC4, ZNF121, C5orf46, EFCAB7, IFIT1B, NABP1, PSMC6, ST6GALNAC5, ZNF124, C5orf47, EFCAB8, IFIT2, NABP2, PSMD1, ST6GALNAC6, ZNF131, C5orf48, EFCAB9, IFIT3, NACA, PSMD10, ST7, ZNF132, C5orf49, EFCC1, IFIT5, NACA2, PSMD11, ST7L, ZNF133, C5orf50, EFEMP1, IFITM1, NACAD, PSMD12, ST8SIA1, ZNF134, C5orf51, EFEMP2, IFITM10, NACC1, PSMD13, ST8SIA2, ZNF135, C5orf52, EFHB, IFITM2, NACC2, PSMD14, ST8SIA3, ZNF136, C5orf55, EFHC1, IFITM3, NADK, PSMD2, ST8SIA4, ZNF138, C5orf58, EFHC2, IFITM5, NADK2, PSMD3, ST8SIA5, ZNF14, C5orf60, EFHD1, IFLTD1, NADSYN1, PSMD4, ST8SIA6, ZNF140, C5orf63, EFHD2, IFNA1, NAE1, PSMD5, STAB1, ZNF141, C5orf64, EFNA1, IFNA10, NAF1, PSMD6, STAB2, ZNF142, C6, EFNA2, IFNA13, NAGA, PSMD7, STAC, ZNF143, C6orf1, EFNA3, IFNA14, NAGK, PSMD8, STAC2, ZNF146, C6orf10, EFNA4, IFNA16, NAGLU, PSMD9, STAC3, ZNF148, C6orf106, EFNA5, IFNA17, NAGPA, PSME1, STAG1, ZNF154, C6orf118, EFNB1, IFNA2, NAGS, PSME2, STAG2, |
| ZNF155, C6orf132, EFNB2, IFNA21, NAIF1, PSME3, STAG3, ZNF157, C6orf136, EFNB3, IFNA4, NAIP, PSME4, STAM, ZNF16, C6orf141, EFR3A, IFNA5, NALCN, PSMF1, STAM2, ZNF160, C6orf15, EFR3B, IFNA6, NAMPT, PSMG1, STAMBP, ZNF165, C6orf163, EFS, IFNA7, NANOG, PSMG2, STAMBPL1, ZNF169, C6orf165, EFTUD1, IFNA8, NANOGNB, PSMG3, STAP1, ZNF17, C6orf183, EFTUD2, IFNAR1, NANOS1, PSMG4, STAP2, ZNF174, C6orf195, EGF, IFNAR2, NANOS2, PSORS1C1, STAR, ZNF175, C6orf201, EGFL6, IFNB1, NANOS3, PSORS1C2, STARD10, ZNF177, C6orf203, EGFL7, IFNE, NANP, PSPC1, STARD13, ZNF18, C6orf211, EGFL8, IFNG, NANS, PSPH, STARD3, ZNF180, C6orf222, EGFLAM, IFNGR1, NAP1L1, PSPN, STARD3NL, ZNF181, C6orf223, EGFR, IFNGR2, NAP1L2, PSRC1, STARD4, ZNF182, C6orf226, EGLN1, IFNK, NAP1L3, PSTK, STARD5, ZNF184, C6orf25, EGLN2, IFNL1, NAP1L4, PSTPIP1, STARD6, ZNF185, C6orf47, EGLN3, IFNL2, NAP1L5, PSTPIP2, STARD7, ZNF189, C6orf48, EGR1, IFNL3, NAPA, PTAFR, STARD8, ZNF19, C6orf52, EGR2, IFNL4, NAPB, PTAR1, STARD9, ZNF195, C6orf57, EGR3, IFNLR1, NAPEPLD, PTBP1, STAT1, ZNF197, C6orf58, EGR4, IFNW1, NAPG, PTBP2, STAT2, ZNF2, C6orf62, EHBP1, IFRD1, NAPRT1, PTBP3, STAT3, ZNF20, C6orf7, EHBP1L1, IFRD2, NAPSA, PTCD1, STAT4, ZNF200, C6orf89, EHD1, IFT122, NARF, PTCD2, STAT5A, ZNF202, C6orf99, EHD2, IFT140, NARFL, PTCD3, STAT5B, ZNF205, C7, EHD3, IFT172, NARG2, PTCH1, STAT6, ZNF207, C7orf10, EHD4, IFT20, NARR, PTCH2, STATH, ZNF208, C7orf25, EHF, IFT27, NARS, PTCHD1, STAU1, ZNF211, C7orf26, EHHADH, IFT43, NARS2, PTCHD2, STAU2, ZNF212, C7orf31, EHMT1, IFT46, NASP, PTCHD3, STBD1, ZNF213, C7orf33, EHMT2, IFT52, NAT1, PTCHD4, STC1, ZNF214, C7orf34, EI24, IFT57, NAT10, PTCRA, STC2, ZNF215, C7orf43, EID1, IFT74, NAT14, PTDSS1, STEAP1, ZNF217, C7orf49, EID2, IFT80, NAT16, PTDSS2, STEAP1B, ZNF219, C7orf50, EID2B, IFT81, NAT2, PTEN, STEAP2, ZNF22, C7orf55, EID3, IFT88, NAT6, PTER, STEAP3, ZNF221, C7orf55-LUC7L2, EIF1, IGBP1, NAT8, PTF1A, STEAP4, ZNF222, C7orf57, EIF1AD, IGDCC3, NAT8B, PTGDR, STH, ZNF223, C7orf60, EIF1AX, IGDCC4, NAT8L, PTGDR2, STIL, ZNF224, C7orf61, EIF1AY, IGF1, NAT9, PTGDS, STIM1, ZNF225, C7orf62, EIF1B, IGF1R, NAV1, PTGER1, STIM2, ZNF226, C7orf63, EIF2A, IGF2, NAV2, PTGER2, STIP1, ZNF227, C7orf65, EIF2AK1, IGF2BP1, NAV3, PTGER3, STK10, ZNF229, C7orf66, EIF2AK2, IGF2BP2, NBAS, PTGER4, STK11, ZNF23, C7orf69, EIF2AK3, IGF2BP3, NBEA, PTGES, STK11IP, ZNF230, C7orf71, EIF2AK4, IGF2R, NBEAL1, PTGES2, STK16, ZNF232, C7orf72, EIF2B1, IGFALS, NBEAL2, PTGES3, STK17A, ZNF233, C7orf73, EIF2B2, IGFBP1, NBL1, PTGES3L, STK17B, ZNF234, C7orf76, EIF2B3, IGFBP2, NBN, PTGES3L-AARSD1, STK19, ZNF235, C8A, EIF2B4, IGFBP3, NBPF14, PTGFR, STK24, ZNF236, C8B, EIF2B5, IGFBP4, NBPF3, PTGFRN, STK25, ZNF239, C8G, EIF2D, IGFBP5, NBPF4, PTGIR, STK3, ZNF24, C8orf22, EIF2S1, IGFBP6, NBPF6, PTGIS, STK31, ZNF248, C8orf31, EIF2S2, IGFBP7, NBPF7, PTGR1, STK32A, ZNF25, C8orf33, EIF2S3, IGFBPL1, NBR1, PTGR2, STK32B, ZNF250, C8orf34, EIF3A, IGFL1, NCALD, PTGS1, STK32C, ZNF251, C8orf37, EIF3B, IGFL2, NCAM1, PTGS2, STK33, ZNF253, C8orf4, EIF3C, IGFL3, NCAM2, PTH, STK35, ZNF254, C8orf44, EIF3CL, IGFL4, NCAN, PTH1R, STK36, ZNF256, |
| C8orf44-SGK3, EIF3D, IGFLR1, NCAPD2, PTH2, STK38, ZNF257, C8orf46, EIF3E, IGFN1, NCAPD3, PTH2R, STK38L, ZNF259, C8orf47, EIF3F, IGHMBP2, NCAPG, PTHLH, STK39, ZNF26, C8orf48, EIF3G, IGIP, NCAPG2, PTK2, STK4, ZNF260, C8orf58, EIF3H, IGJ, NCAPH, PTK2B, STK40, ZNF263, C8orf59, EIF3I, IGLL1, NCAPH2, PTK6, STMN1, ZNF264, C8orf74, EIF3J, IGLL5, NCBP1, PTK7, STMN2, ZNF266, C8orf76, EIF3K, IGLON5, NCBP2, PTMA, STMN3, ZNF267, C8orf82, EIF3L, IGSF1, NCCRP1, PTMS, STMN4, ZNF268, C8orf86, EIF3M, IGSF10, NCDN, PTN, STMND1, ZNF273, C8orf87, EIF4A1, IGSF11, NCEH1, PTOV1, STOM, ZNF274, C9, EIF4A2, IGSF21, NCF1, PTP4A1, STOML1, ZNF275, C9orf106, EIF4A3, IGSF22, NCF2, PTP4A2, STOML2, ZNF276, C9orf114, EIF4B, IGSF23, NCF4, PTP4A3, STOML3, ZNF277, C9orf116, EIF4E, IGSF3, NCK1, PTPDC1, STON1, ZNF28, C9orf117, EIF4E1B, IGSF5, NCK2, PTPLA, STON1-GTF2A1L, ZNF280A, C9orf129, EIF4E2, IGSF6, NCKAP1, PTPLAD1, STON2, ZNF280B, C9orf131, EIF4E3, IGSF8, NCKAP1L, PTPLAD2, STOX1, ZNF280C, C9orf135, EIF4EBP1, IGSF9, NCKAP5, PTPLB, STOX2, ZNF280D, C9orf139, EIF4EBP2, IGSF9B, NCKAP5L, PTPMT1, STPG1, ZNF281, C9orf142, EIF4EBP3, IHH, NCKIPSD, PTPN1, STPG2, ZNF282, C9orf152, EIF4ENIF1, IK, NCL, PTPN11, STRA13, ZNF283, C9orf153, EIF4G1, IKBIP, NCLN, PTPN12, STRA6, ZNF284, C9orf156, EIF4G2, IKBKAP, NCMAP, PTPN13, STRA8, ZNF285, C9orf16, EIF4G3, IKBKB, NCOA1, PTPN14, STRADA, ZNF286A, C9orf163, EIF4H, IKBKE, NCOA2, PTPN18, STRADB, ZNF286B, C9orf169, EIF5, IKBKG, NCOA3, PTPN2, STRAP, ZNF287, C9orf170, EIF5A, IKZF1, NCOA4, PTPN20A, STRBP, ZNF292, C9orf171, EIF5A2, IKZF2, NCOA5, PTPN20B, STRC, ZNF296, C9orf173, EIF5AL1, IKZF3, NCOA6, PTPN21, STRIP1, ZNF3, C9orf24, EIF5B, IKZF4, NCOA7, PTPN22, STRIP2, ZNF30, C9orf3, EIF6, IKZF5, NCOR1, PTPN23, STRN, ZNF300, C9orf37, ELAC1, INTERLEUKIN, IL10, NCOR2, PTPN3, STRN3, ZNF302, C9orf40, ELAC2, IL10RA, NCR1, PTPN4, STRN4, ZNF304, C9orf41, ELANE, IL10RB, NCR2, PTPN5, STS, ZNF311, C9orf43, ELAVL1, IL11, NCR3, PTPN6, STT3A, ZNF316, C9orf47, ELAVL2, IL11RA, NCR3LG1, PTPN7, STT3B, ZNF317, C9orf50, ELAVL3, IL12A, NCS1, PTPN9, STUB1, ZNF318, C9orf57, ELAVL4, IL12B, NCSTN, PTPRA, STX10, ZNF319, C9orf62, ELF1, IL12RB1, NDC1, PTPRB, STX11, ZNF32, C9orf64, ELF2, IL12RB2, NDC80, PTPRC, STX12, ZNF320, C9orf66, ELF3, IL13, NDE1, PTPRCAP, STX16, ZNF322, C9orf69, ELF4, IL13RA1, NDEL1, PTPRD, STX17, ZNF324, C9orf72, ELF5, IL13RA2, NDFIP1, PTPRE, STX18, ZNF324B, C9orf78, ELFN1, IL15, NDFIP2, PTPRF, STX19, ZNF326, C9orf84, ELFN2, IL15RA, NDN, PTPRG, STX1A, ZNF329, C9orf85, ELK1, IL16, NDNF, PTPRH, STX1B, ZNF330, C9orf89, ELK3, IL17A, NDNL2, PTPRJ, STX2, ZNF331, C9orf9, ELK4, IL17B, NDOR1, PTPRK, STX3, ZNF333, C9orf91, ELL, IL17C, NDP, PTPRM, STX4, ZNF334, C9orf92, ELL2, IL17D, NDRG1, PTPRN, STX5, ZNF335, C9orf96, ELL3, IL17F, NDRG2, PTPRN2, STX6, ZNF337, CA1, ELMO1, IL17RA, NDRG3, PTPRO, STX7, ZNF33A, CA10, ELMO2, IL17RB, NDRG4, PTPRQ, STX8, ZNF33B, CA11, ELMO3, IL17RC, NDST1, PTPRR, STXBP1, ZNF34, CA12, ELMOD1, IL17RD, NDST2, PTPRS, STXBP2, ZNF341, CA13, ELMOD2, IL17RE, NDST3, PTPRT, STXBP3, ZNF343, CA14, ELMOD3, IL17REL, NDST4, PTPRU, STXBP4, ZNF345, CA2, |
| ELMSAN1, IL18, NDUFA1, PTPRZ1, STXBP5, ZNF346, CA3, ELN, IL18BP, NDUFA10, PTRF, STXBP5L, ZNF347, CA4, ELOF1, IL18R1, NDUFA11, PTRH1, STXBP6, ZNF35, CA5A, ELOVL1, IL18RAP, NDUFA12, PTRH2, STYK1, ZNF350, CA5B, ELOVL2, IL19, NDUFA13, PTRHD1, STYX, ZNF354A, CA6, ELOVL3, IL1A, NDUFA2, PTS, STYXL1, ZNF354B, CA7, ELOVL4, IL1B, NDUFA3, PTTG1, SUB1, ZNF354C, CA8, ELOVL5, IL1F10, NDUFA4, PTTG1IP, SUCLA2, ZNF358, CA9, ELOVL6, IL1R1, NDUFA4L2, PTTG2, SUCLG1, ZNF362, CAAP1, ELOVL7, ILIR2, NDUFA5, PTX3, SUCLG2, ZNF365, CAB39, ELP2, IL1RAP, NDUFA6, PTX4, SUCNR1, ZNF366, CAB39L, ELP3, IL1RAPL1, NDUFA7, PUF60, SUCO, ZNF367, CABIN1, ELP4, IL1RAPL2, NDUFA8, PUM1, SUDS3, ZNF37A, CABLES1, ELP5, IL1RL1, NDUFA9, PUM2, SUFU, ZNF382, CABLES2, ELP6, IL1RL2, NDUFAB1, PURA, SUGP1, ZNF383, CABP1, ELSPBP1, IL1RN, NDUFAF1, PURB, SUGP2, ZNF384, CABP2, ELTD1, IL2, NDUFAF2, PURG, SUGT1, ZNF385A, CABP4, EMB, IL20, NDUFAF3, PUS1, SULF1, ZNF385B, CABP5, EMC1, IL20RA, NDUFAF4, PUS10, SULF2, ZNF385C, CABP7, EMC10, IL20RB, NDUFAF5, PUS3, SULT1A1, ZNF385D, CABS1, EMC2, IL21, NDUFAF6, PUS7, SULT1A2, ZNF391, CABYR, EMC3, IL21R, NDUFAF7, PUS7L, SULT1A3, ZNF394, CACFD1, EMC4, IL22, NDUFB1, PUSL1, SULT1A4, ZNF395, CACHD1, EMC6, IL22RA1, NDUFB10, PVALB, SULT1B1, ZNF396, CACNA1A, EMC7, IL22RA2, NDUFB11, PVR, SULT1C2, ZNF397, CACNA1B, EMC8, IL23A, NDUFB2, PVRIG, SULT1C3, ZNF398, CACNA1C, EMC9, IL23R, NDUFB3, PVRL1, SULT1C4, ZNF404, CACNA1D, EMCN, IL24, NDUFB4, PVRL2, SULT1E1, ZNF407, CACNA1E, EMD, IL25, NDUFB5, PVRL3, SULT2A1, ZNF408, CACNA1F, EME1, IL26, NDUFB6, PVRL4, SULT2B1, ZNF41, CACNA1G, EME2, IL27, NDUFB7, PWP1, SULT4A1, ZNF410, CACNA1H, EMG1, IL27RA, NDUFB8, PWP2, SULT6B1, ZNF414, CACNA1I, EMID1, IL2RA, NDUFB9, PWWP2A, SUMF1, ZNF415, CACNA1S, EMILIN1, IL2RB, NDUFC1, PWWP2B, SUMF2, ZNF416, CACNA2D1, EMILIN2, IL2RG, NDUFC2, PXDC1, SUMO1, ZNF417, CACNA2D2, EMILIN3, IL3, NDUFC2-KCTD14, PXDN, SUMO2, ZNF418, CACNA2D3, EML1, IL31, NDUFS1, PXDNL, SUMO3, ZNF419, CACNA2D4, EML2, IL31RA, NDUFS2, PXK, SUMO4, ZNF420, CACNB1, EML3, IL32, NDUFS3, PXMP2, SLTN1, ZNF423, CACNB2, EML4, IL33, NDUFS4, PXMP4, SUN2, ZNF425, CACNB3, EML5, IL34, NDUFS5, PXN, SUN3, ZNF426, CACNB4, EML6, IL36A, NDUFS6, PXT1, SUNS, ZNF428, CACNG1, EMP1, IL36B, NDUFS7, PYCARD, SUOX, ZNF429, CACNG2, EMP2, IL36G, NDUFS8, PYCR1, SUPT16H, ZNF43, CACNG3, EMP3, IL36RN, NDUFV1, PYCR2, SUPT20H, ZNF430, CACNG4, EMR1, IL37, NDUFV2, PYCRL, SUPT3H, ZNF431, CACNG5, EMR2, IL3RA, NDUFV3, PYDC1, SUPT4H1, ZNF432, CACNG6, EMR3, IL4, NEB, PYDC2, SUPT5H, ZNF433, CACNG7, EMX1, IL4I1, NEBL, PYGB, SUPT6H, ZNF436, CACNG8, EMX2, IL4R, NECAB1, PYGL, SUPT7L, ZNF438, CACTIN, EN1, IL5, NECAB2, PYGM, SUPV3L1, ZNF439, CACUL1, EN2, IL5RA, NECAB3, PYGO1, SURF1, ZNF44, CACYBP, ENAH, IL6, NECAP1, PYGO2, SURF2, ZNF440, CAD, ENAM, IL6R, NECAP2, PYHIN1, SURF4, ZNF441, CADM1, ENC1, IL6ST, NEDD1, PYROXD1, SURF6, ZNF442, CADM2, ENDOD1, IL7, NEDD4, PYROXD2, SUSD1, ZNF443, CADM3, ENDOG, IL7R, |
| NEDD4L, PYURF, SUSD2, ZNF444, CADM4, ENDOU, IL8, NEDD8, PYY, SUSD3, ZNF445, CADPS, ENDOV, IL9, NEDD8-MDP1, PZP, SUSD4, ZNF446, CADPS2, ENG, IL9R, NEDD9, QARS, SUSD5, ZNF449, CAGE1, ENGASE, ILDR1, NEFH, QDPR, SUV39H1, ZNF45, CALB1, ENHO, ILDR2, NEFL, QKI, SUV39H2, ZNF451, CALB2, ENKD1, ILF2, NEFM, QPCT, SUV420H1, ZNF454, CALCA, ENKUR, ILF3, NEGR1, QPCTL, SUV420H2, ZNF460, CALCB, ENO1, ILK, NEIL1, QPRT, SUZ12, ZNF461, CALCOCO1, ENO2, ILKAP, NEIL2, QRFP, SV2A, ZNF462, CALCOCO2, ENO3, ILVBL, NEIL3, QRFPR, SV2B, ZNF467, CALCR, ENO4, IMMP1L, NEK1, QRICH1, SV2C, ZNF468, CALCRL, ENOPH1, IMMP2L, NEK10, QRICH2, SVEP1, ZNF469, CALD1, ENOSF1, IMMT, NEK11, QRSL1, SVIL, ZNF470, CALHM1, ENOX1, IMP3, NEK2, QSER1, SVIP, ZNF471, CALHM2, ENOX2, IMP4, NEK3, QSOX1, SVOP, ZNF473, CALHM3, ENPEP, IMPA1, NEK4, QSOX2, SVOPL, ZNF474, CALM1, ENPP1, IMPA2, NEK5, QTRT1, SWAP70, ZNF479, CALM2, ENPP2, IMPACT, NEK6, QTRTD1, SWI5, ZNF48, CALM3, ENPP3, IMPAD1, NEK7, R3HCC1, SWSAP1, ZNF480, CALML3, ENPP4, IMPDH1, NEK8, R3HCC1L, SWT1, ZNF483, CALML4, ENPP5, IMPDH2, NEK9, R3HDM1, SYAP1, ZNF484, CALML5, ENPP6, IMPG1, NELFA, R3HDM2, SYBU, ZNF485, CALML6, ENPP7, IMPG2, NELFB, R3HDM4, SYCE1, ZNF486, CALN1, ENSA, INA, NELFCD, R3HDML, SYCE1L, ZNF488, CALR, ENTHD1, INADL, NELFE, RAB10, SYCE2, ZNF490, CALR3, ENTHD2, INCA1, NELL1, RAB11A, SYCE3, ZNF491, CALU, ENTPD1, INCENP, NELL2, RAB11B, SYCN, ZNF492, CALY, ENTPD2, INF2, NEMF, RAB11FIP1, SYCP1, ZNF493, CAMK1, ENTPD3, ING1, NENF, RAB11FIP2, SYCP2, ZNF496, CAMK1D, ENTPD4, ING2, NEO1, RAB11FIP3, SYCP2L, ZNF497, CAMK1G, ENTPD5, ING3, NES, RAB11FIP4, SYCP3, ZNF500, CAMK2A, ENTPD6, ING4, NET1, RAB11FIP5, SYDE1, ZNF501, CAMK2B, ENTPD7, ING5, NETO1, RAB12, SYDE2, ZNF502, CAMK2D, ENTPD8, INHA, NETO2, RAB13, SYF2, ZNF503, CAMK2G, ENY2, INHBA, NEU1, RAB14, SYK, ZNF506, CAMK2N1, EOGT, INHBB, NEU2, RAB15, SYMPK, ZNF507, CAMK2N2, EOMES, INHBC, NEU3, RAB17, SYN1, ZNF510, CAMK4, EP300, INHBE, NEU4, RAB18, SYN2, ZNF511, CAMKK1, EP400, INIP, NEURL1, RAB19, SYN3, ZNF512, CAMKK2, EPAS1, INMT, NEURL1B, RAB1A, SYNC, ZNF512B, CAMKMT, EPB41, INO80, NEURL2, RAB1B, SYNCRIP, ZNF513, CAMKV, EPB41L1, INO80B, NEURL3, RAB20, SYNDIG1, ZNF514, CAMLG, EPB41L2, INO80C, NEURL4, RAB21, SYNDIG1L, ZNF516, CAMP, EPB41L3, INO80D, NEUROD1, RAB22A, SYNE1, ZNF517, CAMSAP1, EPB41L4A, INO80E, NEUROD2, RAB23, SYNE2, ZNF518A, CAMSAP2, EPB41L4B, INPP1, NEUROD4, RAB24, SYNE3, ZNF518B, CAMSAP3, EPB41L5, INPP4A, NEUROD6, RAB25, SYNE4, ZNF519, CAMTA1, EPB42, INPP4B, NEUROG1, RAB26, SYNGAP1, ZNF521, CAMTA2, EPC1, INPP5A, NEUROG2, RAB27A, SYNGR1, ZNF524, CAND1, EPC2, INPP5B, NEUROG3, RAB27B, SYNGR2, ZNF526, CAND2, EPCAM, INPP5D, NEXN, RAB28, SYNGR3, ZNF527, CANT1, EPDR1, INPP5E, NF1, RAB2A, SYNGR4, ZNF528, CANX, EPG5, INPP5F, NF2, RAB2B, SYNJ1, ZNF529, CAP1, EPGN, INPP5J, NFAM1, RAB30, SYNJ2, ZNF530, CAP2, EPHA1, INPP5K, NFASC, RAB31, SYNJ2BP, ZNF532, CAPG, EPHA10, INPPL1, NFAT5, RAB32, SYNJ2BP-COX16, ZNF534, CAPN1, EPHA2, |
| INS, NFATC1, RAB33A, SYNM, ZNF536, CAPN10, EPHA3, INSC, NFATC2, RAB33B, SYNPO, ZNF540, CAPN11, EPHA4, INSIG1, NFATC2IP, RAB34, SYNPO2, ZNF541, CAPN12, EPHA5, INSIG2, NFATC3, RAB35, SYNPO2L, ZNF543, CAPN13, EPHA6, INS-IGF2, NFATC4, RAB36, SYNPR, ZNF544, CAPN14, EPHA7, INSL3, NFE2, RAB37, SYNRG, ZNF546, CAPN15, EPHA8, INSL4, NFE2L1, RAB38, SYP, ZNF547, CAPN2, EPHB1, INSL5, NFE2L2, RAB39A, SYPL1, ZNF548, CAPN3, EPHB2, INSL6, NFE2L3, RAB39B, SYPL2, ZNF549, CAPN5, EPHB3, INSM1, NFIA, RAB3A, SYS1, ZNF550, CAPN6, EPHB4, INSM2, NFIB, RAB3B, SYT1, ZNF551, CAPN7, EPHB6, INSR, NFIC, RAB3C, SYT10, ZNF552, CAPN8, EPHX1, INSRR, NFIL3, RAB3D, SYT11, ZNF554, CAPN9, EPHX2, INTS1, NFIX, RAB3GAP1, SYT12, ZNF555, CAPNS1, EPHX3, INTS10, NFKB1, RAB3GAP2, SYT13, ZNF556, CAPNS2, EPHX4, INTS12, NFKB2, RAB3IL1, SYT14, ZNF557, CAPRIN1, EPM2A, INTS2, NFKBIA, RAB3IP, SYT15, ZNF558, CAPRIN2, EPM2AIP1, INTS3, NFKBIB, RAB40A, SYT16, ZNF559, CAPS, EPN1, INTS4, NFKBID, RAB40AL, SYT17, ZNF559-ZNF177, CAPS2, EPN2, INTS5, NFKBIE, RAB40B, SYT2, ZNF560, CAPSL, EPN3, INTS6, NFKBIL1, RAB40C, SYT3, ZNF561, CAPZA1, EPO, INTS7, NFKBIZ, RAB41, SYT4, ZNF562, CAPZA2, EPOR, INTS8, NFRKB, RAB42, SYT5, ZNF563, CAPZA3, EPPIN, INTS9, NFS1, RAB43, SYT6, ZNF564, CAPZB, EPPIN-WFDC6, INTU, NFU1, RAB44, SYT7, ZNF565, CARD10, EPPK1, INVS, NFX1, RAB4A, SYT8, ZNF566, CARD11, EPRS, IP6K1, NFXL1, RAB4B, SYT9, ZNF567, CARD14, EPS15, IP6K2, NFYA, RAB5A, SYTL1, ZNF568, CARD16, EPS15L1, IP6K3, NFYB, RAB5B, SYTL2, ZNF569, CARD17, EPS8, IPCEF1, NFYC, RAB5C, SYTL3, ZNF57, CARD18, EPS8L1, IPMK, NGB, RAB6A, SYTL4, ZNF570, CARD6, EPS8L2, IPO11, NGDN, RAB6B, SYTL5, ZNF571, CARD8, EPS8L3, IPO13, NGEF, RAB6C, SYVN1, ZNF572, CARD9, EPSTI1, IPO4, NGF, RAB7A, SZT2, ZNF573, CARF, EPT1, IPO5, NGFR, RAB7L1, T, ZNF574, CARHSP1, EPX, IPO7, NGFRAP1, RAB8A, TAAR1, ZNF575, CARKD, EPYC, IPO8, NGLY1, RAB8B, TAAR2, ZNF576, CARM1, EQTN, IPO9, NGRN, RAB9A, TAAR5, ZNF577, CARNS1, ERAL1, IPP, NHEJ1, RAB9B, TAAR6, ZNF578, CARS, ERAP1, IPPK, NHLH1, RABAC1, TAAR8, ZNF579, CARS2, ERAP2, IQCA1, NHLH2, RABEP1, TAAR9, ZNF580, CARTPT, ERAS, IQCB1, NHLRC1, RABEP2, TAB1, ZNF581, CASC1, ERBB2, IQCC, NHLRC2, RABEPK, TAB2, ZNF582, CASC10, ERBB2IP, IQCD, NHLRC3, RABGAP1, TAB3, ZNF583, CASC3, ERBB3, IQCE, NHLRC4, RABGAP1L, TAC1, ZNF584, CASC4, ERBB4, IQCF1, NHP2, RABGEF1, TAC3, ZNF585A, CASC5, ERC1, IQCF2, NHP2L1, RABGGTA, TAC4, ZNF585B, CASD1, ERC2, IQCF3, NHS, RABGGTB, TACC1, ZNF586, CASK, ERCC1, IQCF5, NHSL1, RABIF, TACC2, ZNF587, CASKIN1, ERCC2, IQCF6, NHSL2, RABL2A, TACC3, ZNF587B, CASKIN2, ERCC3, IQCG, NICN1, RABL2B, TACO1, ZNF589, CASP1, ERCC4, IQCH, NID1, RABL3, TACR1, ZNF592, CASP10, ERCC5, IQCJ, NID2, RABL5, TACR2, ZNF593, CASP12, ERCC6, IQCJ-SCHIP1, NIF3L1, RABL6, TACR3, ZNF594, CASP14, ERCC6L, IQCK, NIFK, RAC1, TACSTD2, ZNF595, CASP16, ERCC6L2, IQGAP1, NIM1, RAC2, TADA1, ZNF596, CASP2, ERCC6-PGBD3, IQGAP2, NIN, RAC3, TADA2A, ZNF597, CASP3, ERCC8, IQGAP3, NINJ1, RACGAP1, TADA2B, ZNF598, CASP4, EREG, IQSEC1, NINJ2, RAD1, TADA3, ZNF599, |
| CASP5, ERF, IQSEC2, NINL, RAD17, TAF1, ZNF600, CASP6, ERG, IQSEC3, NIP7, RAD18, TAF10, ZNF605, CASP7, ERGIC1, IQUB, NIPA1, RAD21, TAF11, ZNF606, CASP8, ERGIC2, IRAK1, NIPA2, RAD21L1, TAF12, ZNF607, CASP8AP2, ERGIC3, IRAK1BP1, NIPAL1, RAD23A, TAF13, ZNF608, CASP9, ERH, IRAK2, NIPAL2, RAD23B, TAF15, ZNF609, CASQ1, ERI1, IRAK3, NIPAL3, RAD50, TAF1A, ZNF610, CASQ2, ERI2, IRAK4, NIPAL4, RAD51, TAF1B, ZNF611, CASR, ERI3, IREB2, NIPBL, RAD51AP1, TAF1C, ZNF613, CASS4, ERICH1, IRF1, NIPSNAP1, RAD51AP2, TAF1D, ZNF614, CAST, ERICH2, IRF2, NIPSNAP3A, RAD51B, TAF1L, ZNF615, CASZ1, ERLEC1, IRF2BP1, NIPSNAP3B, RAD51C, TAF2, ZNF616, CAT, ERLIN1, IRF2BP2, NISCH, RAD51D, TAF3, ZNF618, CATSPER1, ERLIN2, IRF2BPL, NIT1, RAD52, TAF4, ZNF619, CATSPER2, ERMAP, IRF3, NIT2, RAD54B, TAF4B, ZNF620, CATSPER3, ERMARD, IRF4, NKAIN1, RAD54L, TAF5, ZNF621, CATSPER4, ERMN, IRF5, NKAIN2, RAD54L2, TAF5L, ZNF622, CATSPERB, ERMP1, IRF6, NKAIN3, RAD9A, TAF6, ZNF623, CATSPERD, ERN1, IRF7, NKAIN4, RAD9B, TAF6L, ZNF624, CATSPERG, ERN2, IRF8, NKAP, RADIL, TAF7, ZNF625, CAV1, ERO1L, IRF9, NKAPL, RAE1, TAF7L, ZNF626, CAV2, ERO1LB, IRG1, NKD1, RAET1E, TAF8, ZNF627, CAV3, ERP27, IRGC, NKD2, RAET1G, TAF9, ZNF628, CBFA2T2, ERP29, IRGM, NKG7, RAET1L, TAF9B, ZNF629, CBFA2T3, ERP44, IRGQ, NKIRAS1, RAF1, TAGAP, ZNF630, CBFB, ERRFI1, IRS1, NKIRAS2, RAG1, TAGLN, ZNF638, CBL, ERVFRD-1, IRS2, NKPD1, RAG2, TAGLN2, ZNF639, CBLB, ERVMER34-1, IRS4, NKRF, RAI1, TAGLN3, ZNF641, CBLC, ERVV-1, IRX1, NKTR, RAT14, TAL1, ZNF644, CBLL1, ERVV-2, IRX2, NKX1-2, RAI2, TAL2, ZNF645, CBLN1, ESAM, IRX3, NKX2-1, RALA, TALDO1, ZNF646, CBLN2, ESCO1, IRX4, NKX2-2, RALB, TAMM41, ZNF648, CBLN3, ESCO2, IRX5, NKX2-3, RALBP1, TANC1, ZNF649, CBLN4, ESD, IRX6, NKX2-4, RALGAPA1, TANC2, ZNF652, CBR1, ESF1, ISCA1, NKX2-5, RALGAPA2, TANGO2, ZNF653, CBR3, ESM1, ISCA2, NKX2-6, RALGAPB, TANGO6, ZNF654, CBR4, ESPL1, ISCU, NKX2-8, RALGDS, TANK, ZNF655, CBS, ESPN, ISG15, NKX3- **1,** RALGPS1, TAOK1, ZNF658, CBWD1, ESPNL, ISG20, NKX3-2, RALGPS2, TAOK2, ZNF660, CBWD2, ESR1, ISG20L2, NKX6-1, RALY, TAOK3, ZNF662, CBWD3, ESR2, ISL1, NKX6-2, RALYL, TAP1, ZNF664, CBWD5, ESRP1, ISL2, NKX6-3, RAMP1, TAP2, ZNF664-FAM101A, CBWD6, ESRP2, ISLR, NLE1, RAMP2, TAPBP, ZNF665, CBWD7, ESRRA, ISLR2, NLGN1, RAMP3, TAPBPL, ZNF667, CBX1, ESRRB, ISM1, NLGN2, RAN, TAPT1, ZNF668, CBX2, ESRRG, ISM2, NLGN3, RANBP1, TARBP1, ZNF669, CBX3, ESX1, ISOC1, NLGN4X, RANBP10, TARBP2, ZNF670, CBX4, ESYT1, ISOC2, NLGN4Y, RANBP17, TARDBP, ZNF671, CBX5, ESYT2, ISPD, NLK, RANBP2, TARM1, ZNF672, CBX6, ESYT3, IST1, NLN, RANBP3, TARP, ZNF674, CBX7, ETAA1, ISX, NLRC3, RANBP3L, TARS, ZNF675, CBX8, ETF1, ISY1, NLRC4, RANBP6, TARS2, ZNF676, CBY1, ETFA, ISY1-RAB43, NLRC5, RANBP9, TARSL2, ZNF677, CBY3, ETFB, ISYNA1, NLRP1, RANGAP1, TAS1R1, ZNF678, CC2D1A, ETFDH, ITCH, NLRP10, RANGRF, TAS1R2, ZNF679, CC2D1B, ETHE1, ITFGl, NLRP11, RAP1A, TAS1R3, ZNF680, CC2D2A, ETNK1, ITFG2, NLRP12, RAP1B, TAS2R1, ZNF681, CC2D2B, ETNK2, ITFG3, NLRP13, RAP1GAP, TAS2R10, ZNF682, CCAR1, |
| ETNPPL, ITGA1, NLRP14, RAP1GAP2, TAS2R13, ZNF683, CCAR2, ETS1, ITGA10, NLRP2, RAP1GDS1, TAS2R14, ZNF684, CCBE1, ETS2, ITGA11, NLRP3, RAP2A, TAS2R16, ZNF687, CCBL1, ETV1, ITGA2, NLRP4, RAP2B, TAS2R19, ZNF688, CCBL2, ETV2, ITGA2B, NLRP5, RAP2C, TAS2R20, ZNF689, CCDC101, ETV3, ITGA3, NLRP6, RAPGEF1, TAS2R3, ZNF69, CCDC102A, ETV3L, ITGA4, NLRP7, RAPGEF2, TAS2R30, ZNF691, CCDC102B, ETV4, ITGA5, NLRP8, RAPGEF3, TAS2R31, ZNF692, CCDC103, ETV5, ITGA6, NLRP9, RAPGEF4, TAS2R38, ZNF695, CCDC104, ETV6, ITGA7, NLRX1, RAPGEF5, TAS2R39, ZNF696, CCDC105, ETV7, ITGA8, NMB, RAPGEF6, TAS2R4, ZNF697, CCDC106, EVA1A, ITGA9, NMBR, RAPGEFL1, TAS2R40, ZNF699, CCDC107, EVA1B, ITGAD, NMD3, RAPH1, TAS2R41, ZNF7, CCDC108, EVA1C, ITGAE, NME1, RAPSN, TAS2R42, ZNF70, CCDC109B, EVC, ITGAL, NME1-NME2, RARA, TAS2R43, ZNF700, CCDC11, EVC2, ITGAM, NME2, RARB, TAS2R46, ZNF701, CCDC110, EVI2A, ITGAV, NME3, RARG, TAS2R5, ZNF703, CCDC112, EVI2B, ITGAX, NME4, RARRES1, TAS2R50, ZNF704, CCDC113, EVI5, ITGB1, NME5, RARRES2, TAS2R60, ZNF705A, CCDC114, EVI5L, ITGB1BP1, NME6, RARRES3, TAS2R7, ZNF705B, CCDC115, EVL, ITGB1BP2, NME7, RARS, TAS2R8, ZNF705D, CCDC116, EVPL, ITGB2, NME8, RARS2, TAS2R9, ZNF705E, CCDC117, EVPLL, ITGB3, NME9, RASA1, TASP1, ZNF705G, CCDC12, EVX1, ITGB3BP, NMI, RASA2, TAT, ZNF706, CCDC120, EVX2, ITGB4, NMNAT1, RASA3, TATDN1, ZNF707, CCDC121, EWSR1, ITGB5, NMNAT2, RASA4, TATDN2, ZNF708, CCDC122, EXD1, ITGB6, NMNAT3, RASA4B, TATDN3, ZNF709, CCDC124, EXD2, ITGB7, NMRAL1, RASAL1, TAX1BP1, ZNF71, CCDC125, EXD3, ITGB8, NMRK1, RASAL2, TAX1BP3, ZNF710, CCDC126, EXO1, ITGBL1, NMRK2, RASAL3, TAZ, ZNF711, CCDC127, EXO5, ITIH1, NMS, RASD1, TBATA, ZNF713, CCDC129, EXOC1, ITIH2, NMT1, RASD2, TBC1D1, ZNF714, CCDC13, EXOC2, ITIH3, NMT2, RASEF, TBC1D10A, ZNF716, CCDC130, EXOC3, ITIH4, NMU, RASGEF1A, TBC1D10B, ZNF717, CCDC132, EXOC3L1, ITIH5, NMUR1, RASGEF1B, TBC1D10C, ZNF718, CCDC134, EXOC3L2, ITIH6, NMUR2, RASGEF1C, TBC1D12, ZNF720, CCDC135, EXOC3L4, ITK, NNAT, RASGRF1, TBC1D13, ZNF721, CCDC136, EXOC4, ITLN1, NNMT, RASGRF2, TBC1D14, ZNF726, CCDC137, EXOC5, ITLN2, NNT, RASGRP1, TBC1D15, ZNF727, CCDC138, EXOC6, ITM2A, NOA1, RASGRP2, TBC1D16, ZNF728, CCDC14, EXOC6B, ITM2B, NOB1, RASGRP3, TBC1D17, ZNF729, CCDC140, EXOC7, ITM2C, NOBOX, RASGRP4, TBC1D19, ZNF730, CCDC141, EXOC8, ITPA, NOC2L, RASIP1, TBC1D2, ZNF732, CCDC142, EXOG, ITPK1, NOC3L, RASL10A, TBC1D20, ZNF735, CCDC144A, EXOSC1, ITPKA, NOC4L, RASL10B, TBC1D21, ZNF736, CCDC144NL, EXOSC10, ITPKB, NOD1, RASL11A, TBC1D22A, ZNF737, CCDC146, EXOSC2, ITPKC, NOD2, RASL11B, TBC1D22B, ZNF74, CCDC147, EXOSC3, ITPR1, NODAL, RASL12, TBC1D23, ZNF740, CCDC148, EXOSC4, ITPR2, NOG, RASSF1, TBC1D24, ZNF746, CCDC149, EXOSC5, ITPR3, NOL10, RASSF10, TBC1D25, ZNF747, CCDC15, EXOSC6, ITPRIP, NOL11, RASSF2, TBC1D26, ZNF749, CCDC150, EXOSC7, ITPRIPL1, NOL12, RASSF3, TBC1D27, ZNF750, CCDC151, EXOSC8, ITPRIPL2, NOL3, RASSF4, TBC1D28, ZNF75A, CCDC152, EXOSC9, ITSN1, NOL4, RASSF5, TBC1D29, ZNF75D, |
| CCDC153, EXPH5, ITSN2, NOL6, RASSF6, TBC1D2B, ZNF76, CCDC154, EXT1, IVD, NOL7, RASSF7, TBC1D3, ZNF761, CCDC155, EXT2, IVL, NOL8, RASSF8, TBC1D30, ZNF763, CCDC157, EXTL1, IVNS1ABP, NOL9, RASSF9, TBC1D31, ZNF764, CCDC158, EXTL2, IWS1, NOLC1, RAVER1, TBC1D32, ZNF765, CCDC159, EXTL3, IYD, NOM1, RAVER2, TBC1D3B, ZNF766, CCDC160, EYA1, IZUMO1, NOMO1, RAX, TBC1D3C, ZNF768, CCDC166, EYA2, IZUMO2, NOMO2, RAX2, TBC1D3F, ZNF77, CCDC167, EYA3, IZUMO3, NOMO3, RB1, TBC1D3G, ZNF770, CCDC168, EYA4, IZUMO4, NONO, RB1CC1, TBC1D3H, ZNF771, CCDC169, EYS, JADE1, NOP10, RBAK, TBC1D4, ZNF772, CCDC169-SOHLH2, EZH1, JADE2, NOP14, RBAK-RBAKDN, TBC1D5, ZNF773, CCDC17, EZH2, JADE3, NOP16, RBBP4, TBC1D7, ZNF774, CCDC170, EZR, JAG1, NOP2, RBBP5, TBC1D8, ZNF775, CCDC171, F10, JAG2, NOP56, RBBP6, TBC1D8B, ZNF776, CCDC172, F11, JAGN1, NOP58, RBBP7, TBC1D9, ZNF777, CCDC173, F11R, JAK1, NOP9, RBBP8, TBC1D9B, ZNF778, CCDC174, F12, JAK2, NOS1, RBBP8NL, TBCA, ZNF780A, CCDC175, F13A1, JAK3, NOS1AP, RBBP9, TBCB, ZNF780B, CCDC176, F13B, JAKMIP1, NOS2, RBCK1, TBCC, ZNF781, CCDC177, F2, JAKMIP2, NOS3, RBFA, TBCCD1, ZNF782, CCDC178, F2R, JAKMIP3, NOSIP, RBFOX1, TBCD, ZNF783, CCDC179, F2RL1, JAM2, NOSTRIN, RBFOX2, TBCE, ZNF784, CCDC18, F2RL2, JAM3, NOTCH1, RBFOX3, TBCEL, ZNF785, CCDC180, F2RL3, JARID2, NOTCH2, RBKS, TBCK, ZNF786, CCDC181, F3, JAZF1, NOTCH2NL, RBL1, TBK1, ZNF787, CCDC19, F5, JDP2, NOTCH3, RBL2, TBKBP1, ZNF789, CCDC22, F7, JHDM1D, NOTCH4, RBM10, TBL1X, ZNF79, CCDC23, F8, JKAMP, NOTO, RBM11, TBL1XR1, ZNF790, CCDC24, F8A1, JMJD1C, NOTUM, RBM12, TBL1Y, ZNF791, CCDC25, F8A2, JMJD4, NOV, RBM12B, TBL2, ZNF792, CCDC27, F8A3, JMJD6, NOVA1, RBM14, TBL3, ZNF793, CCDC28A, F9, JMJD7, NOVA2, RBM14-RBM4, TBP, ZNF799, CCDC28B, FA2H, JMJD7-PLA2G4B, NOX1, RBM15, TBPL1, ZNF8, CCDC3, FAAH, JMJD8, NOX3, RBM15B, TBPL2, ZNF80, CCDC30, FAAH2, JMY, NOX4, RBM17, TBR1, ZNF800, CCDC33, FABP1, JOSD1, NOX5, RBM18, TBRG1, ZNF804A, CCDC34, FABP12, JOSD2, NOXA1, RBM19, TBRG4, ZNF804B, CCDC36, FABP2, JPH1, NOXO1, RBM20, TBX1, ZNF805, CCDC37, FABP3, JPH2, NOXRED1, RBM22, TBX10, ZNF806, CCDC38, FABP4, JPH3, NPAP1, RBM23, TBX15, ZNF808, CCDC39, FABP5, JPH4, NPAS1, RBM24, TBX18, ZNF81, CCDC40, FABP6, JRK, NPAS2, RBM25, TBX19, ZNF812, CCDC41, FABP7, JRKL, NPAS3, RBM26, TBX2, ZNF813, CCDC42, FABP9, JSRP1, NPAS4, RBM27, TBX20, ZNF814, CCDC42B, FADD, JTB, NPAT, RBM28, TBX21, ZNF816, CCDC43, FADS1, JUN, NPB, RBM3, TBX22, ZNF816-ZNF321P, CCDC47, FADS2, JUNB, NPBWR1, RBM33, TBX3, ZNF821, CCDC50, FADS3, JUND, NPBWR2, RBM34, TBX4, ZNF823, CCDC51, FADS6, JUP, NPC1, RBM38, TBX5, ZNF827, CCDC53, FAF1, KAAG1, NPC1L1, RBM39, TBX6, ZNF829, CCDC54, FAF2, KAL1, NPC2, RBM4, TBXA2R, ZNF83, CCDC57, FAH, KALRN, NPDC1, RBM41, TBXAS1, ZNF830, CCDC58, FAHD1, KANK1, NPEPL1, RBM42, TC2N, ZNF831, CCDC59, FAHD2A, KANK2, NPEPPS, RBM43, TCAIM, ZNF835, CCDC6, FAHD2B, KANK3, NPFF, RBM46, TCAP, ZNF836, CCDC60, FAIM, KANK4, NPFFR1, RBM47, TCEA1, ZNF837, CCDC61, FAIM2, |
| KANSL1, NPFFR2, RBM48, TCEA2, ZNF839, CCDC62, FAIM3, KANSL1L, NPHP1, RBM4B, TCEA3, ZNF84, CCDC63, FAM101A, KANSL2, NPHP3, RBM5, TCEAL1, ZNF841, CCDC64, FAM101B, KANSL3, NPHP4, RBM6, TCEAL2, ZNF843, CCDC64B, FAM102A, KARS, NPHS1, RBM7, TCEAL3, ZNF844, CCDC65, FAM102B, KAT2A, NPHS2, RBM8A, TCEAL4, ZNF845, CCDC66, FAM103A1, KAT2B, NPIPA1, RBMS1, TCEAL5, ZNF846, CCDC67, FAM104A, KAT5, NPIPA2, RBMS2, TCEAL6, ZNF85, CCDC68, FAM104B, KAT6A, NPIPA3, RBMS3, TCEAL7, ZNF850, CCDC69, FAM105A, KAT6B, NPIPA5, RBMX, TCEAL8, ZNF852, CCDC7, FAM105B, KAT7, NPIPA7, RBMX2, TCEANC, ZNF853, CCDC70, FAM107A, KAT8, NPIPA8, RBMXL1, TCEANC2, ZNF860, CCDC71, FAM107B, KATNA1, NPIPB11, RBMXL2, TCEB1, ZNF862, CCDC71L, FAM109A, KATNAL1, NPIPB15, RBMXL3, TCEB2, ZNF865, CCDC73, FAM109B, KATNAL2, NPIPB3, RBMY1A1, TCEB3, ZNF878, CCDC74A, FAM110A, KATNB1, NPIPB4, RBMY1B, TCEB3B, ZNF879, CCDC74B, FAM110B, KATNBL1, NPIPB5, RBMY1D, TCEB3C, ZNF880, CCDC77, FAM110C, KAZALD1, NPIPB6, RBMY1E, TCEB3CL, ZNF883, CCDC78, FAM110D, KAZN, NPIPB8, RBMY1F, TCEB3CL2, ZNF888, CCDC79, FAM111A, KBTBD11, NPIPB9, RBMY1J, TCERG1, ZNF891, CCDC8, FAM111B, KBTBD12, NPL, RBP1, TCERG1L, ZNF90, CCDC80, FAM114A1, KBTBD13, NPLOC4, RBP2, TCF12, ZNF91, CCDC81, FAM114A2, KBTBD2, NPM1, RBP3, TCF15, ZNF92, CCDC82, FAM115A, KBTBD3, NPM2, RBP4, TCF19, ZNF93, CCDC83, FAM115C, KBTBD4, NPM3, RBP5, TCF20, ZNF98, CCDC84, FAM117A, KBTBD6, NPNT, RBP7, TCF21, ZNF99, CCDC85A, FAM117B, KBTBD7, NPPA, RBPJ, TCF23, ZNFX1, CCDC85B, FAM118A, KBTBD8, NPPB, RBPJL, TCF24, ZNHIT1, CCDC85C, FAM118B, KCMF1, NPPC, RBPMS, TCF25, ZNHIT2, CCDC86, FAM120A, KCNA1, NPR1, RBPMS2, TCF3, ZNHIT3, CCDC87, FAM120AOS, KCNA10, NPR2, RBX1, TCF4, ZNHIT6, CCDC88A, FAM120B, KCNA2, NPR3, RC3H1, TCF7, ZNRD1, CCDC88B, FAM120C, KCNA3, NPRL2, RC3H2, TCF7L1, ZNRF1, CCDC88C, FAM122A, KCNA4, NPRL3, RCAN1, TCF7L2, ZNRF2, CCDC89, FAM122B, KCNA5, NPS, RCAN2, TCFL5, ZNRF3, CCDC9, FAM122C, KCNA6, NPSR1, RCAN3, TCHH, ZNRF4, CCDC90B, FAM124A, KCNA7, NPTN, RCBTB1, TCHHL1, ZP1, CCDC91, FAM124B, KCNAB1, NPTX1, RCBTB2, TCHP, ZP2, CCDC92, FAM126A, KCNAB2, NPTX2, RCC1, TCIRG1, ZP3, CCDC93, FAM126B, KCNAB3, NPTXR, RCC2, TCL1A, ZP4, CCDC94, FAM127A, KCNB1, NPVF, RCCD1, TCL1B, ZPBP, CCDC96, FAM127B, KCNB2, NPW, RCE1, TCN1, ZPBP2, CCDC97, FAM127C, KCNC1, NPY, RCHY1, TCN2, ZPLD1, CCER1, FAM129A, KCNC2, NPY1R, RCL1, TCOF1, ZRANB1, CCHCR1, FAM129B, KCNC3, NPY2R, RCN1, TCP1, ZRANB2, CCIN, FAM129C, KCNC4, NPY4R, RCN2, TCP10, ZRANB3, CCK, FAM131A, KCND1, NPY5R, RCN3, TCP10L, ZRSR2, CCKAR, FAM131B, KCND2, NQO1, RCOR1, TCP10L2, ZSCAN1, CCKBR, FAM131C, KCND3, NQO2, RCOR2, TCP11, ZSCAN10, CCL1, FAM132A, KCNE1, NROB1, RCOR3, TCP11L1, ZSCAN12, CCL11, FAM132B, KCNE1L, NR0B2, RCSD1, TCP11L2, ZSCAN16, CCL13, FAM133A, KCNE2, NR1D1, RCVRN, TCP11X1, ZSCAN18, CCL14, FAM133B, KCNE3, NR1D2, RD3, TCP11X2, ZSCAN2, CCL15, FAM134A, KCNE4, NR1H2, RD3L, TCTA, ZSCAN20, CCL16, |
| FAM134B, KCNF1, NR1H3, RDH10, TCTE1, ZSCAN21, CCL17, FAM134C, KCNG1, NR1H4, RDH11, TCTE3, ZSCAN22, CCL18, FAM135A, KCNG2, NR1I2, RDH12, TCTEX1D1, ZSCAN23, CCL19, FAM135B, KCNG3, NR1I3, RDH13, TCTEX1D2, ZSCAN25, CCL2, FAM136A, KCNG4, NR2C1, RDH14, TCTEX1D4, ZSCAN26, CCL20, FAM13A, KCNH1, NR2C2, RDH16, TCTN1, ZSCAN29, CCL21, FAM13B, KCNH2, NR2C2AP, RDH5, TCTN2, ZSCAN30, CCL22, FAM13C, KCNH3, NR2E1, RDH8, TCTN3, ZSCAN31, CCL23, FAM149A, KCNH4, NR2E3, RDM1, TDG, ZSCAN32, CCL24, FAM149B1, KCNH5, NR2F1, RDX, TDGF1, ZSCAN4, CCL25, FAM150A, KCNH6, NR2F2, REC8, TDO2, ZSCAN5A, CCL26, FAM150B, KCNH7, NR2F6, RECK, TDP1, ZSCAN5B, CCL27, FAM151A, KCNH8, NR3C1, RECQL, TDP2, ZSCAN9, CCL28, FAM151B, KCNIP1, NR3C2, RECQL4, TDRD1, ZSWIM1, CCL3, FAM153A, KCNIP2, NR4A1, RECQL5, TDRD10, ZSWIM2, CCL3L1, FAM153B, KCNIP3, NR4A2, REEP1, TDRD12, ZSWIM3, CCL3L3, FAM154A, KCNIP4, NR4A3, REEP2, TDRD3, ZSWIM4, CCL4, FAM154B, KCNJ1, NR5A1, REEP3, TDRD5, ZSWIM5, CCL4L1, FAM155A, KCNJ10, NR5A2, REEP4, TDRD6, ZSWIM6, CCL4L2, FAM155B, KCNJ11, NR6A1, REEP5, TDRD7, ZSWIM7, CCL5, FAM156A, KCNJ12, NRAP, REEP6, TDRD9, ZSWIM8, CCL7, FAM156B, KCNJ13, NRARP, REG1A, TDRKH, ZUFSP, CCL8, FAM157A, KCNJ14, NRAS, REG1B, TDRP, ZW10, CCM2, FAM157B, KCNJ15, NRBF2, REG3A, TEAD1, ZWILCH, CCM2L, FAM159A, KCNJ16, NRBP1, REG3G, TEAD2, ZWINT, CCNA1, FAM159B, KCNJ18, NRBP2, REG4, TEAD3, ZXDA, CCNA2, FAM160A1, KCNJ2, NRCAM, REL, TEAD4, ZXDB, CCNB1, FAM160A2, KCNJ3, NRD1, RELA, TEC, ZXDC, CCNB1IP1, FAM160B1, KCNJ4, NRDE2, RELB, TECPR1, ZYG11A, CCNB2, FAM160B2, KCNJ5, NREP, RELL1, TECPR2, ZYG11B, CCNB3, FAM161A, KCNJ6, NRF1, RELL2, TECR, ZYX, CCNC, FAM161B, KCNJ8, NRG1, RELN, TECRL, ZZEF1, CCND1, FAM162A, KCNJ9, NRG2, RELT, TECTA, ZZZ3, CCND2, FAM162B, KCNK1, NRG, RHODOPSIN, RdCVF, RdCVFL, GIRK, DUX4, and DBET (or DBET IncRNA). |

**TABLE 2. List of naturally occurring Cas12f proteins.**

| **Cas protein** | **SEQ ID NO** | **Amino acid sequence** |
|---|---|---|
| Un2Cas12f 1 | 2 | Provided herein (SEQ ID NO: 2) |
| AsCas 12f | 3 | Provided herein (SEQ ID NO: 3) |
| Mi1Cas12f 2 | 13 | MNMSKTTISVKLKIIDLSSEKKEFLDNYFNEYAKATTFCQLRIRRLLRN THWLGKKEKSSKKWIFESGICDLCGENKELVNEDRNSGEPAKICKRCY NGRYGNQMIRKLFVSTKKREVQENMDIRRVAKLNNTHYHRIPEEAFD MIKAADTAEKRRKKNVEYDKKRQMEFIEMFNDEKKRAARPKKPNERE TRYVHISKLESPSKGYTLNGIKRKIDGMGKKIERAEKGLSRKKIFGYQG NRIKLDSNWVRFDLAESEITIPSLFKEMKLRITGPTNVHSKSGQIYFAEW FERINKQPNNYCYLIRKTSSNGKYEYYLQYTYEAEVEANKEYAGCLGV DIGCSKLAAAVYYDSKNKKAQKPIEIFTNPIKKIKMRREKLIKLLSRVK VRHRRRKLMQLSKTEPIIDYTCHKTARKIVEMANTAKAFISMENLETGI KQKQQARETKKQKFYRNMFLFRKLSKLIEYKALLKGIKIVYVKPDYTS QTCSSCGADKEKTERPSQAIFRCLNPTCRYYQRDINADFNAAVNIAKK ALNNTEVVTTLL |
| Mi2Cas12f 2 | 14 | MPSETYITKTLSLKLIPSDEEKQALENYFITFQRAVNFAIDRIVDIRSSFR YLNKNEQFPAVCDCCGKKEKIMYVNISNKTFKFKPSRNQKDRYTKDIY TIKPNAHICKTCYSGVAGNMFIRKQMYPNDKEGWKVSRSYNIKVNAP GLTGTEYAMAIRKAISILRSFEKRRRNAERRIIEYEKSKKEYLELIDDVE KGKTNKIVVLEKEGHQRVKRYKHKNWPEKWQGISLNKAKSKVKDIE KRIKKLKEWKHPTLNRPYVELHKNNVRIVGYETVELKLGNKMYTIHF ASISNLRKPFRKQKKKSIEYLKHLLTLALKRNLETYPSIIKRGKNFFLQY PVRVTVKVPKLTKNFKAFGIDRGVNRLAVGCIISKDGKLTNKNIFFFHG KEAWAKENRYKKIRDRLYAMAKKLRGDKTKKIRLYHEIRKKFRHKV KYFRRNYLHNISKQIVEIAKENTPTVIVLEDLRYLRERTYRGKGRSKKA KKTNYKLNTFTYRMLIDMIKYKAEEAGVPVMIIDPRNTSRKCSKCGYV DENNRKQASFKCLKCGYSLNADLNAAVNIAKAFYECPTFRWEEKLHA YVCSEPDK |
| AuCas12f2 | 15 | MKSFKLKLLPTDEQNVLLNEVFCKWASLCTRMASKGHDKERLAPPDS SGNYFNKTQLNQVNTDVTDHMGALEESASQKERAVEKVKRRLKLISD MLSEPNLRDVSQQKPTTFRPLEWVKEGLLKTKYHTVHYWQKECDKLT KQKERMEKTIEKIKKGKITFKPTKMSLHQNCFSLSFGKGTFSMRPFSDT KRGINLDMLTAPIQPAIGKNDGKSSLRSKEFIARNIENYIIFSIHSQLFGLS RSEELLLNAKKEELVAKRDAMLKKKSDSLSKKIKELEKIVGRKITDSER SEIMSQGGKLSSEKFSEDNSYLKTLKVLAKDIIGREELFRLKKYPIVIRK PLNERKKLKNLKPDEWEYYLQLSYDELEKKEFTPKTIMGIDRGLKHIL AIAIYDPVQNKFVKNMLIPNPILGWKWKLRKIKRSIQHMERRIRAQQN AHVPENQLKKRLKSIENKIDYYYHNVSRQILNLAHDFKSAIVVEDLQN MKQHGRKKSKGLRGLNYALSNFDYGKIMGLVKYKAESENVPLLTVLP AGTSQNCAYCLLYGKEQGNYVRNNVNSKIGKCKLHGEIDADINAARTI AICYHKNINEPKPYGERKTFKRK |
| PtCas12f1 | 16 | MKYTKVMRYQIIKPLNAEWDELGMVLRDIQKETRAALNKTIQLCWEY QGFSADYKQIHGQYPKPKDVLGYTSMHGYAYDRLKNEFSKIASSNLSQ TIKRAVDKWNSDLKEILRGDRSIPNFRKDCPIDIVKQSTKIQKCNDGYV LSLGLINREYKNELGRKNGVFDVLIKANDKTQQTILERIINGDYTYTAS QIINHKNKWFINLTYQFETKETALDPNNVMGVDLGIVYPVYIAFNNSL HRYHIKGGEIERFRRQVEKRKRELLNQGKYCGDGRKGHGYATRTKSIE SISDKIARFRDTCNHKYSRFIVDMALKHNCGIIQMEDLTGISKESTFLKN WTYYDLQQKIEYKAREAGIQVIKIEPQYTSQRCSKCGYIDKENRQEQA TFKCIECGFKTNADYNAARNIAIPNIDKIIRKTLKMQ |
| RuCas 12f1 | 17 | MTLLVKVVKIHLISEQFDKAGNRIDYEEVNKILWELQKQTREAKNKTV QLLWEWNNFSSDYVKASGIYPKAKDIFGYSSVHGQANKELRTKLALN SSNLSTTTMDVCKNFNTYKKEVWKGKRSVPSYKSDQPLDLHKDSIKLI YENNEFYVRLALLKKAEFAKYGFKDGFRFKMQVKDNSTKTILERCFD EVYKINASKLLYDQKKKKWKLNLSYSFDNKNISELDKEKILGVDVGV NCPLVASVFGDRDRFIIKGGEIEKFRKSVEARRRSMLEQTKYCGDGRIG HGRKKRTEPALNIGDKIARFRDTTNHKYSRALIEYAVKKGCGTIQMEK LTGITSKSDRFLKDWTYYDLQTKIENKAKEVGINVVYIAPKYTSQRCSK CGYIHKDNRPNQAKFRCLECDFESNADYNASQNIGIKNIDKIIEKDLQK QESEVQVNENK |
| SpCas12f1 | 18 | MGESVKAIKLKILDMFLDPECTKQDDNWRKDLSTMSRFCAEAGNMCL RDLYNYFSMPKEDRISSKDLYNAMYHKTKLLHPELPGKVANQIVNHA KDVWKRNAKLIYRNQISMPTYKITTAPIRLQNNIYKLIKNKNKYIIDVQ LYSKEYSKDSGKGTHRYFLVAVRDSSTRMIFDRIMSKDHIDSSKSYTQ GQLQIKKDHQGKWYCIIPYTFPTHETVLDPDKVMGVDLGVAKAVYW AFNSSYKRGCIDGGEIEHFRKMIRARRVSIQNQIKHSGDARKGHGRKR ALKPIETLSEKEKNFRDTINHRYANRIVEAAIKQGCGTIQIENLEGIADT TGSKFLKNWPYYDLQTKIVNKAKEHGITVVAINPQYTSQRCSMCGYIE KTNRSSQAVFECKQCGYGSRTICINCRHVQVSGDVCEECGGIVKKENV NADYNAAKNISTPYIDQIIMEKCLELGIPYRSITCKECGHIQASGNTCEV CGSTNILKPKKIRKAK |
| CnCas 12f1 | 19 | MITVRKIKLTIMGDKDTRNSQYKWIRDEQYNQYRALNMGMTYLAVN DILYMNESGLEIRTIKDLKDCEKDIDKNKKEIEKLTARLEKEQNKKNSS SEKLDEIKYKISLVENKIEDYKLKIVELNKILEETQKERMDIQKEFKEKY VDDLYQVLDKIPFKHLDNKSLVTQRIKADIKSDKSNGLLKGERSIRNYK RNFPLMTRGRDLKFKYDDNDDIEIKWMEGIKFKVILGNRIKNSLELRHT LHKVIEGKYKICDSSLQFDKNNNLILNLTLDIPIDIVNKKVSGRVVGVD LGLKIPAYCALNDVEYIKKSIGRIDDFLKVRTQMQSRRRRLQIAIQSAK GGKGRVNKLQALERFAEKEKNFAKTYNHFLSSNIVKFAVSNQAEQIN MELLSLKETQNKSILRNWSYYQLQTMIEYKAQREGIKVKYIDPYHTSQ TCSKCGNYEEGQRESQADFICKKCGYKVNADYNAARNIAMSNKYITK KEESKYYYKIKESMV |

**TABLE 3A. List of engineered nuclease variants, such as dCasMINI and chimeric engineered nuclease variants. The number of asterisks represent the relative degree of gene modulation activity of the engineered nuclease variants.**

| **Engineered nuclease variant** | **Size (amino acids)** | **Activation of IFN gamma** | **Activation of CD2** | **Chimera design** |
|---|---|---|---|---|
| dCasMINI | 529 | ** | ** | SEQ ID NO: 10 |
| cA1 | 495 | - | - | Substitute K31-L105 of dCasMINI with K32-L72 of Un2Cas12f1 |
| cA2 | 495 | *** | * | Substitute K31-L77 of dCasMINI with K32-L44 of Un2Cas12f1 |
| cA3 | 496 | - | - | Substitute M1-L77 of dCasMINI with M1-L44 of Un2Cas12f1 |
| cA4 | 495 | * | - | Substitute P17-L77 of dCasMINI with P18-L44 of Un2Cas12f1 |
| cA5 | 451 | - | - | Substitute M1-W95 of dCasMINI with M1-W17 of AsCas12f1 |
| cA6 | 485 | - | - | Substitute M1-D91 of dCasMINI with M1-D47 of CnCas12f1 |
| cA7 | 462 | - | - | Substitute M1-W95 of dCasMINI with M1-W28 of SpCas12f1 |
| cA8 | 453 | - | - | Substitute M1-W95 of dCasMINI with M1-W19 of PtCas12f1 |
| cA9 | 460 | - | - | Substitute M1-E97 of dCasMINI with M1-E28 of RuCas12f1 |

**TABLE 3B. Amino acid sequences of chimeric engineered nuclease variants from TABLE 3A.**

| **Engineered nuclease variant** | **SEQ ID NO** | **Amino acid sequence** |
|---|---|---|
| cA1 | 20 | MAKNTITKTLKLRIVRPYNSAEVEKIVADEKERRKQAGGTGELDGGFYKKL EKKHSEMFSFDRLNLLLNQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNISNPKLKSTKERP |
| cA3 | 21 | MEVQKTVMKTLSLRILRPLYSQEIEKEIKEEKERRKQAGGTGELDDKFYQK LRGQFPDAVFWQEISEIFRQLQKQAAEIYNQSLIELYYEIFIKGKGIANASSV EHYLSRVCYRRAAELFKNAAIASGLRSKIKSNFRLKELKNMKSGLPTTKSD NFPIPLVKQKGGQYTGFEISNHNSDFIIKIPFGRWQVKKEIDKYRPWEKFDFE QVQKSPKPISLLLSTQRRKRNKGWSKDEGTEAEIKKVMNGDYQTSYIEVKR GSKICEKSAWMLNLSIDVPKIDKGVDPSIIGGIAVGVRSPLVCAINNAFSRYS ISDNDLFHFNKKMFARRRILLKKNRHKRAGHGAKNKLKPITILTEKSERFRK KLIERWACEIADFFIKNKVGTVQMENLESMKRKEDSYFNIRLRGFWPYAEM QNKIEFKLKQYGIEIRKVAPNNTSKTCSKCGHLNNYFNFEYRKKNKFPHFK CEKCNFKENAAYNAALNISNPKLKSTKERP |
| cA4 | 22 | MAKNTITKTLKLRIVRPLYSQEIEKEIKEEKERRKQAGGTGELDDKFYQKLR GQFPDAVFWQEISEIFRQLQKQAAEIYNQSLIELYYEIFIKGKGIANASSVEH YLSRVCYRRAAELFKNAAIASGLRSKIKSNFRLKELKNMKSGLPTTKSDNFP IPLVKQKGGQYTGFEISNHNSDFIIKIPFGRWQVKKEIDKYRPWEKFDFEQV QKSPKPISLLLSTQRRKRNKGWSKDEGTEAEIKKVMNGDYQTSYIEVKRGS KICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNISNPKLKSTKERP |
| cA5 | 23 | MIKVYRYEIVKPLDLDWQEISEIFRQLQKQAAEIYNQSLIELYYEIFIKGKGIA NASSVEHYLSRVCYRRAAELFKNAAIASGLRSKIKSNFRLKELKNMKSGLP TTKSDNFPIPLVKQKGGQYTGFEISNHNSDFIIKIPFGRWQVKKEIDKYRPWE KFDFEQVQKSPKPISLLLSTQRRKRNKGWSKDEGTEAEIKKVMNGDYQTSY IEVKRGSKICEKSAWMLNLSIDVPKIDKGVDPSIIGGIAVGVRSPLVCAINNA FSRYSISDNDLFHFNKKMFARRRILLKKNRHKRAGHGAKNKLKPITILTEKS ERFRKKLIERWACEIADFFIKNKVGTVQMENLESMKRKEDSYFNIRLRGFW PYAEMQNKIEFKLKQYGIEIRKVAPNNTSKTCSKCGHLNNYFNFEYRKKNK FPHFKCEKCNFKENAAYNAALNISNPKLKSTKERP |
| cA6 | 24 | MITVRKIKLTIMGDKDTRNSQYKWIRDEQYNQYRALNMGMTYLAVNDAV FWQEISEIFRQLQKQAAEIYNQSLIELYYEIFIKGKGIANASSVEHYLSRVCY RRAAELFKNAAIASGLRSKIKSNFRLKELKNMKSGLPTTKSDNFPIPLVKQK GGQYTGFEISNHNSDFIIKIPFGRWQVKKEIDKYRPWEKFDFEQVQKSPKPIS LLLSTQRRKRNKGWSKDEGTEAEIKKVMNGDYQTSYIEVKRGSKICEKSA WMLNLSIDVPKIDKGVDPSIIGGIAVGVRSPLVCAINNAFSRYSISDNDLFHF NKKMFARRRILLKKNRHKRAGHGAKNKLKPITILTEKSERFRKKLIERWAC EIADFFIKNKVGTVQMENLESMKRKEDSYFNIRLRGFWPYAEMQNKIEFKL KQYGIEIRKVAPNNTSKTCSKCGHLNNYFNFEYRKKNKFPHFKCEKCNFKE NAAYNAALNISNPKLKSTKERP |
| cA7 | 25 | MGESVKAIKLKILDMFLDPECTKQDDNWQEISEIFRQLQKQAAEIYNQSLIE LYYEIFIKGKGIANASSVEHYLSRVCYRRAAELFKNAAIASGLRSKIKSNFRL KELKNMKSGLPTTKSDNFPIPLVKQKGGQYTGFEISNHNSDFIIKIPFGRWQ VKKEIDKYRPWEKFDFEQVQKSPKPISLLLSTQRRKRNKGWSKDEGTEAEI KKVMNGDYQTSYIEVKRGSKICEKSAWMLNLSIDVPKIDKGVDPSIIGGIAV GVRSPLVCAINNAFSRYSISDNDLFHFNKKMFARRRILLKKNRHKRAGHGA KNKLKPITILTEKSERFRKKLIERWACEIADFFIKNKVGTVQMENLESMKRK EDSYFNIRLRGFWPYAEMQNKIEFKLKQYGIEIRKVAPNNTSKTCSKCGHL NNYFNFEYRKKNKFPHFKCEKCNFKENAAYNAALNISNPKLKSTKERP |
| cA8 | 26 | MKYTKVMRYQIIKPLNAEWQEISEIFRQLQKQAAEIYNQSLIELYYEIFIKGK GIANASSVEHYLSRVCYRRAAELFKNAAIASGLRSKIKSNFRLKELKNMKS GLPTTKSDNFPIPLVKQKGGQYTGFEISNHNSDFIIKIPFGRWQVKKEIDKYR PWEKFDFEQVQKSPKPISLLLSTQRRKRNKGWSKDEGTEAEIKKVMNGDY QTSYIEVKRGSKICEKSAWMLNLSIDVPKIDKGVDPSIIGGIAVGVRSPLVCA INNAFSRYSISDNDLFHFNKKMFARRRILLKKNRHKRAGHGAKNKLKPITIL TEKSERFRKKLIERWACEIADFFIKNKVGTVQMENLESMKRKEDSYFNIRLR GFWPYAEMQNKIEFKLKQYGIEIRKVAPNNTSKTCSKCGHLNNYFNFEYRK KNKFPHFKCEKCNFKENAAYNAALNISNPKLKSTKERP |
| cA9 | 27 | MTLLVKVVKIHLISEQFDKAGNRIDYEEISEIFRQLQKQAAEIYNQSLIELYY EIFIKGKGIANASSVEHYLSRVCYRRAAELFKNAAIASGLRSKIKSNFRLKEL KNMKSGLPTTKSDNFPIPLVKQKGGQYTGFEISNHNSDFIIKIPFGRWQVKK EIDKYRPWEKFDFEQVQKSPKPISLLLSTQRRKRNKGWSKDEGTEAEIKKV MNGDYQTSYIEVKRGSKICEKSAWMLNLSIDVPKIDKGVDPSIIGGIAVGVR SPLVCAINNAFSRYSISDNDLFHFNKKMFARRRILLKKNRHKRAGHGAKNK LKPITILTEKSERFRKKLIERWACEIADFFIKNKVGTVQMENLESMKRKEDS YFNIRLRGFWPYAEMQNKIEFKLKQYGIEIRKVAPNNTSKTCSKCGHLNNY FNFEYRKKNKFPHFKCEKCNFKENAAYNAALNISNPKLKSTKERP |

**TABLE 4A. List of engineered nuclease variants. The number of asterisks represent the relative degree of gene modulation activity of the engineered nuclease variants.**

| **Engineered nuclease variant** | **Size (amino acids)** | **Activation of IFN gamma** | **Activation of CD2** | **Repression of eGFP (repressor type A)** | **Repression of eGFP (repressor type B)** |
|---|---|---|---|---|---|
| dCasMINI | 529 | ** | ** | ** | ** |
| cA2 | 495 | *** | * | **** | ** |
| mA8 | 529 | **** | ** | ** | - |
| mA10 | 529 | - | ** | * | - |
| mA11 | 529 | * | * | ** | - |
| mA12 | 529 | * | * | * | * |
| mA14 | 529 | * | * | * | * |
| mB9 | 529 | - | - | * | - |
| mC7 | 529 | ** | * | ** | (no data) |
| mC16 | 529 | - | * | * | * |
| mC18 | 529 | *** | ** | (no data) | *** |
| mC21 | 529 | - | * | *** | * |
| mD2 | 529 | **** | **** | **** | (no data) |
| mD4 | 529 | **** | ** | *** | *** |
| mD5 | 529 | - | * | *** | - |
| mD6 | 529 | - | * | ** | - |
| mD7 | 529 | - | ** | *** | (no data) |
| mD15 | 529 | - | * | * | ** |

**TABLE 4B. Amino acid sequences of chimeric engineered nuclease variants from TABLE 4A.**

| **Engineered nuclease variant** | **SEQ ID NO** | **Amino acid sequence** |
|---|---|---|
| mA1 | 28 | MAKNTITKTLKLRIVRPYYSQEIEKIVAEEKNRREKIALEKNDKVKEACSK HLKVAAYCTTQVERNACLFCKARKLDDKFYQKLRGQFPDAVFWQEISEIFR QLQKQAAEIYNQSLIELYYEIFIKGKGIANASSVEHYLSRVCYRRAAELFKN AAIASGLRSKIKSNFRLKELKNMKSGLPTTKSDNFPIPLVKQKGGQYTGFEIS NHNSDFIIKIPFGRWQVKKEIDKYRPWEKFDFEQVQKSPKPISLLLSTQRRKR NKGWSKDEGTEAEIKKVMNGDYQTSYIEVKRGSKICEKSAWMLNLSIDVP KIDKGVDPSIIGGIAVGVRSPLVCAINNAFSRYSISDNDLFHFNKKMFARRRI LLKKNRHKRAGHGAKNKLKPITILTEKSERFRKKLIERWACEIADFFIKNKV GTVQMENLESMKRKEDSYFNIRLRGFWPYAEMQNKIEFKLKQYGIEIRKVA PNNTSKTCSKCGHLNNYFNFEYRKKNKFPHFKCEKCNFKENAAYNAALNIS NPKLKSTKERP |
| mA2 | 29 | MAKNTITKTLKLRIVRPYYSAEVEKIVAEEKNNREKIALDKNKDKVKEACS KHLKVAAYCTTQVERNACLFCKARKLDDKFYKKLRGQFPDAVFWQEISEI FRQLQKQAREIYNQSLIELYYEIFIKGKGIANASSVEHYLSRVCYRRAAELFK NAAIASGLRSKIKSNFRLKELKNMKSGLPTTKSDNFPIPLVKQKGGQYTGFE ISNHNSDFIIKIPFGRWQVKKEIDKYRPWEKFDFEQVQKSPKPISLLLSTQRR KRNKGWSKDEGTEAEIKKVMNGDYQTSYIEVKRGSKICEKSAWMLNLSID VPKIDKGVDPSIIGGIAVGVRSPLVCAINNAFSRYSISDNDLFHFNKKMFARR RILLKKNRHKRAGHGAKNKLKPITILTEKSERFRKKLIERWACEIADFFIKNK VGTVQMENLESMKRKEDSYFNIRLRGFWPYAEMQNKIEFKLKQYGIEIRKV APNNTSKTCSKCGHLNNYFNFEYRKKNKFPHFKCEKCNFKENAAYNAALN ISNPKLKSTKERP |
| mA3 | 30 | MAKNTITKTLKLRIVRPYYSAEIEKIVADEKNRREKIALEKNKDKVKEACSK HLKVAAYCTTQVERNACLFCKARKLDDKFYQKLRKQFPDAVFWQEISEIFR QLQKQAREIYNQSLIELYYEIFIKGKGIANASSVEHYLSRVCYRRAAELFKN AAIASGLRSKIKSNFRLKELKNMKSGLPTTKSDNFPIPLVKQKGGQYTGFEIS NHNSDFIIKIPFGRWQVKKEIDKYRPWEKFDFEQVQKSPKPISLLLSTQRRKR NKGWSKDEGTEAEIKKVMNGDYQTSYIEVKRGSKICEKSAWMLNLSIDVP KIDKGVDPSIIGGIAVGVRSPLVCAINNAFSRYSISDNDLFHFNKKMFARRRI LLKKNRHKRAGHGAKNKLKPITILTEKSERFRKKLIERWACEIADFFIKNKV GTVQMENLESMKRKEDSYFNIRLRGFWPYAEMQNKIEFKLKQYGIEIRKVA PNNTSKTCSKCGHLNNYFNFEYRKKNKFPHFKCEKCNFKENAAYNAALNIS NPKLKSTKERP |
| mA4 | 31 | MAKNTITKTLKLRIVRPYNSQEVEKIVAEEKNRREKIALDKNKDVKEACS KHLKVAAYCTTQVERNACLFCKARKLDDKFYQKLRKQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPHFKCEKCNFKENAAYNAAL NISNPKLKSTKERP |
| mA5 | 32 | MAKNTITKTLKLRIVRPYNSQEVEKIVAEEKNNREKIALDKNKDKVKEACS KHLKVAAYCTTQVERNACLFCKARKLDDKFYKKLRKQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPHFKCEKCNFKENAAYNAAL NISNPKLKSTKERP |
| mA6 | 33 | MAKNTITKTLKLRIVRPYNSQEVEKIVAEEKNNREKIALDKNKDKVKEACS KHLKVAAYCTTQVERNACLFCKARKLDDKFYKKLRGQFPDAVFWQEISEI FRQLQKQAREIYNQSLIELYYEIFIKGKGIANASSVEHYLSRVCYRRAAELFK NAAIASGLRSKIKSNFRLKELKNMKSGLPTTKSDNFPIPLVKQKGGQYTGFE ISNHNSDFIIKIPFGRWQVKKEIDKYRPWEKFDFEQVQKSPKPISLLLSTQRR KRNKGWSKDEGTEAEIKKVMNGDYQTSYIEVKRGSKICEKSAWMLNLSID VPKIDKGVDPSIIGGIAVGVRSPLVCAINNAFSRYSISDNDLFHFNKKMFARR RILLKKNRHKRAGHGAKNKLKPITILTEKSERFRKKLIERWACEIADFFIKNK VGTVQMENLESMKRKEDSYFNIRLRGFWPYAEMQNKIEFKLKQYGIEIRKV APNNTSKTCSKCGHLNNYFNFEYRKKNKFPHFKCEKCNFKENAAYNAALN ISNPKLKSTKERP |
| mA7 | 34 | MAKNTITKTLKLRIVRPYYSAEVEKIVAEEKNNREKIALDKNKDKVKEACS KHLKVAAYCTTQVERNACLFCKARKLDDKFYKKLRKQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPHFKCEKCNFKENAAYNAAL NISNPKLKSTKERP |
| mA8 | 35 | MAKNTITKTLKLRIVRPYNSAEIEKIVADEKNRREKIALDKNKDKVKEACS KHLKVAAYCTTQVERNACLFCKARKLDDKFYKKLRKQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPHFKCEKCNFKENAAYNAAL NISNPKLKSTKERP |
| mA9 | 36 | MAKNTITKTLKLRIVRPYNSAEIEKIVADEKNRREKIALDKNKDKVKEACS KHLKVAAYCTTQVERNACLFCKARKLDDKFYKKLRGQFPDAVFWQEISEI FRQLQKQAREIYNQSLIELYYEIFIKGKGIANASSVEHYLSRVCYRRAAELFK NAAIASGLRSKIKSNFRLKELKNMKSGLPTTKSDNFPIPLVKQKGGQYTGFE ISNHNSDFIIKIPFGRWQVKKEIDKYRPWEKFDFEQVQKSPKPISLLLSTQRR KRNKGWSKDEGTEAEIKKVMNGDYQTSYIEVKRGSKICEKSAWMLNLSID VPKIDKGVDPSIIGGIAVGVRSPLVCAINNAFSRYSISDNDLFHFNKKMFARR RILLKKNRHKRAGHGAKNKLKPITILTEKSERFRKKLIERWACEIADFFIKNK VGTVQMENLESMKRKEDSYFNIRLRGFWPYAEMQNKIEFKLKQYGIEIRKV APNNTSKTCSKCGHLNNYFNFEYRKKNKFPHFKCEKCNFKENAAYNAALN ISNPKLKSTKERP |
| mA10 | 37 | MAKNTITKTLKLRIVRPYNSQEIEKIVAEEKNRREKIALDKNKDKVKEACSK HLKVAAYCTTQVERNACLFCKARKLDDKFYQKLRGQFPDAVFWQEISEIFR QLQKQAAEIYNQSLIELYYEIFIKGKGIANASSVEHYLSRVCYRRAAELFKN AAIASGLRSKIKSNFRLKELKNMKSGLPTTKSDNFPIPLVKQKGGQYTGFEIS NHNSDFIIKIPFGRWQVKKEIDKYRPWEKFDFEQVQKSPKPISLLLSTQRRKR NKGWSKDEGTEAEIKKVMNGDYQTSYIEVKRGSKICEKSAWMLNLSIDVP KIDKGVDPSIIGGIAVGVRSPLVCAINNAFSRYSISDNDLFHFNKKMFARRRI LLKKNRHKRAGHGAKNKLKPITILTEKSERFRKKLIERWACEIADFFIKNKV GTVQMENLESMKRKEDSYFNIRLRGFWPYAEMQNKIEFKLKQYGIEIRKVA PNNTSKTCSKCGHLNNYFNFEYRKKNKFPHFKCEKCNFKENAAYNAALNIS NPKLKSTKERP |
| mA11 | 38 | MAKNTITKTLKLRIVRPYNSAEIEKIVAEEKNRREKIALDKNKDKVKEACSK HLKVAAYCTTQVERNACLFCKARKLDDKFYKKLRGQFPDAVFWQEISEIFR QLQKQAAEIYNQSLIELYYEIFIKGKGIANASSVEHYLSRVCYRRAAELFKN AAIASGLRSKIKSNFRLKELKNMKSGLPTTKSDNFPIPLVKQKGGQYTGFEIS NHNSDFIIKIPFGRWQVKKEIDKYRPWEKFDFEQVQKSPKPISLLLSTQRRKR NKGWSKDEGTEAEIKKVMNGDYQTSYIEVKRGSKICEKSAWMLNLSIDVP KIDKGVDPSIIGGIAVGVRSPLVCAINNAFSRYSISDNDLFHFNKKMFARRRI LLKKNRHKRAGHGAKNKLKPITILTEKSERFRKKLIERWACEIADFFIKNKV GTVQMENLESMKRKEDSYFNIRLRGFWPYAEMQNKIEFKLKQYGIEIRKVA PNNTSKTCSKCGHLNNYFNFEYRKKNKFPHFKCEKCNFKENAAYNAALNIS NPKLKSTKERP |
| mA12 | 39 | MAKNTITKTLKLRIVRPYNSAEVEKIVAEEKNRREKIALDKNKDKVKEACS KHLKVAAYCTTQVERNACLFCKARKLDDKFYKKLRKQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPHFKCEKCNFKENAAYNAAL NISNPKLKSTKERP |
| mA13 | 40 | MAKNTITKTLKLRIVRPYNSAEVEKIVADEKNRREKIALDKNKDKVKEACS KHLKVAAYCTTQVERNACLFCKARKLDDKFYKKLRKQFPDAVFWQEISEI FRQLQKQAREIYNQSLIELYYEIFIKGKGIANASSVEHYLSRVCYRRAAELFK NAAIASGLRSKIKSNFRLKELKNMKSGLPTTKSDNFPIPLVKQKGGQYTGFE ISNHNSDFIIKIPFGRWQVKKEIDKYRPWEKFDFEQVQKSPKPISLLLSTQRR KRNKGWSKDEGTEAEIKKVMNGDYQTSYIEVKRGSKICEKSAWMLNLSID VPKIDKGVDPSIIGGIAVGVRSPLVCAINNAFSRYSISDNDLFHFNKKMFARR RILLKKNRHKRAGHGAKNKLKPITILTEKSERFRKKLIERWACEIADFFIKNK VGTVQMENLESMKRKEDSYFNIRLRGFWPYAEMQNKIEFKLKQYGIEIRKV APNNTSKTCSKCGHLNNYFNFEYRKKNKFPHFKCEKCNFKENAAYNAALN ISNPKLKSTKERP |
| mA14 | 41 | MAKNTITKTLKLRIVRPYYSAEIEKIVADEKNRREKIALDKNKDKVKEACS KHLKVAAYCTTQVERNACLFCKARKLDDKFYQKLRKQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPHFKCEKCNFKENAAYNAAL NISNPKLKSTKERP |
| mA15 | 42 | MAKNTITKTLKLRIVRPYYSAEIEKIVAEEKNRREKIALEKNKDKVKEACSK HLKVAAYCTTQVERNACLFCKARKLDDKFYQKLRKQFPDAVFWQEISEIFR QLQKQAAEIYNQSLIELYYEIFIKGKGIANASSVEHYLSRVCYRRAAELFKN AAIASGLRSKIKSNFRLKELKNMKSGLPTTKSDNFPIPLVKQKGGQYTGFEIS NHNSDFIIKIPFGRWQVKKEIDKYRPWEKFDFEQVQKSPKPISLLLSTQRRKR NKGWSKDEGTEAEIKKVMNGDYQTSYIEVKRGSKICEKSAWMLNLSIDVP KIDKGVDPSIIGGIAVGVRSPLVCAINNAFSRYSISDNDLFHFNKKMFARRRI LLKKNRHKRAGHGAKNKLKPITILTEKSERFRKKLIERWACEIADFFIKNKV GTVQMENLESMKRKEDSYFNIRLRGFWPYAEMQNKIEFKLKQYGIEIRKVA PNNTSKTCSKCGHLNNYFNFEYRKKNKFPHFKCEKCNFKENAAYNAALNIS NPKLKSTKERP |
| mA16 | 43 | MAKNTITKTLKLRIVRPYYSAEIEKIVAEEKNNREKIALDKNKDKVKEACSK HLKVAAYCTTQVERNACLFCKARKLDDKFYQKLRGQFPDAVFWQEISEIFR QLQKQAREIYNQSLIELYYEIFIKGKGIANASSVEHYLSRVCYRRAAELFKN AAIASGLRSKIKSNFRLKELKNMKSGLPTTKSDNFPIPLVKQKGGQYTGFEIS NHNSDFIIKIPFGRWQVKKEIDKYRPWEKFDFEQVQKSPKPISLLLSTQRRKR NKGWSKDEGTEAEIKKVMNGDYQTSYIEVKRGSKICEKSAWMLNLSIDVP KIDKGVDPSIIGGIAVGVRSPLVCAINNAFSRYSISDNDLFHFNKKMFARRRI LLKKNRHKRAGHGAKNKLKPITILTEKSERFRKKLIERWACEIADFFIKNKV GTVQMENLESMKRKEDSYFNIRLRGFWPYAEMQNKIEFKLKQYGIEIRKVA PNNTSKTCSKCGHLNNYFNFEYRKKNKFPHFKCEKCNFKENAAYNAALNIS NPKLKSTKERP |
| mB1 | 44 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAARLF KNAAIASGLRSKIKSNFRLKELKNMKSGLPTTKSDNFPIPLVKQKGGQYTGF KISNHNSDFIIKIPFGRWQVKKEIDKYRPWEKFDFRQVQKSPKPISLLLSTQR RKRNKGWSKDEGTEAEIRKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPIEQLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mB2 | 45 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAALF KNAAIASGLRSKIKSNFRLKELKNMKSGLPTTKSDNFPIPLVKQKGGQYTGF KISNHNSDFIIKIPFGRWQVKKEIDKYRPWEKFDFRQVQKSPKPISLLLSTQR RKRNKGWSKDEGTEAEIRKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPIEQLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mB3 | 46 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAGLF KNAAIASGLRSKIKSNFRLKELKNMKSGLPTTKSDNFPIPLVKQKGGQYTGF KISNHNSDFIIKIPFGRWQVKKEIDKYRPWEKFDFRQVQKSPKPISLLLSTQR RKRNKGWSKDEGTEAEIRKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPIEQLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mB4 | 47 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAARLF KNAAIASGLRSKIKSNFRLKELKNMKSGLPTTKSDNFPIPLVKQKGGQYTGF RISNHNSDFIIKIPFGRWQVKKEIDKYRPWEKFDFRQVQKSPKPISLLLSTQR RKRNKGWSKDEGTEAEIRKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPIEQLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mB5 | 48 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAALF KNAAIASGLRSKIKSNFRLKELKNMKSGLPTTKSDNFPIPLVKQKGGQYTGF RISNHNSDFIIKIPFGRWQVKKEIDKYRPWEKFDFRQVQKSPKPISLLLSTQR RKRNKGWSKDEGTEAEIRKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPIEQLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mB6 | 49 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAGLF KNAAIASGLRSKIKSNFRLKELKNMKSGLPTTKSDNFPIPLVKQKGGQYTGF RISNHNSDFIIKIPFGRWQVKKEIDKYRPWEKFDFRQVQKSPKPISLLLSTQR RKRNKGWSKDEGTEAEIRKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPIEQLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mB7 | 50 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAARLF KNAAIASGLRSKIKSNFRLKELKNMKSGLPTTKSDNFPIPLVKQKGGQYTGF SISNHNSDFIIKIPFGRWQVKKEIDKYRPWEKFDFRQVQKSPKPISLLLSTQR RKRNKGWSKDEGTEAEIRKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPIEQLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mB8 | 51 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAALF KNAAIASGLRSKIKSNFRLKELKNMKSGLPTTKSDNFPIPLVKQKGGQYTGF SISNHNSDFIIKIPFGRWQVKKEIDKYRPWEKFDFRQVQKSPKPISLLLSTQR RKRNKGWSKDEGTEAEIRKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPIEQLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mB9 | 52 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAGLF KNAAIASGLRSKIKSNFRLKELKNMKSGLPTTKSDNFPIPLVKQKGGQYTGF SISNHNSDFIIKIPFGRWQVKKEIDKYRPWEKFDFRQVQKSPKPISLLLSTQR RKRNKGWSKDEGTEAEIRKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPIEQLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC1 | 53 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLENFNKKMFAR RRILLKKNRHKRGGHGAKNKLKPIEQLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC2 | 54 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIEGGDLFHFNKKMFAR RRILLKKNRHKRAGHGAKNKLKPIEQLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC3 | 55 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIEGGDLENFNKKMFA RRRILLKKNRHKRGGHGRDKKLKPIEQLTEKSERFRKKLIERWACEIADFFI KNKVGTVQMENLESMKRKEDSYFNIRLRGFWPYAEMQNKIEFKLKQYGIEI RKVAPNNTSKTCSKCGHLNNYFNFEYRKKNKFPHFKCEKCNFKENAAYNA ALNISNPKLKSTKERP |
| mC4 | 56 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIEGGDLENFNKKMFA RRRILLKKNRHKRAGHGAKNKLKPITILTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC5 | 57 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGRDKKLKPIEQLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC6 | 58 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLENFNKKMFAR RRILLKKNRHKRAGHGRDKKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPHFKCEKCNFKENAAYNAAL NISNPKLKSTKERP |
| mC7 | 59 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLEHFNKKMFAR RRILLKKNRHKRKGHGAKNKLKPIETLTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC8 | 60 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIDGGDLEHFNKKMFA RRRILLKKNRHKRKGHGAKNKLKPITILTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC9 | 61 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIDGGDLFHFNKKMFA RRRILLKKNRHKRAGHGAKNKLKPIETLTEKSERFRKKLIERWACEIADFFI KNKVGTVQMENLESMKRKEDSYFNIRLRGFWPYAEMQNKIEFKLKQYGIEI RKVAPNNTSKTCSKCGHLNNYFNFEYRKKNKFPHFKCEKCNFKENAAYNA ALNISNPKLKSTKERP |
| mC10 | 62 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIDGGDLEHFNKKMFA RRRILLKKNRHKRKGHGAKNKLKPIETLTEKSERFRKKLIERWACEIADFFI KNKVGTVQMENLESMKRKEDSYFNIRLRGFWPYAEMQNKIEFKLKQYGIEI RKVAPNNTSKTCSKCGHLNNYFNFEYRKKNKFPHFKCEKCNFKENAAYNA ALNISNPKLKSTKERP |
| mC11 | 63 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNSFSRYSIDSNDLFKFNKKMFAR RRILLKKNRHKRKGHGAKNKLKPITELTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC12 | 64 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNSFSRYSIDSNDLFHFNKKMFAR RRILLKKNRHKRAGHGAAHKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPHFKCEKCNFKENAAYNAAL NISNPKLKSTKERP |
| mC13 | 65 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNSFSRYSIDSNDLFKFNKKMFAR RRILLKKNRHKRAGHGAAHKLKPITELTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC14 | 66 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIDSNDLFKFNKKMFAR RRILLKKNRHKRAGHGAAHKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPHFKCEKCNFKENAAYNAAL NISNPKLKSTKERP |
| mC15 | 67 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNSFSRYSIDSNDLFKFNKKMFAR RRILLKKNRHKRAGHGAKNKLKPITELTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC16 | 68 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNSFSRYSISDNDLFKFNKKMFAR RRILLKKNRHKRKGHGAKNKLKPITELTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC17 | 69 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIDSNDLFKFNKKMFAR RRILLKKNRHKRKGHGAKNKLKPITELTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC18 | 70 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFKFNKKMFAR RRILLKKNRHKRKGHGAAHKLKPITELTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC19 | 71 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIKGGDLERFNKKMFA RRRILLKKNRHKRKGHGAKNKLKPITILTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC20 | 72 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIKGGDLERFNKKMFA RRRILLKKNRHKRAGHGAKNKLKPITILTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC21 | 73 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIKGGDLEKFNKKMFA RRRILLKKNRHKRAGHGAKNKLKPITILTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC22 | 74 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIKGGDLFHFNKKMFA RRRILLKKNRHKRAGHGRKKKLKPITILTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mC23 | 75 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLEKFNKKMFAR RRILLKKNRHKRAGHGRKKKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPHFKCEKCNFKENAAYNAAL NISNPKLKSTKERP |
| mC24 | 76 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSIKGGDLEKFNKKMFA RRRILLKKNRHKRAGHGRKKKLKPITILTEKSERFRKKLIERWACEIADFFIK NKVGTVQMENLESMKRKEDSYFNIRLRGFWPYAEMQNKIEFKLKQYGIEIR KVAPNNTSKTCSKCGHLNNYFNFEYRKKNKFPHFKCEKCNFKENAAYNAA LNISNPKLKSTKERP |
| mD1 | 77 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWAKEIADFFIKN KVGTVQMEDLSTMKRKEDSYFNIRLRGFWPYYEMQNKIEFKLKQYGIEIRK VAPNNTSQLCSKCGHLNNYFNFEYRKKNKFPKFKCEKCNFKENAAYNAAR NISTPDIKSTKERP |
| mD2 | 78 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMEDLESMKRKEDSYFNIRLRGFWPYAEMQNKIEFKLKQYGIEIRK VAPNNTSQLCSKCGHLNNYFNFEYRKKNKFPHFKCEKCNFKENAAYNAAL NISNPDIKSTKERP |
| mD3 | 79 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWAKEIADFFIKN KVGTVQMEDLSTMKRKEDSYFNIRLRGFWPYYEMQNKIEFKLKQYGIEIRK VAPNNTSKTCSKCGHLNNYFNFEYRKKNKFPHFKCEKCNFKENAAYNAAL NISNPKLKSTKERP |
| mD4 | 80 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSQLCSKCGHLNNYFNFEYRKKNKFPKFKCEKCNFKENAAYNAAL NISNPDIKSTKERP |
| mD5 | 81 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSQLCSKCGHLNNYFNFEYRKKNKFPHFKCEKCNFKENAAYNAAL NISTPDIKSTKERP |
| mD6 | 82 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMEDLSTMKRKEDSYFNIRLRGFWPYYEMQNKIEFKLKQYGIEIRK VAPNNTSKTCSKCGHLNNYFNFEYRKKNKFPKFKCEKCNFKENAAYNAAL NISNPKLKSTKERP |
| mD7 | 83 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMEDLESMKRKEDSYFNIRLRGFWPYAEMQNKIEFKLKQYGIEIRK VAPNNTSQLCSKCGHLNNYFNFEYRKKNKFPKFKCEKCNFKENAAYNAAL NISTPDIKSTKERP |
| mD8 | 84 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPKFKCEKCNFKENAAYNAAR NISTPDIKSTKERP |
| mD9 | 85 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWSRYIADFFIKN KVGTVQMEDLESMKRKEDSYFNIRLRGFWPYYEMQNKIEFKLKQYGIKIR KVAPNNTSQRCSKCGHLNNYFNFEYRKKNKFPHFKCEKCNFKANAAYNA ARNISNPNIKSTKERP |
| mD10 | 86 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACYIADFFIK NKVGTVQMEDLESMKRKEDSYFNIRLRGFWPYAEMQNKIEFKLKQYGIEIR KVAPNNTSQRCSKCGHLNNYFNFEYRKKNKFPHFKCEKCNFKENAAYNAA RNISNPNIKSTKERP |
| mD11 | 87 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACYIADFFIK NKVGTVQMENLESMKRKEDSYFNIRLRGFWPYAEMQNKIEFKLKQYGIEIR KVAPNNTSQRCSKCGHLNNYFNFEYRKKNKFPHFKCEKCNFKRNAAYNA ARNISNPKLKSTKERP |
| mD12 | 88 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACYIADFFIK NKVGTVQMEDLESMKRKEDSYFNIRLRGFWPYAEMQNKIEFKLKQYGIEIR KVAPNNTSKTCSKCGHLNNYFNFEYRKKNKFPHFKCEKCNFKENAAYNAA RNISNPNIKSTKERP |
| mD13 | 89 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWARYIADFFIK NKVGTVQMEDLESMKRKEDSYFNIRLRGFWPYYEMQNKIEFKLKQYGIKI RKVAPNNTSKTCSKCGHLNNYFNFEYRKKNKFPHFKCEKCNFKENAAYNA ALNISNPKLKSTKERP |
| mD14 | 90 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSQRCSKCGHLNNYFNFEYRKKNKFPHFKCEKCNFKRNAAYNAAR NISNPNIKSTKERP |
| mD15 | 91 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMEDLESMKRKEDSYFNIRLRGFWPYAEMQNKIEFKLKQYGIEIRK VAPNNTSQRCSKCGHLNNYFNFEYRKKNKFPHFKCEKCNFKENAAYNAAR NISNPNIKSTKERP |
| mD16 | 92 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPHFKCEKCNFKRNAAYNAAR NISNPNIKSTKERP |
| mD17 | 93 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWANRIADFFIK NKVGTVQMENLESMKRKEDSYFNIRLRGFWPYAEMQNKIEFKLKQYGIKI RKVAPNNTSQRCSKCGHLNNYFNFEYRKKNKFPHFKCEKCNFKRNAAYNA AKNISNPKLKSTKERP |
| mD18 | 94 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSQRCSKCGHLNNYFNFEYRKKNKFPHFKCEKCNFKRNAAYNAA KNISNPKLKSTKERP |
| mD19 | 95 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIKIR KVAPNNTSQRCSKCGHLNNYFNFEYRKKNKFPHFKCEKCNFKRNAAYNA AKNISNPKLKSTKERP |
| mD20 | 96 | MAKNTITKTLKLRIVRPYNSAEVEKIVADEKNNREKIALEKNKDKVEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWANRIADFFIK NKVGTVQMENLESMKRKEDSYFNIRLRGFWPYAEMQNKIEFKLKQYGIKI RKVAPNNTSQRCSKCGHLNNYFNFEYRKKNKFPHFKCEKCNFKENAAYNA ALNISNPKLKSTKERP |
| mD21 | 97 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWANRIADFFIK NKVGTVQMENLESMKRKEDSYFNIRLRGFWPYAEMQNKIEFKLKQYGIEIR KVAPNNTSQRCSKCGHLNNYFNFEYRKKNKFPHFKCEKCNFKRNAAYNA ALNISNPKLKSTKERP |
| mD22 | 98 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWSRFIADFFIKN KVGTVQMEDLESMKRKEDSYFNIRLRGFWPYYEMQNKIEFKLKQYGIEIRK VAPNNTSQRCSKCGHLNNYFNFEYRKKNKFPHFKCEKCNFKENAAYNAAR NISNPNIKSTKERP |
| cB2 | 99 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIDVQLYSKEYSKDSGKGTHRYFLLSTQRRKRNKGWSKDEGT EAEIKKVMNGDYQTSYIEVKRGSKICEKSAWMLNLSIDVPKIDKGVDPSIIG GIAVGVRSPLVCAINNAFSRYSISDNDLFHFNKKMFARRRILLKKNRHKRA GHGAKNKLKPITILTEKSERFRKKLIERWACEIADFFIKNKVGTVQMENLES MKRKEDSYFNIRLRGFWPYAEMQNKIEFKLKQYGIEIRKVAPNNTSKTCSK CGHLNNYFNFEYRKKNKFPHFKCEKCNFKENAAYNAALNISNPKLKSTKE RP |
| cB3 | 100 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIASLSLLSNPAKQEMNVKRKISLLLSTQRRKRNKGWSKDEGTE AEIKKVMNGDYQTSYIEVKRGSKICEKSAWMLNLSIDVPKIDKGVDPSIIGG IAVGVRSPLVCAINNAFSRYSISDNDLFHFNKKMFARRRILLKKNRHKRAG HGAKNKLKPITILTEKSERFRKKLIERWACEIADFFIKNKVGTVQMENLESM KRKEDSYFNIRLRGFWPYAEMQNKIEFKLKQYGIEIRKVAPNNTSKTCSKC GHLNNYFNFEYRKKNKFPHFKCEKCNFKENAAYNAALNISNPKLKSTKERP |
| cD1 | 101 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPIYERKPNRSIVGGLAVGIRSPLVCAINNSFSRYSVDSNDVFKFSKQVFAF RRRLLSKNSLKRKGHGAAHKLEPITEMTEKNDKFRKKIIERWAKEVTNFFV KNQVGIVQIEDLSTMKDREDHFFNQYLRGFWPYYQMQTLIENKLKEYGIEV KRVQAKYTSQLCSNPNCRYWNNYFNFEYRKVNKFPKFKCEKCNLEISAAY NAARNLSTPDIEKFVAKATKGINLPEK |
| cD2 | 102 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPTHETVLDPDKVMGVALGVAKAVYWAFNSSYKRGCIDGGEIEHFRKMI RARRVSIQNQIKHSGDARKGHGRKRALKPIETLSEKEKNFRDTINHRYANRI VEAAIKQGCGTIQIENLEGIADTTGSKFLKNWPYYDLQTKIVNKAKEHGITV VAINPQYTSQRCSMCGYIEKTNRSSQAVFECKQCGYGSRTICINCRHVQVSG DVCEECGGIVKKENVNAAYNAAKNISTPYIDQIIMEKCLELGIPYRSITCKEC GHIQASGNTCEVCGSTNILKPKK |
| cD3 | 103 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPQTRVLDLNKIMGIALGVAVAVYMAFQHTPARYKLEGGEIENFRRQVE SRRISMLRQGKYAGGARGGHGRDKRIKPIEQLRDKIANFRDTTNHRYSRYI VDMAIKEGCGTIQMEDLTNIRDIGSRFLQNWTYYDLQQKIIYKAEEAGIKVI KIDPQYTSQRCSECGNIDSGNRIGQAIFKCRACGYEANAAYNAARNIAIPNI DKIIAESIK |
| cD4 | 104 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPIDIVNKKVSGRVVGVALGLKIPAYCALNDVEYIKKSIGRIDDFLKVRTQ MQSRRRRLQIAIQSAKGGKGRVNKLQALERFAEKEKNFAKTYNHFLSSNIV KFAVSNQAEQINMELLSLKETQNKSILRNWSYYQLQTMIEYKAQREGIKVK YIDPYHTSQTCSKCGNYEEGQRESQADFICKKCGYKVNAAYNAARNIAMS NKYITKKEESKYYKIKESMV |
| cD5 | 105 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVETKETALDPNNVMGVALGIVYPVYIAFNNSLHRYHIKGGEIERFRRQVE KRKRELLNQGKYCGDGRKGHGYATRTKSIESISDKIARFRDTCNHKYSRFIV DMALKHNCGIIQMEDLTGISKESTFLKNWTYYDLQQKIEYKAREAGIQVIKI EPQYTSQRCSKCGYIDKENRQEQATFKCIECGFKTNAAYNAARNIAIPNIDK IIRKTLKMQ |
| cD6 | 106 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVNRSIVGGLAVGIRSPLVCAINNSFSRYSVDSNDVFKFSKQVFA FRRRLLSKNSLKRKGHGAAHKLEPITEMTEKNDKFRKKIIERWAKEVTNFF VKNQVGIVQIEDLSTMKDREDHFFNQYLRGFWPYYQMQTLIENKLKEYGIE VKRVQAKYTSQLCSNPNCRYWNNYFNFEYRKVNKFPKFKCEKCNLEISAA YNAARNLSTPDIEKFVAKATKGINLPEK |
| cD7 | 107 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPDKVMGVALGVAKAVYWAFNSSYKRGCIDGGEIEHFRKMI RARRVSIQNQIKHSGDARKGHGRKRALKPIETLSEKEKNFRDTINHRYANRI VEAAIKQGCGTIQIENLEGIADTTGSKFLKNWPYYDLQTKIVNKAKEHGITV VAINPQYTSQRCSMCGYIEKTNRSSQAVFECKQCGYGSRTICINCRHVQVSG DVCEECGGIVKKENVNAAYNAAKNISTPYIDQIIMEKCLELGIPYRSITCKEC GHIQASGNTCEVCGSTNILKPKK |
| cD8 | 108 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDLNKIMGIALGVAVAVYMAFQHTPARYKLEGGEIENFRRQV ESRRISMLRQGKYAGGARGGHGRDKRIKPIEQLRDKIANFRDTTNHRYSRYI VDMAIKEGCGTIQMEDLTNIRDIGSRFLQNWTYYDLQQKIIYKAEEAGIKVI KIDPQYTSQRCSECGNIDSGNRIGQAIFKCRACGYEANAAYNAARNIAIPNI DKIIAESIK |
| cD9 | 109 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPNNVMGVALGIVYPVYIAFNNSLHRYHIKGGEIERFRRQVE KRKRELLNQGKYCGDGRKGHGYATRTKSIESISDKIARFRDTCNHKYSRFIV DMALKHNCGIIQMEDLTGISKESTFLKNWTYYDLQQKIEYKAREAGIQVIKI EPQYTSQRCSKCGYIDKENRQEQATFKCIECGFKTNAAYNAARNIAIPNIDK IIRKTLKMQ |
| cD10 | 110 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDKEKILGVAVGVNCPLVASVFGDRDRFIIKGGEIEKFRKSVEA RRRSMLEQTKYCGDGRIGHGRKKRTEPALNIGDKIARFRDTTNHKYSRALI EYAVKKGCGTIQMEKLTGITSKSDRFLKDWTYYDLQTKIENKAKEVGINVV YIAPKYTSQRCSKCGYIHKDNRPNQAKFRCLECDFESNAAYNASQNIGIKNI DKIIEKDLQKQESEVQVNENK |
| t1 | 111 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAELF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR RRILLKKNRHKRAGHGAKNKLKPITILTEKSERFRKKLIERWACEIADFFIKN KVGTVQMENLESMKRKEDSYFNIRLRGFWPYAEMQNKIEFKLKQYGIEIRK VAPNNTSKTCSKCGHLNNYFNFEYRKKNKFPHFKCEKCNFKENAAYNAAL NI |

**TABLE 5A. Engineered nuclease variants and their set of modification(s) relative to SEQ ID NO: 1 or SEQ ID NO: 10.**

| **Engineered nuclease variant** | **Set of non-limiting modifications relative to the amino acid sequence of Un1Cas12f1 (SEQ ID NO: 1) or dCasMINI (SEQ ID NO: 10), for enhanced activity as compared to dCasMINI (SEQ ID NO: 10)** |
|---|---|
| cA2 | deletions: amino acids 39-72; mutations: N32E, N33R, E35K, K36Q, I37A, A38G, K73G, A74T, R75G, K76E |
| t1 | deletions: amino acids 518-529 |
| mA8 | mutations: V23I, N33R, E40D, Q83K, and G87K |
| mA10 | mutations: A21Q, V23I, D29E, N33R, and E40D |
| mC16 | mutations: A340S, H353K, A374K, and I387E |
| mD2 | mutations: N423D, K473Q, T474L, K521D, and L522I |
| mD4 | mutations: K473Q, T474L, H497K, K521D, and L522I |
| mD7 | mutations: N423D, K473Q, T474L, H497K, N519T, K521D, and L522I |
| mD15 | mutations: N423D, K473Q, T474R, L515R, K521N, and L522I |
| additional variant 1 | mutations: E151A |
| additional variant 2 | mutations: N423D |
| additional variant 3 | mutations: K473Q, and T474L |
| additional variant 4 | mutations: K521D, and L522I |
| additional variant 5 | mutations: K473Q, T474L, K521D, and L522I |

**TABLE 5B. Engineered nuclease variants generated by grafting different combinations of modifications onto "cA2" starting sequence, along with their efficacy in enhancing target gene activity as compared to dCasMINI (SEQ ID NO: 10).**

| **Engineered nuclease variant** | **Combination of modifications, from TABLE 5A** | **Enhanced activity as compared to dCasMINI (SEQ ID NO: 10)?** | **Size (amino acids)** |
|---|---|---|---|
| cA2 | - | yes | 495 |
| cA2.1 | cA2, E151A, mC16, mD2 | yes | 495 |
| cA2.2 | cA2, E151A, mC16, mD4 | yes | 495 |
| cA2.3 | cA2, E151A, mC16, mD7 | yes | 495 |
| cA2.4 | cA2, E151A, mC16, mD15 | yes | 495 |
| cA2.5 | cA2, E151A, mC16 | yes | 495 |
| cA2.6 | cA2, E151A, mC16, mD2, t1 | yes | 483 |
| cA2.7 | cA2, E151A, mC16, mD4, t1 | yes | 483 |
| cA2.8 | cA2, E151A, mC16, mD7, t1 | yes | 483 |
| cA2.9 | cA2, E151A, mC16, mD15 ,t1 | yes | 483 |
| cA2.10 | cA2, E151A, mC16, t1 | yes | 483 |
| cA2.11 | cA2, E151A, mD2 | yes | 495 |
| cA2.12 | cA2, E151A, mD4 | yes | 495 |
| cA2.13 | cA2, E151A, mD7 | yes | 495 |
| cA2.14 | cA2, E151A, mD15 | yes | 495 |
| cA2.15 | cA2, E151A | yes | 495 |
| cA2.16 | cA2, E151A, mD2, t1 | yes | 483 |
| cA2.17 | cA2, E151A, mD4, t1 | yes | 483 |
| cA2.18 | cA2, E151A, mD7, t1 | yes | 483 |
| cA2.19 | cA2, E151A, mD15 ,t1 | yes | 483 |
| cA2.20 | cA2, E151A, t1 | yes | 483 |
| cA2.21 | cA2, mC16, mD2 | yes | 495 |
| cA2.22 | cA2, mC16, mD4 | yes | 495 |
| cA2.23 | cA2, mC16, mD7 | yes | 495 |
| cA2.24 | cA2, mC16, mD15 | yes | 495 |
| cA2.25 | cA2, mC16 | yes | 495 |
| cA2.26 | cA2, mC16, mD2, t1 | yes | 483 |
| cA2.27 | cA2, mC16, mD4, t1 | yes | 483 |
| cA2.28 | cA2, mC16, mD7, t1 | yes | 483 |
| cA2.29 | cA2, mC16, mD15 ,t1 | yes | 483 |
| cA2.30 | cA2, mC16, t1 | yes | 483 |
| cA2.31 | cA2, mD2 | yes | 495 |
| cA2.32 | cA2, mD4 | yes | 495 |
| cA2.33 | cA2, mD7 | yes | 495 |
| cA2.34 | cA2, mD15 | yes | 495 |
| cA2.36 | cA2, mD2, t1 | yes | 483 |
| cA2.37 | cA2, mD4, t1 | yes | 483 |
| cA2.38 | cA2, mD7, t1 | yes | 483 |
| cA2.39 | cA2, mD15, t1 | yes | 483 |
| cA2.40 | cA2, t1 | yes | 483 |
| cA2.41 | cA2, mA10, E151A, mC16, mD2 | yes | 495 |
| cA2.42 | cA2, mA10, E151A, mC16, mD4 | yes | 495 |
| cA2.43 | cA2, mA10, E151A, mC16, mD7 | yes | 495 |
| cA2.44 | cA2, mA10, E151A, mC16, mD15 | yes | 495 |
| cA2.45 | cA2, mA10, E151A, mC16 | yes | 495 |
| cA2.46 | cA2, mA10, E151A, mC16, mD2, t1 | yes | 483 |
| cA2.47 | cA2, mA10, E151A, mC16, mD4, t1 | yes | 483 |
| cA2.48 | cA2, mA10, E151A, mC16, mD7, t1 | yes | 483 |
| cA2.49 | cA2, mA10, E151A, mC16, mD15 ,t1 | yes | 483 |
| cA2.50 | cA2, mA10, E151A, mC16, t1 | yes | 483 |
| cA2.51 | cA2, mA10, E151A, mD2 | yes | 495 |
| cA2.52 | cA2, mA10, E151A, mD4 | yes | 495 |
| cA2.53 | cA2, mA10, E151A, mD7 | yes | 495 |
| cA2.54 | cA2, mA10, E151A, mD15 | yes | 495 |
| cA2.55 | cA2, mA10, E151A | no | 495 |
| cA2.56 | cA2, mA10, E151A, mD2, t1 | yes | 483 |
| cA2.57 | cA2, mA10, E151A, mD4, t1 | yes | 483 |
| cA2.58 | cA2, mA10, E151A, mD7, t1 | yes | 483 |
| cA2.59 | cA2, mA10, E151A, mD15 ,t1 | yes | 483 |
| cA2.60 | cA2, mA10, E151A, t1 | yes | 483 |
| cA2.61 | cA2, mA10, mC16, mD2 | yes | 495 |
| cA2.62 | cA2, mA10, mC16, mD4 | yes | 495 |
| cA2.63 | cA2, mA10, mC16, mD7 | yes | 495 |
| cA2.64 | cA2, mA10, mC16, mD15 | yes | 495 |
| cA2.65 | cA2, mA10, mC16 | yes | 495 |
| cA2.66 | cA2, mA10, mC16, mD2, t1 | yes | 483 |
| cA2.67 | cA2, mA10, mC16, mD4, t1 | yes | 483 |
| cA2.68 | cA2, mA10, mC16, mD7, t1 | yes | 483 |
| cA2.69 | cA2, mA10, mC16, mD15 ,t1 | yes | 483 |
| cA2.70 | cA2, mA10, mC16, t1 | yes | 483 |
| cA2.71 | cA2, mA10, mD2 | yes | 495 |
| cA2.72 | cA2, mA10, mD4 | yes | 495 |
| cA2.73 | cA2, mA10, mD7 | yes | 495 |
| cA2.74 | cA2, mA10, mD15 | yes | 495 |
| cA2.75 | cA2, mA10 | yes | 495 |
| cA2.76 | cA2, mA10, mD2, t1 | yes | 483 |
| cA2.77 | cA2, mA10, mD4, t1 | yes | 483 |
| cA2.78 | cA2, mA10, mD7, t1 | yes | 483 |
| cA2.79 | cA2, mA10, mD15 ,t1 | yes | 483 |
| cA2.80 | cA2, mA10, t1 | yes | 483 |
| cA2.81 | E151A, mC16, mD2 | yes | 529 |
| cA2.82 | E151A, mD2 | yes | 529 |
| cA2.83 | cA2, mA8, E151A, mD2 | yes | 495 |
| cA2.84 | cA2, mA8, E151A, mD4 | no | 495 |
| cA2.85 | cA2, mA8, mD2 | yes | 495 |
| cA2.86 | cA2, mA8, mD4 | yes | 495 |
| cA2.87 | cA2, N423D | yes | 495 |
| cA2.88 | cA2, K473Q, T474L | yes | 495 |
| cA2.89 | cA2, K521D, L522I | yes | 495 |
| cA2.90 | cA2, K473Q, T474L, K521D, L522I | yes | 495 |

**TABLE 5C. The amino acid sequence of each of the engineered nuclease variants listed in TABLE 5B**

| **Engineered nuclease variant** | **SEQ ID NO** | **Amino acid sequence** |
|---|---|---|
| cA2.1 | 112 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNISNPDIKSTKERP |
| cA2.2 | 113 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISNPDIKSTKERP |
| cA2.3 | 114 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISTPDIKSTKERP |
| cA2.4 | 115 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNISNPNIKSTKERP |
| cA2.5 | 116 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ |
| | | NKIEFKLKQYGIEIRKVAPNNTSKTCSKCGHLNNYFNFEYRKKNKFPHFKCE KCNFKENAAYNAALNISNPKLKSTKERP |
| cA2.6 | 117 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNI |
| cA2.7 | 118 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.8 | 119 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.9 | 120 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNI |
| cA2.10 | 121 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNI |
| cA2.11 | 122 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNISNPDIKSTKERP |
| cA2.12 | 123 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ |
| | | NKIEFKLKQYGIEIRKVAPNNTSQLCSKCGHLNNYFNFEYRKKNKFPKFKCE KCNFKENAAYNAALNISNPDIKSTKERP |
| cA2.13 | 124 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISTPDIKSTKERP |
| cA2.14 | 125 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNISNPNIKSTKERP |
| cA2.15 | 126 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNISNPKLKSTKERP |
| cA2.16 | 127 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNI |
| cA2.17 | 128 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.18 | 129 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.19 | 130 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ |
| | | NKIEFKLKQYGIEIRKVAPNNTSQRCSKCGHLNNYFNFEYRKKNKFPHFKC EKCNFKENAAYNAARNI |
| cA2.20 | 131 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNI |
| cA2.21 | 132 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNISNPDIKSTKERP |
| cA2.22 | 133 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISNPDIKSTKERP |
| cA2.23 | 134 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISTPDIKSTKERP |
| cA2.24 | 135 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNISNPNIKSTKERP |
| cA2.25 | 136 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNISNPKLKSTKERP |
| cA2.26 | 137 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ |
| | | NKIEFKLKQYGIEIRKVAPNNTSQLCSKCGHLNNYFNFEYRKKNKFPHFKCE KCNFKENAAYNAALNI |
| cA2.27 | 138 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.28 | 139 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.29 | 140 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNI |
| cA2.30 | 141 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNI |
| cA2.31 | 142 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNISNPDIKSTKERP |
| cA2.32 | 143 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISNPDIKSTKERP |
| cA2.33 | 144 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ |
| | | NKIEFKLKQYGIEIRKVAPNNTSQLCSKCGHLNNYFNFEYRKKNKFPKFKCE KCNFKENAAYNAALNISTPDIKSTKERP |
| cA2.34 | 145 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNISNPNIKSTKERP |
| cA2.35 | 146 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNISNPKLKSTKERP |
| cA2.36 | 147 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNI |
| cA2.37 | 148 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.38 | 149 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.39 | 150 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNI |
| cA2.40 | 151 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ |
| | | NKIEFKLKQYGIEIRKVAPNNTSKTCSKCGHLNNYFNFEYRKKNKFPHFKCE KCNFKENAAYNAALNI |
| cA2.41 | 152 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNISNPDIKSTKERP |
| cA2.42 | 153 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISNPDIKSTKERP |
| cA2.43 | 154 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISTPDIKSTKERP |
| cA2.44 | 155 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNISNPNIKSTKERP |
| cA2.45 | 156 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNISNPKLKSTKERP |
| cA2.46 | 157 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNI |
| cA2.47 | 158 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ |
| | | NKIEFKLKQYGIEIRKVAPNNTSQLCSKCGHLNNYFNFEYRKKNKFPKFKCE KCNFKENAAYNAALNI |
| cA2.48 | 159 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.49 | 160 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNI |
| cA2.50 | 161 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNI |
| cA2.51 | 162 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNISNPDIKSTKERP |
| cA2.52 | 163 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISNPDIKSTKERP |
| cA2.53 | 164 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISTPDIKSTKERP |
| cA2.54 | 165 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ |
| | | NKIEFKLKQYGIEIRKVAPNNTSQRCSKCGHLNNYFNFEYRKKNKFPHFKC EKCNFKENAAYNAARNISNPNIKSTKERP |
| cA2.55 | 166 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNISNPKLKSTKERP |
| cA2.56 | 167 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNI |
| cA2.57 | 168 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.58 | 169 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.59 | 170 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNI |
| cA2.60 | 171 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNI |
| cA2.61 | 172 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ |
| | | NKIEFKLKQYGIEIRKVAPNNTSQLCSKCGHLNNYFNFEYRKKNKFPHFKCE KCNFKENAAYNAALNISNPDIKSTKERP |
| cA2.62 | 173 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISNPDIKSTKERP |
| cA2.63 | 174 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISTPDIKSTKERP |
| cA2.64 | 175 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNISNPNIKSTKERP |
| cA2.65 | 176 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNISNPKLKSTKERP |
| cA2.66 | 177 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNI |
| cA2.67 | 178 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.68 | 179 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ |
| | | NKIEFKLKQYGIEIRKVAPNNTSQLCSKCGHLNNYFNFEYRKKNKFPKFKCE KCNFKENAAYNAALNI |
| cA2.69 | 180 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNI |
| cA2.70 | 181 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNSFSRYSIS DNDLFKFNKKMFARRRILLKKNRHKRKGHGAKNKLKPITELTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNI |
| cA2.71 | 182 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNISNPDIKSTKERP |
| cA2.72 | 183 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISNPDIKSTKERP |
| cA2.73 | 184 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISTPDIKSTKERP |
| cA2.74 | 185 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNISNPNIKSTKERP |
| cA2.75 | 186 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ |
| | | NKIEFKLKQYGIEIRKVAPNNTSKTCSKCGHLNNYFNFEYRKKNKFPHFKCE KCNFKENAAYNAALNISNPKLKSTKERP |
| cA2.76 | 187 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNI |
| cA2.77 | 188 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.78 | 189 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNI |
| cA2.79 | 190 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQRCSKCGHLNNYFNFEYRKKNKFPHFKC EKCNFKENAAYNAARNI |
| cA2.80 | 191 | MAKNTITKTLKLRIVRPYNSQEIEKIVAEEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNI |
| cA2.81 | 192 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAAALF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNSFSRYSISDNDLFKFNKKMFAR RRILLKKNRHKRKGHGAKNKLKPITELTEKSERFRKKLIERWACEIADFFIK NKVGTVQMEDLESMKRKEDSYFNIRLRGFWPYAEMQNKIEFKLKQYGIEIR KVAPNNTSQLCSKCGHLNNYFNFEYRKKNKFPHFKCEKCNFKENAAYNAA LNISNPDIKSTKERP |
| cA2.82 | 193 | MAKNTITKTLKLRIVRPYNSAEVEKIVADEKNNREKIALEKNKDKVKEACS KHLKVAAYCTTQVERNACLFCKARKLDDKFYQKLRGQFPDAVFWQEISEI FRQLQKQAAEIYNQSLIELYYEIFIKGKGIANASSVEHYLSRVCYRRAALF KNAAIASGLRSKIKSNFRLKELKNMKSGLPTTKSDNFPIPLVKQKGGQYTGF EISNHNSDFIIKIPFGRWQVKKEIDKYRPWEKFDFEQVQKSPKPISLLLSTQR RKRNKGWSKDEGTEAEIKKVMNGDYQTSYIEVKRGSKICEKSAWMLNLSI DVPKIDKGVDPSIIGGIAVGVRSPLVCAINNAFSRYSISDNDLFHFNKKMFAR |
| | | RRILLKKNRHKRAGHGAKNKLKPITILTEKSERFRKKLIERWACEIADFFIKN KVGTVQMEDLESMKRKEDSYFNIRLRGFWPYAEMQNKIEFKLKQYGIEIRK VAPNNTSQLCSKCGHLNNYFNFEYRKKNKFPHFKCEKCNFKENAAYNAAL NISNPDIKSTKERP |
| cA2.83 | 194 | MAKNTITKTLKLRIVRPYNSAEIEKIVADEKERRKQAGGTGELDDKFYKKL RKQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNISNPDIKSTKERP |
| cA2.84 | 195 | MAKNTITKTLKLRIVRPYNSAEIEKIVADEKERRKQAGGTGELDDKFYKKL RKQFPDAVFWQEISEIFRQLQKQAAEIYNQSLIELYYEIFIKGKGIANASSVE HYLSRVCYRRAAALFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISNPDIKSTKERP |
| cA2.85 | 196 | MAKNTITKTLKLRIVRPYNSAEIEKIVADEKERRKQAGGTGELDDKFYKKL RKQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNISNPDIKSTKERP |
| cA2.86 | 197 | MAKNTITKTLKLRIVRPYNSAEIEKIVADEKERRKQAGGTGELDDKFYKKL RKQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPKFKCE KCNFKENAAYNAALNISNPDIKSTKERP |
| cA2.87 | 198 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMEDLESMKRKEDSYFNIRLRGFWPYAEMQ NKIEFKLKQYGIEIRKVAPNNTSKTCSKCGHLNNYFNFEYRKKNKFPHFKCE KCNFKENAAYNAALNISNPKLKSTKERP |
| cA2.88 | 199 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNISNPKLKSTKERP |
| cA2.89 | 200 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ |
| | | NKIEFKLKQYGIEIRKVAPNNTSKTCSKCGHLNNYFNFEYRKKNKFPHFKCE KCNFKENAAYNAALNISNPDIKSTKERP |
| cA2.90 | 201 | MAKNTITKTLKLRIVRPYNSAEVEKIVADEKERRKQAGGTGELDDKFYQKL RGQFPDAVFWQEISEIFRQLQKQAAEIYNQSLIELYYEIFIKGKGIANASSVE HYLSRVCYRRAAELFKNAAIASGLRSKIKSNFRLKELKNMKSGLPTTKSDN FPIPLVKQKGGQYTGFEISNHNSDFIIKIPFGRWQVKKEIDKYRPWEKFDFEQ VQKSPKPISLLLSTQRRKRNKGWSKDEGTEAEIKKVMNGDYQTSYIEVKRG SKICEKSAWMLNLSIDVPKIDKGVDPSIIGGIAVGVRSPLVCAINNAFSRYSIS DNDLFHFNKKMFARRRILLKKNRHKRAGHGAKNKLKPITILTEKSERFRKK LIERWACEIADFFIKNKVGTVQMENLESMKRKEDSYFNIRLRGFWPYAEMQ NKIEFKLKQYGIEIRKVAPNNTSQLCSKCGHLNNYFNFEYRKKNKFPHFKCE KCNFKENAAYNAALNISNPDIKSTKERP |

**TABLE 6A. List of gRNA scaffold variants from first round. The number of asterisks represent the relative degree of gene modulation activity of the gRNA scaffold variants.**

| **Guide NA scaffold (first round)** | **Length w/o spacer (bp)** | **Suppression of GFP (5 d.p.t.)** | **Comparable or improved activity to SQ w/ reduced size** |
|---|---|---|---|
| SQ (positive control) | 159 | *** | |
| 2 | 139 | - | |
| 3 | 138 | * | |
| 4 | 137 | *** | yes |
| 5 | 136 | *** | yes |
| 6 | 151 | *** | yes |
| 7 | 148 | *** | yes |
| 8 | 157 | *** | yes |
| 9 | 153 | *** | yes |
| 10 | 153 | *** | yes |
| 11 | 151 | *** | yes |
| 12 | 153 | *** | yes |
| 13 | 149 | *** | yes |
| 14 | 132 | * | |
| 15 | 132 | * | |
| 16 | 131 | * | |
| 17 | 134 | * | |
| 18 | 134 | * | |
| 19 | 136 | ** | |
| 20 | 135 | *** | yes |
| 21 | 133 | * | |
| 22 | 131 | * | |
| 23 | 129 | * | |
| 24 | 126 | ** | |
| 25 | 124 | *** | yes |
| 26 | 122 | - | |
| 27 | 120 | * | |
| 28 | 118 | ** | |
| 29 | 116 | *** | yes |
| 30 | 114 | - | |
| 31 | 161 | - | |
| 32 | 164 | - | |
| 33 | 164 | ** | |
| 34 | 169 | ** | |
| 35 | 140 | * | |
| 36 | 149 | ** | |
| 37 | 142 | * | |
| 38 | 140 | - | |
| 39 | 122 | ** | |
| 40 | 120 | * | |
| 41 | 100 | ** | |
| 42 | 98 | - | |
| 43 | 110 | ** | |
| 44 | 108 | * | |
| 45 | 104 | *** | yes |
| 46 | 109 | ** | |

**TABLE 6B. List of gRNA scaffold variants and the respective polynucleotide sequences from TABLE 6A.**

| **Guide NA scaffold** | **Position relative to spacer** | **SEQ ID NO** | **Guide nucleic acid (NA) scaffold sequence (without spacer)** |
|---|---|---|---|
| SQ | 5' of spacer | 500 | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAATTCATTTGAATGAAGGAATGCAAC |
| 2 | 5' of spacer | 501 | GAACCGCTTCACCAAAAGCTGTCCCTTAGGGGATTAGAACTTG AGTGAAGGTGGGCTGCTTGCATCAGCCTAATGTCGAGAAGTGC TTTCTTCGGAAAGTAACCCTCGAAACAAATTCATTTGAATGAA GGAATGCAAC |
| 3 | 5' of spacer | 502 | AACCGCTTCACCAAAAGCTGTCCCTTAGGGGATTAGAACTTGA GTGAAGGTGGGCTGCTTGCATCAGCCTAATGTCGAGAAGTGCT TTCTTCGGAAAGTAACCCTCGAAACAAATTCATTTGAATGAAG GAATGCAAC |
| 4 | 5' of spacer | 503 | ACCGCTTCACCAAAAGCTGTCCCTTAGGGGATTAGAACTTGAG TGAAGGTGGGCTGCTTGCATCAGCCTAATGTCGAGAAGTGCTT TCTTCGGAAAGTAACCCTCGAAACAAATTCATTTGAATGAAGG AATGCAAC |
| 5 | 5' of spacer | 504 | CCGCTTCACCAAAAGCTGTCCCTTAGGGGATTAGAACTTGAGT GAAGGTGGGCTGCTTGCATCAGCCTAATGTCGAGAAGTGCTTT CTTCGGAAAGTAACCCTCGAAACAAATTCATTTGAATGAAGGA ATGCAAC |
| 6 | 5' of spacer | *505* | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAGCAATAAGGAATGCAAC |
| 7 | 5' of spacer | 506 | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAGAAAGGAATGCAAC |
| 8 | 5' of spacer | 507 | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAATCTTCGGATTAAGGAATGCAAC |
| 9 | 5' of spacer | 508 | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAATTGCAAAAGGAATGCAAC |
| 10 | 5' of spacer | 509 | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAATTCGTTAAGGAATGCAAC |
| 11 | 5' of spacer | 510 | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAATGCAAAGGAATGCAAC |
| 12 | 5' of spacer | 511 | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAAGCAATTAAGGAATGCAAC |
| 13 | 5' of spacer | 512 | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAAGAAAGGAATGCAAC |
| 14 | 5' of spacer | 513 | GGCTTCACTGATAAAGTGGAGAACCGCTTCACTTAGAGTGAAG GTGGGCTGCTTGCATCAGCCTAATGTCGAGAAGTGCTTTCTTCG GAAAGTAACCCTCGAAACAAATTCATTTGAATGAAGGAATGCA AC |
| 15 | 5' of spacer | 514 | GGCTTCACTGATAAAGTGGAGAACCGCTTCACTTCGAGTGAAG GTGGGCTGCTTGCATCAGCCTAATGTCGAGAAGTGCTTTCTTCG GAAAGTAACCCTCGAAACAAATTCATTTGAATGAAGGAATGCA AC |
| 16 | 5' of spacer | *515* | GGCTTCACTGATAAAGTGGAGAACCGCTTCACTTCGGTGAAGG TGGGCTGCTTGCATCAGCCTAATGTCGAGAAGTGCTTTCTTCGG AAAGTAACCCTCGAAACAAATTCATTTGAATGAAGGAATGCAA C |
| 17 | 5' of spacer | 516 | GGCTTCACTGATAAAGTGGAGAACCGCTTCACCTTAGGAGTGA AGGTGGGCTGCTTGCATCAGCCTAATGTCGAGAAGTGCTTTCT TCGGAAAGTAACCCTCGAAACAAATTCATTTGAATGAAGGAAT GCAAC |
| 18 | 5' of spacer | 517 | GGCTTCACTGATAAAGTGGAGAACCGCTTCACCTTCGGAGTGA AGGTGGGCTGCTTGCATCAGCCTAATGTCGAGAAGTGCTTTCT TCGGAAAGTAACCCTCGAAACAAATTCATTTGAATGAAGGAAT GCAAC |
| 19 | 5' of spacer | 518 | GGCTTCACTGATAAAGTGGAGAACCGCTTCACGCTTCGGCAGT GAAGGTGGGCTGCTTGCATCAGCCTAATGTCGAGAAGTGCTTT CTTCGGAAAGTAACCCTCGAAACAAATTCATTTGAATGAAGGA ATGCAAC |
| 20 | 5' of spacer | 519 | ACCGCTTCACCAAAAGCTGTCCTTAGGGATTAGAACTTGAGTG AAGGTGGGCTGCTTGCATCAGCCTAATGTCGAGAAGTGCTTTC TTCGGAAAGTAACCCTCGAAACAAATTCATTTGAATGAAGGAA TGCAAC |
| 21 | 5' of spacer | 520 | ACCGCTTCACCAAAAGCTGTCTTAGGATTAGAACTTGAGTGAA GGTGGGCTGCTTGCATCAGCCTAATGTCGAGAAGTGCTTTCTTC GGAAAGTAACCCTCGAAACAAATTCATTTGAATGAAGGAATGC AAC |
| 22 | 5' of spacer | 521 | ACCGCTTCACCAAAAGCTGTTTAGATTAGAACTTGAGTGAAGG TGGGCTGCTTGCATCAGCCTAATGTCGAGAAGTGCTTTCTTCGG AAAGTAACCCTCGAAACAAATTCATTTGAATGAAGGAATGCAA C |
| 23 | 5' of spacer | 522 | ACCGCTTCACCAAAAGCTGTTAGTTAGAACTTGAGTGAAGGTG GGCTGCTTGCATCAGCCTAATGTCGAGAAGTGCTTTCTTCGGA AAGTAACCCTCGAAACAAATTCATTTGAATGAAGGAATGCAAC |
| 24 | 5' of spacer | 523 | ACCGCTTCACCAAAAGCTTTAGAGAACTTGAGTGAAGGTGGGC TGCTTGCATCAGCCTAATGTCGAGAAGTGCTTTCTTCGGAAAG TAACCCTCGAAACAAATTCATTTGAATGAAGGAATGCAAC |
| 25 | 5' of spacer | 524 | ACCGCTTCACCAAAAGCTTCGGCACTTGAGTGAAGGTGGGCTG CTTGCATCAGCCTAATGTCGAGAAGTGCTTTCTTCGGAAAGTA ACCCTCGAAACAAATTCATTTGAATGAAGGAATGCAAC |
| 26 | 5' of spacer | 525 | ACCGCTTCACCAAAAGTTCGCACTTGAGTGAAGGTGGGCTGCT TGCATCAGCCTAATGTCGAGAAGTGCTTTCTTCGGAAAGTAAC CCTCGAAACAAATTCATTTGAATGAAGGAATGCAAC |
| 27 | 5' of spacer | 526 | ACCGCTTCACCAAAATTCGTCTTGAGTGAAGGTGGCTGCTTG CATCAGCCTAATGTCGAGAAGTGCTTTCTTCGGAAAGTAACCC TCGAAACAAATTCATTTGAATGAAGGAATGCAAC |
| 28 | 5' of spacer | 527 | ACCGCTTCACCAAGTTCGCTTGAGTGAAGGTGGGCTGCTTGCA TCAGCCTAATGTCGAGAAGTGCTTTCTTCGGAAAGTAACCCTC GAAACAAATTCATTTGAATGAAGGAATGCAAC |
| 29 | 5' of spacer | 528 | ACCGCTTCACCAATTCGTTGAGTGAAGGTGGGCTGCTTGCATC AGCCTAATGTCGAGAAGTGCTTTCTTCGGAAAGTAACCCTCGA AACAAATTCATTTGAATGAAGGAATGCAAC |
| 30 | 5' of spacer | 529 | ACCGCTTCACCATTCGTGAGTGAAGGTGGGCTGCTTGCATCAG CCTAATGTCGAGAAGTGCTTTCTTCGGAAAGTAACCCTCGAAA CAAATTCATTTGAATGAAGGAATGCAAC |
| 31 | 5' of spacer | 530 | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAATTCATTTGAATGAAGGAATGCAAC |
| | 3' of spacer | 531 | TT |
| 32 | 5' of spacer | 532 | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAATTCATTTGAATGAAGGAATGCAAC |
| | 3' of spacer | 533 | TTTTA |
| 33 | 5' of spacer | 534 | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAATTCATTTGAATGAAGGAATGCAAC |
| | 3' of spacer | 535 | TTTTG |
| 34 | 5' of spacer | 536 | GGCTTCACTGATAAAGTGGAGAACCGCTTCACCAAAAGCTGTC CCTTAGGGGATTAGAACTTGAGTGAAGGTGGGCTGCTTGCATC AGCCTAATGTCGAGAAGTGCTTTCTTCGGAAAGTAACCCTCGA AACAAATTCATTTGAATGAAGGAATGCAAC |
| | 3' of spacer | 537 | TTTTATTTTT |
| 35 | 5' of spacer | 538 | GGCTTCACTGATAAAGTGGAGAACCGCTTCACCGAGTGAAGGT GGGCTGCTTGCATCAGCCTAATGTCGAGAAGTGCTTTCTTCGG AAAGTAACCCTCGAAACAAATTCATTTGAATGAAGGAATGCAA C |
| | 3' of spacer | 539 | TTTTATTTTT |
| 36 | 5' of spacer | 540 | GGCTTCACTGATAAAGTGGAGAACCGCTTCACCAAAAGCTGTC CCTTAGGGGATTAGAACTTGAGTGAAGGTGGGCTGCTTGCATC |
| | | | AGCCTAATGTCGAGAAGTGCTTTCTTCGGAAAGTAACCCTCGA AACAAAGAAAGGAATGCAAC |
| 37 | 5' of spacer | 541 | GGCTTCACTGATAAAGTGGAGAACCGCTTCACTTAGAGTGAAG GTGGGCTGCTTGCATCAGCCTAATGTCGAGAAGTGCTTTCTTCG GAAAGTAACCCTCGAAACAAATTCATTTGAATGAAGGAATGCA AC |
| | 3' of spacer | 542 | TTTTATTTTT |
| 38 | 5' of spacer | 543 | GGCTTCACTGATAAAGTGGAGAACCGCTTCACCGAGTGAAGGT GGGCTGCTTGCATCAGCCTAATGTCGAGAAGTGCTTTCTTCGG AAAGTAACCCTCGAAACAAATTCATTTGAATGAAGGAATGCAA C |
| | 3' of spacer | 544 | TTTTATTTTT |
| 39 | 5' of spacer | 545 | GGCTTCACTGATAAAGTGGAGAACCGCTTCACTTAGAGTGAAG GTGGGCTGCTTGCATCAGCCTAATGTCGAGAAGTGCTTTCTTCG GAAAGTAACCCTCGAAACAAAGAAAGGAATGCAAC |
| 40 | 5' of spacer | 546 | GGCTTCACTGATAAAGTGGAGAACCGCTTCACCGAGTGAAGGT GGGCTGCTTGCATCAGCCTAATGTCGAGAAGTGCTTTCTTCGG AAAGTAACCCTCGAAACAAAGAAAGGAATGCAAC |
| 41 | 5' of spacer | 547 | ACCGCTTCACTTAGAGTGAAGGTGGGCTGCTTGCATCAGCCTA ATGTCGAGAAGTGCTTTCTTCGGAAAGTAACCCTCGAAACAAA GAAAGGAATGCAAC |
| 42 | 5' of spacer | 548 | ACCGCTTCACCGAGTGAAGGTGGGCTGCTTGCATCAGCCTAAT GTCGAGAAGTGCTTTCTTCGGAAAGTAACCCTCGAAACAAAGA AAGGAATGCAAC |
| 43 | 5' of spacer | 549 | ACCGCTTCACTTAGAGTGAAGGTGGGCTGCTTGCATCAGCCTA ATGTCGAGAAGTGCTTTCTTCGGAAAGTAACCCTCGAAACAAA GAAAGGAATGCAAC |
| | 3' of spacer | *550* | TTTTATTTTT |
| 44 | 5' of spacer | *551* | ACCGCTTCACCGAGTGAAGGTGGGCTGCTTGCATCAGCCTAAT GTCGAGAAGTGCTTTCTTCGGAAAGTAACCCTCGAAACAAAGA AAGGAATGCAAC |
| | 3' of spacer | 552 | TTTTATTTTT |
| 45 | 5' of spacer | 553 | ACCGCTTCACGCTTCGGCAGTGAAGGTGGGCTGCTTGCATCAG CCTAATGTCGAGAAGTGCTTTCTTCGGAAAGTAACCCTCGAAA CAAAGAAAGGAATGCAAC |
| 46 | 5' of spacer | 554 | ACCGCTTCACGCTTCGGCAGTGAAGGTGGGCTGCTTGCATCAG CCTAATGTCGAGAAGTGCTTTCTTCGGAAAGTAACCCTCGAAA CAAAGAAAGGAATGCAAC |
| | 3' of spacer | 602 | TTTTA |

**TABLE 7A. List of gRNA scaffold variants from second round. The number of asterisks represent the relative degree of gene modulation activity of the gRNA scaffold variants.**

| **Guide NA scaffold (second round)** | **Length w/o spacer (bp)** | **Suppression of GFP (5 d.p.t.)** | **Comparable or improved activity to SQ w/ reduced size** |
|---|---|---|---|
| SQ (positive control) | 159 | *** | |
| 45.1 | 103 | *** | yes |
| 2-5 | 103 | ** | |
| 2-6 | 103 | *** | yes |
| 2-7 | 103 | *** | yes |
| 2-8 | 104 | ** | |
| 2-9 | 105 | * | |
| 2-10 | 102 | ** | |
| 2-11 | 101 | - | |
| 2-12 | 102 | * | |
| 2-13 | 111 | - | |
| 2-14 | 112 | * | |
| 2-15 | 102 | ** | |
| 2-16 | 101 | ** | |
| 2-17 | 100 | *** | yes |
| 2-18 | 99 | *** | yes |
| 2-19 | 98 | ** | |
| 2-20 | 97 | ** | |
| 2-21 | 96 | - | |
| 2-22 | 95 | - | |
| 2-23 | 94 | - | |
| 2-24 | 93 | - | |
| 2-25 | 97 | ** | |
| 2-26 | 99 | ** | |
| 2-27 | 98 | *** | yes |
| 2-28 | 102 | * | |
| 2-29 | 100 | *** | yes |
| 2-30 | 99 | - | |
| 2-31 | 96 | ** | |
| 2-32 | 95 | - | |
| 2-33 | 103 | ** | |
| 2-34 | 103 | - | |
| 2-35 | 103 | - | |
| 2-36 | 97 | - | |
| 2-37 | 97 | * | |

**TABLE 7B. List of gRNA scaffold variants and the respective polynucleotide sequences from TABLE 7A.**

| **Guide NA scaffold** | **Position relative to spacer** | **SEQ ID NO** | **Guide nucleic acid (NA) scaffold sequence (without spacer)** |
|---|---|---|---|
| 45.1 | 5' of spacer | *555* | CCGCTTCACGCTTCGGCAGTGAAGGTGGGCTGCTTGCATCAGC CTAATGTCGAGAAGTGCTTTCTTCGGAAAGTAACCCTCGAAAC AAAGAAAGGAATGCAAC |
| 2-5 | 5' of spacer | 556 | CCGCTTCACGCTTAGGCAGTGAAGGTGGGCTGCTTGCATCAGC CTAATGTCGAGAAGTGCTTTCTTCGGAAAGTAACCCTCGAAAC AAAGAAAGGAATGCAAC |
| 2-6 | 5' of spacer | 557 | CCGCTTCACTCTTAGGAAGTGAAGGTGGGCTGCTTGCATCAGC CTAATGTCGAGAAGTGCTTTCTTCGGAAAGTAACCCTCGAAAC AAAGAAAGGAATGCAAC |
| 2-7 | 5' of spacer | 558 | CCGCTTCACGTTTAGACAGTGAAGGTGGGCTGCTTGCATCAGC CTAATGTCGAGAAGTGCTTTCTTCGGAAAGTAACCCTCGAAAC AAAGAAAGGAATGCAAC |
| 2-8 | 5' of spacer | 559 | GCCGCTTCACGCTTCGGCAGTGAAGGTGGGCTGCTTGCATCAG CCTAATGTCGAGAAGTGCTTTCTTCGGAAAGTAACCCTCGAAA CAAAGAAAGGAATGCAAC |
| 2-9 | 5' of spacer | 560 | GGCCGCTTCACGCTTCGGCAGTGAAGGTGGGCTGCTTGCATCA GCCTAATGTCGAGAAGTGCTTTCTTCGGAAAGTAACCCTCGAA ACAAAGAAAGGAATGCAAC |
| 2-10 | 5' of spacer | 561 | CGCTTCACGCTTCGGCAGTGAAGGTGGGCTGCTTGCATCAGCC TAATGTCGAGAAGTGCTTTCTTCGGAAAGTAACCCTCGAAACA AAGAAAGGAATGCAAC |
| 2-11 | 5' of spacer | 562 | GCTTCACGCTTCGGCAGTGAAGGTGGGCTGCTTGCATCAGCCT AATGTCGAGAAGTGCTTTCTTCGGAAAGTAACCCTCGAAACAA AGAAAGGAATGCAAC |
| 2-12 | 5' of spacer | 563 | GGCTTCACGCTTCGGCAGTGAAGGTGGGCTGCTTGCATCAGCC TAATGTCGAGAAGTGCTTTCTTCGGAAAGTAACCCTCGAAACA AAGAAAGGAATGCAAC |
| 2-13 | 5' of spacer | 564 | GCTTCACGCTTCGGCAGTGAAGGTGGGCTGCTTGCATCAGCCT AATGTCGAGAAGTGCTTTCTTCGGAAAGTAACCCTCGAAACAA AGAAAGGAATGCAAC |
| | 3' of spacer | | TTTTATTTTT |
| 2-14 | 5' of spacer | 565 | GGCTTCACGCTTCGGCAGTGAAGGTGGGCTGCTTGCATCAGCC TAATGTCGAGAAGTGCTTTCTTCGGAAAGTAACCCTCGAAACA AAGAAAGGAATGCAAC |
| | 3' of spacer | | TTTTATTTTT |
| 2-15 | 5' of spacer | 566 | CCGCTTCACGCTTCGGCAGTGAAGGTGGGCTGCTTGCATCAGC CTAATGTCGAGAAGTGCTTTCTTCGGAAAGTAACCCTCGAAAC AAAGAAGGAATGCAAC |
| 2-16 | 5' of spacer | 567 | CCGCTTCACGCTTCGGCAGTGAAGGTGGGCTGCTTGCATCAGC CTAATGTCGAGAAGTGCTTTCTTCGGAAAGTAACCCTCGAAAC AAAGAGGAATGCAAC |
| 2-17 | 5' of spacer | 568 | CCGCTTCACGCTTCGGCAGTGAAGGTGGGCTGCTTGCATCAGC CTAATGTCGAGAAGTGCTTTCTTCGGAAAGTAACCCTCGAAAC AAAGGGAATGCAAC |
| 2-18 | 5' of spacer | 569 | CCGCTTCACGCTTCGGCAGTGAAGGTGGGCTGCTTGCATCAGC CTAATGTCGAGAAGTGCTTTCTTCGGAAAGTAACCCTCGAAAC AAAGGAATGCAAC |
| 2-19 | 5' of spacer | 570 | CCGCTTCACGCTTCGGCAGTGAAGGTGGGCTGCTTGCATCAGC CTAATGTCGAGAAGTGCTTTCTTCGGAAAGTAACCCTCGAAAC AAGGAATGCAAC |
| 2-20 | 5' of spacer | 571 | CCGCTTCACGCTTCGGCAGTGAAGGTGGGCTGCTTGCATCAGC CTAATGTCGAGAAGTGCTTTCTTCGGAAAGTAACCCTCGAAAC AGGAATGCAAC |
| 2-21 | 5' of spacer | 572 | CCGCTTCACGCTTCGGCAGTGAAGGTGGGCTGCTTGCATCAGC CTAATGTCGAGAAGTGCTTTCTTCGGAAAGTAACCCTCGAAAC GGAATGCAAC |
| 2-22 | 5' of spacer | 573 | CCGCTTCACGCTTCGGCAGTGAAGGTGGGCTGCTTGCATCAGC CTAATGTCGAGAAGTGCTTTCTTCGGAAAGTAACCCTCGAAAG GAATGCAAC |
| 2-23 | 5' of spacer | 574 | CCGCTTCACGCTTCGGCAGTGAAGGTGGGCTGCTTGCATCAGC CTAATGTCGAGAAGTGCTTTCTTCGGAAAGTAACCCTCGAAGG AATGCAAC |
| 2-24 | 5' of spacer | 575 | CCGCTTCACGCTTCGGCAGTGAAGGTGGGCTGCTTGCATCAGC CTAATGTCGAGAAGTGCTTTCTTCGGAAAGTAACCCTCGAGGA ATGCAAC |
| 2-25 | 5' of spacer | 576 | CCGCTTCACGCTTCGGCAGTGAAGGTGGGCTGCTTGCATCAGC CTAATGTCGAGAAGTGCTTTCTTCGGAAAGTAACCCTCGAAAC AAGAATGCAAC |
| 2-26 | 5' of spacer | 577 | CCGCTTCACGCTTCGGCAGTGAAGGTGGGCTGCTTGCATCAGC CTAATGTCGAGAAGTGCTTTCTTCGGAAAGTAACCCTCGAAAC AAAGGAATGCAAC |
| 2-27 | 5' of spacer | 578 | CCGCTTCACGCTTCGGCAGTGAAGGTGGGCTGCTTGCATCAGC CTAATGTCGAGAAGTGCTTTCTTCGGAAAGTAACCCTCGAAAC AAAGAATGCAAC |
| 2-28 | 5' of spacer | 579 | CCGCTTCACGCTTCGGCAGTGAAGGTGGGCTGCTTGCATCAGC CTAATGTCGAGAAGTGCTTTCTTCGGAAAGTAACCCTCGAAAC AAAGAAAGAATGCAAC |
| 2-29 | 5' of spacer | 580 | CCGCTTCACGCTTCGGCAGTGAAGGTGGGCTGCTTGCATCAGC CTAATGTCGAGAAGTGCTTTCTTCGGAAAGTAACCCTCGAAAC AAAGAGAATGCAAC |
| 2-30 | 5' of spacer | 581 | CCGCTTCACGCTTCGGCAGTGAAGGTGGGCTGCTTGCATCAGC CTAATGTCGAGAAGTGCTTTCTTCGGAAAGTAACCCTCGAAAC AAAGAAATGCAAC |
| 2-31 | 5' of spacer | 582 | CCGCTTCACGCTTCGGCAGTGAAGGTGGGCTGCTTGCATCAGC CTAATGTCGAGAAGTGCTTTCTTCGGAAAGTAACCCTCGAAAC AGAATGCAAC |
| 2-32 | 5' of spacer | 583 | CCGCTTCACGCTTCGGCAGTGAAGGTGGGCTGCTTGCATCAGC CTAATGTCGAGAAGTGCTTTCTTCGGAAAGTAACCCTCGAAAC AAATGCAAC |
| 2-33 | 5' of spacer | 584 | CCGCTTCACGCTTCGGCAGTGAAGGTGGGCTGATTGCATCAGC CTAATGTCGAGAAGTGCTTTCTTCGGAAAGTAACCCTCGAAAC AAAGAAAGGAATGCAAC |
| 2-34 | 5' of spacer | 585 | CCGCTTCACGCTTCGGCAGTGAAGGTAGGCTGCTTGCATCAGC CTAATGTCGAGAAGTGCTTTCTTCGGAAAGTAACCCTCGAAAC AAAGAAAGGAATGCAAC |
| 2-35 | 5' of spacer | 586 | CCGCTTCACGCTTCGGCAGTGAAGGTGGGCTGCTTGCATCAGC CCAATGTCGAGAAGTGCTTTCTTCGGAAAGTAACCCTCGAAAC AAAGAAAGGAATGCAAC |
| 2-36 | 5' of spacer | 587 | GGCTTCACGCTTCGGCAGTGAAGGTAGGCTGCTTGCATCAGCC TAATGTCGAGAAGTGCTTTCTTCGGAAAGTAACCCTCGAAACA AGGAATGCAAC |
| 2-37 | 5' of spacer | 588 | GGCTTCACGCTTCGGCAGTGAAGGTGGGCTGCTTGCATCAGCC CAATGTCGAGAAGTGCTTTCTTCGGAAAGTAACCCTCGAAACA AGGAATGCAAC |

**TABLE 8A. List of additional gRNA scaffold variants. The number of asterisks represent the relative degree of gene modulation activity of the gRNA scaffold variants.**

| **Guide NA scaffold** | **Length w/o spacer (bp)** | **Activation of CD2 (2 d.p.t.)** | **Comparable or improved activity to SQ w/ reduced size** | **SEQ ID NO** |
|---|---|---|---|---|
| SQ (positive control) | 159 | ** | | 500 |
| 45.1 | 103 | **** | yes | *555* |
| 2-6 | 103 | **** | yes | 557 |
| 2-17 | 100 | *** | yes | 568 |
| 2-18 | 99 | *** | yes | 569 |
| 2-25 | 97 | ** | yes | 576 |
| 2-26 | 99 | ** | yes | 577 |
| 2-27 | 98 | *** | yes | 578 |
| 2-29 | 100 | ** | yes | 580 |
| 2-33 | 103 | - | | 584 |
| 2-31 | 96 | * | | 582 |
| 3-1 | 103 | * | | 589 |
| 3-2 | 100 | * | | 590 |
| 3-3 | 99 | - | | 591 |
| 3-4 | 98 | - | | 592 |
| 3-5 | 97 | ** | yes | 593 |
| 3-6 | 99 | * | | 594 |
| 3-7 | 98 | - | | 595 |
| 3-8 | 96 | - | | 596 |
| 6 | 151 | - | | 505 |
| 20 | 135 | ** | yes | 519 |
| 29 | 116 | *** | yes | 528 |

**TABLE 8B. List of additional gRNA scaffold variants from TABLE 8A.**

| **Guide NA scaffold** | **SEQ ID NO** | **Guide nucleic acid (NA) scaffold sequence (without spacer)** |
|---|---|---|
| 3-1 | 589 | CCGCTTCACTCTTAGGAAGTGAAGGTGGGCTGATTGCATCAGCCTAATG TCGAGAAGTGCTTTCTTCGGAAAGTAACCCTCGAAACAAAGAAAGGAA TGCAAC |
| 3-2 | 590 | CCGCTTCACGCTTCGGCAGTGAAGGTGGGCTGATTGCATCAGCCTAATG TCGAGAAGTGCTTTCTTCGGAAAGTAACCCTCGAAACAAAGGGAATGCA AC |
| 3-3 | 591 | CCGCTTCACGCTTCGGCAGTGAAGGTGGGCTGATTGCATCAGCCTAATG TCGAGAAGTGCTTTCTTCGGAAAGTAACCCTCGAAACAAAGGAATGCAA C |
| 3-4 | 592 | CCGCTTCACGCTTCGGCAGTGAAGGTGGGCTGATTGCATCAGCCTAATG TCGAGAAGTGCTTTCTTCGGAAAGTAACCCTCGAAACAAGGAATGCAAC |
| 3-5 | 593 | CCGCTTCACGCTTCGGCAGTGAAGGTGGGCTGATTGCATCAGCCTAATG TCGAGAAGTGCTTTCTTCGGAAAGTAACCCTCGAAACAAGAATGCAAC |
| 3-6 | 594 | CCGCTTCACGCTTCGGCAGTGAAGGTGGGCTGATTGCATCAGCCTAATG TCGAGAAGTGCTTTCTTCGGAAAGTAACCCTCGAAACAAAGGAATGCAA C |
| 3-7 | 595 | CCGCTTCACGCTTCGGCAGTGAAGGTGGGCTGATTGCATCAGCCTAATG TCGAGAAGTGCTTTCTTCGGAAAGTAACCCTCGAAACAAAGAATGCAAC |
| 3-8 | 596 | CCGCTTCACGCTTCGGCAGTGAAGGTGGGCTGATTGCATCAGCCTAATG TCGAGAAGTGCTTTCTTCGGAAAGTAACCCTCGAAACAGAATGCAAC |

**TABLE 9. List of fragments derived from gRNA scaffold variants.**

| **Guide NA scaffold fragment** | **SEQ ID NO** | **Guide nucleic acid (NA) scaffold fragment sequence** |
|---|---|---|
| Fragment of 45.1, 2-27, 2-17, and/or 2-18 | 597 | CCGCTTCACGCTTCGGCAGTGAAGGTGGGC |
| Fragment of 2-6 | 598 | CCGCTTCACTCTTAGGAAGTGAAGGTGGGC |
| Fragment of 2-27 | 599 | GAAAGTAACCCTCGAAACAAAGAATGCAAC |
| Fragment of 2-17 | 600 | AAGTAACCCTCGAAACAAAGGGAATGCAAC |
| Fragment of 2-18 | 601 | AAAGTAACCCTCGAAACAAAGGAATGCAAC |

## Claims

1. An engineered polypeptide comprising an engineered nuclease, wherein said engineered nuclease comprises the amino acid sequence of any one of SEQ ID NOs: 132, 134, 135, 140, or 146.

2. The engineered polypeptide of claim 1, wherein the engineered nuclease consists of the amino acid sequence of any one of SEQ ID NOs: 132, 134, 135, 140, or 146.

3. The engineered polypeptide of claim 1, wherein the engineered nuclease comprises the amino acid sequence of SEQ ID NO:132.

4. The engineered polypeptide of claim 1, wherein the engineered nuclease comprises the amino acid sequence of SEQ ID NO:134.

5. The engineered polypeptide of claim 1, wherein the engineered nuclease comprises the amino acid sequence of SEQ ID NO:135.

6. The engineered polypeptide of claim 1, wherein the engineered nuclease comprises the amino acid sequence of SEQ ID NO:140.

7. The engineered polypeptide of claim 1, wherein the engineered nuclease comprises the amino acid sequence of SEQ ID NO:146.

8. The engineered polypeptide of claim 1, wherein the engineered nuclease consists of the amino acid sequence of SEQ ID NO:132.

9. The engineered polypeptide of claim 1, wherein the engineered nuclease consists of the amino acid sequence of SEQ ID NO:134.

10. The engineered polypeptide of claim 1, wherein the engineered nuclease consists of the amino acid sequence of SEQ ID NO:135.

11. The engineered polypeptide of claim 1, wherein the engineered nuclease consists of the amino acid sequence of SEQ ID NO:140.

12. The engineered polypeptide of claim 1, wherein the engineered nuclease consists of the amino acid sequence of SEQ ID NO:146.

13. The engineered polypeptide of claim 1, wherein the engineered nuclease has a length of at most 495 amino acids.

## Patentansprüche

1. Modifiziertes Polypeptid, das eine modifizierte Nuklease umfasst, wobei die modifizierte Nuklease die Aminosäuresequenz einer der SEQ ID NOs: 132, 134, 135, 140 oder 146 umfasst.

2. Modifiziertes Polypeptid nach Anspruch 1, wobei die modifizierte Nuklease aus der Aminosäuresequenz einer der SEQ ID NOs: 132, 134, 135, 140 oder 146 besteht.

3. Modifiziertes Polypeptid nach Anspruch 1, wobei die modifizierte Nuklease die Aminosäuresequenz von SEQ ID NO: 132 umfasst.

4. Modifiziertes Polypeptid nach Anspruch 1, wobei die modifizierte Nuklease die Aminosäuresequenz von SEQ ID NO: 134 umfasst.

5. Modifiziertes Polypeptid nach Anspruch 1, wobei die modifizierte Nuklease die Aminosäuresequenz von SEQ ID NO: 135 umfasst.

6. Modifiziertes Polypeptid nach Anspruch 1, wobei die modifizierte Nuklease die Aminosäuresequenz von SEQ ID NO: 140 umfasst.

7. Modifiziertes Polypeptid nach Anspruch 1, wobei die modifizierte Nuklease die Aminosäuresequenz von SEQ ID NO: 146 umfasst.

8. Modifiziertes Polypeptid nach Anspruch 1, wobei die modifizierte Nuklease aus der Aminosäuresequenz von SEQ ID NO: 132 besteht.

9. Modifiziertes Polypeptid nach Anspruch 1, wobei die modifizierte Nuklease aus der Aminosäuresequenz von SEQ ID NO: 134 besteht.

10. Modifiziertes Polypeptid nach Anspruch 1, wobei die modifizierte Nuklease aus der Aminosäuresequenz von SEQ ID NO: 135 besteht.

11. Modifiziertes Polypeptid nach Anspruch 1, wobei die modifizierte Nuklease aus der Aminosäuresequenz von SEQ ID NO: 140 besteht.

12. Modifiziertes Polypeptid nach Anspruch 1, wobei die modifizierte Nuklease aus der Aminosäuresequenz von SEQ ID NO: 146 besteht.

13. Modifiziertes Polypeptid nach Anspruch 1, wobei die modifizierte Nuklease eine Länge von höchstens 495 Aminosäuren aufweist.

## Revendications

1. Polypeptide modifié comprenant une nucléase modifiée, dans lequel ladite nucléase modifiée comprend la séquence d'acides aminés de l'une quelconque des SEQ ID NO: 132, 134, 135, 140 ou 146.

2. Polypeptide modifié selon la revendication 1, dans lequel la nucléase modifiée est constituée de la séquence d'acides aminés de l'une quelconque des SEQ ID NO: 132, 134, 135, 140 ou 146.

3. Polypeptide modifié selon la revendication 1, dans lequel la nucléase modifiée comprend la séquence d'acides aminés de SEQ ID NO: 132.

4. Polypeptide modifié selon la revendication 1, dans lequel la nucléase modifiée comprend la séquence d'acides aminés de SEQ ID NO: 134.

5. Polypeptide modifié selon la revendication 1, dans lequel la nucléase modifiée comprend la séquence d'acides aminés de SEQ ID NO: 135.

6. Polypeptide modifié selon la revendication 1, dans lequel la nucléase modifiée comprend la séquence d'acides aminés de SEQ ID NO: 140.

7. Polypeptide modifié selon la revendication 1, dans lequel la nucléase modifiée comprend la séquence d'acides aminés de SEQ ID NO: 146.

8. Polypeptide modifié selon la revendication 1, dans lequel la nucléase modifiée est constituée de la séquence d'acides aminés de SEQ ID NO: 132.

9. Polypeptide modifié selon la revendication 1, dans lequel la nucléase modifiée est constituée de la séquence d'acides aminés de SEQ ID NO: 134.

10. Polypeptide modifié selon la revendication 1, dans lequel la nucléase modifiée est constituée de la séquence d'acides aminés de SEQ ID NO: 135.

11. Polypeptide modifié selon la revendication 1, dans lequel la nucléase modifiée est constituée de la séquence d'acides aminés de SEQ ID NO: 140.

12. Polypeptide modifié selon la revendication 1, dans lequel la nucléase modifiée est constituée de la séquence d'acides aminés de SEQ ID NO: 146.

13. Polypeptide modifié selon la revendication 1, dans lequel la nucléase modifiée a une longueur d'au plus 495 acides aminés.
